# EUROPEAN PATENT APPLICATION

(11) **EP 3 929 185 A1**
(43) Date of publication of application: **29.12.2021**
(21) Application number: 20758649.6
(22) Date of filing: 10.02.2020
(51) Int. Cl.: C07D 401/04, C07D 495/04, C07D 215/02, A61K 31/4709, A61K 31/4365, A61P 35/00

(54) **NITROGEN-CONTAINING FUSED CYCLIC COMPOUND, PREPARATION METHOD THEREFOR AND USE THEREOF**

(30) Priority: 19.02.2019 CN 201910124048; 06.06.2019 CN 201910488969; 29.09.2019 CN 201910932156
(71) Applicant: Sichuan Kelun-Biotech Biopharmaceutical Co., Ltd., Chengdu, Sichuan 611138 (CN)
(72) Inventor: LI, Guiying, Chengdu, Sichuan 611138 (CN); YOU, Zejin, Chengdu, Sichuan 611138 (CN); HE, Yun, Chengdu, Sichuan 611138 (CN); TIAN, Qiang, Chengdu, Sichuan 611138 (CN); SONG, Hongmei, Chengdu, Sichuan 611138 (CN); XUE, Tongtong, Chengdu, Sichuan 611138 (CN); WANG, Jingyi, Chengdu, Sichuan 611138 (CN)
(74) Representative: Turner, Craig Robert
(86) International application number: PCT/CN2020/074572
(87) International publication number: WO 2020/168927

(57) **Abstract**

The present invention relates to a nitrogen-containing fused ring compound, a preparation method and use thereof. Specifically, the present invention relates to a compound having the structure of Formula (X), a stereoisomer, tautomer or mixture thereof, a pharmaceutically acceptable salt, co-crystal, polymorph or solvate thereof, or a stable isotope derivative, metabolite or prodrug thereof. The compound of the invention may have the structure of Formula (I) or Formula (II). These compounds are useful for the treatment of an abnormal cell proliferation disease (e.g., cancer).

R-L-R³ (X)

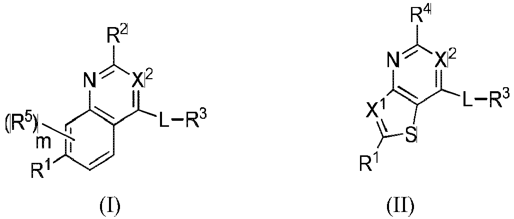

## Description

### FIELD OF THE INVENTION

The present invention relates to a nitrogen-containing fused ring compound, a stereoisomer, tautomer or mixture thereof, a pharmaceutically acceptable salt, co-crystal, polymorph or solvate thereof, or a stable isotope derivative, metabolite or prodrug thereof. The compound of the invention is useful as a NLRP3 (NLR family pyrin domain containing 3) modulator (e.g., an agonist or a partial agonist), and is useful for the treatment of an abnormal cell proliferation disease (e.g., cancer).

### BACKGROUND OF THE INVENTION

NLRP3 belongs to the family of NOD-like receptors, and is one of the most widely studied intracellular pattern recognition receptors in recent years. It is mainly expressed in macrophages and neutrophils, and is involved in the innate immunity of a body and fights pathogen infection and stress damage. The effect of NLRP3 inflammasomes in inflammatory and metabolic diseases is quite clear, and its excessive activation will lead to type 2 diabetes, immune diseases such as rheumatoid arthritis, and atherosclerosis. However, recent studies have shown that NLRP3 has anti-tumor effects that inhibit tumor growth and metastasis.

After NLRP3 protein recognizes a pathogen associated molecular pattern (PAMP) or an endogenous damage associated molecular pattern (DAMP), its NOD domain oligomerizes and recruits proteins such as ASC and pro-caspase-1 to form functional NLRP3 inflammasomes. After pro-caspase-1 is cleaved and activated to caspase-1, caspase-1 cleaves a large amount of pro-IL-1β and pro-IL-18 and converts them to active forms of IL-1β and IL-18, which are released out of the cells so that inflammatory response is amplified. Agitated NLRP3 inflammasomes can significantly increase the levels of the immune factors IL-1β and IL-18 in tumor microenvironment, initiate natural immune killing and subsequent adaptive immune response to exert its anti-tumor effects. Specifically, IL-1β can induce CD8+T cells to secrete interferon γ (IFN-γ), and can also induce CD4+ cells to secrete IL-17, leading to effective anti-tumor immune effects. IL-18 can promote maturation of NK cells, and activate the downstream signaling pathway of STAT1 in immune cells to enhance the killing function of the immune cells. Clinical studies have shown that down-regulation of NLRP3 is significantly negatively correlated with the prognosis of liver cancer patients. Preclinical studies have also shown that NLRP3-deficient mice have a higher rate of colorectal tumor formation and worsening liver metastasis of colorectal cancer. It can be seen that NLRP3 plays an important role in tumor microenvironment and can be a key target of tumor immunotherapy as well as a tumor prognostic marker.

WO2017184746, WO2017184735, WO2018152396 and WO2019014402 disclose NLRP3 modulators. Although NLRP3 agonists are potential agents in tumor immunotherapy, only one compound, BMS-986299, is currently in the stage of clinical phase I study. Therefore, it is necessary to develop new, high-efficient and low-toxic NLRP3 agonists to meet the needs in clinical treatment.

### SUMMARY OF THE INVENTION

The present inventors, through creative works, have obtained a new class of nitrogen-containing fused ring compounds, which are useful as NLRP3 modulators (e.g., agonists), and which directly bind or modify NLRP3 at protein levels and enhance the function of NLRP3 inflammasomes by activating, stabilizing, changing NLRP3 distribution or in other ways, thereby providing the following invention.

### Compound

In one aspect, the present invention provides a compound having the structure of Formula X, a stereoisomer, tautomer or mixture thereof, a pharmaceutically acceptable salt, co-crystal, polymorph or solvate thereof, or a stable isotope derivative, metabolite or prodrug thereof:

R-L-R³ (X)

wherein:
R³ is selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, C(=NR³⁸)NR³¹R³², NR³³C(=NR³⁸)NR³¹R³², P(R³⁹)₂, P(OR³⁹)₂, P(O)R³⁹R⁴⁰, P(O)OR³⁹OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9-to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³², NR³³C(O)OR³⁰, C(=NR³⁸)NR³¹R³², NR³³C(=NR³⁸)NR³¹R³², =NNR³¹R³², P(R³⁹)₂, P(OR³⁹)₂, P(O)R³⁹R⁴⁰ and P(O)OR³⁹OR³⁰;
L is -(L¹)ₙ-(L²)ₚ-(L³)_{q}-, wherein L¹, L² and L³ are the same or different and are each independently selected from the group consisting of C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl, C₁₋₄ alkoxy and NR³¹R³²; n, p and q are each independently 0, 1 or 2.
R³⁰, R³⁷, R³⁹ and R⁴⁰ are each independently selected from the group consisting of H, C₁₋₈ alkyl (e.g., C₁₋₆ alkyl or C₁₋₄ alkyl), C₁₋₈ alkoxy (e.g., C₁₋₆ alkoxy or C₁₋₄ alkoxy), C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl) are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)R³⁵, S(O)₂R³⁵, S(O)NR³¹R³² and S(O)₂NR³¹R³²;
R³¹, R³², R³³ and R³⁴ are each independently selected from the group consisting of H, C₁₋₈ alkyl, C₁₋₈ alkoxy, C₃₋₈ cycloalkyl, 4- to 10-membered heterocyclyl, C₆₋₁₂ aryl and 5- 10-membered heteroaryl, or R³¹ and R³² together with the N atom to which they are attached form a 4- to 8-membered heterocyclyl, or R³³ and R³⁴ together with the C and N atoms to which they are respectively attached form a 4- to 8-membered heterocyclyl, wherein the C₁₋₈ alkyl, C₁₋₈ alkoxy, C₃₋₈ cycloalkyl, 4- to 8-membered heterocyclyl, 4- to 10-membered heterocyclyl, C₆₋₁₂ aryl and 5- to 10-membered heteroaryl are each optionally substituted by one or more of the following substituents: OH, CN, halogen, NO₂, C₁₋₄ alkyl, C₁₋₄ alkoxy, C₁₋₄ hydroxyalkyl, C₁₋₄ haloalkyl, C₁₋₄ haloalkoxy, 4- to 10-membered heterocyclyl, C₆₋₁₂ aryl and 5- to 10-membered heteroaryl;
R³⁵ is selected from the group consisting of C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl and 5- to 10-membered heteroaryl are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)Me, S(O)₂Me, S(O)NR³¹R³² and S(O)₂NR³¹R³², wherein R³¹, R³², R³³ and R³⁴ are as defined above;
R^{36a} and R^{36b} are the same or different and are each independently selected from the group consisting of H, C₁₋₈ alkyl and C₁₋₈ alkoxy, wherein the C₁₋₈ alkyl and C₁₋₈ alkoxy are each optionally substituted by one or more of the following groups: OH, CN, halogen, NH₂, NHCH₃ and N(CH₃)₂, or R^{36a} and R^{36b} together with the C atom to which they are attached form 3- to 7-membered cycloalkyl or heterocyclyl;
R³⁸ is selected from the group consisting of H, OH, CN, NO₂, S(O)R³⁵ and S(O)₂R³⁵;
R is selected from the group consisting of 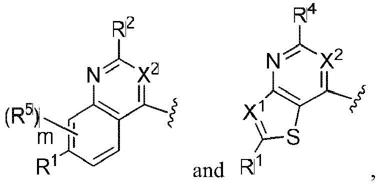 wherein:
X² is selected from the group consisting of C-L-R³ and N, and wherein R³ and L are each independently as defined above at each occurrence;
R¹ is selected from the group consisting of C₁₋₈ alkyl, C₃₋₈ cycloalkyl, 4- to 10-membered heterocyclyl, C₆₋₁₂ aryl, 5-to 10-membered heteroaryl and 9- to 12-membered aryl fused heterocyclyl, wherein the C₁₋₈ alkyl, C₃₋₈ cycloalkyl, 4- to 10-membered heterocyclyl, C₆₋₁₂ aryl, 5- to 10-membered heteroaryl and 9- to 12-membered aryl fused heterocyclyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², OC(O)R³⁰, OC(O)NR³¹R³², NR³³C(O)NR³¹R³², NR³³C(O)OR³⁰, S(O)NR³¹R³², S(O)₂NR³¹R³², S(O)R³⁵, S(O)₂R³⁵, OR³⁷ and SR³⁷;
R² is selected from the group consisting of H, NR^{41a}R^{41b}, C₁₋₈ alkyl, C₂₋₈ alkenyl, C₂₋₈ alkynyl, C₃₋₈ cycloalkyl and 4-to 10-membered heterocyclyl, wherein the C₁₋₈ alkyl, C₂₋₈ alkenyl, C₂₋₈ alkynyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, CN, NO₂, OR³⁷, SR³⁷, C(O)R³⁰, C(O)NR³¹R³², NR³³C(O)R³⁴, C(O)OR³⁰, OC(O)R³⁰, OC(O)NR³¹R³², NR³³C(O)NR³¹R³² and NR³¹R³²;
R⁵ is null or selected from the group consisting of halogen, C₁₋₆ alkyl, C₁₋₄ alkoxy, C₃₋₈ cycloalkyl and 4- to 10-membered heterocyclyl, wherein the C₁₋₆ alkyl, C₁₋₄ alkoxy, C₃₋₈ cycloalkyl and 4- to 10-membered heterocyclyl are each optionally substituted by one or more of the following groups: halogen, OH, CN, C₁₋₄ alkoxy, C₁₋₄ hydroxyalkyl and NR³¹R³²;
m is 0, 1 or 2, preferably 0 or 1;
X¹ is selected from the group consisting of CR⁶ and N;
R⁴ is selected from the group consisting of H, NR^{41a}R^{41b}, C₁₋₁₅ alkyl, C₁₋₈ alkoxy, C₂₋₈ alkenyl, C₂₋₈ alkynyl, C₃₋₈ cycloalkyl and 4- to 10-membered heterocyclyl, wherein the C₁₋₁₅ alkyl, C₁₋₈ alkoxy, C₂₋₈ alkenyl, C₂₋₈ alkynyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, CN, NO₂, OR³⁷, SR³⁷, C(O)R³⁰, C(O)NR³¹R³², NR³³C(O)R³⁴, C(O)OR³⁰, OC(O)R³⁰, OC(O)NR³¹R³², NR³³C(O)NR³¹R³² and NR³¹R³²;
R⁶ is selected from the group consisting of H, halogen, C₁₋₆ alkyl, C₁₋₄ alkoxy, C₃₋₈ cycloalkyl and 4- to 10-membered heterocyclyl, wherein the C₁₋₆ alkyl, C₁₋₄ alkoxy, C₃₋₈ cycloalkyl and 4- to 10-membered heterocyclyl are each optionally substituted by one or more of the following groups: halogen, OH, CN, C₁₋₄ alkoxy, C₁₋₄ hydroxyalkyl and NR³¹R³²;
R^{41a} and R^{41b} are each independently selected from the group consisting of H, C₁₋₆ alkyl, C₁₋₆ alkoxy and C₃₋₈ cycloalkyl, or R^{41a} and R^{41b} together with the N atom to which they are attached form a 4- to 7-membered heterocyclyl, wherein the C₁₋₆ alkyl, C₁₋₆ alkoxy, C₃₋₈ cycloalkyl and 4- to 7-membered heterocyclyl are each optionally substituted by one or more of the following groups: OH, CN and NR³¹R³², and when R is 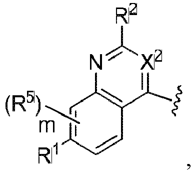 R^{41a} and R^{41b} are not simultaneously H;
when multiple R³⁰ are simultaneously present, each R³⁰ may be the same or different;
when multiple R³¹ are simultaneously present, each R³¹ may be the same or different;
when multiple R³² are simultaneously present, each R³² may be the same or different;
when multiple R³³ are simultaneously present, each R³³ may be the same or different;
when multiple R³⁴ are simultaneously present, each R³⁴ may be the same or different;
when multiple R³⁵ are simultaneously present, each R³⁵ may be the same or different;
when multiple R³⁷ are simultaneously present, each R³⁷ may be the same or different;
when multiple R³⁸ are simultaneously present, each R³⁸ may be the same or different;
when multiple R³⁹ are simultaneously present, each R³⁹ may be the same or different;
when multiple R⁴⁰ are simultaneously present, each R⁴⁰ may be the same or different.

In some embodiments, the present invention provides a compound having the structure of Formula I, a stereoisomer, tautomer or mixture thereof, a pharmaceutically acceptable salt, co-crystal, polymorph or solvate thereof, or a stable isotope derivative, metabolite or prodrug thereof: 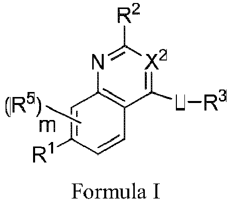 wherein:
X² is selected from the group consisting of C-L-R³ and N;
R¹ is selected from the group consisting of C₁₋₈ alkyl, C₃₋₈ cycloalkyl, 4- to 10-membered heterocyclyl, C₆₋₁₂ aryl, 5-to 10-membered heteroaryl and 9- to 12-membered aryl fused heterocyclyl, wherein the C₁₋₈ alkyl, C₃₋₈ cycloalkyl, 4- to 10-membered heterocyclyl, C₆₋₁₂ aryl, 5- to 10-membered heteroaryl and 9- to 12-membered aryl fused heterocyclyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², OC(O)R³⁰, OC(O)NR³¹R³², NR³³C(O)NR³¹R³², NR³³C(O)OR³⁰, S(O)NR³¹R³², S(O)₂NR³¹R³², S(O)R³⁵, S(O)₂R³⁵, OR³⁷ and SR³⁷;
R² is selected from the group consisting of H, NR^{41a}R^{41b}, C₁₋₈ alkyl, C₂₋₈ alkenyl, C₂₋₈ alkynyl, C₃₋₈ cycloalkyl and 4-to 10-membered heterocyclyl, wherein the C₁₋₈ alkyl, C₂₋₈ alkenyl, C₂₋₈ alkynyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, CN, NO₂, OR³⁷, SR³⁷, C(O)R³⁰, C(O)NR³¹R³², NR³³C(O)R³⁴, C(O)OR³⁰, OC(O)R³⁰, OC(O)NR³¹R³², NR³³C(O)NR³¹R³² and NR³¹R³²;
R³ at each occurrence is independently selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, C(=NR³⁸)NR³¹R³², NR³³C(=NR³⁸)NR³¹R³², P(R³⁹)₂, P(OR³⁹)₂, P(O)R³⁹R⁴⁰, P(O)OR³⁹OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³², NR³³C(O)OR³⁰, C(=NR³⁸)NR³¹R³², NR³³C(=NR³⁸)NR³¹R³², =NNR³¹R³², P(R³⁹)₂, P(OR³⁹)₂, P(O)R³⁹R⁴⁰ and P(O)OR³⁹OR³⁰;
R⁵ is null or selected from the group consisting of halogen, C₁₋₆ alkyl, C₁₋₄ alkoxy, C₃₋₈ cycloalkyl and 4- to 10-membered heterocyclyl, wherein the C₁₋₆ alkyl, C₁₋₄ alkoxy, C₃₋₈ cycloalkyl and 4- to 10-membered heterocyclyl are each optionally substituted by one or more of the following groups: halogen, OH, CN, C₁₋₄ alkoxy, C₁₋₄ hydroxyalkyl and NR³¹R³²;
m is 0, 1 or 2, preferably 0 or 1;
L is -(L¹)ₙ-(L²)ₚ-(L³)_{q}-, wherein L¹, L² and L³ are the same or different and at each occurrence are each independently selected from the group consisting of C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl, C₁₋₄ alkoxy and NR³¹R³²;
n, p and q are each independently 0, 1 or 2 at each occurrence;
R³⁰, R³⁷, R³⁹ and R⁴⁰ are each independently selected from the group consisting of H, C₁₋₈ alkyl (e.g., C₁₋₆ alkyl or C₁₋₄ alkyl), C₁₋₈ alkoxy (e.g., C₁₋₆ alkoxy or C₁₋₄ alkoxy), C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl) are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)R³⁵, S(O)₂R³⁵, S(O)NR³¹R³² and S(O)₂NR³¹R³²;
R³¹, R³², R³³ and R³⁴ are each independently selected from the group consisting of H, C₁₋₈ alkyl, C₁₋₈ alkoxy, C₃₋₈ cycloalkyl, 4- to 10-membered heterocyclyl, C₆₋₁₂ aryl and 5- 10-membered heteroaryl, or R³¹ and R³² together with the N atom to which they are attached form a 4- to 8-membered heterocyclyl, or R³³ and R³⁴ together with the C and N atoms to which they are respectively attached form a 4- to 8-membered heterocyclyl, wherein the C₁₋₈ alkyl, C₁₋₈ alkoxy, C₃₋₈ cycloalkyl, 4- to 8-membered heterocyclyl, 4- to 10-membered heterocyclyl, C₆₋₁₂ aryl and 5- to 10-membered heteroaryl are each optionally substituted by one or more of the following substituents: OH, CN, halogen, NO₂, C₁₋₄ alkyl, C₁₋₄ alkoxy, C₁₋₄ hydroxyalkyl, C₁₋₄ haloalkyl, C₁₋₄ haloalkoxy, 4- to 10-membered heterocyclyl, C₆₋₁₂ aryl and 5- to 10-membered heteroaryl;
R³⁵ is selected from the group consisting of C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl and 5- to 10-membered heteroaryl are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆alkyl), CONR³¹R³², NR³¹R³², NR³³C(O)R³⁴, S(O)Me, S(O)₂Me, S(O)NR³¹R³² and S(O)₂NR³¹R³², wherein R³¹, R³², R³³ and R³⁴ are as defined above;
R^{36a} and R^{36b} are the same or different and are each independently selected from the group consisting of H, C₁₋₈ alkyl and C₁₋₈ alkoxy, wherein the C₁₋₈ alkyl and C₁₋₈ alkoxy are each optionally substituted by one or more of the following groups: OH, CN, halogen, NH₂, NHCH₃ and N(CH₃)₂, or R^{36a} and R^{36b} together with the C atom to which they are attached form 3- to 7-membered cycloalkyl or heterocyclyl;
R³⁸ is selected from the group consisting of H, OH, CN, NO₂, S(O)R³⁵ and S(O)₂R³⁵;
R^{41a} and R^{41b} are each independently selected from the group consisting of H, C₁₋₆ alkyl, C₁₋₆ alkoxy and C₃₋₈ cycloalkyl, or R^{41a} and R^{41b} together with the N atom to which they are attached form a 4- to 7-membered heterocyclyl, wherein the C₁₋₆ alkyl, C₁₋₆ alkoxy, C₃₋₈ cycloalkyl and 4- to 7-membered heterocyclyl are each optionally substituted by one or more of the following groups: OH, CN and NR³¹R³², and R^{41a} and R^{41b} are not simultaneously H; and when multiple R³⁰ are simultaneously present, each R³⁰ may be the same or different;
when multiple R³¹ are simultaneously present, each R³¹ may be the same or different;
when multiple R³² are simultaneously present, each R³² may be the same or different;
when multiple R³³ are simultaneously present, each R³³ may be the same or different;
when multiple R³⁴ are simultaneously present, each R³⁴ may be the same or different;
when multiple R³⁵ are simultaneously present, each R³⁵ may be the same or different;
when multiple R³⁷ are simultaneously present, each R³⁷ may be the same or different;
when multiple R³⁸ are simultaneously present, each R³⁸ may be the same or different;
when multiple R³⁹ are simultaneously present, each R³⁹ may be the same or different;
when multiple R⁴⁰ are simultaneously present, each R⁴⁰ may be the same or different.

In some embodiments, the present invention provides a compound having the structure of Formula III, a stereoisomer, tautomer or mixture thereof, a pharmaceutically acceptable salt, co-crystal, polymorph or solvate thereof, or a stable isotope derivative, metabolite or prodrug thereof: 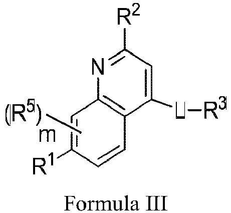 wherein R¹, R², R³, R⁵, L and m are as defined above for Formula I.

In some embodiments, the present invention provides a compound having the structure of Formula III-A, a stereoisomer, tautomer or mixture thereof, a pharmaceutically acceptable salt, co-crystal, polymorph or solvate thereof, or a stable isotope derivative, metabolite or prodrug thereof: 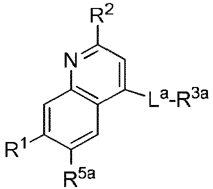 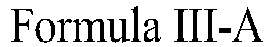 wherein:
R¹ and R² are as defined above for Formula I;
R^{5a} is H, C₁₋₃ alkyl, F or Cl;
L^{a} is -L^{1a}-(L²)ₚ-(L³)_{q}-, wherein L^{1a} is O, S or NR³³, and L², L³, p and q are as defined above for Formula I;
R^{3a} is selected from the group consisting of C₁₋₈ alkyl, C₃₋₈ cycloalkyl, 4- to 10-membered heterocyclyl, C₆₋₁₂ aryl, 5-to 10-membered heteroaryl, 9- to 12-membered aryl fused heterocyclyl, 9- to 12-membered aryl fused heteroaryl, 9-to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9-to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³² and NR³³C(O)OR³⁰; and
R³⁰, R³¹, R³², R³³, R³⁴, R³⁵and R³⁷ are as defined above for Formula I.

In some embodiments, the present invention provides a compound having the structure of Formula III-B, a stereoisomer, tautomer or mixture thereof, a pharmaceutically acceptable salt, co-crystal, polymorph, solvate thereof, or a stable isotope derivative, metabolite or prodrug thereof: 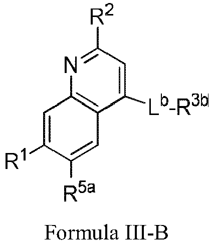 wherein:
R¹ and R² are as defined above for Formula I;
R^{5a} is H, C₁₋₃ alkyl, F or Cl;
L^{b} is -(L^{1b})ₙ-(L^{2b})ₚ-(L^{3b})_{q}-, wherein L^{1b}, L^{2b} and L^{3b} are the same or different and are each independently selected from the group consisting of C₁₋₈ alkylene, C₂₋₈ alkenylene, C₂₋₈ alkynylene, C₁₋₈ alkyleneoxy, C₃₋₈ cycloalkylene, 4-to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl and C₁₋₄ alkoxy;
n, p and q are each independently 0, 1 or 2;
R^{3b} is selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³² and NR³³C(O)OR³⁰; and
R³⁰, R³¹, R³², R³³, R³⁴, R³⁵, R^{36a}, R^{36b} and R³⁷ are as defined above for Formula I;
   provided that:
   when n+p+q≥1, L^{1b} or L^{2b} or L^{3b} of -(L¹b)ₙ-(L^{2b})ₚ-(L^{3b})_{q}- attached to the C atom of the pyridine ring in Formula III-B is not O, S, NR³³, S(O), S(O)₂ or C(O);
   when n+p+q=0, R^{3b} is not H, halogen, CN, NO₂, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, S(O)R³⁵, S(O)NR³¹R³² or S(O)₂NR³¹R³².

In some embodiments, the present invention provides a compound having the structure of Formula II, a stereoisomer, tautomer or mixture thereof, a pharmaceutically acceptable salt, co-crystal, polymorph or solvate thereof, or a stable isotope derivative, metabolite or prodrug thereof: 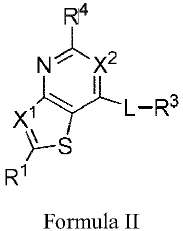 wherein:
X¹ is selected from the group consisting of CR⁶ and N,
X² is selected from the group consisting of C-L-R³ and N,
R¹ is selected from the group consisting of C₁₋₈ alkyl, C₃₋₈ cycloalkyl, 4- to 10-membered heterocyclyl, C₆₋₁₂ aryl, 5-to 10-membered heteroaryl and 9- to 12-membered aryl fused heterocyclyl, wherein the C₁₋₈ alkyl, C₃₋₈ cycloalkyl, 4- to 10-membered heterocyclyl, C₆₋₁₂ aryl, 5- to 10-membered heteroaryl and 9- to 12-membered aryl fused heterocyclyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², OC(O)R³⁰, OC(O)NR³¹R³², NR³³C(O)NR³¹R³², NR³³C(O)OR³⁰, S(O)NR³¹R³², S(O)₂NR³¹R³², S(O)R³⁵, S(O)₂R³⁵, OR³⁷ and SR³⁷;
R³ at each occurrence is independently selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR¹³C(O)OR³⁰, C(=NR³⁸)NR³¹R³², NR³³C(=NR³⁸)NR³¹R³², P(R³⁹)₂, P(OR³⁹)₂, P(O)R³⁹R⁴⁰, P(O)OR³⁹OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³², NR³³C(O)OR³⁰, C(=NR³⁸)NR³¹R³², NR³³C(=NR³⁸)NR³¹R³², =NNR³¹R³², P(R³⁹)₂, P(OR³⁹)₂, P(O)R³⁹R⁴⁰ and P(O)OR³⁹OR³⁰;
R⁴ is selected from the group consisting of H, NR^{41a}R^{41b}C₁₋₁₅ alkyl, C₁₋₈ alkoxy, C₂₋₈ alkenyl, C₂₋₈ alkynyl, C₃₋₈ cycloalkyl and 4- to 10-membered heterocyclyl, wherein the C₁₋₁₅ alkyl, C₁₋₈ alkoxy, C₂₋₈ alkenyl, C₂₋₈ alkynyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, CN, NO₂, OR³⁷, SR³⁷, C(O)R³⁰, C(O)NR³¹R³², NR³³C(O)R³⁴, C(O)OR³⁰, OC(O)R³⁰, OC(O)NR³¹R³², NR³³C(O)NR³¹R³² and NR³¹R³²;
R⁶ is selected from the group consisting of H, halogen, C₁₋₆ alkyl, C₁₋₄ alkoxy, C₃₋₈ cycloalkyl and 4- to 10-membered heterocyclyl, wherein the C₁₋₆ alkyl, C₁₋₄ alkoxy, C₃₋₈ cycloalkyl and 4- to 10-membered heterocyclyl are each optionally substituted by one or more of the following groups: halogen, OH, CN, C₁₋₄ alkoxy, C₁₋₄ hydroxyalkyl and NR³¹R³²;
L is -(L¹)ₙ-(L²)ₚ-(L³)_{q}-, wherein L¹, L² and L³ are the same or different and at each occurrence are each independently selected from the group consisting of C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl, C₁₋₄ alkoxy and NR³¹R³²;
n, p and q are each independently 0, 1 or 2 at each occurrence;
R³⁰, R³⁷, R³⁹ and R⁴⁰ are each independently selected from the group consisting of H, C₁₋₈ alkyl (e.g., C₁₋₆ alkyl or C₁₋₄ alkyl), C₁₋₈ alkoxy (e.g., C₁₋₆ alkoxy or C₁₋₄ alkoxy), C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl) are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)R³⁵, S(O)₂R³⁵, S(O)NR³¹R³² and S(O)₂NR³¹R³²;
R³¹, R³², R³³ and R³⁴ are each independently selected from the group consisting of H, C₁₋₈ alkyl, C₁₋₈ alkoxy, C₃₋₈ cycloalkyl, 4- to 10-membered heterocyclyl, C₆₋₁₂ aryl and 5- 10-membered heteroaryl, or R³¹ and R³² together with the N atom to which they are attached form a 4- to 8-membered heterocyclyl, or R³³ and R³⁴ together with the C and N atoms to which they are respectively attached form a 4- to 8-membered heterocyclyl, wherein the C₁₋₈ alkyl, C₁₋₈ alkoxy, C₃₋₈ cycloalkyl, 4- to 8-membered heterocyclyl, 4- to 10-membered heterocyclyl, C₆₋₁₂ aryl and 5- to 10-membered heteroaryl are each optionally substituted by one or more of the following substituents: OH, CN, halogen, NO₂, C₁₋₄ alkyl, C₁₋₄ alkoxy, C₁₋₄ hydroxyalkyl, C₁₋₄ haloalkyl, C₁₋₄ haloalkoxy, 4- to 10-membered heterocyclyl, C₆₋₁₂ aryl and 5- to 10-membered heteroaryl;
R³⁵ is selected from the group consisting of C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl and 5- to 10-membered heteroaryl are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)Me, S(O)₂Me, S(O)NR³¹R³² and S(O)₂NR³¹R³², wherein R³¹, R³², R³³ and R³⁴ are as defined above;
R^{36a} and R^{36b} are the same or different and are each independently selected from the group consisting of H, C₁₋₈ alkyl and C₁₋₈ alkoxy, wherein the C₁₋₈ alkyl and C₁₋₈ alkoxy are each optionally substituted by one or more of the following groups: OH, CN, halogen, NH₂, NHCH₃ and N(CH₃)₂, or R^{36a} and R^{36b} together with the C atom to which they are attached form 3- to 7-membered cycloalkyl or heterocyclyl;
R³⁸ is selected from the group consisting of H, OH, CN, NO₂, S(O)R³⁵ and S(O)₂R³⁵;
R^{41a} and R^{41b} are each independently selected from the group consisting of H, C₁₋₆ alkyl, C₁₋₆ alkoxy and C₃₋₈ cycloalkyl, or R^{41a} and R^{41b} together with the N atom to which they are attached form a 4- to 7-membered heterocyclyl, wherein the C₁₋₆ alkyl, C₁₋₆ alkoxy, C₃₋₈ cycloalkyl and 4- to 7-membered heterocyclyl may optionally be substituted by one or more of the following groups: OH, CN and NR³¹R³²; and
when multiple R³⁰ are simultaneously present, each R³⁰ may be the same or different;
when multiple R³¹ are simultaneously present, each R³¹ may be the same or different;
when multiple R³² are simultaneously present, each R³² may be the same or different;
when multiple R³³ are simultaneously present, each R³³ may be the same or different;
when multiple R³⁴ are simultaneously present, each R³⁴ may be the same or different;
when multiple R³⁵ are simultaneously present, each R³⁵ may be the same or different;
when multiple R³⁷ are simultaneously present, each R³⁷ may be the same or different;
when multiple R³⁸ are simultaneously present, each R³⁸ may be the same or different;
when multiple R³⁹ are simultaneously present, each R³⁹ may be the same or different;
when multiple R⁴⁰ are simultaneously present, each R⁴⁰ may be the same or different.

In some embodiments, the present invention provides a compound having the structure of Formula IV, a stereoisomer, tautomer or mixture thereof, a pharmaceutically acceptable salt, co-crystal, polymorph or solvate thereof, or a stable isotope derivative, metabolite or prodrug thereof: 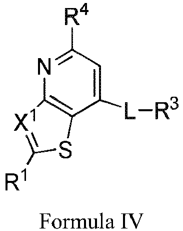 wherein R¹, R³, R⁴, X¹ and L are as defined above for Formula II.

In some embodiments, the present invention provides a compound having the structure of Formula IV-A, a stereoisomer, tautomer or mixture thereof, a pharmaceutically acceptable salt, co-crystal, polymorph or solvate thereof, or a stable isotope derivative, metabolite or prodrug thereof: 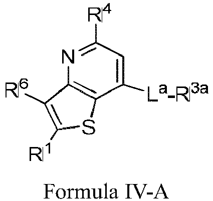 wherein:
R¹, R⁴and R⁶ are as defined above for Formula II;
L^{a} is -L^{1a}-(L²)ₚ-(L³)_{q}-, wherein L^{1a} is O, S or NR³³, and L², L³, p and q are as defined above for Formula II;
R^{3a} is selected from the group consisting of C₁₋₈ alkyl, C₃₋₈ cycloalkyl, 4- to 10-membered heterocyclyl, C₆₋₁₂ aryl, 5-to 10-membered heteroaryl, 9- to 12-membered aryl fused heterocyclyl, 9- to 12-membered aryl fused heteroaryl, 9-to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9-to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³² and NR³³C(O)OR³⁰; and
R³⁰, R³¹, R³², R³³, R³⁴, R³⁵and R³⁷ are as defined above for Formula II.

In certain embodiments of the compound of Formula IV-A of the invention, R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl;
R⁴ is NR^{41a}R^{41b}, wherein R^{41a} and R^{41b} are each independently selected from the group consisting of H, C₁₋₄ alkyl and C₃₋₆ cycloalkyl;
R⁶ is selected from the group consisting of H, halogen, C₁₋₄ alkyl and C₃₋₆ cycloalkyl;
-L^{a}-R^{3a} is selected from the group consisting of:

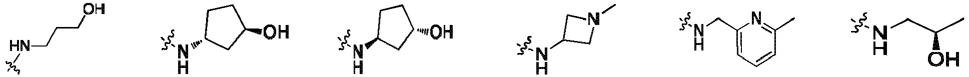

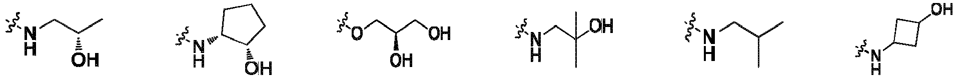

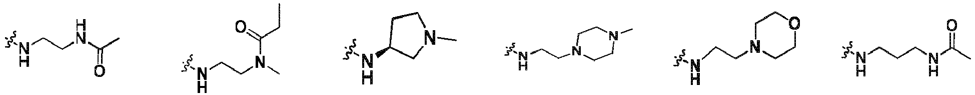

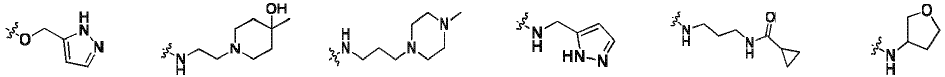

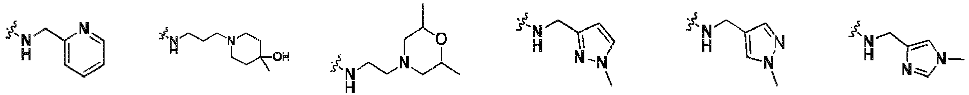

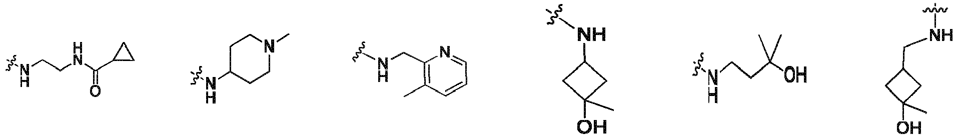

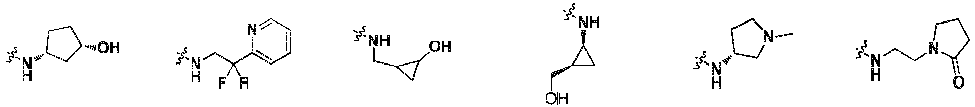

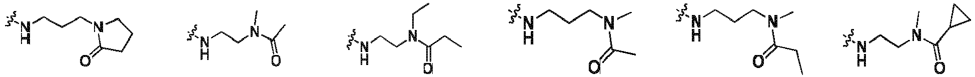

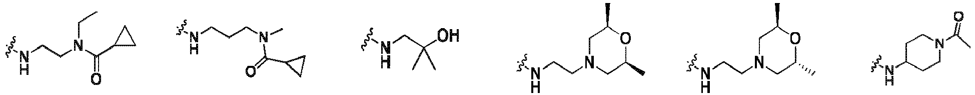

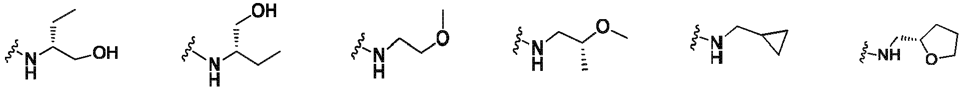

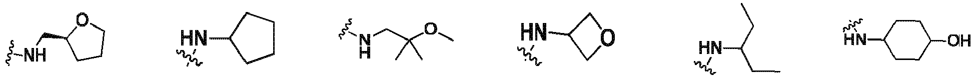

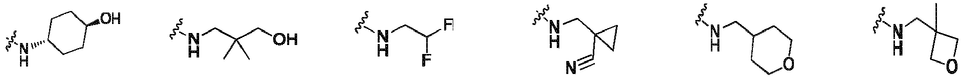

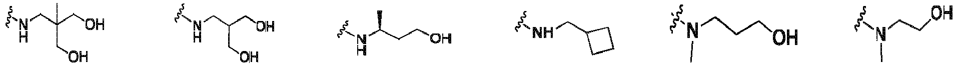

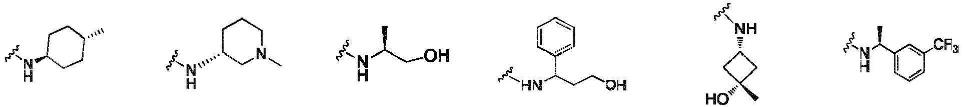

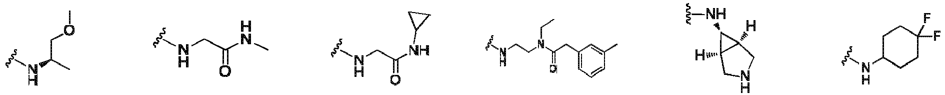

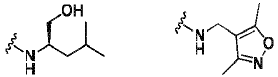

In certain embodiments of the compound of Formula IV-A of the invention, R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl; preferably, R¹ is 5- to 6-membered heteroaryl (especially 5- to 6-membered nitrogen-containing heteroaryl such as pyrazolyl or pyridyl), and the 5- to 6-membered heteroaryl is optionally substituted by one or more of the following substituents: halogen (e.g., F), 4- to 10-membered heterocyclyl (e.g., 2-tetrahydropyranyl) and C₁₋₃ alkyl (e.g., methyl); or R¹ is phenyl;
R⁴ is NR^{41a}R^{41b}, wherein R^{41a} and R^{41b} are each independently selected from the group consisting of H, C₁₋₄ alkyl and C₃₋₆ cycloalkyl; preferably, R⁴ is NH₂;
R⁶ is selected from the group consisting of H, halogen, C₁₋₄ alkyl and C₃₋₆ cycloalkyl; preferably, R⁶ is H, Cl or methyl;
L^{a} is -NH-(L²)ₚ-(L³)_{q}-, wherein L² and L³ are the same or different and at each occurrence are each independently selected from the group consisting of methylene, ethylene, n-propylene, cyclopropylene, cyclobutylene, cyclopentylene, cyclohexylene, 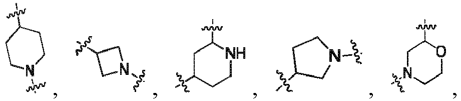 imidazolylene, pyrazolylene, isoxazolylene, pyridylene, phenylene, O, NH, N(CH₂CH₃), N(CH₃) and C(O), wherein the methylene, ethylene, n-propylene, cyclopropylene, cyclobutylene, cyclopentylene, cyclohexylene, 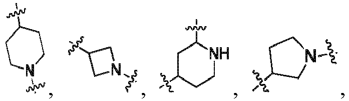 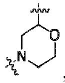 , imidazolylene, pyrazolylene, isoxazolylene, pyridylene and phenylene are each optionally substituted by one or more of the following substituents: F, OH, hydroxymethyl, hydroxyethyl, methyl and ethyl; and p and q are each independently 1 or 2 at each occurrence;
R^{3a} is selected from the group consisting of methyl, difluoromethyl, trifluoromethyl, ethyl, methoxy, cyclopropyl, cyclobutyl, cyclopentyl, 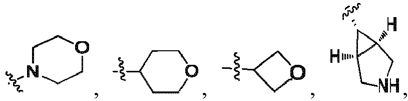 tetrahydrofuranyl, pyrazolyl, pyridyl, phenyl, CN and OH.

In some embodiments, the present invention provides a compound having the structure of Formula IV-B, a stereoisomer, tautomer or mixture thereof, a pharmaceutically acceptable salt, co-crystal, polymorph, solvate thereof, or a stable isotope derivative, metabolite or prodrug thereof: 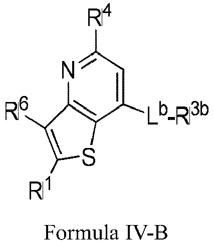 wherein:
R¹, Rand R⁶ are as defined above for Formula II;
L^{b} is -(L^{1b})ₙ-(L^{2b})ₚ-(L^{3b})_{q}-, wherein L^{1b}, L^{2b} and L^{3b} are the same or different and are each independently selected from the group consisting of C₁₋₈ alkylene, C₂₋₈ alkenylene, C₂₋₈ alkynylene, C₁₋₈ alkyleneoxy, C₃₋₈ cycloalkylene, 4-to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl and C₁₋₄ alkoxy;
n, p and q are each independently 0, 1 or 2;
R^{3b} is selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³² and NR³³C(O)OR³⁰; and
R³⁰, R³¹, R³², R³³, R³⁴, R³⁵, R^{36a}, R^{36b} and R³⁷ are as defined above for Formula II;
   provided that:
   when n+p+q≥1, L^{1b} or L^{2b} or L^{3b} of -(L^{1b})ₙ-(L^{2b})ₚ-(L^{3b})_{q}- attached to the C atom of the pyridine ring in Formula IV-B is not O, S, NR³³, S(O), S(O)₂ or C(O);
   when n+p+q=0, R^{3b} is not H, halogen, CN, NO₂, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, S(O)R³⁵, S(O)NR³¹R³² or S(O)₂NR³¹R³².

In certain embodiments of the compound of Formula IV-B of the invention, R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl; preferably, R¹ is 5- to 6-membered heteroaryl (especially 5- to 6-membered nitrogen-containing heteroaryl such as pyrazolyl or pyridyl), and the 5- to 6-membered heteroaryl is optionally substituted by one or more of the following substituents: halogen (e.g., F), 4- to 10-membered heterocyclyl (e.g., 2-tetrahydropyranyl) and C₁₋₃ alkyl (e.g., methyl);
R⁴ is NR^{41a}R^{41b}, wherein R^{41a} and R^{41b} are each independently selected from the group consisting of H, C₁₋₄ alkyl and C₃₋₆ cycloalkyl;
R⁶ is selected from the group consisting of H, halogen, C₁₋₆ alkyl and C₃₋₆ cycloalkyl;
when n+p+q=0, R^{3b} is selected from the group consisting of piperazinyl, morpholinyl, piperidinyl, tetrahydropyrrolyl and 3,6-diazabicyclo[3.1.1]hept-3-yl, each of which is optionally substituted by one or more of the following substituents: C₁₋₄ alkyl, C₃₋₆ cycloalkyl, S(O)₂R³⁵, OR³⁷, C(O)R³⁰ and phenyl; preferably, the substituents are selected from the group consisting of methyl, propyl, cyclopropyl, cyclopentyl, cyclohexyl, S(O)₂CH₃, methoxy, -OH, -C(O)H and phenyl; and
when n+p+q≥1, -L^{b}- is selected from the group consisting of methylene, ethylene, butylene, and R^{3b} is OH.

In certain embodiments of the compounds of Formula I, Formula II, Formula III and Formula IV of the invention, R³ at each occurrence is independently selected from the group consisting of H, halogen, CN, C₁₋₆ alkyl, C₃₋₆ cycloalkyl, 4- to 7-membered heterocyclyl, C₆₋₁₀ aryl, 5- to 6-membered heteroaryl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, NR³³C(O)NR³¹R³² and S(O)₂NR³¹R³², wherein the C₁₋₆ alkyl, C₃₋₆ cycloalkyl, 4- to 7-membered heterocyclyl, C₆₋₁₀ aryl and the 5- to 6-membered heteroaryl are each optionally substituted by one or more of the following substituents: halogen, CN, C₁₋₄ alkyl, C₃₋₆ cycloalkyl, C₁₋₄ haloalkyl, C₁₋₄ hydroxyalkyl, 4- to 7-membered heterocyclyl, C₆₋₁₀ aryl, 5- to 6-membered heteroaryl, CO₂R³⁰, C(O)R³⁰, C(O)NR³¹R³², NR³³C(O)R³⁴, NR³¹R³², S(O)₂R³⁵, S(O)₂NR³¹R³², OR³⁷, SR³⁷ and NR³³C(O)NR³¹R³².

In certain embodiments of the compounds of Formula I, Formula II, Formula III and Formula IV of the invention, R³ at each occurrence is independently selected from the group consisting of H, halogen, CN, C₁₋₆ alkyl, C₃₋₆ cycloalkyl, 4- to 7-membered heterocyclyl, C₆₋₁₀ aryl, 5- to 6-membered heteroaryl, C(O)NR³¹R³², NR³³C(O)R³⁴, S(O)₂R³⁵, OR³⁷ and C(O)R³⁰, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₆₋₁₀ aryl, C(O)R³⁰, S(O)₂R³⁵ and OR³⁷.

In certain embodiments of the compounds of Formula I, Formula II, Formula III and Formula IV of the invention, R³ at each occurrence is independently selected from the group consisting of methyl, difluoromethyl, trifluoromethyl, ethyl, propyl, methoxy, cyclopropyl, cyclobutyl, cyclopentyl, piperazinyl, morpholinyl, piperidinyl, 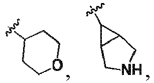 tetrahydrofuranyl, tetrahydropyrrolyl, 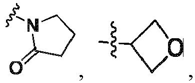 pyrazolyl, pyridyl, pyridazinyl, phenyl, 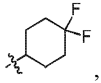 CN, OH, C(O)R³⁰, S(O)₂CH₃ and 3,6-diazabicyclo[3.1.1]hept-3-yl, each of which is optionally substituted by one or more of the following substituents: C₁₋₄ alkyl, C₃₋₆ cycloalkyl, S(O)₂R³¹, OR³⁷, C(O)R³⁰ and phenyl.

In certain embodiments of the compounds of Formula I, Formula II, Formula III and Formula IV of the invention, R³ at each occurrence is independently selected from the group consisting of methyl, difluoromethyl, trifluoromethyl, ethyl, methoxy, cyclopropyl, cyclobutyl, cyclopentyl, 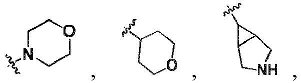 tetrahydrofuranyl, tetrahydropyrrolyl, 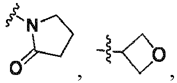 pyrazolyl, pyridyl, pyridazinyl, phenyl, 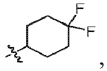 CN, OH and C(O)R³⁰.

In certain embodiments of the compounds of Formula I, Formula II, Formula III and Formula IV of the invention, R³ at each occurrence is independently selected from the group consisting of propyl and S(O)₂R³⁵.

In certain embodiments of the compounds of Formula I, Formula II, Formula III and Formula IV of the invention, R³ at each occurrence is independently selected from the group consisting of piperazinyl, morpholinyl, piperidinyl, tetrahydropyrrolyl and 3,6-diazabicyclo [3.1.1]heptan-3-yl, each of which is optionally substituted by one or more of the following substituents: C₁₋₄ alkyl, C₃₋₆ cycloalkyl, S(O)₂R³¹, OR³⁷, C(O)R³⁰ and phenyl. Preferably, the substituents are selected from the group consisting of methyl, propyl, cyclopropyl, cyclopentyl, cyclohexyl, S(O)₂CH₃, methoxy, -OH, -C(O)H and phenyl.

In certain embodiments of the compounds of Formula I, Formula II, Formula III and Formula IV of the invention, R³ at each occurrence is independently selected from the group consisting of methyl, difluoromethyl, trifluoromethyl, ethyl, methoxy, cyclopropyl, cyclobutyl, cyclopentyl, 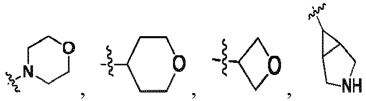 (e.g., 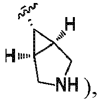 tetrahydrofuranyl, pyrazolyl, pyridyl, phenyl, CN and OH.

In certain embodiments of the compounds of Formula I, Formula II, Formula III and Formula IV of the invention, L is -(L¹)ₙ-(L²)ₚ-(L³)_{q}-, wherein L¹, L² and L³ are the same or different and at each occurrence are each independently selected from the group consisting of C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl, C₁₋₄ alkoxy and NR³¹R³²; and R³¹, R³², R³³, R^{36a}, R^{36b}, n, p, and q are as defined above.

In certain embodiments of the compounds of Formula I, Formula II, Formula III and Formula IV of the invention, L is -(L¹)ₙ-(L²)ₚ-(L³)_{q}-, wherein L¹, L² and L³ are the same or different and at each occurrence are each independently selected from the group consisting of C₁₋₄ alkylene (e.g., methylene, ethylene and n-propylene), C₃₋₆ cycloalkylene (e.g., cyclopropylene, cyclobutylene, cyclopentylene, and cyclohexylene), 4- to 6-membered heterocyclylene (e.g., 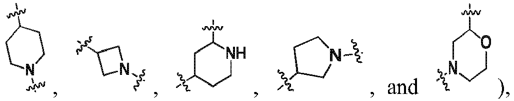 5- to 6-membered heteroarylene (e.g., imidazolylene, pyrazolylene, isoxazolylene and pyridylene), phenylene, O, NH, N(CH₂CH₃), N(CH₃), C(O) and C(R^{36a}R^{36b}), wherein the C₁₋₄ alkylene, C₃₋₆ cycloalkylene, 4- to 6-membered heterocyclylene and 5- to 6-membered heteroarylene are each optionally substituted by one or more of the following substituents: halogen (e.g., F), OH, C₁₋₃ hydroxyalkyl (e.g., hydroxymethyl and hydroxyethyl) and C₁₋₃ alkyl (e.g., methyl and ethyl), and R^{36a}R^{36b}n, p, and q are as defined above.

In certain embodiments of the compounds of Formula I, Formula II, Formula III and Formula IV of the invention, L is -(L¹)ₙ-(L²)ₚ-(L³)_{q}-, wherein L¹, L² and L³ are the same or different and at each occurrence are independently a 4-to 10-membered heterocyclylene, for example,

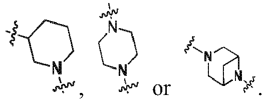

In certain embodiments of the compounds of Formula I, Formula II, Formula III and Formula IV of the invention, L is -(L¹)ₙ-(L²)ₚ-(L³)_{q}-, wherein L¹, L² and L³ are the same or different and at each occurrence are each independently selected from the group consisting of methylene, ethylene, n-propylene, cyclopropylene, cyclobutylene, cyclopentylene, cyclohexylene, 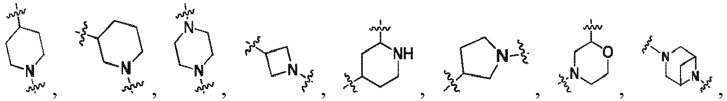 imidazolylene, pyrazolylene, isoxazolylene, pyridylene, phenylene, O, NH, N(CH₂CH₃), N(CH₃) and C(O), wherein the methylene, ethylene, n-propylene, cyclopropylene, cyclobutylene, cyclopentylene, cyclohexylene, 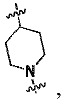 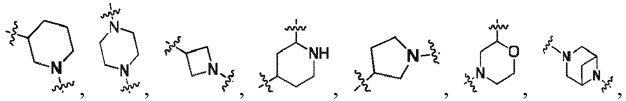 imidazolylene, pyrazolylene, isoxazolylene, pyridylene and phenylene are each optionally substituted by one or more of the following substituents: F, OH, hydroxymethyl, hydroxyethyl, methyl and ethyl; and n, p and q are each independently 1 or 2 at each occurrence.

In certain embodiments of the compounds of Formula I, Formula II, Formula III and Formula IV of the invention, L is -(L¹)ₙ-(L²)ₚ-(L³)_{q}-, wherein L¹, L² and L³ are the same or different and at each occurrence are each independently selected from the group consisting of C₁₋₆ alkylene (e.g., methylene, ethylene and n-propylene) and C₃₋₆ cycloalkylene (e.g., cyclopropylene, cyclobutylene, cyclopentylene and cyclohexylene).

In certain embodiments of the compounds of Formula I, Formula II, Formula III, Formula III-A, Formula III-B, Formula IV, Formula IV-A or Formula IV-B of the invention, -L-R³, -L^{a}-R^{3a} or -L^{b}-R^{3b} is selected from the group consisting of:

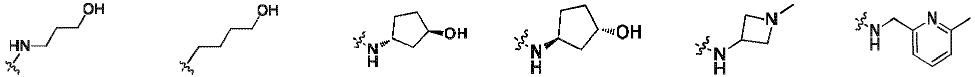

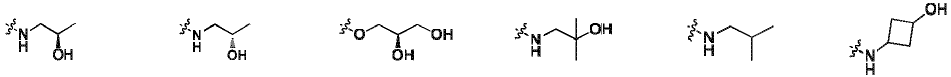

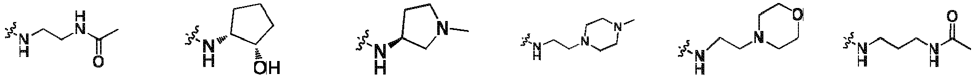

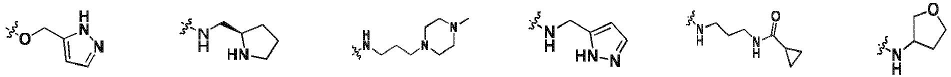

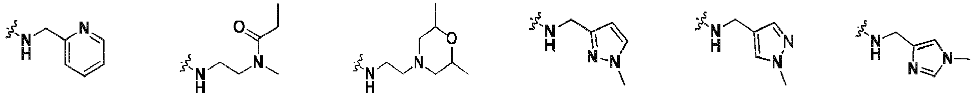

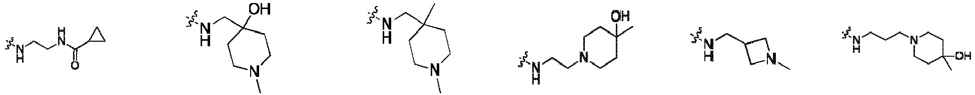

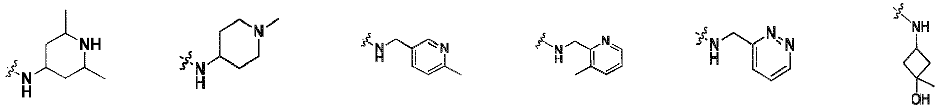

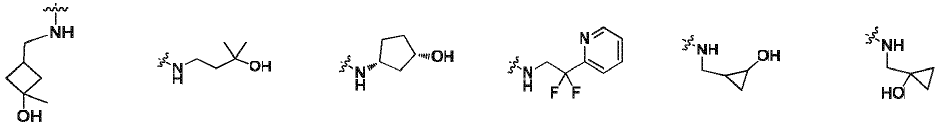

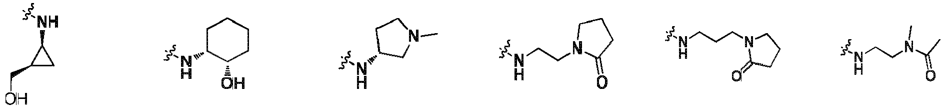

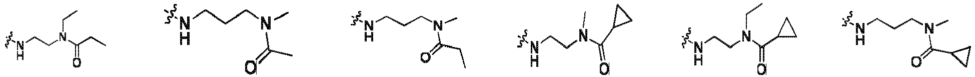

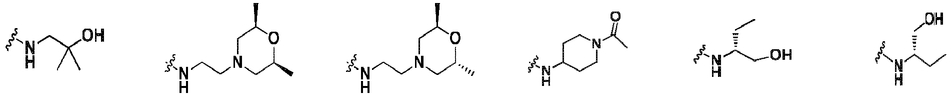

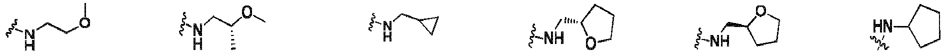

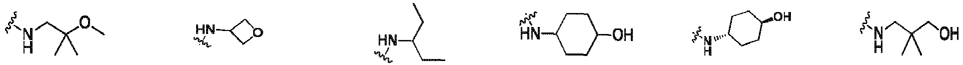

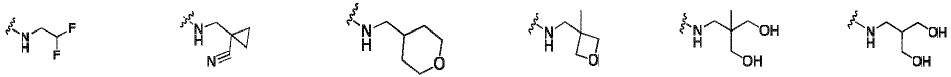

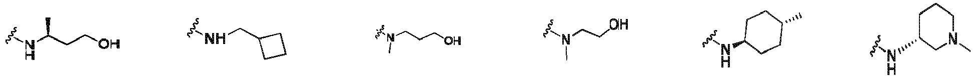

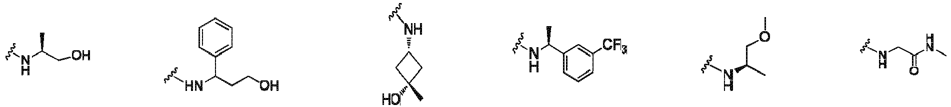

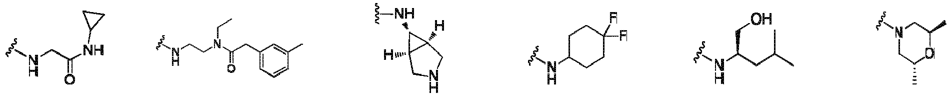

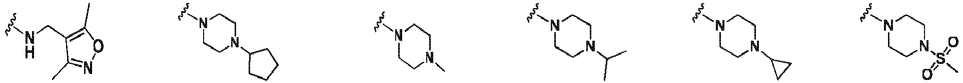

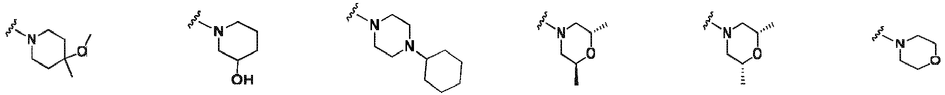

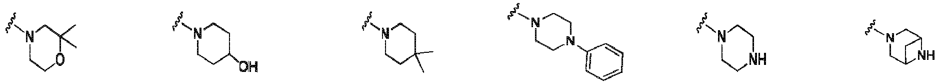

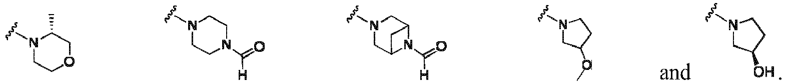

In certain embodiments of the compounds of Formula I, Formula II, Formula III, Formula III-A, Formula III-B, Formula IV, Formula IV-A or Formula IV-B of the invention, R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl. Preferably, R¹ is 5- to 6-membered heteroaryl, especially 5- to 6-membered nitrogen-containing heteroaryl such as pyrazolyl or pyridyl, which is optionally substituted by one or more of the following substituents: halogen (preferably F) and C₁₋₃ alkyl (preferably methyl); or R¹ is phenyl.

In certain embodiments of the compounds of Formula I, Formula III, Formula III-A or Formula III-B of the invention, R² is C₁₋₆ alkyl, preferably C₁₋₄ alkyl, and more preferably methyl.

In certain embodiments of the compounds of Formula I and Formula III of the invention, R⁵ is null or selected from the group consisting of halogen, C₁₋₄ alkyl and C₃₋₆ cycloalkyl, wherein the C₁₋₄ alkyl and C₃₋₆ cycloalkyl are each optionally substituted by one or more of the following groups: halogen, OH, CN, C₁₋₄ alkoxy, C₁₋₄ hydroxyalkyl and NR³¹R³².

In certain embodiments of the compounds of Formula I and Formula III of the invention, R⁵ is null or F.

In certain embodiments of the compounds of Formula II, Formula IV, Formula IV-A or Formula IV-B of the invention, R⁴ is C₁₋₆ alkyl, preferably C₁₋₄ alkyl, more preferably methyl; or R⁴ is NR^{41a}R^{41b} wherein R^{41a} and R^{41b} are each independently selected from the group consisting of H, C₁₋₄ alkyl and C₃₋₆ cycloalkyl, preferably R⁴ is NH₂.

In certain embodiments of the compounds of Formula II, Formula IV, Formula IV-A or Formula IV-B of the invention, R⁶ is H, halogen (preferably Cl), C₁₋₄ alkyl (preferably methyl) or C₃₋₆ cycloalkyl.

In certain embodiments of the compounds of Formula II, Formula IV, Formula IV-A or Formula IV-B of the invention, R⁶ is H, Cl or methyl.

In the above Formula I to Formula IV-B of the invention, the groups of all embodiments can be appropriately selected and arbitrarily combined, so as to obtain different general formula ranges or specific solutions. These ranges and solutions all belong to the present invention.

In certain embodiments of the invention, the compounds of the invention and pharmaceutically acceptable salts thereof include, but are not limited to:

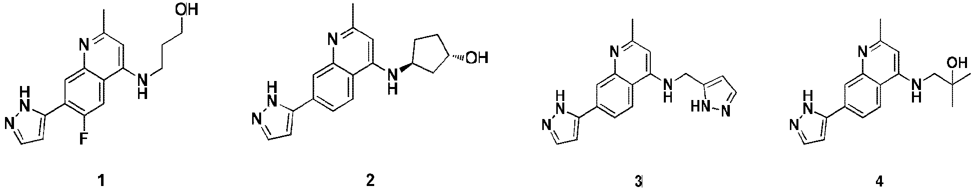

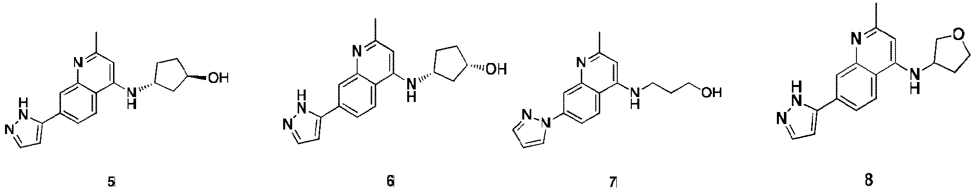

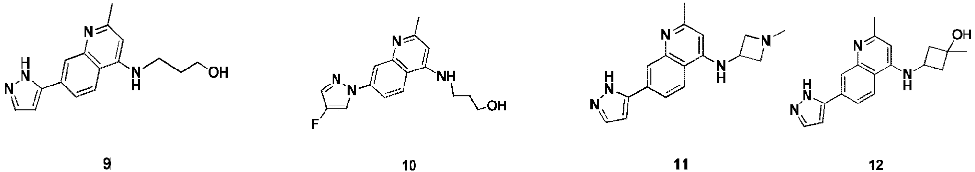

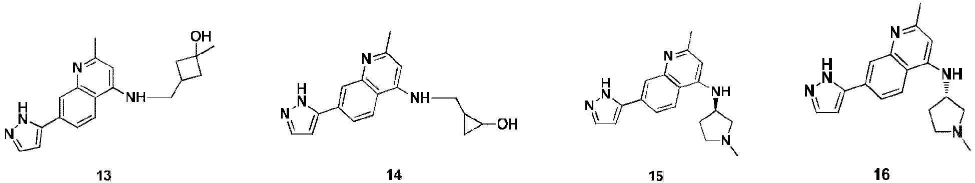

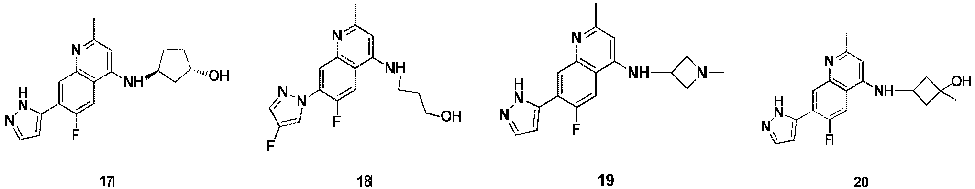

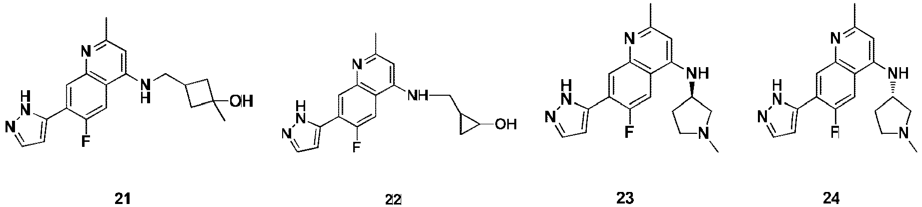

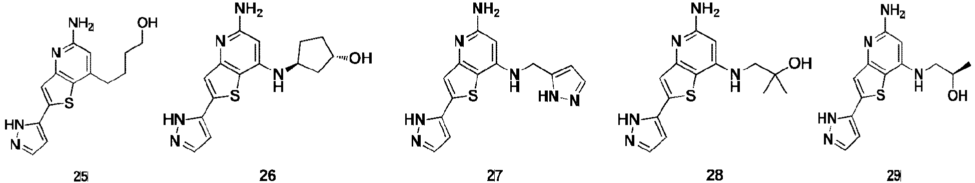

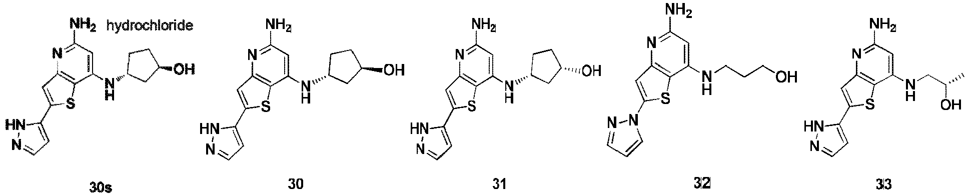

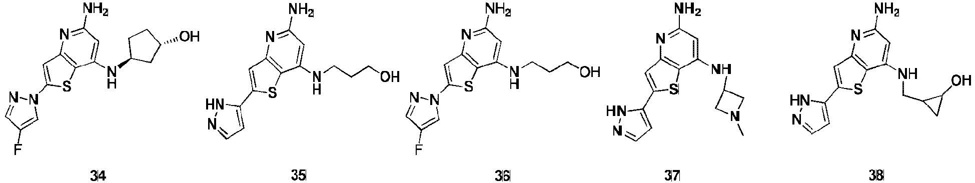

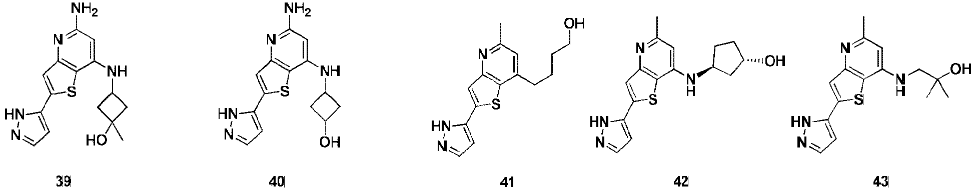

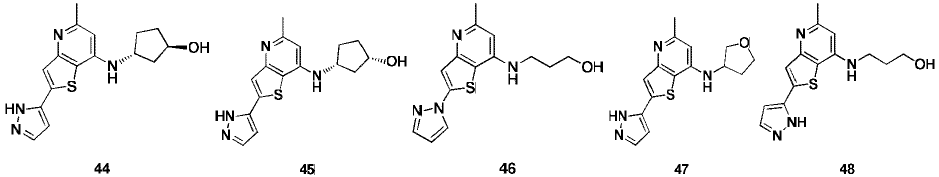

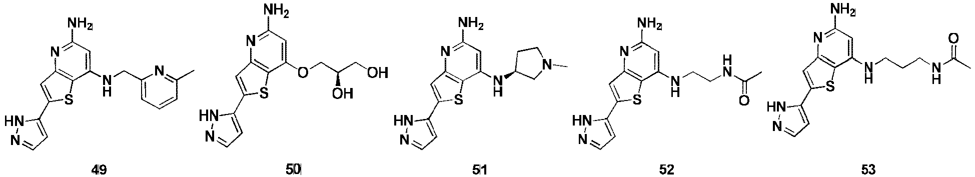

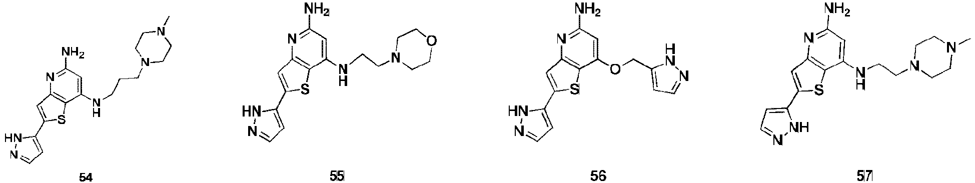

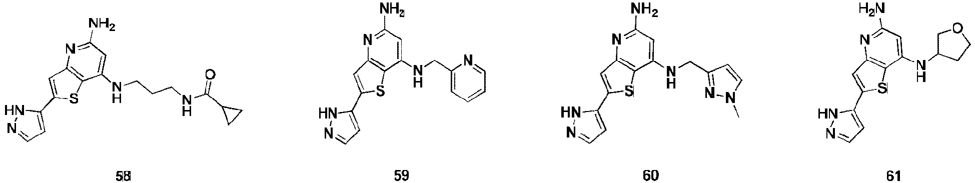

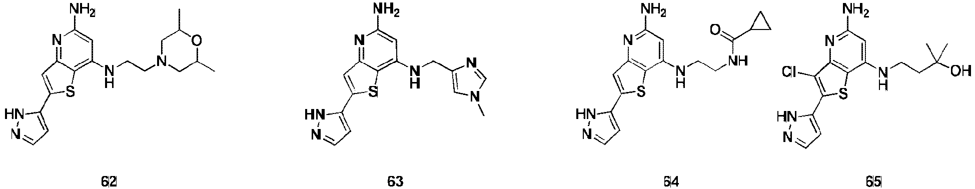

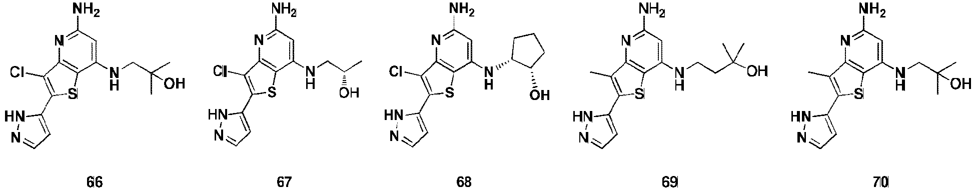

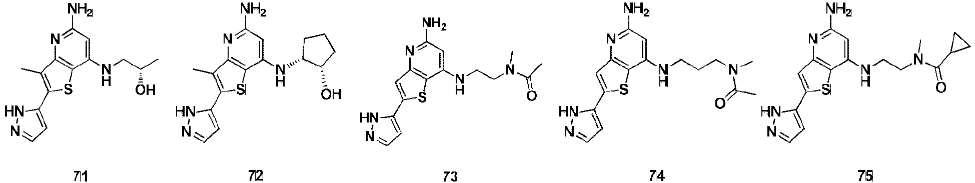

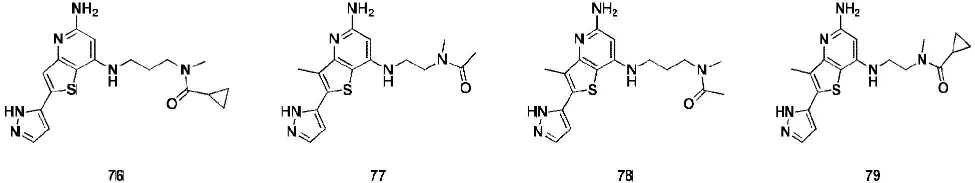

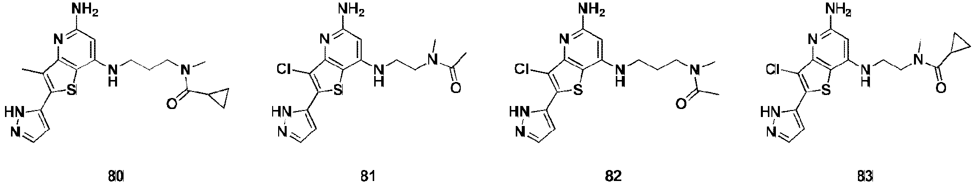

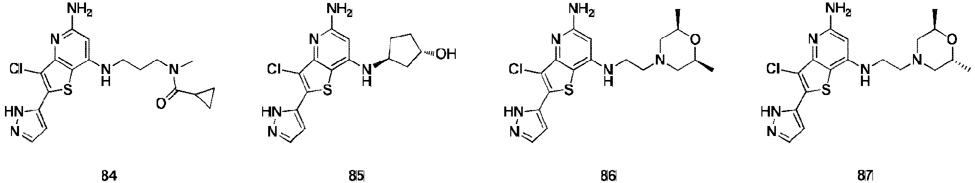

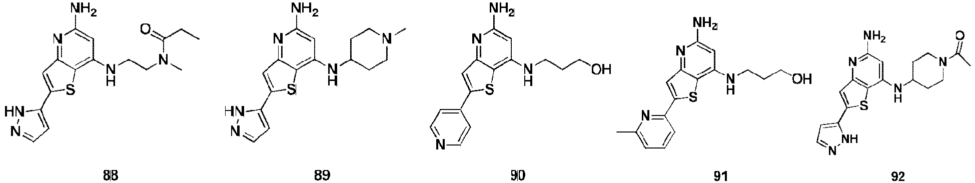

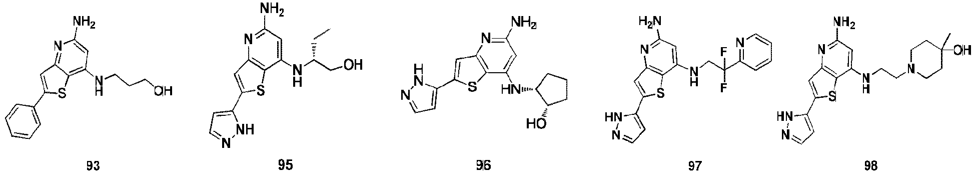

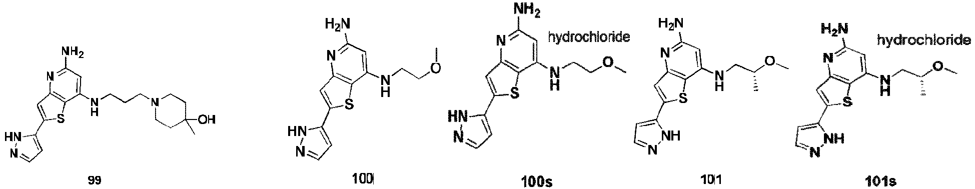

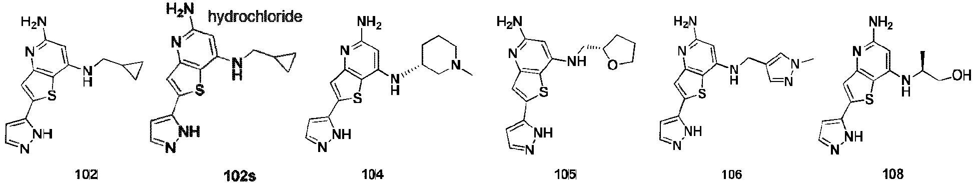

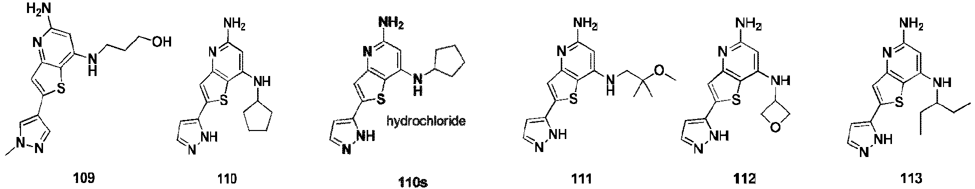

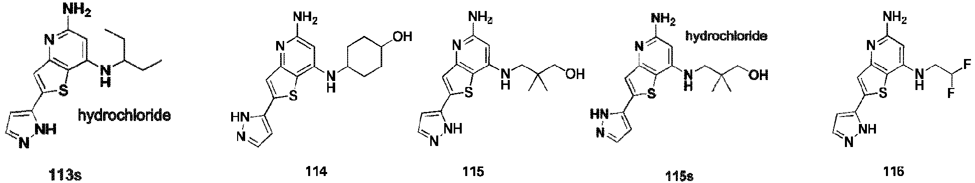

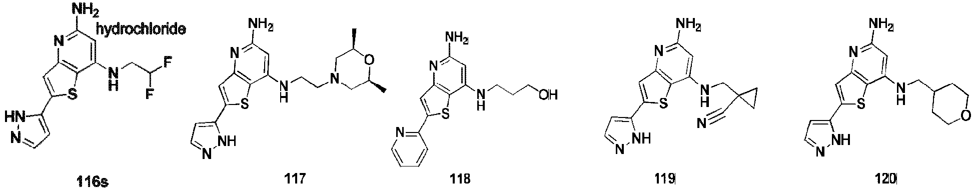

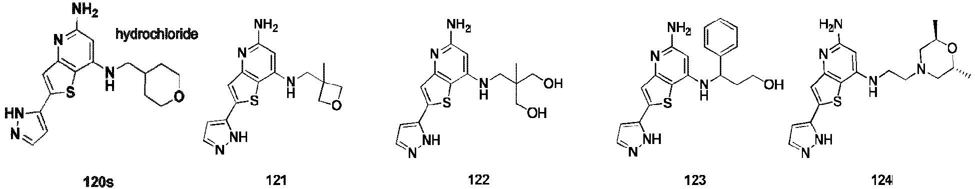

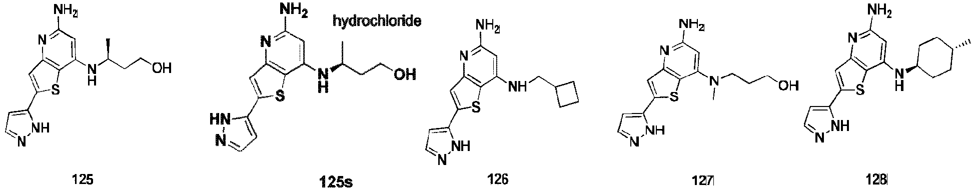

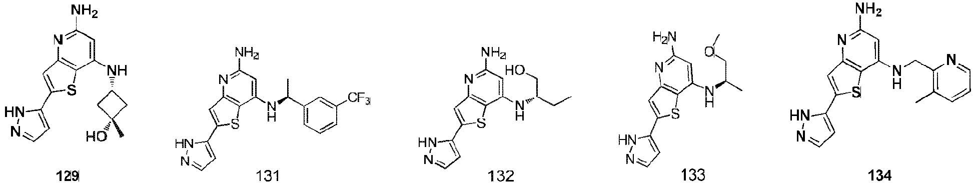

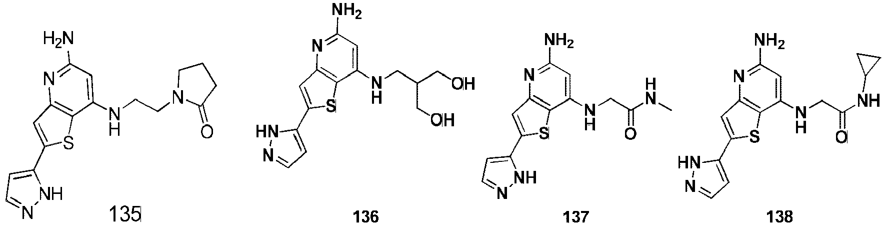

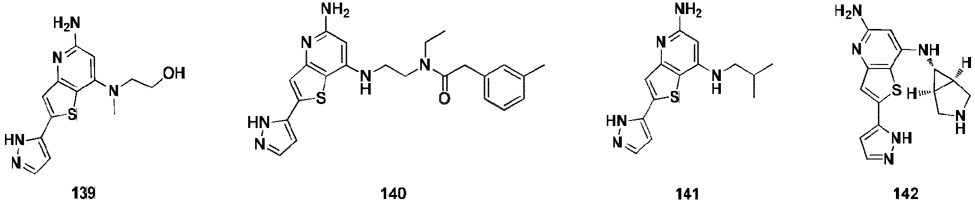

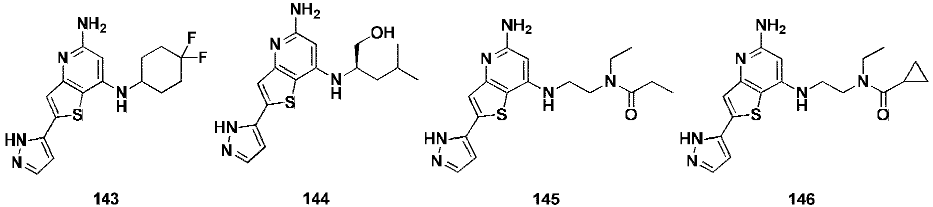

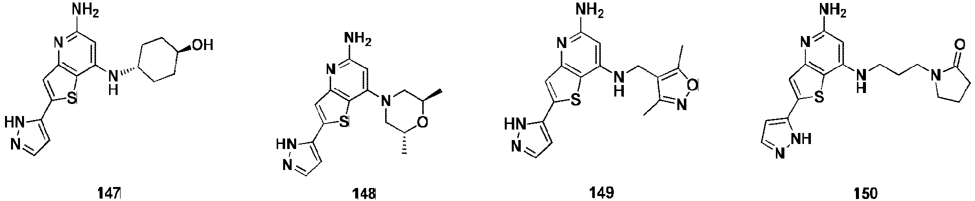

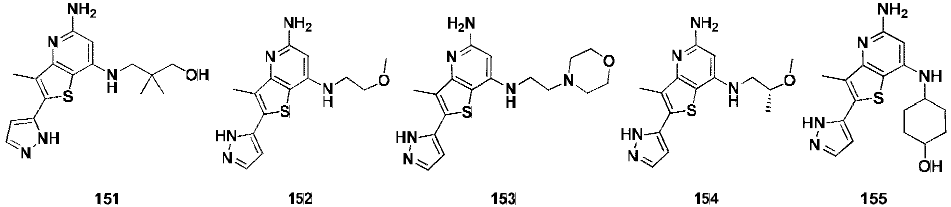

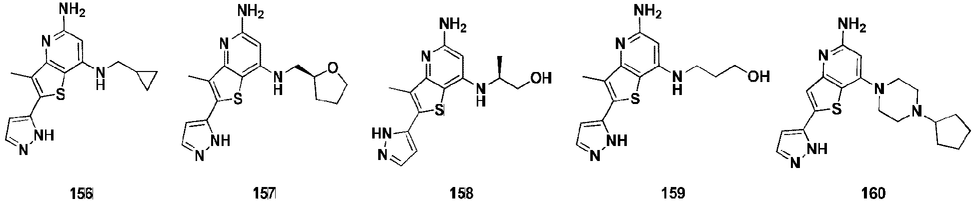

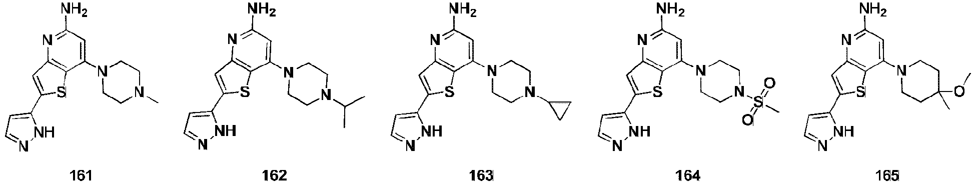

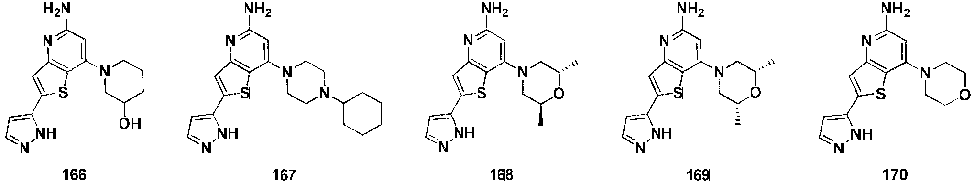

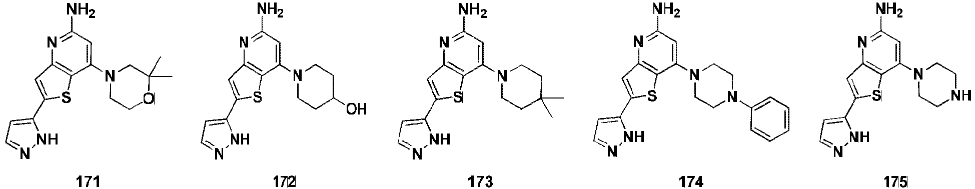

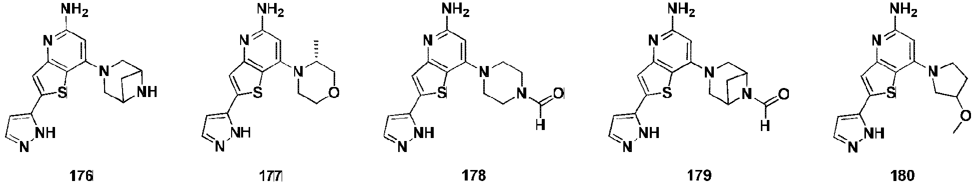

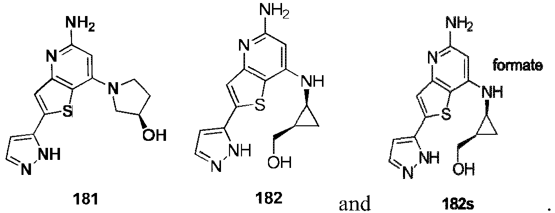

### Composition, Preparation and Use

In another aspect, the present invention provides a pharmaceutical composition comprising a compound as describe above, a stereoisomer, tautomer or mixture thereof, a pharmaceutically acceptable salt, co-crystal, polymorph or solvate thereof, or a stable isotope derivative, metabolite or prodrug thereof. Optionally, the pharmaceutical composition further comprises one or more pharmaceutically acceptable carriers.

In some embodiments, the pharmaceutical composition is for use in the prophylaxis and/or treatment of a disease related to the activity of NLRP3 inflammasomes (e.g., a neoplastic disease).

In another aspect, the present invention provides a pharmaceutical preparation comprising a compound as describe above, a stereoisomer, tautomer or mixture thereof, a pharmaceutically acceptable salt, co-crystal, polymorph or solvate thereof, or a stable isotope derivative, metabolite or prodrug thereof, or comprising a pharmaceutical composition as describe above.

In another aspect, the present invention provides a use of a compound as describe above, a stereoisomer, tautomer or mixture thereof, a pharmaceutically acceptable salt, co-crystal, polymorph or solvate thereof, or a stable isotope derivative, metabolite or prodrug thereof, or a pharmaceutical composition as described above in the manufacture of a medicament for the prophylaxis and/or treatment of a disease related to the activity of NLRP3 inflammasomes (e.g., a neoplastic disease).

In another aspect, the present invention provides a compound as describe above, a stereoisomer, tautomer or mixture thereof, a pharmaceutically acceptable salt, co-crystal, polymorph or solvate thereof, or a stable isotope derivative, metabolite or prodrug thereof, or a pharmaceutical composition as described above for use in the prophylaxis and/or treatment of a diseases related to the activity of NLRP3 inflammasomes (e.g., a neoplastic disease).

In another aspect, the present invention provides a use of a compound as describe above, a stereoisomer, tautomer or mixture thereof, a pharmaceutically acceptable salt, co-crystal, polymorph or solvate thereof, or a stable isotope derivative, metabolite or prodrug thereof, or a pharmaceutical composition as described above in the manufacture of a preparation for regulating (e.g., increasing) the activity of NLRP3 inflammasomes.

In some embodiments, the preparation is administered to a subject (e.g., a mammal, including for example, bovine, equine, ovine, swine, canine, feline, rodent, primates such as human) in order to increase the activity of NLRP3 inflammasomes in the cells of the subject. Alternatively, the preparation is administered to cells *in vitro* (e.g., a cell line or cells from a subject) to increase the activity of NLRP3 inflammasomes in the cells.

In another aspect, the present invention provides a method for modulating (e.g., increasing) the activity of NLRP3 inflammasomes in cells, comprising administering to the cells an effective amount of a compound as described above, a stereoisomer, tautomer, or mixture thereof, a pharmaceutically acceptable salt, co-crystal, polymorph or solvate thereof, a stable isotope derivative, metabolite or prodrug thereof, a pharmaceutical composition as described above, or a pharmaceutical preparation as described above.

In another aspect, the present invention provides a kit for regulating (e.g., increasing) the activity of NLRP3 inflammasomes, comprising a compound as described above, a stereoisomer, tautomer or mixture thereof, a pharmaceutically acceptable salt, co-crystal, polymorph or solvate thereof, a stable isotope derivative, metabolite or prodrug thereof, a pharmaceutical composition as described above, or a pharmaceutical preparation as described above.

In another aspect, the present invention provides a method for the treatment and/or prophylaxis of a disease related to the activity of NLRP3 inflammasomes (e.g., a neoplastic disease), comprising administering to a subject in need thereof a therapeutically and/or prophylactically effective amount of a compound as described above, a stereoisomer, tautomer or mixture of the compound, the pharmaceutically acceptable salt, co-crystal, polymorph or solvate thereof, a stable isotope derivative, metabolite or prodrug thereof, a pharmaceutical composition as described above, or a pharmaceutical preparation as described above.

In the present invention, the neoplastic disease include, but is not limited to brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, esophageal squamous cell carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma or sarcoma.

In some embodiments, the compound of the invention is a NLRP3 full agonist. In some embodiments, the compound of the invention is a NLRP3 partial agonist.

### DETAILED DESCRIPTION OF THE INVENTION

### Definitions

Unless otherwise defined hereinafter, all the technical and scientific terms used herein are intended to have the same meaning as commonly understood by a person skilled in the art. References to the techniques employed herein are intended to refer to the techniques as commonly understood in the art, including variations to those techniques or substitutions with equivalent techniques, which would be apparent to a person skilled in the art. While it is believed that the following terms will be readily understood by a person skilled in the art, the following definitions are put forth to better illustrate the invention.

The term "agonist" means a compound that binds to and activates a receptor to trigger a downstream biological effect or response, including a full agonist and a partial agonist. A full agonist can activate the receptor and produce a maximal effect (Eₘₐₓ). A partial agonist can bind to and activate the receptor, but only produces a partial effect compared to a full agonist. In the case of coexistence of a full agonist and a partial agonist, the partial agonist can sometimes become a partial antagonist by competing with the full agonist for a binding site on the receptor or by another mechanism. The potency of a partial agonist, which can be measured by EC₅₀ (the compound concentration offering 50% Eₘₐₓ), may be higher or lower than the potency of a full agonist. The NLRP3 agonists of the invention include NLRP3 full agonists and NLRP3 partial agonists.

The full name of the term "NLRP3" is NLR family pyrin domain containing 3, which is an inflammasome. In the present invention, reference to "NLRP3" means a nucleic acid, a polynucleotide, an oligonucleotide, a sense and antisense polynucleotide chain, a complementary sequence, a short peptide, a polypeptide, a protein, a homologous or heterologous molecule, a subtype, a precursor, a mutant, a variant, a derivative, various splicing bodies, alleles, different species and activated fragments of NLRP3.

The terms "contain", "include", "comprise", "have", or "relate to", as well as other variations used herein are inclusive or open-ended, and do not exclude additional, unrecited elements or method steps.

The term "halo" means substitution by a halogen atom, and the "halogen" includes F, Cl, Br or I.

The term "alkyl" is a linear or branched saturated aliphatic hydrocarbon group. The terms "C₁₋₁₅ alkyl", "C₁₋₈ alkyl", "C₁₋₆ alkyl" and "C₁₋₄ alkyl" respectively mean a linear or branched alkyl group having 1 to 15 carbon atoms, 1 to 8 carbon atoms, 1 to 6 carbon atoms and 1 to 4 carbon atoms, e.g., methyl, ethyl, n-propyl, isopropyl, n-butyl, isobutyl, sec-butyl, tert-butyl, n-pentyl or n-hexyl. The alkyl group may optionally be substituted by one or more (e.g., 1 to 3) same or different substituents.

The term "alkylene" means a saturated divalent hydrocarbon group obtained by removing two hydrogen atoms from a linear or branched saturated hydrocarbon group, which comprises a specified number of carbon atoms. For example, the term "C₁₋₈ alkylene" means an alkylene group having 1 to 8 carbon atoms, e.g., methylene (-CH₂-), ethylene (-CH₂CH₂-), iso-propylene (-CH(CH₃)CH₂-). The alkylene group may optionally be substituted by one or more (e.g., 1 to 3) same or different substituents.

The term "haloalkyl" means an alkyl substituted by one or more (e.g., 1 to 3) same or different halogen atoms, and the terms "C₁₋₈ haloalkyl", "C₁₋₆ haloalkyl" and "C₁₋₄ haloalkyl" respectively mean a haloalkyl group having 1 to 8 carbon atoms, 1 to 6 carbon atoms and 1 to 4 carbon atoms, e.g., -CF₃, -C₂F₅, -CHF₂, -CH₂F, -CH₂CF₃, -CH₂Cl,-CH₂CH₂CF₃, or the like.

The term "hydroxyalkyl" means a group formed by replacing one or more hydrogen atoms of an alkyl group with one or more hydroxy groups, e.g., a C₁₋₄ hydroxyalkyl group or a C₁₋₃ hydroxyalkyl group, examples of which include, but are not limited to hydroxymethyl, hydroxyethyl, hydroxypropyl, hydroxybutyl, -CH(OH)CH₃, or the like.

The term "alkenyl" means a monovalent linear or branched hydrocarbon group having one or more carbon-carbon double bonds, e.g., -CH=CH₂, -CH₂CH=CH₂, -C(CH₃)=CH₂, -CH₂-CH=CH-CH₃, the alkenyl may optionally be substituted by one or more (e.g., 1 to 3) same or different substituents.

The term "alkenylene" means a divalent linear or branched aliphatic hydrocarbon group having one or more carbon-carbon double bonds and comprising a specified number of carbon atoms, e.g., 2 to 8 carbon atoms, e.g., -CH=CH-, -CH₂CH=CH-, -C(CH₃)=CH-, or the like, and the alkenylene may optionally be substituted by one or more (e.g., 1 to 3) same or different substituents.

The term "alkynyl" means a monovalent linear or branched hydrocarbon group having one or more carbon-carbon triple bonds, including but not limited to ethynyl, 2-propynyl, 2-butynyl, 1,3-dialkynyl, or the like, and the alkynyl may optionally be substituted by one or more (e.g., 1 to 3) same or different substituents.

The term "alkynylene" means a divalent linear or branched hydrocarbon group having one or more carbon-carbon triple bonds and comprising a specified number of carbon atoms, e.g., 2 to 8 carbon atoms, including but not limited to 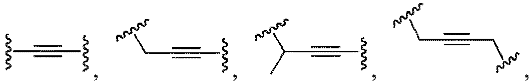 or the like, and the alkynylene may optionally be substituted by one or more (e.g., 1 to 3) same or different substituents.

The term "alkoxy" means a group having an oxygen atom inserted at any reasonable position of an alkyl (as defined above), e.g., C₁₋₈ alkoxy, C₁₋₆ alkoxy, C₁₋₄ alkoxy or C₁₋₃ alkoxy. Representative examples of C₁₋₆ alkoxy include, but are not limited to methoxy, ethoxy, propoxy, iso-propoxy, n-propoxy, iso-propoxy, n-butoxy, iso-butoxy, tert-butoxy, pentyloxy, hexyloxy, -CH₂-OCH₃, or the like, and the alkoxy may optionally be substituted by one or more (e.g., 1 to 3) same or different substituents.

The term "alkyleneoxy" means a divalent alkoxy group, e.g., -OCH₂-, -OCH(CH₃)CH₂-, -OCH₂CH₂O-, -CH₂CH₂O-, or the like, and the alkyleneoxy may optionally be substituted by one or more (e.g., 1 to 3) same or different substituents.

The term "fused ring" or "condensed ring" means a ring system formed by two or more cyclic structures that share two adjacent atoms.

The term "spirocyclic ring" means a ring system formed by two or more cyclic structures that share one ring atom.

The term "bridged ring" means a ring system formed by two or more cyclic structures that share two atoms that are not directly connected.

The term "cycloalkyl" means a saturated or unsaturated non-aromatic monocyclic or polycyclic (e.g., bicyclic) hydrocarbon ring group, including but not limited to a monocyclic alkyl group (e.g., cyclopropyl, cyclobutyl, cyclopentyl, cyclohexyl, cycloheptyl, cyclooctyl, cyclononyl) and a bicyclic alkyl group, including spiro, fused (condensed) or bridged ring systems (i.e., spiro cycloalkyl, fused (condensed) cycloalkyl and bridged cycloalkyl, such as bicyclo[1.1.1]pentyl, bicyclo[2.2.1]heptyl, or the like). In the present invention, the cycloalkyl may optionally be substituted by one or more (e.g., 1 to 3) same or different substituents. A carbon atom in the cycloalkyl is optionally substituted by oxo (i.e., forming C=O).

The term "3- to 7-membered cycloalkyl" means a cycloalkyl group having 3 to 7 ring-forming carbon atoms, which may be a monocyclic alkyl group, e.g., cyclopropyl, cyclobutyl, cyclopentyl, cyclohexyl or cycloheptyl, or may also be a bicyclic alkyl group, e.g., C₅₋₇ spirocycloalkyl, C₅₋₇ bridged cycloalkyl or C₄₋₇ fused cycloalkyl.

The term "C₃₋₈ cycloalkyl" means a cycloalkyl group having 3 to 8 ring-forming carbon atoms, e.g., a C₃₋₆ cycloalkyl, which may be a monocyclic alkyl group, e.g., cyclopropyl, cyclobutyl, cyclopentyl, cyclohexyl, cycloheptyl, cyclooctyl, or a bicyclic alkyl, e.g., C₃₋₈ spirocycloalkyl, C₃₋₈ bridged cycloalkyl, C₃₋₈ fused cycloalkyl, C₃₋₆ spirocycloalkyl, C₃₋₆ bridged cycloalkyl and C₃₋₆ fused cycloalkyl.

The term "cycloalkylene" means a cycloalkyl group as defined herein, which has two monovalent group centers obtained by removing two hydrogen atoms from the same carbon atom or two different carbon atoms of the parent cycloalkyl. Typical cycloalkylene groups include, but are not limited to cyclopropylene, cyclobutylene, cyclopentylene, cyclohexylene, cycloheptylene, cyclooctylene, cyclononylene, cyclohexenylene, or the like.

The term "aryl" means an all-carbon monocyclic or fused polycyclic aromatic group having a conjugated π-electron system. As used herein, the term "C₆₋₁₂ aryl" means an aryl group having 6 to 12 carbon atoms, e.g., C₆₋₁₀ aryl, and specific examples are phenyl or naphthyl. The aryl is optionally substituted by one or more (e.g., 1 to 3) same or different substituents (e.g., halogen, OH, CN, NO₂, C₁-C₆ alkyl, or the like).

The term "arylene" means an aryl group as defined herein, which has two monovalent group centers obtained by removing two hydrogen atoms from the same carbon atom or two different carbon atoms of the parent aryl group. Typical arylene groups include, but are not limited to phenylene and naphthylene.

The term "aryl fused cycloalkyl" means a fused ring group formed by aryl and cycloalkyl (e.g., monocyclic alkyl) that share two adjacent atoms, and the point of attachment to other groups may be on the aryl group or on the cycloalkyl. The term "9- to 12-membered aryl fused cycloalkyl" means an aryl fused cycloalkyl group having 9 to 12 ring atoms in total, e.g., phenyl fused cyclopentyl, phenyl fused cyclohexyl, for example, 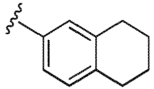 or

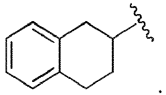

The term "heterocyclyl" means a monocyclic or polycyclic (e.g., fused, spiro or bridged) group having 2 or more (e.g., 3, 4, 5, 6, 7, 8, 9, 10, 11, 12, 13 or 14) carbon atoms and one or more (e.g., 1, 2, 3 or 4) heteroatoms, which include but are not limited to oxygen atom, nitrogen atom, sulfur atom, and the carbon atoms and heteroatoms in the heterocyclyl are optionally substituted by oxo (e.g., to form C=O, S(=O) or S(=O)₂).

The term "heterocyclylene" means a heterocyclyl group as defined herein, which has two monovalent group centers obtained by removing two hydrogen atoms from the same carbon atom or two different carbon atoms, one carbon atom and one heteroatom, or two heteroatoms of the parent heterocyclyl group.

The term "3- to 14-membered heterocyclyl" means a heterocyclic group having 3-14 ring atoms, which include but are not limited to 4- to 10-membered heterocyclyl, 4- to 7-membered heterocyclyl, 5- to 6-membered heterocyclyl, 4- to 7-membered nitrogen-containing heterocyclyl, 4- to 7-membered oxygen-containing heterocyclyl, 4- to 7-membered sulfur-containing heterocyclyl, 5- to 6-membered nitrogen-containing heterocyclyl, 5- to 6-membered oxygen-containing heterocyclyl, 5- to 6-membered sulfur-containing heterocyclyl, and the "nitrogen-containing heterocyclyl", "oxygen-containing heterocyclyl" and "sulfur-containing heterocyclyl" optionally further contain one or more other heteroatoms selected from the group consisting of oxygen, nitrogen and sulfur. Examples of 3- to 14-membered heterocyclyl include, but are not limited to oxiranyl, aziridinyl, azetidinyl, oxetanyl, tetrahydrofuryl, pyrrolidinyl, pyrrolidonyl, imidazolidinyl, pyrazolidinyl, tetrahydropyranyl, piperidinyl, morpholinyl, dithianyl, thiomorpholinyl, piperazinyl, trithianyl, 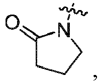 or the like.

In the present invention, the heterocyclyl also includes a fused ring structure, and the point of attachment of the fused ring structure to other groups can be on any ring in the fused ring structure. Therefore, the heterocyclic group in the invention also includes (but is not limited to) heterocyclyl fused heterocyclyl, heterocyclyl fused cycloalkyl, monoheterocyclyl fused monoheterocyclyl, monoheterocyclyl fused monocycloalkyl, e.g., 3- to 7-membered (mono)heterocyclyl fused 3- to 7-membered (mono)heterocyclyl, 3- to 7-membered (mono)heterocyclyl fused (mono)cycloalkyl, 3- to 7-membered (mono)heterocyclyl fused C₄₋₆ (mono)cycloalkyl, examples of which include but are not limited to pyrrolidinyl fused cyclopropyl, cyclopentyl fused azacyclopropyl, pyrrolidinyl fused cyclobutyl, pyrrolidinyl fused pyrrolidinyl, pyrrolidinyl fused piperidinyl, pyrrolidinyl fused piperazinyl, piperidinyl fused morpholinyl, 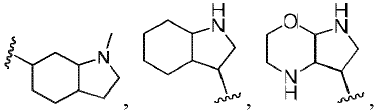 or the like.

In the present invention, the heterocyclyl also includes bridged heterocyclyl and spiro heterocyclyl.

The term "bridged heterocyclyl" means a cyclic structure, which is formed by two saturated rings sharing two ring atoms that are not directly connected, which contains one or more (e.g., 1, 2, 3 or 4) heteroatoms (e.g., oxygen, nitrogen and sulfur atoms), and which includes but is not limited to 7- to 10-membered bridged heterocyclyl, 8- to 10-membered bridged heterocyclyl, 7- to 10-membered nitrogen-containing bridged heterocyclyl, 7- to 10-membered oxygen-containing bridged heterocyclyl, 7- to 10-membered sulfur-containing bridged heterocyclyl, e.g., 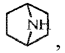 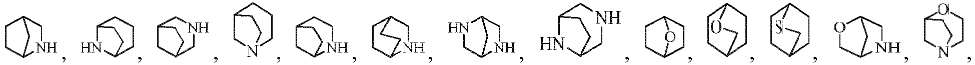 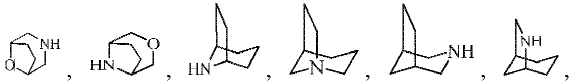 or the like. The "nitrogen-containing bridged heterocyclyl", "oxygen-containing bridged heterocyclyl", and "sulfur-containing bridged heterocyclyl" optionally further contain one or more other heteroatoms selected from the group consisting of oxygen, nitrogen and sulfur.

The term "spiro heterocyclyl" means a cyclic structure, which is formed by two or more saturated rings sharing one ring atom, which contains one or more (e.g., 1, 2, 3 or 4) heteroatoms (e.g., oxygen, nitrogen, sulfur atoms), and which includes but is not limited to 5- to 10-membered spiro heterocyclyl, 6- to 10-membered spiro heterocyclyl, 6-to 10-membered nitrogen-containing spiro heterocyclyl, 6- to 10-membered oxygen-containing spiro heterocyclyl, 6- to 10-membered sulfur-containing spiro heterocyclyl, e.g., 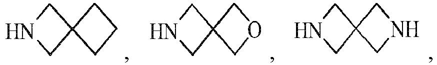 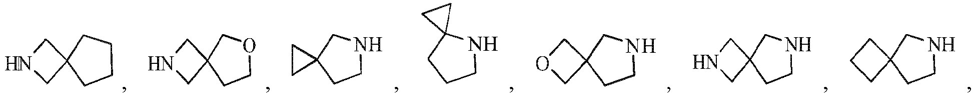 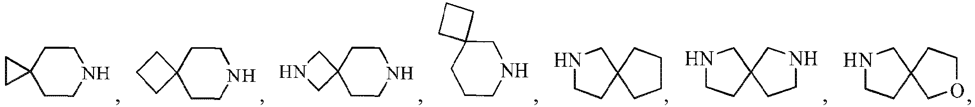 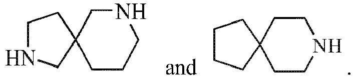 The "nitrogen-containing spiro heterocyclyl", "oxygen-containing spiro heterocyclyl" and "sulfur-containing spiro heterocyclyl" optionally further contain one or more other heteroatoms selected from the group consisting of oxygen, nitrogen and sulfur. The term "6- to 10-membered nitrogen-containing spiro heterocyclyl" means a spiroheterocyclic group having 6-10 ring atoms in total, at least one of which is a nitrogen atom.

The term "aryl fused heterocyclyl" means a cyclic group, which is formed by an aryl group and a heterocyclic group sharing two adjacent carbon atoms (wherein the aryl group and heterocyclic group are as defined above), and the point of attachment of which may be on the aryl group or on the heterocyclic group. For example, as used herein, the term "9- to 12-membered aryl fused heterocyclyl" means an aryl fused heterocyclyl group having a total of 9-12 ring atoms, and includes but is not limited to 9- to 10-membered benzoheterocyclyl, for example benzo 5- to 8-membered heterocyclyl, for example benzo 5- to 6-membered heterocyclyl, for example benzo 5- to 6-membered monoheterocyclyl, benzo 5- to 6-membered nitrogen-containing monoheterocyclyl, benzo 5- to 6-membered oxygen-containing monoheterocyclyl, benzo 5- to 6-membered sulfur-containing heterocyclyl, and the "nitrogen-containing heterocyclyl", "oxygen-containing heterocyclyl" and "sulfur-containing heterocyclyl" optionally further contain one or more other heteroatoms selected from the group consisting of oxygen, nitrogen and sulfur. The carbon atoms and heteroatoms in the heterocyclyl are optionally substituted by oxo (e.g., forming C=O, S(=O) or S(=O)₂).

Examples of aryl fused heterocyclyl include, but are not limited to indazolyl,

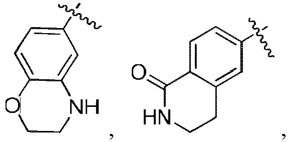

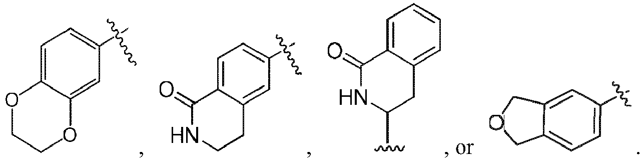

The term "heteroaryl" means a monocyclic or polycyclic aromatic group having one or more same or different heteroatoms, including monocyclic heteroaryl and a bicyclic or polycyclic system having at least one heteroaromatic ring (an aromatic ring system containing at least one heteroatom), which may have 5, 6, 7, 8, 9, 10, 11, 12, 13 or 14 ring atoms, e.g., 5, 6, 7, 8, 9 or 10 ring atoms. The heteroatom may be oxygen, nitrogen or sulfur. The carbon atoms and heteroatoms in the heteroaryl are optionally substituted by oxo (e.g., forming C=O, S(=O) or S(=O)₂).

The term "heteroarylene" means a heteroaryl group as described above, which has two monovalent group centers resulting from removing two hydrogen atoms from a same carbon atom or two different carbon atoms of the parent heteroaryl group or by removing one hydrogen atom from the carbon atom and removing another hydrogen atom from the nitrogen atom.

The term "5- to 10-membered heteroaryl" means a heteroaryl group having 5 to 10 ring atoms, including 5- to 6-membered heteroaryl, 5- to 6-membered monoheteroaryl, 5- to 10-membered nitrogen-containing heteroaryl, 5- to 10-membered oxygen-containing heteroaryl, 5- to 10-membered sulfur-containing heteroaryl, 5- to 6-membered nitrogen-containing heteroaryl, 5- to 6-membered oxygen-containing heteroaryl, 5- to 6-membered sulfur-containing heteroaryl, 5- to 6-membered nitrogen-containing monoheteroaryl, 5- to 6-membered oxygen-containing monoheteroaryl, and 5- to 6-membered sulfur-containing monoheteroaryl. The "nitrogen-containing heteroaryl", "oxygen-containing heteroaryl", "sulfur-containing heteroaryl", "nitrogen-containing monoheteroaryl", "oxygen-containing monoheteroaryl" and "sulfur-containing monoheteroaryl" optionally further contain one or more other heteroatoms selected from the group consisting of oxygen, nitrogen and sulfur. Examples of 5- to 10-membered heteroaryl include, but are not limited to thienyl, furyl, pyrrolyl, oxazolyl, thiazolyl, imidazolyl, pyrazolyl, isoxazolyl, isothiazolyl, triazolyl, tetrazolyl, oxadiazolyl, thiadiazolyl, pyridinyl, pyridazinyl, pyrimidinyl, pyrazinyl, triazinyl, and a 5- to 10-membered fused cyclic group containing these groups.

In the present invention, the term "heteroaryl" encompasses fused ring structure, i.e., fused ring structure that is formed by heteroaryl (e.g., monoheteroaryl) and aryl (e.g., monocyclic aryl, e.g., phenyl), heterocyclyl (e.g., monoheterocyclyl), cycloalkyl (e.g., monocycloalkyl) or another heteroaryl (e.g., another monoheteroaryl) sharing two adjacent atoms, and the point of attachment to other groups of which is on any heteroaromatic ring or another ring. The heteroaryl includes but is not limited to (mono)heteroaryl fused (mono)heteroaryl, (mono)heteroaryl fused (monocyclic)aryl, (mono)heteroaryl fused (mono)heterocyclyl or (mono)heteroaryl fused (mono)cycloalkyl, e.g., 5- to 6-membered (mono)heteroaryl fused 5- to 6-membered (mono)heteroaryl, 5- to 6-membered (mono)heteroaryl fused phenyl, 5- to 6-membered (mono)heteroaryl fused 5- to 6-membered (mono)heterocyclyl, or 5- to 6-membered (mono)heteroaryl fused C₄₋₆ (mono)cycloalkyl (e.g., 5- to 6-membered heteroaryl fused cyclobutyl, 5- to 6-membered heteroaryl fused cyclopentyl, 5- to 6-membered heteroaryl fused cyclohexyl), and examples thereof include but are not limited to indolyl, isoindolyl, indazolyl, benzimidazolyl, quinolinyl, isoquinolinyl, 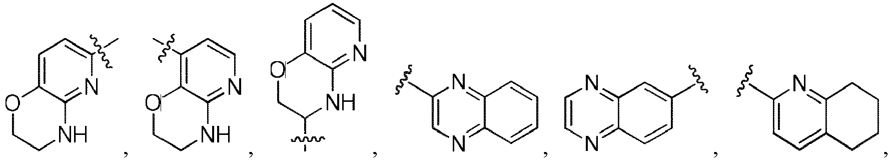 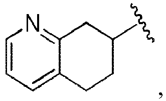 or the like.

The aryl-containing fused ring structure encompassed by the term "heteroaryl" is also referred to as "aryl fused heteroaryl", which means a fused ring group formed by aryl (e.g., monocyclic aryl e.g., phenyl) and heteroaryl (e.g., monoheteroaryl e.g., 5- to 6-membered monoheteroaryl), and the point of attachment to other groups of which may be on the aromatic ring or on the heteroaromatic ring. The "aryl fused heteroaryl" includes but is not limited to monocyclic aryl fused monoheteroaryl. The term "9- to 12-membered aryl fused heteroaryl" means an aryl fused heteroaryl group containing a total of 9-12 ring atoms, e.g., benzo 5- to 6-membered nitrogen-containing monoheteroaryl.

The cycloalkyl-containing fused ring structure encompassed by the term "heteroaryl" is also referred to as "heteroaryl fused cycloalkyl", which means a fused ring group formed by heteroaryl (e.g., monoheteroaryl e.g., 5- to 6-membered monoheteroaryl) and cycloalkyl (e.g., C₄₋₆ cycloalkyl), and the point of attachment to other groups of which may be on the heteroaromatic ring or on the cycloalkyl. The "heteroaryl fused cycloalkyl" includes but is not limited to monoheteroaryl fused monocycloalkyl. The term "9- to 10-membered heteroaryl fused cycloalkyl" means heteroaryl fused cycloalkyl containing a total of 9-10 ring atoms, e.g., 4- to 6-membered nitrogen-containing monoheteroaryl fused C₄₋₆ monocycloalkyl.

The term "substituted" means that one or more ((e.g., 1, 2, 3 or 4) hydrogen atoms on a specified atom are replaced with a selection from the specified groups, provided that the specified atom has normal valency under the situation, and that the substitution leads to a stable compound. Combinations of substituents and/or variables are permissible only if such combinations result in stable compounds.

If a substituent is described as being "optionally substituted by ...", the substituent may be either (1) unsubstituted or (2) substituted. If a carbon atom in a substituent is described as being optionally substituted with one or more from a list of substituents, one or more of the hydrogen atoms (to the extent that any hydrogen atoms existed) on the carbon may each be optionally replaced with an independently selected substituent. If a nitrogen atom in a substituent is described as being optionally substituted with one or more from a list of substituents, one or more of the hydrogen atoms (to the extent that any hydrogen atoms existed) on the nitrogen may each be optionally replaced with an independently selected substituent.

If substituents are described as being "independently selected" from a group, each substituent is selected independent of the other(s). Each substituent therefore may be identical to or different from the other substituent(s).

As used herein, the term "one or more" means one or more than one, e.g., 2, 3, 4, 5, 6, 7, 8, 9 or 10, where appropriate.

As used herein, unless otherwise specified, the point of attachment of a substituent can be from any suitable position of the substituent.

The present invention also encompasses all pharmaceutically acceptable isotope compounds of the compounds of the invention, which are identical to those of the invention except one or more atoms are replaced by atom(s) having the same atomic number but having an atomic mass or mass number different from the predominant atomic mass or mass number in nature. Examples of isotopes suitable for incorporating in the compounds of the invention include but are not limited to isotopes of hydrogen (e.g., ²H, ³H); isotopes of carbon (e.g., ¹¹C, ¹³C and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O and ¹⁸O); isotopes of phosphorus (e.g., ³²P); and isotopes of sulfur (e.g., ³⁵S). The term "stable isotope derivative" means a stable compound formed by replacing one or more atoms in the compound of the invention with atom(s) having the same atomic number but having different atomic mass or mass number from the predominant atomic mass or mass number in nature.

The term "stereoisomer" means an isomer formed by a compound containing at least one asymmetric center. A compound having one or more (e.g., 1, 2, 3 or 4) asymmetric centers can give rise to a racemic mixture, single enantiomer, diastereomer mixture and individual diastereomer. Certain individual molecules may exist as geometric isomers (cis/trans). The compound of the invention may exist as a mixture of two or more structurally different forms in rapid equilibrium (generally referred to as tautomer). Representative examples of a tautomer include a keto-enol tautomer, phenol-keto tautomer, nitroso-oxime tautomer, imine-enamine tautomer, or the like. For example, nitroso-oxime can exist in equilibrium in the following tautomeric forms in a solution: 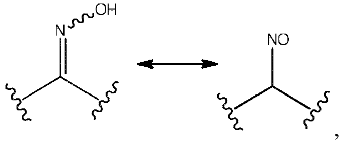 and the pyrazole ring can exist in equilibrium in the following tautomeric forms:

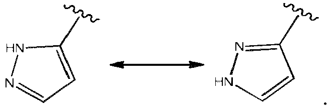

It is to be understood that all such isomers and mixtures thereof in any proportion (e.g., 60%, 65%, 70%, 75%, 80%, 85%, 90%, 95%, 96%, 97%, 98% or 99%) are encompassed within the scope of the present application.

Unless stated otherwise, it is intended that the compound of the invention can exist as stereoisomers, which include cis and trans isomers, optical isomers (e.g., R and S enantiomers), diastereomers, geometric isomers, rotational isomers, conformational isomers, atropisomers, or mixtures thereof. The compound of the invention may exhibit more than one type of isomerism, and consist of mixtures thereof (e.g., racemates and diastereomeric pairs).

The present invention encompasses all possible crystalline forms or polymorphs of the compound of the invention, either as a single polymorph, or as a mixture of more than one polymorphs in any ratio. It should also be understood that certain compounds of the invention can be used in a free from for treatment, or where appropriate, in a form of a pharmaceutically acceptable derivative. In the present invention, a pharmaceutically acceptable derivative includes but is not limited to a pharmaceutically acceptable salt, solvate, metabolite or prodrug, which can directly or indirectly provide the compound of the invention or a metabolite or residue thereof after administration to a patient in need thereof. Therefore, a reference to "the compound of the invention" herein also intends to encompass various derivative forms of the compound as mentioned above.

A pharmaceutically acceptable salt of the compound of the invention includes an acid addition salt and a base addition salt thereof, e.g., hexafluorophosphate, meglumine salt, or the like. For a review of suitable salts, see "Handbook of Pharmaceutical Salts: Properties, Selection, and Use" by Stahl and Wermuth (Wiley-VCH, 2002).

The term "pharmaceutically acceptable carrier" in the present invention means a diluent, auxiliary material, excipient or vehicle with which a therapeutic is administered, and it is, within the scope of a sound medical judgment, suitable for contacting with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the invention includes but is not limited to sterile liquids, e.g., water and oils, including those of petroleum, animal, vegetable or synthetic origin, e.g., peanut oil, soybean oil, mineral oil, sesame oil, or the like. Water is an exemplary carrier when the pharmaceutical composition is administered intravenously. Physiological saline as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, skim milk powder, glycerol, propylene glycol, water, ethanol, or the like. The composition, if desired, can also contain minor amounts of wetting agents, emulsifying agents, or pH buffering agents. Oral formulations can include standard carriers such as pharmaceutical grades mannitol, lactose, starch, magnesium stearate, sodium saccharine, cellulose, magnesium carbonate, or the like. Examples of suitable pharmaceutical carriers are described in e.g., Remington's Pharmaceutical Sciences (1990).

The pharmaceutical composition of the invention can act systemically and/or topically. To this end, it can be administered through a suitable route, e.g., through injection, intravenous, intraarterial, subcutaneous, intraperitoneal, intramuscular or transdermal administration; or administered orally, buccally, nasally, transmucosally, topically, as an ophthalmic formulation, or by inhalation.

For these routes of administration, the pharmaceutical composition of the invention can be administered in a suitable dosage form.

The dosage forms include but are not limited to tablets, capsules, lozenges, hard candies, powders, sprays, creams, salves, suppositories, gels, pastes, lotions, ointments, aqueous suspensions, injectable solutions, elixirs, and syrups.

As used herein, the term "effective amount" means an amount of a compound that will relieve to some extent one or more of the symptoms of the disorder to be treated after administration.

Dosage regimens may be adjusted to provide an optimal desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment by the person administering or supervising the administration of the composition.

The amount of the compound of the invention administered will be dependent on the subject to be treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a human of 70 kg, this would amount to about 0.007 mg/day to about 3500 mg/day, for example about 0.7 mg/day to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or amount of the compound of the invention in the pharmaceutical composition may be about 0.01 mg to about 1000 mg.

Unless otherwise indicated, the term "treating" or "treatment", as used herein, means reversing, alleviating, suppressing the disorder or condition to which such term applies, or inhibiting the progress of one or more symptoms of such disorder or condition, or preventing such disorder or condition or one or more symptoms thereof.

As used herein, the term "subject" includes a human or non-human animal. An exemplary human subject includes a human subject having a disease (e.g., one described herein) (referred to as a patient), or a normal subject. The term "non-human animal" as used herein includes all vertebrates, e.g., non-mammals (e.g., birds, amphibians, reptiles) and mammals, e.g., non-human primates, livestock and/or domesticated animals (e.g., sheep, dog, cat, cow, pig, or the like).

The compound of the invention can exist as a solvate (preferably a hydrate), wherein the compound of the invention contains a polar solvent, in particular water, methanol or ethanol for example, as a structural element of the crystal lattice of the compound. The amount of the polar solvent, in particular water, may exist in a stoichiometric or non-stoichiometric ratio.

The metabolite of the compound of the invention, namely a substance formed in vivo upon administration of the compound of the invention, is also encompassed within the scope of the invention. Such a product may result e.g., from the oxidation, reduction, hydrolysis, amidation, de-amidation, esterification, degreasing, enzymolysis, or the like, of the administered compound. Accordingly, the present invention encompasses the metabolite of the compound of the invention, including a compound produced by a method comprising contacting the compound of the invention with a mammal for a period of time sufficient to result in a metabolic product thereof.

Also within the scope of the invention is a prodrug of the compound of the invention, which is certain derivative of the compound of the invention that may have little or no pharmacological activity itself, but when administered into or onto the body, can be converted into the compound of the invention having the desired activity, for example, by hydrolytic cleavage. In general, such prodrug will be a functional derivative of the compound which is readily converted in vivo into the compound with desired therapeutic activity. For additional information on the use of prodrugs, see "Pro-drugs as Novel Delivery Systems", Vol. 14, ACS Symposium Series (T. Higuchi and V. Stella) and "Bioreversible Carriers in Drug Design," Pergamon Press, 1987 (edited by EB Roche, American Pharmaceutical Association). The prodrug in accordance with the invention, for example, can be produced by replacing appropriate functionalities present in the compound of the invention with certain moieties known to those skilled in the art as "pro-moieties" as described, for example, in "Design of Prodrugs" by H. Bundgaard (Elsevier, 1985).

The present invention further encompasses the compound of the invention having a protecting group. During any of the processes for preparing the compound of the invention, it may be necessary and/or desirable to protect sensitive or reactive groups on any of the molecules concerned, thereby resulting in the chemically protected form of the compound of the invention. This may be achieved by means of conventional protecting groups, e.g., those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which are incorporated herein by reference. The protecting groups may be removed at an appropriate subsequent stage using methods known in the art.

### Preparation Method

### Synthetic methods of Compounds III-A-1 and III-B-1

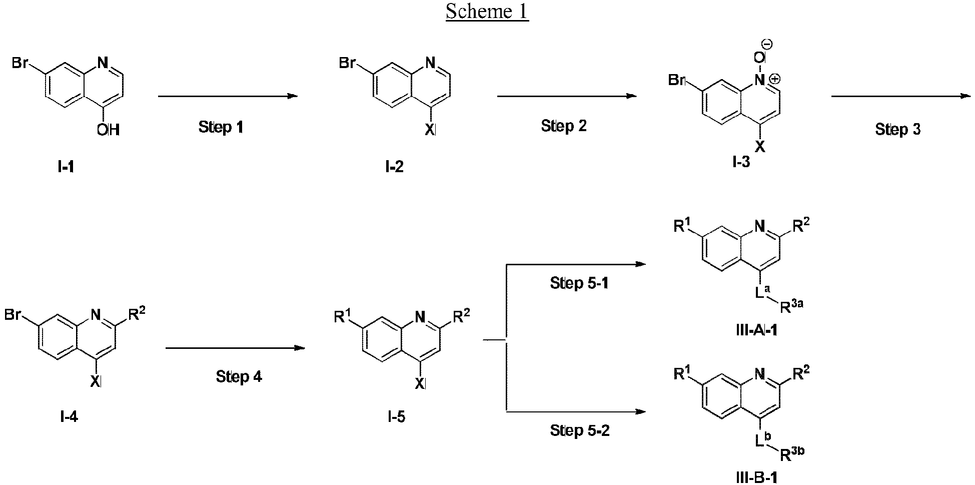

wherein R¹, R², R^{3a}, R^{3b}, L^{a} and L^{b} are as defined above for Formula III-A or III-B; and X is selected from the group consisting of chlorine, bromine and iodine.

In particular, R² is selected from the group consisting of C₁₋₈ alkyl, C₃₋₈ cycloalkyl and 4- to 10-membered heterocyclyl, the C₁₋₈ alkyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, OR³⁷, SR³⁷ and NR³¹R³², or R² is NR^{41a}R^{41b} wherein R³¹, R³², R³⁷, R^{41a} and R^{41b} are as defined above for Formula I.

Step 1: Halogenation of Compound 1-1 to produce Compound 1-2.

In certain embodiments, a useful halogenating agent is phosphorus oxychloride, PCl₅, phosphorus oxybromide, HBr, or the like. In certain embodiments, a useful solvent is 1,4-dioxane, DMF, EA, or the like. In certain embodiments, the reaction temperature is -20°C to 100°C.

Step 2: Oxidation of Compound 1-2 to produce Compound 1-3.

In some embodiments, a useful oxidizing agent is m-chloroperoxybenzoic acid, hydrogen peroxide, carbamide peroxide, or the like. In certain embodiments, a useful solvent is DCM, chloroform, 1,2-dichloroethane, 1,4-dioxane, or the like. In certain embodiments, the reaction temperature is -20°C to 100°C.

Step 3: Reaction of Compound 1-3 with R²MgX (when R² in Compound 1-4 is attached to the pyridine ring via its carbon atom), R²H (when R² in Compound 1-4 is attached to the pyridine ring via its nitrogen atom) to produce Compound 1-4.

In certain embodiments, a useful solvent is THF, 1,4-dioxane, toluene, or the like. In certain embodiments, the reaction temperature is -20°C to 100°C.

Step 4: Coupling (e.g., Suzuki reaction or Stille reaction) of Compound 1-4 with R¹-boronic acid or R¹-borate or R¹-organotin compound (e.g., R¹Sn(*n*-Bu)₃) to produce Compound 1-5.

In certain embodiments, a useful catalyst is Pd(PPh₃)₄, Pd(dppf)Cl₂•CH₂Cl₂, or the like. In certain embodiments, a useful base is Cs₂CO₃, K₃PO₄, Na₂CO₃, AcOK, NaHCO₃, K₂CO₃, or the like. In certain embodiments, a useful solvent is 1,4-dioxane/H₂O, DMF/H₂O, DMSO/H₂O, CH₃CN/H₂O, toluene/H₂O, or the like. In certain embodiments, the reaction temperature is 60°C to 180°C.

Step 5-1: Substitution of Compound I-5 with R^{3a}-L^{a}-H to produce Compound III-A-1.

In certain embodiments, a useful solvent is DMF, DMSO, THF, CH₃CN, DCM, or the like. In certain embodiments, a useful base is triethylamine, *N,N-*diisopropylethylamine, potassium carbonate, potassium tert-butoxide, sodium hydroxide, or the like. In certain embodiments, the reaction temperature is -20°C to 180°C.

Step 5-2: Coupling (e.g., Suzuki reaction or Stille reaction) of Compound 1-5 with R^{3b}-L^{b}-boronic acid or R^{3b}-L^{b}-borate or R^{3b}-L^{b}-organotin compound (e.g., R^{3b}-L^{b}-Sn(*n*-Bu)₃) to produce Compound III-B-1.

In certain embodiments, a useful catalyst is Pd(PPh₃)₄, Pd(dppf)Cl₂•CH₂Cl₂, or the like. In certain embodiments, a useful base is Cs₂CO₃, K₃PO₄, Na₂CO₃, AcOK, NaHCO₃, K₂CO₃, or the like. In certain embodiments, a useful solvent is 1,4-dioxane/H₂O, DMF/H₂O, DMSO/H₂O, CH₃CN/H₂O, toluene/H₂O, or the like. In certain embodiments, the reaction temperature is 60°C to 180°C.

### Synthetic methods of Compounds III-A-2 and III-B-2

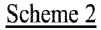

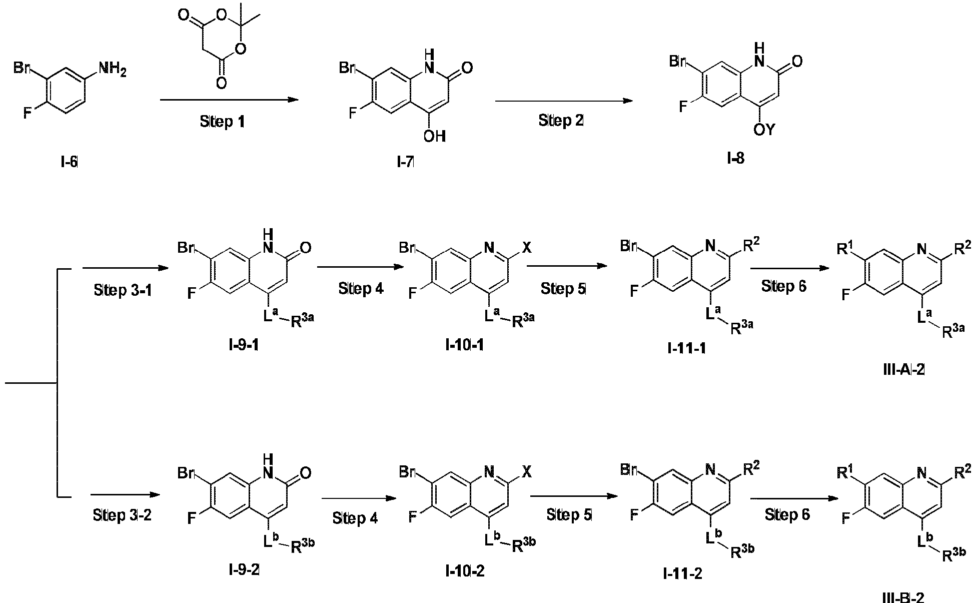

wherein R¹, R², R^{3a}, R^{3b}, L^{a} and L^{b} are as defined above for Formula III-A or III-B; X is selected from the group consisting of chlorine, bromine and iodine; and Y is selected from the group consisting of Ms, Ts, Tf.

In particular, R² is selected from the group consisting of C₁₋₈ alkyl, C₃₋₈ cycloalkyl and 4- to 10-membered heterocyclyl, the C₁₋₈ alkyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, OR³⁷, SR³⁷ and NR³¹R³², or R² is NR^{41a}R^{41b} wherein R³¹, R³², R³⁷, R^{41a} and R^{41b} are as defined above for Formula I.

Step 1: Ring closure of Compound 1-6 with Meldrum's acid under the catalysis of Lewis acid to produce Compound 1-7.

In certain embodiments, a useful Lewis acid is Eaton's reagent, aluminum trichloride, boron trifluoride, iron bromide, or the like. In certain embodiments, a useful solvent is THF, 1,4-dioxane, toluene, or the like. In certain embodiments, the reaction temperature is 20°C to 100°C.

Step 2: Substitution of Compound 1-7 to produce Compound 1-8.

In certain embodiments, a useful base is triethylamine, *N,N*-diisopropylethylamine, potassium carbonate, or the like. In certain embodiments, a useful solvent is THF, 1,4-dioxane, toluene, or the like. In certain embodiments, the reaction temperature is 20°C to 100°C.

Step 3-1: Substitution of Compound 1-8 with R^{3a}-L^{a}-H to produce Compound 1-9-1.

In certain embodiments, a useful solvent is DMF, DMSO, THF, CH₃CN, DCM, or the like. In certain embodiments, a that can be used base is triethylamine, *N,N*-diisopropylethylamine, potassium carbonate, potassium tert-butoxide, sodium hydroxide, or the like. In certain embodiments, the reaction temperature is -20°C to 180°C.

Step 3-2: Coupling (e.g., Suzuki reaction, Stille reaction) of Compound 1-8 with R^{3b}-L^{b}-boronic acid or R^{3b}-L^{b}-borate or R^{3b}-L^{b}-organotin compound (e.g., R^{3b}-L_{b}-Sn(n-Bu)₃) to produce Compound 1-9-2.

In certain embodiments, a useful catalyst is Pd(PPh₃)₄, Pd(dppf)Cl₂•CH₂Cl₂, or the like. In certain embodiments, a useful base is Cs₂CO₃, K₃PO₄, Na₂CO₃, AcOK, NaHCO₃, K₂CO₃, or the like. In certain embodiments, a useful solvent is 1,4-dioxane/H₂O, DMF/H₂O, DMSO/H₂O, CH₃CN/H₂O, toluene/H₂O, or the like. In certain embodiments, the reaction temperature is 60°C to 180°C.

Step 4: Halogenation of Compound 1-9-1 and Compound 1-9-2 to produce Compound 1-10-1 and Compound 1-10-2, respectively.

In certain embodiments, a useful halogenating agent is phosphorus oxychloride, PCl₅, phosphorus oxybromide, HBr, or the like. In certain embodiments, a useful solvent is 1,4-dioxane, DMF, EA, or the like. In certain embodiments, the reaction temperature is -20°C to 100°C.

Step 5: Reaction of Compounds I-10-1 and 1-10-2 with R²MgX (when R² in Compounds I-11-1 and I-11-2 is attached to the pyridine ring via its carbon atom) or R²H (when R² in Compounds I-11-1 and I-11-2 is attached to the pyridine ring via its nitrogen atom) to produce Compounds I-11-1 and I-11-2, respectively.

In certain embodiments, a useful solvent is THF, 1,4-dioxane, toluene, or the like. In certain embodiments, the reaction temperature is -20°C to 100°C.

Step 6: Coupling (e.g., Suzuki reaction, Stille reaction) of Compounds I-11-1 and I-11-2 with R¹-boronic acid or R¹-borate or R¹-organotin compound (e.g., R¹Sn(n-Bu)₃) to produce Compounds III-A-2 and III-B-2, respectively.

In certain embodiments, a useful catalyst is Pd(PPh₃)₄, Pd(dppf)Cl₂•CH₂Cl₂, or the like. In certain embodiments, a useful base is selected as Cs₂CO₃, K₃PO₄, Na₂CO₃, AcOK, NaHCO₃, K₂CO₃, or the like. In some embodiments, a useful solvent is 1,4-dioxane/H₂O, DMF/H₂O, DMSO/H₂O, CH₃CN/H₂O, toluene/H₂O, or the like. In certain embodiments, the reaction temperature is 60°C to 180°C.

### Synthetic methods of Compound IV-A-1 and IV-B-1

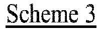

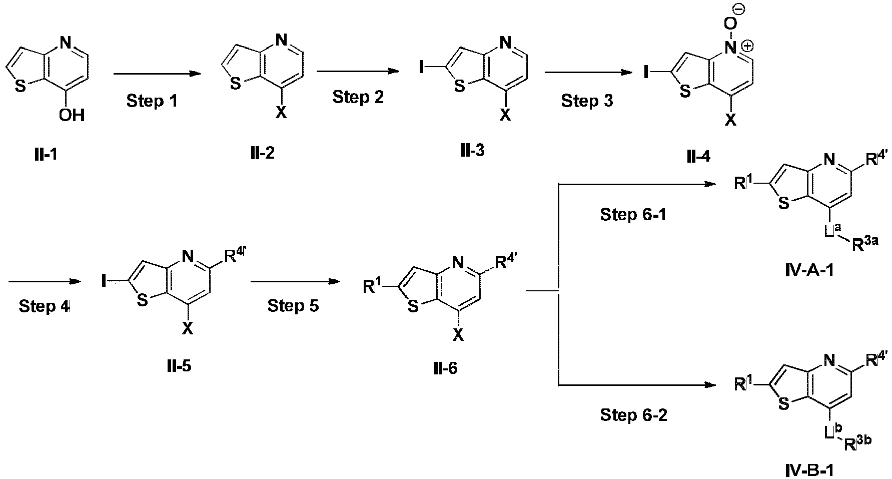

wherein R¹, R^{3a}, R^{3b}, L^{a} and L^{b} are as defined above for Formula IV-A or IV-B; R⁴ is selected from the group consisting of NR^{41a}R^{41b} C₁₋₁₅ alkyl, C₁₋₈ alkoxy, C₂₋₈ alkenyl, C₂₋₈ alkynyl, C₃₋₈ cycloalkyl, 4- to 10-membered heterocyclyl, and the C₁₋₁₅ alkyl, C₁₋₈ alkoxy, C₂₋₈ alkenyl, C₂₋₈ alkynyl, C₃₋₈ cycloalkyl, 4- to 10-membered heterocyclyl may optionally be substituted by one or more of the following substituents: halogen, C₁₋₄ haloalkyl, C₁₋₄ hydroxyalkyl, 4- to 7-membered heterocyclyl, CN, OR³⁷, SR³⁷, C(O)R³⁰, C(O)NR³¹R³², NR³¹R³²C(O), C(O)OR³⁰, OC(O)R³⁰, OC(O)NR³¹R³², NR³³C(O)NR³¹R³², and NR³¹R³²; X is selected from the group consisting of chlorine, bromine and iodine.

In certain embodiments, R^{4'} is NR^{41a}R^{41b}, wherein R^{41a} and R^{41b} are as defined above for Formula I.

In certain embodiments, R^{4'} is C₁₋₁₅ alkyl, C₁₋₈ alkoxy, C₃₋₈ cycloalkyl, or 4- to 10-membered heterocyclyl, and the C₁₋₁₅ alkyl, C₁₋₈ alkoxy, C₃₋₈ cycloalkyl or 4- to 10-membered heterocyclyl may optionally be substituted by one or more of the following substituents: halogen, C₁₋₄ haloalkyl, C₁₋₄ hydroxyalkyl, 4- to 7-membered heterocyclyl, OR³⁷, SR³⁷, and NR³¹R³².

Step 1: Halogenation of Compound II-1 to produce Compound II-2.

In certain embodiments, a useful halogenating agent is phosphorus oxychloride, PCl₅, phosphorus oxybromide, HBr, or the like. In certain embodiments, a useful solvent is 1,4-dioxane, DMF, EA, or the like. In certain embodiments, the reaction temperature is -20°C to 100°C.

Step 2: Iodination of Compound II-2 to produce Compound II-3.

In certain embodiments, a useful iodinating agent is I2, NIS, HI, or the like. In certain embodiments, a useful solvent is acetic acid, 1,4-dioxane, DMF, EA, or the like. In certain embodiments, the reaction temperature is -20°C to 100°C.

Step 3: Oxidation of Compound II-3 to produce Compound II-4.

In certain embodiments, a useful oxidizing agent is m-chloroperoxybenzoic acid, hydrogen peroxide, carbamide peroxide, or the like. In certain embodiments, a useful solvent is DCM, chloroform, 1,2-dichloroethane, 1,4-dioxane, or the like. In certain embodiments, the reaction temperature is -20°C to 100°C.

Step 4: Reaction of Compound II-4 with R⁴MgX (when R⁴ in Compound II-5 is attached to the pyridine ring via its carbon atom) or R⁴H (when R⁴ in Compound II-5 is attached to the pyridine ring via its nitrogen atom) to produce Compound II-5.

In certain embodiments, a useful solvent is THF, 1,4-dioxane, toluene, or the like. In certain embodiments, the reaction temperature is -20°C to 100°C.

Step 5: Coupling (e.g., Suzuki reaction, Stille reaction) of Compound II-5 with R¹-boronic acid or R¹-borate or R¹-organotin compound (e.g., R¹Sn(n-Bu)₃) to produce Compound II-6.

In certain embodiments, a useful catalyst is Pd(PPh₃)₄, Pd(dppf)Cl₂•CH₂Cl₂, or the like. In certain embodiments, a base is CS₂CO₃, K₃PO₄, Na₂CO₃, AcOK, NaHCO₃, K₂CO₃, or the like. In some embodiments, a useful solvent is 1,4-dioxane/H₂O, DMF/H₂O, DMSO/H₂O, CH₃CN/H₂O, toluene/H₂O, or the like. In certain embodiments, the reaction temperature is 60°C to 180°C.

Step 6-1: Substitution of Compound II-6 with R^{3a}-L^{a}-H to produce Compound IV-A-1.

In certain embodiments, a useful solvent is DMF, DMSO, THF, CH₃CN, DCM, or the like. A useful base is triethylamine, N,N-diisopropylethylamine, potassium carbonate, potassium tert-butoxide, sodium hydroxide, or the like. In certain embodiments, the reaction temperature is -20°C to 180°C.

Step 6-2: Coupling (e.g., Suzuki reaction, Stille reaction) of Compound II-6 with R^{3b}-L^{b}-boronic acid or R^{3b}-L^{b}-borate or R^{3b}-L^{b}-organotin compound (e.g., R³-L^{b}-Sn(n-Bu)₃) to produce Compound IV-B-1.

In certain embodiments, a useful catalyst is Pd(PPh₃)₄, Pd(dppf)Cl₂•CH₂Cl₂, or the like. In certain embodiments, a useful base is Cs₂CO₃, K₃PO₄, Na₂CO₃, AcOK, NaHCO₃, K₂CO₃, or the like. In some embodiments, a useful solvent is 1,4-dioxane/H₂O, DMF/H₂O, DMSO/H₂O, CH₃CN/H₂O, toluene/H₂O, or the like. In certain embodiments, the reaction temperature is 40°C to 180°C.

### Synthetic methods of Compound IV-A-2 and IV-B-2

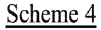

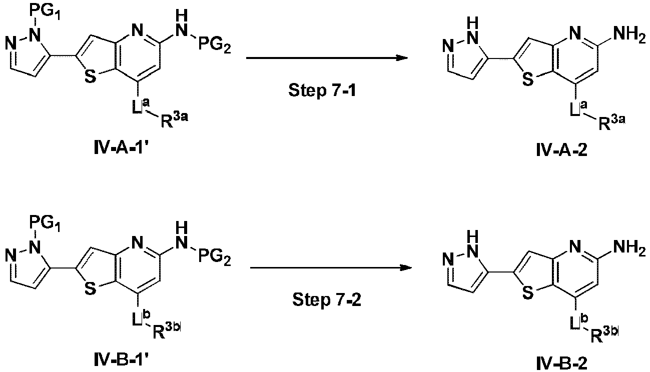

wherein R^{3a}, R^{3b}, L^{a} and L^{b} are as defined above for Formula IV-A or IV-B; PG₁ is a protecting group such as THP, Boc, Cbz, or the like; and PG₂ is an amine protecting group such as tert-butyl, tert-octyl, or the like.

Step 7-1: Deprotection of Compound IV-A-1' to produce Compound IV-A-2.

Step 7-2: Deprotection of Compound IV-B-1' to produce Compound IV-B-2.

In certain embodiments, a useful acid in steps 7-1 and 7-2 is hydrochloric acid, hydrobromic acid, sulfuric acid, nitric acid, AlCl₃, p-toluenesulfonic acid, or the like, and a useful solvent in steps 7-1 and 7-2 is DCM, EA, 1,4-dioxane, DMSO, CH₃CN, toluene, or the like, and the reaction temperature is -10°C to 180°C.

### Beneficial Effects

The compound of the invention has significant agonistic activity on NLRP3 and the signaling pathway thereof, has no apparent side effect, and is useful for the treatment of an abnormal cell proliferation disease (e.g., cancer).

### EXAMPLES

The present invention will be further described with reference to the following examples, the provision of which is not intended to limit the scope of the invention.

The abbreviations as used herein have the following meanings:

| NMR | nuclear magnetic resonance | MS | mass spectrum |
|---|---|---|---|
| TLC | thin layer chromatography | LC-MS | liquid chromatography-mass spectrometry |
| HPLC | high performance liquid chromatography | min | minute |
| Et | ethyl | m-CPBA | meta-chloroperoxybenzoic acid |
| Prep-HPLC | preparative high performance liquid chromatography | CDCl₃ | deuterated chloroform |
| DMSO-*d₆* | deuterated dimethyl sulfoxide | DCM/CH₂Cl₂ | dichloromethane |
| DMSO | dimethyl sulfoxide | DCE | 1,2-dichloroethane |
| PE | petroleum ether | DMF | *N*,*N*-diimethylformainide |
| NMP | *N*-methylpyrrolidone | EA/EtOAc | ethyl acetate |
| DIEA/DIPE A | *N*,*N*-diisopropylethylamine | THF | tetrahydrofuran |
| TFA | trifluoroacetic acid | LDA | lithium diisopropylamide |
| Pd(PPh₃)₄ | tetrakis(triphenylphosphine )palladi um | Pd | palladium |
| Pd₂(dba)₃ | tris(dibenzylideneacetone)dipalladi um | Pd(dppf)Cl₂•CH₂C l₂ | [1,1'-bis(diphenylphosphino)ferrocene]palladi um chloride-dichloromethane complex |
| Boc | tert-butoxycarbonyl | (BOC)₂O | di-tert-butyl dicarbonate |
| Me | methyl | Ms | mesyl |
| HBTU | benzotriazole-N,N,N',N'-tetramethylurea hexafluorophosphate | DavePhos | 2-dicyclohexylphosphino-2'-(*N*,*N-*dimethylamine)-biphenyl |
| *i*-Pr | isopropyl | t-Bu | tert-butyl |
| Bn | benzyl | Ts | *p*-toluenesulfonyl |
| DAVE-Phos | 2-dicyclohexylphosphino-2' -(N ,N-dimethylamine)-biphenyl | LDA | lithium diisopropylamide |
| BrettPhos | dicyclohexyl(2',4',6'-triisopropyl-3,6-dimethoxy-[1,1'-biphenyl]-2-yl)phosphine | | |

The structure of the compound of the invention was identified by nuclear magnetic resonance (¹H NMR) and/or mass spectrometry (MS).

¹H NMR chemical shift (δ) was recorded in parts per million (ppm). ¹H NMR was measured with a Bruker AVACE III HD 400 MHz nuclear magnetic spectrometer, using a the solvent of deuterated methanol (CD₃OD), deuterated chloroform (CDCl₃) or hexadeuterated dimethyl sulfoxide (DMSO-d₆), and an internal standard of tetramethylsilane (TMS). s: singlet, d: doublet, t: triplet, q: quartet, dd: double doublet, qd: quartet doublet, m: multiplet, br: broad, *J*: coupling constant, Hz: Hertz.

The reaction was monitored with TLC or LC-MS.

Silica gel GF 254 was used as the stationary phase in TLC.

¹H NMR spectrum: Bruker superconducting nuclear magnetic resonance spectrometer (Model AVACE III HD 400 MHz).

LC/MS mass spectrometer: Aglient 1260 Infinity/Aglient 6120 Quadrupole

The compounds of the invention may be separated and purified by preparative TLC, silica gel column chromatography, Prep-HPLC and/or flash column chromatography.

Agilent 1260 preparative liquid chromatograph was used in Prep-HPLC, and the detection wavelength was 214 nm or 254 nm. The chromatographic column was Waters SunFire Prep C18 OBD (19 mm × 150 mm × 5.0 µm). The column temperature was 25°C, and the elution conditions were as follows:
Condition 1: 10%-90% acetonitrile and 90%-10% ammonium formate aqueous solution (0.05%), 0-16 min; flow rate: 24 mL/min;
Condition 2: 10%-46% acetonitrile and 90%-54% ammonium bicarbonate aqueous solution (0.05%), 0-7.2 min; flow rate: 24 mL/min;
Condition 3: 10%-90% acetonitrile and 90%-10% formic acid aqueous solution (0.05%), 0-16 min; flow rate: 28 mL/min;
Condition 4: 10%-90% acetonitrile and 90%-10% ammonium bicarbonate aqueous solution (0.05%), 0-16 min; flow rate: 28 mL/min;
Condition 5: 10%-60% acetonitrile and 90%-40% formic acid aqueous solution (0.05%), 0-16 min; flow rate: 28 mL/min;
Condition 6: 5%-95% acetonitrile and 95%-5% formic acid aqueous solution (0.05%), 0-20 min; flow rate: 28 mL/min.

In the column chromatography, 200-300 mesh silica gel (Qingdao Ocean) was generally used as the stationary phase. Eluent System A: dichloromethane and methanol; Eluent System B: petroleum ether and ethyl acetate. The volume ratio of the solvents was adjusted according to the polarity of the compound.

Biotage flash column chromatograph was used in the fast column chromatography.

The microwave reaction was carried out using BiotageInitiator + microwave reactor.

In the following examples, unless otherwise specified, the reaction temperature was room temperature (15°C to 30°C).

The reagents used herein were purchased from companies such as Acros Organics, Aldrich Chemical Company, or

### Top Biochemical.

### Intermediate Preparation Example 1:

7-chloro-2-(1-(tetrahydro-2*H*-pyran-2-yl)-1*H*-pyrazol-5-yl)thieno[3,2-b]pyridine-5-amine (Compound 1g)

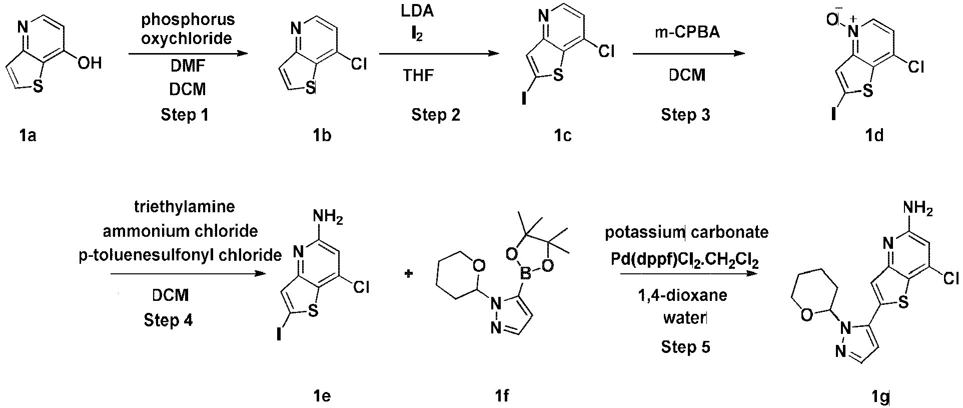

### Step 1: Synthesis of 7-chlorothieno[3,2-b]pyridine (Compound 1b)

DMF (7.24 g) and phosphorus oxychloride (30.36 g) were added to a solution of Compound 1a (15 g) in DCM (60 mL) at room temperature. The reaction proceeded at an elevated temperature of 50°C for 5 hours. The reaction liquid was poured slowly into warm water and was adjusted to pH > 8 with sodium hydroxide. The organic layer from DCM extraction was dried and concentrated. Purification by column chromatography (Eluent System B) gave Compound 1b (16 g).

MS (ESI, m/z): 170.1 [M+H]⁺.

### Step 2: Synthesis of 7-chloro-2-iodothieno[3,2-b]pyridine (Compound 1c)

LDA (2 M, 9.73 mL) was slowly added to a solution of Compound 1b (3 g) in THF (20 mL) at -65°C. The reaction was stirred for 1 hour. A solution of I₂ (4.92 g) in THF (20 mL) was slowly added to the reaction system, and the reaction continued for 2 hours. The reaction proceeded at room temperature for 2 hours. The reaction was quenched by adding a saturated ammonium chloride aqueous solution. The organic layer from EA extraction was dried and concentrated. Purification by column chromatography (Eluent System B) gave Compound 1c (4.5 g).

MS (ESI, m/z): 295.9 [M+H]⁺.

### Step 3: Synthesis of 7-chloro-2-iodothieno[3,2-b]pyridine-4-oxide (Compound 1d)

m-Chloroperoxybenzoic acid (2.18 g) was added to a solution of Compound 1c (2.5 g) in DCM (10 mL) at room temperature, and the reaction proceeded at room temperature for 4 hours. The reaction was quenched by adding a saturated sodium carbonate aqueous solution, and was extracted with DCM (15 mL^{∗}3). The organic layer was dried, concentrated, slurried with DCM, and filtered to give Compound Id (2.4 g).

MS (ESI, m/z): 311.9 [M+H]⁺.

### Step 4: Synthesis of 7-chloro-2-iodothieno[3,2-b]pyridine-5-amine (Compound 1e)

p-Toluenesulfonyl chloride (1.47 g) was added to a solution of Compound Id (2.4 g) in chloroform (10 mL) at room temperature, and the reaction proceeded for 4 hours. A solution of triethylamine (3.90 g) and ammonium chloride (2.04 g) in DCM (10 mL) was added to the reaction liquid. The reaction proceeded at 50°C for 4 hours. The reaction liquid was filtered, and the mother liquor was concentrated. Purification by column chromatography (Eluent System A) gave Compound 1e (700 mg).

MS (ESI, m/z): 310.9 [M+H]⁺.

### Step 5: Synthesis of 7-chloro-2-(1-(tetrahydro-2H-pyran-2-yl)-1H-pyrazol-5-yl)thieno[3,2-b]pyridine-5-amine (Compound 1g)

Potassium carbonate (933.21 mg) and Pd(dppf)Cl₂•CH₂Cl₂ (82.39 mg) were added to a solution of Compound If (752.39 mg) and Compound 1e (700 mg) in 1,4-dioxane (4 mL)/water (0.5 mL) at room temperature, and the reaction proceeded at 80°C for 2 hours. The reaction was quenched by adding water, and was extracted with EA. The organic layer was dried, concentrated, and then separated and purified by column chromatography (Eluent System B) to give Compound 1g (600 mg).

MS (ESI, m/z): 335.1 [M+H]⁺.

### Intermediate Preparation Example 2:

(7-bromo-*N*-(tert-butyl)-2-(1-(tetrahydro-2*H*-pyran-2-yl)-1*H*-pyrazol-5-yl)thieno[3,2-b]pyridine-5-amine (Compound 2f)

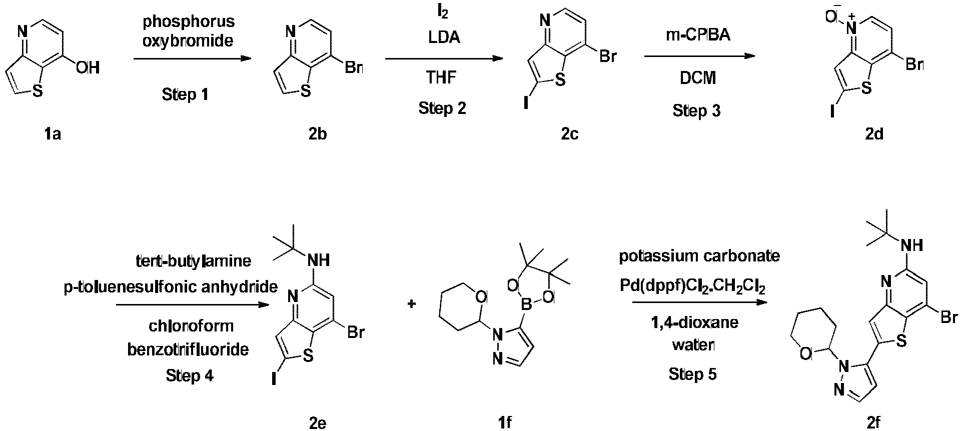

### Step 1: Synthesis of 7-bromothieno[3,2-b]pyridine (Compound 2b)

Phosphorus oxybromide (158.90 g) was heated to 60°C to a molten state, and Compound 1a (14 g) was added thereto. The reaction proceeded at an elevated temperature of 100°C for 2 hours. The reaction liquid was poured slowly into ice water and was adjusted to pH > 8 with sodium hydroxide. The organic layer from DCM extraction was dried and concentrated. Purification by column chromatography (Eluent System B) gave Compound 2b (18.5 g).

MS (ESI, m/z): 215.9[M+H]⁺.

### Step 2: Synthesis of 7-bromo-2-iodothieno[3,2-b]pyridine (Compound 2c)

LDA (4 M, 14.2 mL) was slowly added to a solution of Compound 2b (10 g) in THF (100 mL) at -65°C, and the reaction proceeded at a low temperature for 1 hour with stirring. A solution of I₂ (14.18 g) in THF (80 mL) was slowly added to the reaction system, and the reaction proceeded at a low temperature for 2 hours. Slowly warming to room temperature and the reaction proceeded for 2 hours. The reaction was quenched by adding a saturated ammonium chloride aqueous solution. The organic layer from EA extraction was dried and concentrated. Purification by column chromatography (Eluent System B) gave Compound 2c (13.5 g).

MS (ESI, m/z): 341.9[M+H]⁺.

### Step 3: Synthesis of 7-bromo-2-iodothieno[3,2-b]pyridine-4-oxide (Compound 2d)

m-Chloroperoxybenzoic acid (3.57 g) was added to a solution of Compound 2c (4.08 g) in DCM (50 mL) at room temperature, and the reaction proceeded at room temperature for 4 hours. The reaction was quenched by adding a saturated sodium carbonate aqueous solution, and was extracted with DCM. The organic layer was dried, concentrated, slurried with DCM, and filtered to give Compound 2d (3.0 g).

MS (ESI, m/z): 357.9[M+H]⁺.

### Step 4: Synthesis of 7-bromo-N-(tert-butyl)-2-iodothieno[3,2-b]pyridine-5-amine (Compound 2e)

tert-Butylamine (3.22 g) and p-toluenesulfonic anhydride (7.19 g) were sequentially added to a solution of Compound 2d (3.0 g) in chloroform (15 mL)/benzotrifluoride (15 mL) at 0°C. The reaction proceeded at 0°C for 1 hour. The reaction liquid was filtered, and the mother liquor was concentrated. Purification by column chromatography (Eluent System B) gave Compound 2e (2.3 g).

MS (ESI, m/z): 435.0[M+H]⁺.

### Step 5: Synthesis of 7-bromo-N-(tert-butyl)-2-(1-(tetrahydro-2H-pyran-2-yl)-1H-pyrazol-5-yl)thieno[3,2-b]pyridine-5-amine (Compound 2f)

Potassium carbonate (1.54 g) and Pd(dppf)Cl₂•CH₂Cl₂ (204.49 mg) were sequentially added to a solution of Compound 1f (1.87 g) and Compound 2e (2.3 g) in 1,4-dioxane (20 mL)/water (2 mL), and the reaction proceeded at 50°C for 8 hours with stirring. The reaction was quenched by adding water, and was extracted with EA (40 mL^{∗}3). The organic layer was dried, concentrated, and was separated and purified by column chromatography (PE:EA=5:1) to give Compound 2f (2.0 g).

MS (ESI, m/z): 435.0[M+H]⁺.

### Example 1:

*N*⁷-(1-methylazetidin-3-yl)-2-(1*H*-pyrazol-5-yl)thieno[3,2-b]pyridine-5,7-diamine (Compound 37)

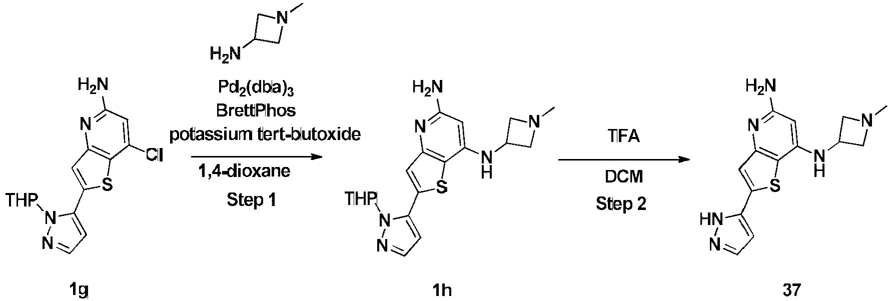

### Step 1: Synthesis of N⁷-(1-methylazetidin-3-yl)-2-(1-(tetrahydro-2H-pyran-2-yl)-1H-pyrazol-5-yl)thieno[3,2-b]pyridine-5,7-diamine (Compound 1h)

Compound 1g (50 mg), 1-methylazetidin-3-amine (80 mg), Pd₂(dba)₃ (27.4 mg), BrettPhos (27.9 mg) and potassium tert-butoxide (77 mg) were added to 1,4-dioxane (2.5 mL), and the microwave reaction proceeded at an elevated temperature of 120°C for 5 hours. The system was filtrated with Celite, and the filtrate was concentrated, diluted with a small amount of methanol, and purified by preparative TLC (Eluent System A) to give Compound 1h (40 mg).

MS (ESI, m/z): 385.2 [M+H]⁺.

### Step 2: Synthesis of N⁷-(1-methylazetidin-3-yl)-2-(1H-pyrazol-5-yl)thieno[3,2-b]pyridine-5,7-diamine (Compound 37)

Compound 1h (35 mg) was added to TFA (3 mL) and DCM (15 mL), and the reaction proceeded at room temperature for 2 hours. The system was concentrated, diluted with methanol, and adjusted to pH = 9 with a saturated sodium bicarbonate aqueous solution. The system was suction filtrated. The filtrate was separated and purified by Prep-HPLC (Elution Condition 3), and lyophilized to give Compound 37 (17 mg).

MS (ESI, m/z): 301.1 [M+H]⁺.

¹H NMR (DMSO-*d₆*, 400 MHz) δ 13.27 (s, 1H), 8.31 (s, 1H), 7.92 (s, 1H), 7.47 (s, 1H), 7.40 (s, 2H), 6.89 (s, 1H), 5.65 (s, 1H), 4.70-4.00 (m, 5H), 2.92 (s, 3H).

### Example 2:

3-((5-amino-2-(1*H*-pyrazol-5-yl)thieno[3,2-b]pyridin-7-yl)amino)-1-propanol (Compound 35)

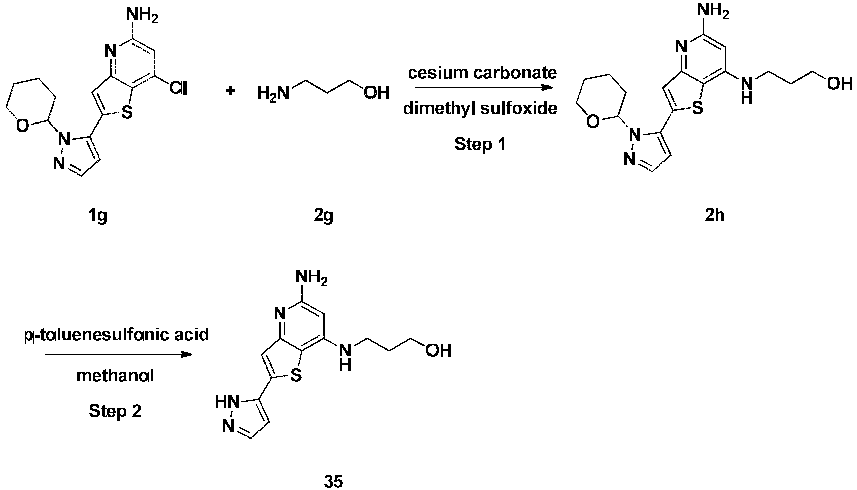

### Step 1: Synthesis of 3-((5-amino-2-(1-(tetrahydro-2H-pyran-2-yl)-1H-pyrazol-5-yl)thieno[3,2-b]pyridin-7-yl)amino)-1-propanol (Compound 2h)

A solution of Compound 1g (50 mg), Compound 2g (11.22 mg) and cesium carbonate (97.07 mg) in dimethyl sulfoxide (2 mL) was placed in a microwave tube, and the reaction proceeded at an elevated temperature of 120°C for 5 hours. The reaction was quenched by adding water, and was extracted with EA. the organic layer was dried, concentrated, and was separated and purified by preparative TLC (Eluent System A) to give Compound 2h (30 mg).

MS (ESI, m/z): 374.2 [M+H]⁺.

### Step 2: Synthesis of 3-((5-amino-2-(1H-pyrazol-5-yl)thieno[3,2-b]pyridin-7-yl)amino)-1-propanol (Compound 35)

p-Toluenesulfonic acid (16.58 mg) was added to a solution of Compound 2h (30 mg) in methanol (3 mL). The reaction proceeded at room temperature for 16 hours. Separation and purification by Prep-HPLC (Elution Condition 1) gave Compound 35 (4 mg).

MS (ESI, m/z): 290.1 [M+H]⁺.

¹H NMR (CD₃OD, 400 MHz) 8 7.73 (d, *J* = 2.1 Hz, 1H), 7.40 (s, 1H), 6.71 (d, *J* = 1.9 Hz, 1H), 6.22 (s, 1H), 4.32 (t, *J=* 5.5 Hz, 2H), 3.19 (s, 2H), 2.25 (s, 2H).

### Example 3:

*N*⁷-(6-methyl-pyridin-2-yl)methyl-2-(1*H*-pyrazol-5-yl)thieno[3,2-b]pyridine-5,7-diamine (Compound 49)

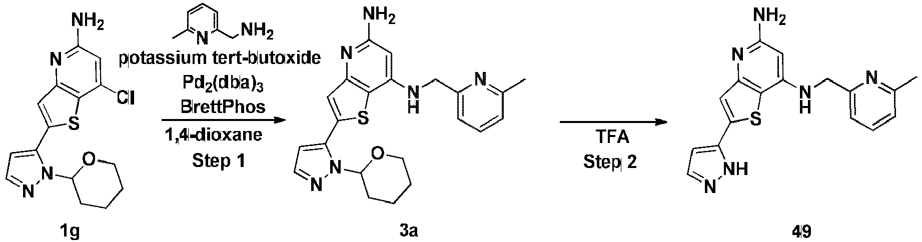

### Step 1: Synthesis of (N⁷-(6-methylpyridin-2-yl)methyl)-2-(1-tetrahydro-2H-pyran-2-yl)-1H-pyrazol-5-yl)thieno[3,2-b]pyridine-5,7-diamine (Compound 3a)

Compound 1g (75 mg), 6-methylpyridin-2-ylmethylamine (54.73 mg), potassium tert-butoxide (125.67 mg), Pd₂(dba)₃ (40.99 mg) and BrettPhos (48.03 mg) were added to 1,4-dioxane (2 mL), and the microwave reaction proceeded at 120 °C for 2 hours under N₂ protection. The system was dried in a rotary dryer, and was purified by flash column chromatography (Eluent System A) to give Compound 3a (50 mg).

MS (ESI, m/z): 421.2 [M+H]⁺.

### Step 2: Synthesis of N⁷-(6-methyl-pyridin-2-yl)methyl-2-(1H-pyrazol-5-yl)thieno[3,2-b]pyridine-5,7-diamine (Compound 49)

Compound 3a (50 mg) was added to TFA (2 mL), and the reaction proceeded at an elevated temperature of 75°C for 5 hours. The reaction liquid was concentrated under a reduced pressure, separated and purified by Prep-HPLC (Elution Condition 3), and lyophilized to give Compound 49 (2 mg).

MS (ESI, m/z): 337.1 [M+H]⁺.

¹H NMR (DMSO-*d₆*, 400 MHz) δ 13.27 (s, 1H), 13.02 (s, 1H), 8.59 (t, 1H), 7.91 (s, 1H), 7.72 (t, 1H), 7.46 (s, 1H), 7.33-7.14 (m, 4H), 6.88 (s, 1H), 5.67 (s, 1H), 4.57 (d, *J* = 6 Hz, 1H), 2.51(s, 3H).

### Example 4:

(*R*)-3-((5-amino-2-(1*H*-pyrazol-5-yl)thieno[3,2-b]pyridin-7-yl)oxy)propane-1,2-diol (Compound 50)

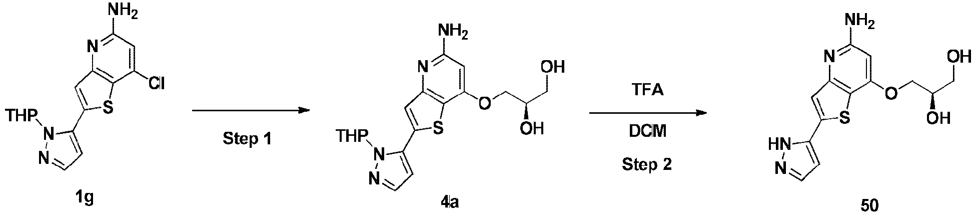

### Step 1: Synthesis of (2R)-3-((5-amino-2-(1-(tetrahydro-2H-pyran-2-yl)-1H-pyrazol-5-yl)thieno[3,2-b]pyridine-7-yl)oxy)propane-1,2-diol (Compound 4a)

Compound 1g (50 mg) was added to NMP (3 mL), and (4*R*)-2,2-dimethyl-1,3-dioxolane-4-methanol (39.47 mg) and potassium tert-butoxide (33.51 mg) were added thereto. The reaction proceeded at 100°C for 10 hours. The reaction was quenched by adding 10 mL water, and was extracted with EA. The organic layer was washed with saturated brine, dried and filtered. The filtrate was concentrated, and was separated and purified by TLC (Eluent System B) to give Compound 4a (45 mg).

MS (ESI, m/z): 391.1 [M+H]⁺.

### Step 2: Synthesis of (R)-3-((5-amino-2-(1H-pyrazol-5-yl)thieno[3,2-b]pyridin-7-yl)oxy)propane-1,2-diol (Compound 50)

Compound 4a (45 mg) was added to DCM (2 mL) and TFA (1 mL), and the reaction proceeded at 25°C for 10 hours with stirring. The reaction liquid was concentrated under a reduced pressure. The residual was dissolved again with methanol, and was adjusted to pH 8 to 9 with a saturated sodium bicarbonate aqueous solution. The system was filtrated. The filtrate was separated and purified by Prep-HPLC (Elution Condition 2), and lyophilized to give Compound 50 (25 mg).

MS (ESI, m/z): 307.0 [M+H]⁺.

¹H NMR (DMSO-*d₆*, 400 MHz) δ 13.00 (s, 1H), 7.83 (s, 1H), 7.36 (s, 1H), 6.76 (t, *J* = 2.0 Hz, 1H), 6.07 (s, 1H), 5.92 (s, 2H), 5.08 (d, *J* = 5.6 Hz, 1H), 4.75 (t, *J* = 5.6 Hz, 1H), 4.10-4.11 (m, 1H), 3.98-3.99 (m, 1H), 3.84-3.85 (m, 1H), 3.49 (t, *J=* 5.6 Hz, 2H).

### Example 5:

3-((5-amino-2-(1*H*-pyrazol-5-yl)thieno[3,2-b]pyridin-7-yl)amino)cyclobutanol (Compound 40)

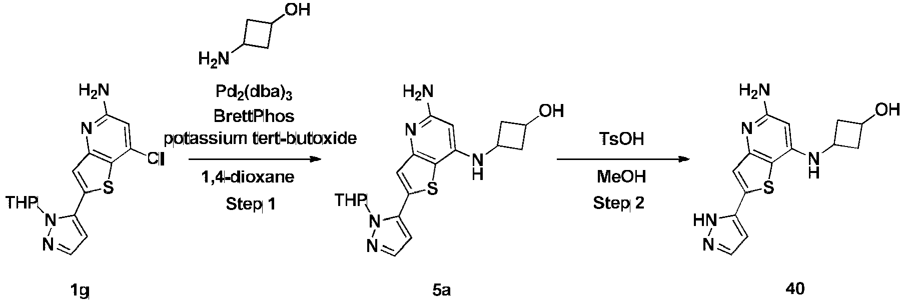

### Step 1: Synthesis of 3-((5-amino-2-(1-(tetrahydro-2H-pyran-2-yl)-1H-pyrazol-5-yl)thieno[3,2-b]pyridin-7-yl)amino)cyclobutanol (Compound 5a)

Compound 1g (70 mg), 3-aminocyclobutanol (140 mg), Pd₂(dba)₃ (39.4 mg), BrettPhos (39.4 mg) and potassium tert-butoxide (108 mg) were added to 1,4-dioxane (2.5 mL), and the microwave reaction proceeded at an elevated temperature of 120°C for 5 hours. The system was filtrated with Celite, and the filtrate was concentrated, then diluted with a small amount of methanol, and purified by preparative TLC (Eluent System A) to give Compound 5a (13 mg).

MS (ESI, m/z): 386.2 [M+H]⁺.

### Step 2: Synthesis of 3-((5-amino-2-(1H-pyrazol-5-yl)thieno[3,2-b]pyridin-7-yl)amino)cyclobutanol (Compound 40)

Compound 5a (13 mg) was added in absolute methanol (2 mL), and p-toluenesulfonic acid monohydrate (10 mg) was added. The reaction proceeded at room temperature for 1 hour. Separation and purification by Prep-HPLC (Elution Condition 2) and lyophilization gave Compound 40 (2 mg).

MS (ESI, m/z): 302.1 [M+H]⁺.

¹H NMR (DMSO-*d₆*, 400 MHz) δ 12.96 (s, 1H), 7.80 (s, 1H), 7.27 (s, 1H), 6.69 (s, 1H), 6.43-6.32 (t, *J=* 6.1 Hz, 1H), 5.60-5.40 (m, 3H), 5.18-5.02 (m, 1H), 4.01-3.84 (m, 1H), 2.78-2.62 (m, 2H), 2.36-2.26 (m, 1H), 2.25-2.14 (m, 1H), 1.93-1.82 (m, 1H).

### Example 6:

(*S*)-*N*⁷-(1-methylpyrrolidin-3-yl)-2-(1*H*-pyrazol-5-yl)thieno[3,2-b]pyridine-5,7-diamine (Compound 51)

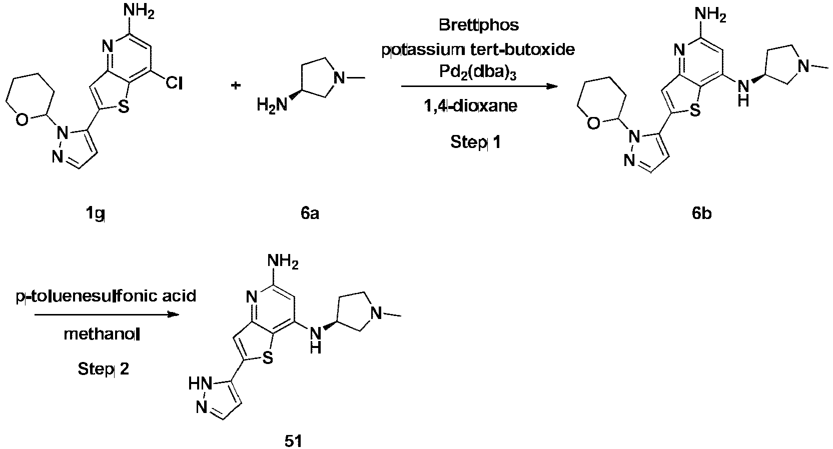

### Step 1: Synthesis of N⁷-((S)-1-methylpyrrolidin-3-yl)-2-(1-(tetrahydro-2H-pyran-2-yl)-1H-pyrazol-5-yl)thieno[3,2-b]pyridine-5,7-diamine (Compound 6b)

Pd₂(dba)₃ (4.10 mg), BrettPhos (4.80 mg) and potassium tert-butoxide (30.16 mg) were added to a solution of Compound 6a (13.19 mg) and Compound 1g (30 mg) in 1,4-dioxane (2 mL) at room temperature. The reaction system was placed in microwave and reacted at an elevated temperature of 120°C for 5 hours under N₂ protection. Drying, concentration, and separation and purification by preparative TLC (Eluent System A) gave Compound 6b (15 mg).

MS (ESI, m/z): 399.1 [M+H]⁺.

### Step 2: Synthesis of (S)-N⁷-(1-methylpyrrolidin-3-yl)-2-(1H-pyrazol-5-yl)thieno[3,2-b]pyridine-5,7-diamine (Compound 51)

p-Toluenesulfonic acid (7.77 mg) was added to a solution of Compound 6b (15 mg) in methanol (4 mL) at room temperature, and the reaction proceeded at 25°C for 4 hours with stirring. Separation and purification by Prep-HPLC (Elution Condition 1) gave Compound 51 (4 mg).

MS (ESI, m/z): 315.1 [M+H]⁺.

¹H NMR (DMSO-*d₆*, 400 MHz) δ 13.20 (s, 1H), 7.86 (s, 1H), 7.54 (s, 1H), 7.43 (s, 1H), 6.82 (s, 3H), 5.76 (d, *J=* 7.4 Hz, 1H), 4.24 (d, *J* = 2.9 Hz, 1H), 3.18-2.98 (m, 4H), 2.66 (s, 3H), 2.39 (dd, *J* = 13.5, 7.1 Hz, 1H), 2.04 (dt, *J* = 13.2, 7.5 Hz, 1H).

### Example 7:

*N*-(2-(5-amino-2-(1*H*-pyrazol-5-yl)thieno[3,2-b]pyridin-7-yl)amino)ethyl)acetamide (Compound 52)

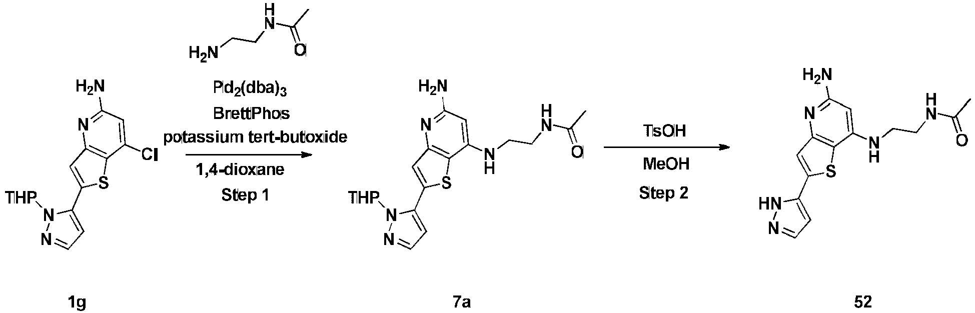

### Step 1: Synthesis of N-(2-((5-amino-2-(1-(tetrahydro-2H-pyran-2-yl)-1H-pyrazol-5-yl)thieno[3,2-b]pyridin-7-yl)amino)ethyl)acetamide (Compound 7a)

Compound 1g (60 mg), N-acetylethylenediamine (91 mg), Pd₂(dba)₃ (33.mg), BrettPhos (34 mg) and potassium tert-butoxide (100 mg) were added to 1,4-dioxane (2.5 mL), and the reaction proceeded at an elevated temperature of 120°C for 5 hours. The system was filtrated with Celite, and the filtrate was concentrated, diluted with a small amount of methanol, and purified by preparative TLC (Eluent System A) to give Compound 7a (60 mg).

MS (ESI, m/z): 401.2 [M+H]⁺.

### Step 2: Synthesis of N-(2-(5-amino-2-(1H-pyrazol-5-yl)thieno[3,2-b]pyridin-7-yl)amino)ethyl)acetamide (Compound 52)

Compound 7a (60 mg) was added to absolute methanol (2 mL), and p-toluenesulfonic acid monohydrate (51 mg) was added. The pH of the solution at this time was 4-5. The reaction proceeded at room temperature for 1 hour. Separation and purification by Prep-HPLC (Elution Condition 2) and lyophilization gave Compound 52 (12 mg).

MS (ESI, m/z): 317.1 [M+H]⁺.

¹H NMR (DMSO-*d₆*, 400 MHz) δ 12.96 (s, 1H), 8.05 (t, *J* = 5.4 Hz, 1H), 7.80 (s, 1H), 7.28 (s, 1H), 6.71 (s,1H), 6.14 (s, 1H), 5.65 (s, 1H), 5.48 (s, 2H), 3.31-3.27 (m, 2H), 3.20-3.22 (m, 2H), 1.84 (s,3H).

### Example 8:

*N*-(3-(5-amino-2-(1*H*-pyrazol-5-yl)thieno[3,2-b]pyridin-7-yl)amino)propyl)acetamide (Compound 53)

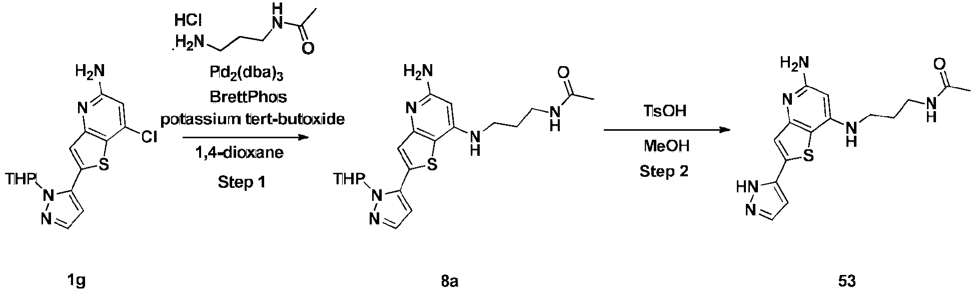

### Step 1: Synthesis of N-(3-((5-amino-2-(1-(tetrahydro-2H-pyran-2-yl)-1H-pyrazol-5-yl)thieno[3,2-b]pyridin-7-yl)amino)propyl)acetamide (Compound 8a)

Compound 1g (50 mg), *N*-(3-aminopropyl)acetamide hydrochloride (114 mg), Pd₂(dba)₃ (27 mg), BrettPhos (28 mg) and potassium tert-butoxide (151 mg) were added to 1,4-dioxane (2.5 mL), and the reaction proceeded at an elevated temperature of 120°C for 5 hours. The system was filtrated with Celite, and the filtrate was concentrated, diluted with a small amount of methanol, and purified by preparative TLC (Eluent System A) to give Compound 8a (20 mg).

MS (ESI, m/z): 415.2 [M+H]⁺.

### Step 2: Synthesis of N-(2-(5-amino-2-(1H-pyrazol-5-yl)thieno[3,2-b]pyridin-7-yl)amino)propyl)acetamide (Compound 53)

Compound 8a (18 mg) was added to absolute methanol (2 mL), and p-toluenesulfonic acid monohydrate (15 mg) was added. The pH of the solution at this time was 4 to 5. The reaction proceeded at room temperature for 1 hour. Separation and purification by Prep-HPLC (Elution Condition 2) and lyophilization gave Compound 53 (5 mg).

MS (ESI, m/z): 331.1 [M+H]⁺.

¹H NMR (DMSO-*d₆*, 400 MHz) δ 12.97 (s, 1H), 7.88 (t*, J* = 5.2 Hz, 1H), 7.78 (s, 1H), 7.28 (s, 1H), 6.69 (s,1H), 6.13 (t, *J* = 5.4 Hz, 1H), 5.63 (s, 1H), 5.48 (s, 2H), 3.10-3.18 (m, 4H), 1.82 (s, 3H), 1.73 (p, *J =* 6.8 Hz, 2H).

### Example 9:

*N*⁷-(3-(4-methylpiperazin-1-yl)propyl)-2-(1*H*-pyrazol-5-yl)thieno[3,2-b]pyridine-5,7-diamine (Compound 54)

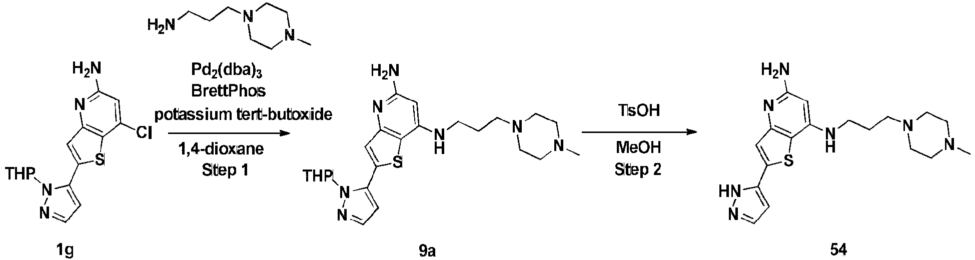

### Step 1: Synthesis of N⁷-(3-(4-methylpiperazin-1-yl)propyl)-2-(1-(tetrahydro-2H-pyran-2-yl)-1H-pyrazol-5-yl)thieno[3,2-b]pyridine-5,7-diamine (Compound 9a)

Compound 1g (50 mg), 1-(3-aminopropyl)-4-methylpiperazine (180.5 mg), Pd₂(dba)₃ (28.1 mg), BrettPhos (28.13 mg) and potassium tert-butoxide (77 mg) were added to 1,4-dioxane (2.5 mL), and the reaction proceeded at an elevated temperature of 120°C for 4 hours. The system was filtrated with Celite, and the filtrate was concentrated, then diluted with a small amount of methanol, and purified by preparative TLC (Eluent System A) to give Compound 9a (27 mg).

MS (ESI, m/z): 456.2 [M+H]⁺.

### Step 2: Synthesis of N⁷(3-(4-methylpiperazin-1-yl)propyl)-2-(1H-pyrazol-5-yl)thieno[3,2-b]pyridine-5,7-diamine (Compound 54)

Compound 9a (27 mg) was added to absolute methanol (2 mL), and p-toluenesulfonic acid monohydrate (23 mg) was added. The pH of the solution at this time was 5. The reaction proceeded at room temperature for 1 hour. Separation and purification by Prep-HPLC (Elution Condition 2) and lyophilization gave Compound 54 (2 mg).

MS (ESI, m/z): 372.2 [M+H]⁺.

¹H NMR (DMSO-*d₆*, 400 MHz) δ 12.95 (s, 1H), 7.80 (s, 1H), 7.27 (s, 1H), 6.70 (s, 1H), 6.37 (s, 1H), 5.62 (s, 1H), 5.48 (s, 2H), 3.22-3.14 (m, 2H), 2.47-2.31 (m, 10H), 2.19 (s, 3H), 1.82-1.75 (m, 2H).

### Example 10:

((*R*)-1-((5-amino-2-(1*H*-pyrazol-5-yl)thieno[3,2-b]pyridin-7-yl)amino)-2-propanol (Compound 29)

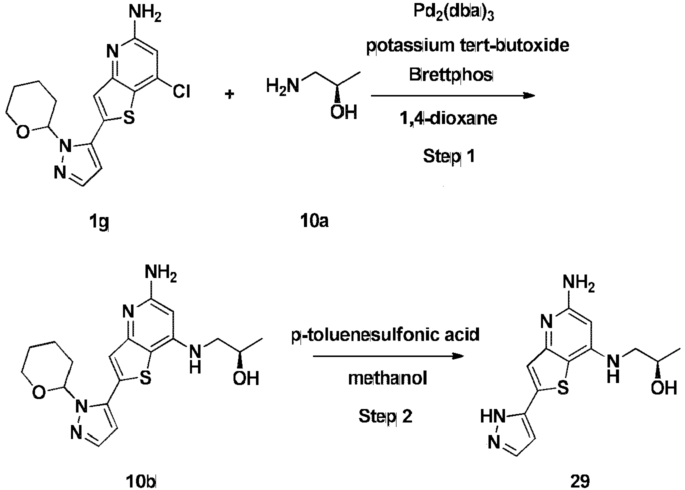

### Step 1: Synthesis of (2R)-1-((5-amino-2-(1-(tetrahydro-2H-pyran-2-yl)-1H-pyrazol-5-yl)thieno[3,2-b]pyridin-7-yl)amino)-2-propanol (Compound 10b)

Pd₂(dba)₃ (6.83 mg), BrettPhos (8.00 mg) and potassium tert-butoxide (50.27 mg) were added to a solution of Compound 10a (16.82 mg) and Compound 1g (50 mg) in 1,4-dioxane (2 mL) at room temperature. The reaction proceeded at an elevated temperature of 110°C for 5 hours under N₂ protection. The mother liquor from filtration was concentrated, and was separated and purified by preparative TLC (Eluent System A) to give Compound 10b (15 mg).

MS (ESI, m/z):374.2 [M+H]⁺.

### Step 2: Synthesis of (R)-1-((5-amino-2-(1H-pyrazol-5-yl)thieno[3,2-b]pyridin-7-yl)amino)-2-propanol (Compound 29)

p-Toluenesulfonic acid (8.29 mg) was added to a solution of Compound 10b (15 mg) in methanol (4 mL) at room temperature. The reaction proceeded at 25°C for 16 hours. Separation and purification by Prep-HPLC (Elution Condition 1) gave Compound 29 (4 mg).

MS (ESI, m/z): 290.1 [M+H]⁺.

¹H NMR (DMSO-*d₆*, 400 MHz) δ 12.96 (s, 1H), 7.79 (s, 1H), 7.28 (s, 1H), 6.70 (s, 1H), 5.93 (t, *J* = 5.2 Hz, 1H), 5.66 (s, 1H), 5.48 (s, 2H), 4.82 (d, *J=* 4.8 Hz, 1H), 3.95-3.84 (m, 1H), 3.07 (t, *J=* 5.8 Hz, 2H), 1.13 (d, *J=* 6.2 Hz, 3H).

### Example 11:

*N*⁷-(2-morpholinoethyl)-2-(1*H*-pyrazol-5-yl)thieno[3,2-b]pyridine-5,7-diamine (Compound 55)

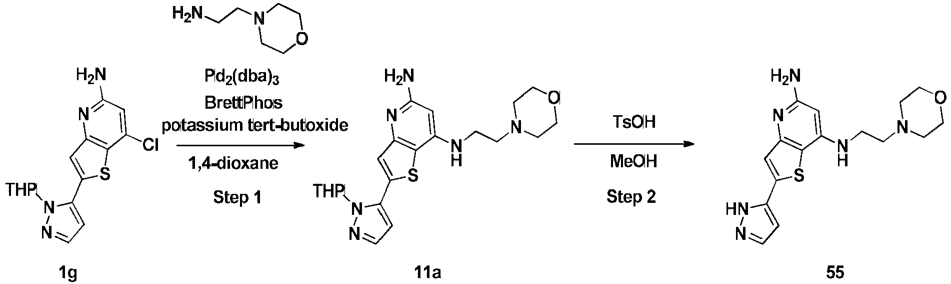

### Step 1: Synthesis of N⁷-(2-morpholinoethyl)-2-(1-(tetrahydro-2H-pyran-2-yl)-1H-pyrazol-5-yl)thieno[3,2-b]pyridine -5,7-diamine (Compound 11a)

Compound 1g (50 mg), 2-morpholinoethylamine (149.4 mg), Pd₂(dba)₃ (28.1 mg), BrettPhos (28.13 mg) and potassium tert-butoxide (77 mg) were added to 1,4-dioxane (2.5 mL), and the reaction proceeded at an elevated temperature of 120°C for 4 hours. The system was filtrated with Celite, and the filtrate was concentrated, then diluted with a small amount of methanol, and purified by preparative TLC (Eluent System A) to give Compound 11a (20 mg).

MS (ESI, m/z): 429.2 [M+H]⁺.

### Step 2: Synthesis of N⁷-(2-morpholinoethyl)-2-(1H-pyrazol-5-yl)thieno[3,2-b]pyridine-5,7-diamine (Compound 55)

Compound 11a (20 mg) was added to absolute methanol (2 mL), and p-toluenesulfonic acid monohydrate (18 mg) was added. The pH of the solution at this time was 5. The reaction proceeded at room temperature for 1 hour. Separation and purification by Prep-HPLC (Elution Condition 2) and lyophilization gave Compound 55 (8 mg).

MS (ESI, m/z): 345.1 [M+H]⁺.

¹H NMR (DMSO-*d₆*, 400 MHz) δ 12.95 (s, 1H), 7.80 (s, 1H), 7.28 (s, 1H), 6.71 (s, 1H), 5.95 (s, 1H), 5.66 (s, 1H), 5.50 (s, 2H), 3.60 (t, *J=* 3.6 Hz, 4H), 3.29-3.25 (m, 2H), 2.56 (t, *J=* 6.9 Hz, 2H), 2.45 (t, *J* = 4.0 Hz, 4H).

### Example 12:

((*S*)-1-((5-amino-2-(1*H*-pyrazol-5-yl)thieno[3,2-b]pyridin-7-yl)amino)-2-propanol (Compound 33)

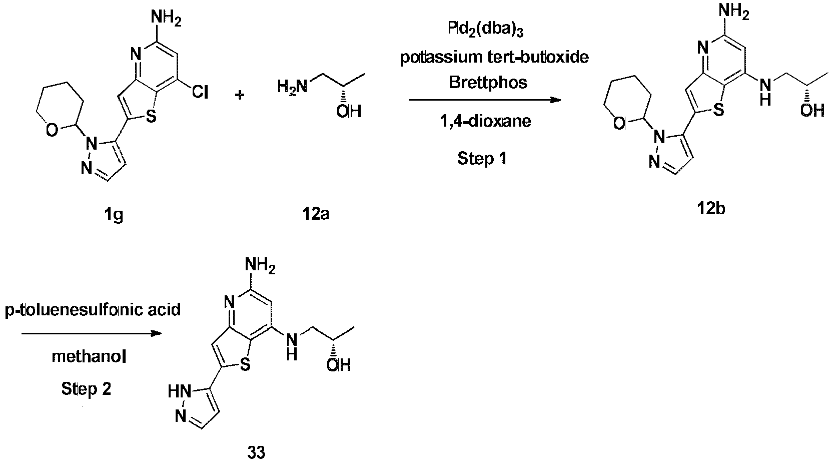

### Step 1: Synthesis of (2s)-1-((5-amino-2-(1-(tetrahydro-2H-pyran-2-yl)-1H-pyrazol-5-yl)thieno[3,2-b]pyridin-7-yl)amino)-2-propanol (Compound 12b)

Brettphos (8.00 mg), Pd₂(dba)₃ (6.83 mg) and potassium tert-butoxide (50.27 mg) were added to a solution of Compound 12a (16.82 mg) and Compound 1g (50 mg) in 1,4-dioxane (2 mL), and the reaction proceeded at an elevated temperature of 110°C for 5 hours. EA (10 mL) was added, and the system was filtered. The mother liquor was concentrated, and was separated and purified by preparative TLC (Eluent System A) to give Compound 12b (15 mg).

MS (ESI, m/z): 374.2 [M+H]⁺.

### Step 2: Synthesis of ((S)-1-((5-amino-2-(1H-pyrazol-5-yl)thieno[3,2-b]pyridin-7-yl)amino)-2-propanol (Compound 33)

p-Toluenesulfonic acid (8.29 mg) was added to a solution of Compound 12b (15 mg) in methanol (4 mL) at room temperature, and the reaction proceeded at 25°C for 16 hours. Separation and purification by Prep-HPLC (Elution Condition 2) gave Compound 33 (3 mg).

MS (ESI, m/z): 290.1 [M+H]⁺.

¹H NMR (DMSO-*d₆*, 400 MHz) δ 12.95 (s, 1H), 7.80 (s, 1H), 7.28 (s, 1H), 6.70 (s, 1H), 5.92 (s, 1H), 5.66 (s, 1H), 5.47 (s, 2H), 4.82 (d, *J* = 4.8 Hz, 1H), 3.90 (dt, *J* = 11.9, 6.0 Hz, 1H), 3.07 (t, *J=* 5.8 Hz, 2H), 1.13 (d, *J* = 6.2 Hz, 3H).

### Example 13:

7-((1*H*-pyrazol-5-yl)methoxy)-2-(1*H*-pyrazol-5-yl)thieno[3,2-b]pyridine-5-amine (Compound 56)

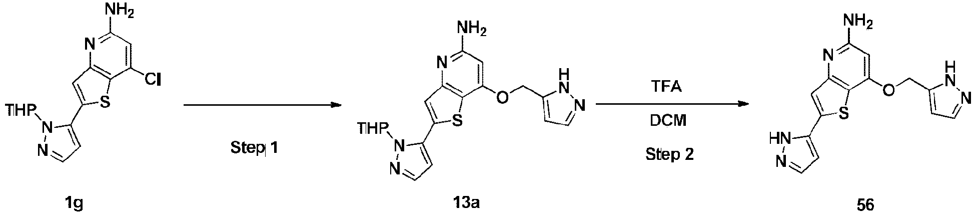

### Step 1: Synthesis of 7-((1H-pyrazol-5-yl)methoxy)-2-(1-(tetrahydro-2H-pyran-2-yl)-1H-pyrazol-5-yl)thieno[3,2-b]pyridine-5-amine (Compound 13a)

Compound 1g (20 mg) was added to NMP (2 mL), and then ((1*H*-pyrazol-5-yl)methanol (9 mg) and potassium tert-butoxide (20.62 mg) were added. The reaction proceeded at 100°C for 10 hours. The reaction was quenched by adding 10 mL water, and was extracted with EA. The organic layer was washed with saturated brine, dried and filtered. The filtrate was concentrated, and was separated and purified by preparative TLC (Eluent System A) to give Compound 13a (15 mg).

MS (ESI, m/z): 397.1 [M+H]⁺.

### Step 2: Synthesis of 7-((1H-pyrazol-5-yl)methoxy)-2-(1H-pyrazol-5-yl)thieno[3,2-b]pyridine-5-amine (Compound 56)

Compound 13a (15 mg) was added to DCM (2 mL) and TFA (1 mL), and the reaction proceeded at 25°C for 10 hours with stirring. The reaction liquid was concentrated under a reduced pressure. The residual was dissolved again with methanol, and was adjusted to pH 8 to 9 with a saturated sodium bicarbonate aqueous solution. The system was filtrated. The filtrate was separated and purified by Prep-HPLC (Elution Condition 3) to give Compound 56 (4 mg).

MS (ESI, m/z): 313.0 [M+H]⁺.

¹H NMR (DMSO-*d₆*, 400 MHz) δ 13.23 (s, 1H), 8.59 (s, 1H), 7.89 (s, 1H), 7.53 (s, 1H), 6.99 (s, 1H), 6.89 (s, 1H), 6.73 (s, 1H), 4.61 (s, 2H).

### Example 14:

*N*⁷-(2-(4-methylpiperazin-1-yl)ethyl)-2-(1*H*-pyrazol-5-yl)thieno[3,2-b]pyridine-5,7-diamine (Compound 57)

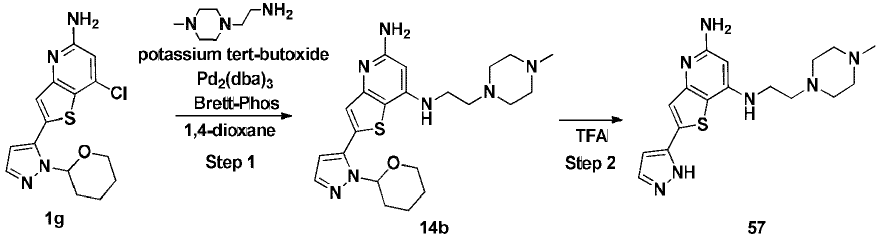

### Step 1: Synthesis of N⁷-(2-(4-methylpiperazin-1-yl)ethyl)-2-(1-(tetrahydro-2H-pyran-2-yl)-1H-pyrazol-5-yl)thieno[3,2-b]pyridine-5,7-diamine (Compound 14b)

Compound 1g (75 mg), 4-methylpiperazin-1-ylethylamine (64.35 mg), potassium tert-butoxide (125.67 mg), Pd₂(dba)₃ (40.99 mg) and BrettPhos (48.03 mg) were added to 1,4-dioxane (2 mL). The microwave reaction proceeded at 120°C for 2 hours under N₂ protection. The system was dried in a rotary dryer, and was purified by flash column chromatography (Eluent System A) to give Compound 14b (50 mg)

MS (ESI, m/z): 498.1 [M+H]⁺.

### Step 2: Synthesis of N⁷-(2-(4-methylpiperazin-1-yl)ethyl)-2-(1H-pyrazol-5-yl)thieno[3,2-b]pyridine-5,7-diamine (Compound 57)

Compound 14b (50 mg) was added to TFA (2 mL), and the reaction proceeded at an elevated temperature of 75°C for 5 hours with stirring. The reaction liquid was concentrated under a reduced pressure, separated and purified by Prep-HPLC (Elution Condition 2), and lyophilized to give Compound 57 (10 mg).

MS (ESI, m/z): 358.2 [M+H]⁺.

¹H NMR (DMSO-*d₆*, 400 MHz) δ 12.97 (s, 1H), 7.79 (s, 1H), 7.29 (s, 1H), 6.70 (s, 1H), 5.71 (t, *J* = 5.2 Hz, 5.6 Hz, 1H), 5.66 (s, 1H), 5.52 (s, 2H), 3.31-3.23 (q, 2H), 2.56 (t, *J =* 7.2 Hz, 6.8 Hz, 2H), 2.49-2.45 (m, 4H), 2.34-2.32 (m, 4H), 2.16 (s, 3H).

### Example 15:

3-(5-amino-2-(1*H*-pyrazol-5-yl)thieno[3,2-b]pyridin-7-yl)amino)propyl)cyclopropyl formamide (Compound 58)

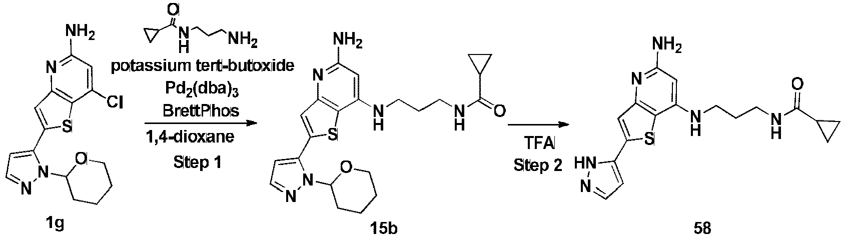

### Step 1: Synthesis of N-(3-((5-amino-2-(1-(tetrahydro-2H-pyran-2-yl)-1H-pyrazol-5-yl)thieno[3,2-b]pyridin-7-yl)amino)propyl)cyclopropyl formamide (Compound 15b)

Compound 1g (75 mg), *N*-(3-aminopropyl)cyclopropyl formamide (63.92 mg), potassium tert-butoxide (125.67 mg), Pd₂(dba)₃ (40.99 mg) and BrettPhos (48.03 mg) were added to 1,4-dioxane (2 mL), and the microwave reaction proceeded at 120°C for 2 hours under N₂ protection. The system was dried in a rotary dryer, and was purified by flash column chromatography (Eluent System A) to give Compound 15b (30 mg).

MS (ESI, m/z): 497.1 [M+H]⁺.

### Step 2: Synthesis of N-(3-(5-amino-2-(1H-pyrazol-5-yl)thieno[3,2-b]pyridin-7-yl)amino)propyl)cyclopropyl formamide (Compound 58)

Compound 15b (30 mg) was added to TFA (2 mL), and the reaction proceeded at 75°C for 5 hours. The reaction liquid was concentrated under a reduced pressure, separated and purified by Prep-HPLC (Elution Condition 2), and lyophilized to give Compound 58 (4 mg).

MS (ESI, m/z): 357.1 [M+H]⁺.

¹H NMR (DMSO-*d₆*, 400 MHz) δ 12.99 (s, 1H), 8.10 (s, 1H), 7.81 (s, 1H), 7.29 (s, 1H), 6.72 (s, 1H), 6.29 (s, 1H), 5.65 (s, 3H), 3.31-3.17 (m, 4H), 1.75-1.53 (m, 3H), 0.65 (m, 4H).

### Example 16:

2-(1*H*-pyrazol-5-yl)-*N*⁷-(pyridin-2-ylmethyl)thieno[3,2-b]pyridine-5,7-diamine (Compound 59)

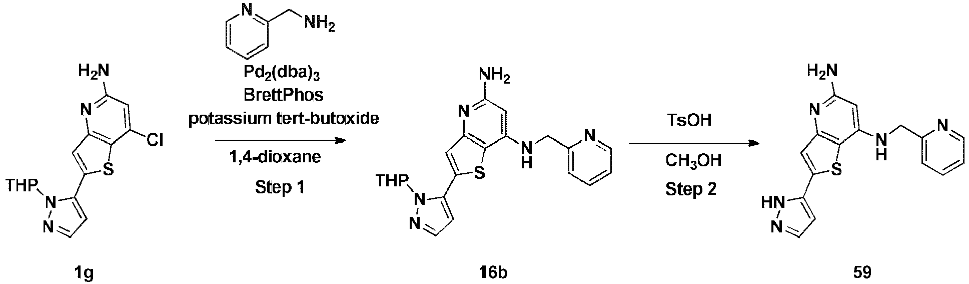

### Step 1: Synthesis of 2-(1-(tetrahydro-2H-pyran-2-yl)-1H-pyrazol-5-yl)-N⁷-(pyridin-2-ylmethyl)thieno[3,2-b]pyridine-5,7-diamine (Compound 16b)

Compound 1g (60 mg), 2-aminomethylpyridine (38.76 mg), Pd₂(dba)₃ (32.79 mg), BrettPhos (38.42 mg) and potassium tert-butoxide (100.54 mg) were added to 1,4-dioxane (2 mL), and the microwave reaction was performed at 120°C for 5 hours. The system was filtrated with Celite, and the filtrate was concentrated, diluted with a small amount of methanol, and purified by preparative TLC (Eluent System A) to give Compound 16b (29 mg).

MS (ESI, m/z): 407.2 [M+H]⁺.

### Step 2: Synthesis of 2-(1H-pyrazol-5-yl)-N⁷-(pyridin-2-ylmethyl)thieno[3,2-b]pyridine-5,7-diamine (Compound 59)

Compound 16b (29 mg) was added to methanol (3 mL), and p-toluenesulfonic acid (24.57 mg) was added. The reaction proceeded at room temperature for 2 hours. The system was dried in a rotary dryer, separated and purified by Prep-HPLC (Elution Condition 2), and lyophilized to give Compound 59 (5 mg).

MS (ESI, m/z): 323.1 [M+H]⁺.

¹H NMR (DMSO-*d₆*, 400 MHz) δ 12.97 (s, 1H), 8.55 (dd, *J* = 4.8, 0.8 Hz, 1H), 7.79 (br, 1H), 7.75 (td, *J* = 7.7, 1.8 Hz, 1H), 7.33 (d, *J* = 4.8 Hz, 1H), 7.30 (s, 1H), 7.28-7.25 (m, 1H), 6.91 (t, *J* = 5.9 Hz, 1H), 6.72 (s, 1H), 5.50 (s, 1H), 5.46 (s, 2H), 4.48 (d, *J* = 6.0 Hz, 2H).

### Example 17:

*N*⁷-(1-methyl-1*H*-pyrazol-3-yl)methyl-2-(1*H*-pyrazol-5-yl)thieno[3,2-b]pyridine-5,7-diamine (Compound 60)

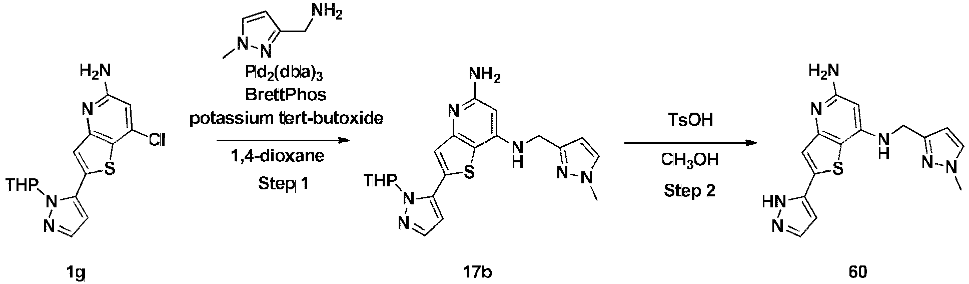

### Step 1: Synthesis of N⁷-((1-methyl-1H-pyrazol-3-yl)methyl)-2-(1-(tetrahydro-2H-pyran-2-yl)-1H-pyrazol-5-yl)thieno[3,2-b]pyridine-5,7-diamine (Compound 17b)

Compound 1g (60 mg), (1-methyl-1*H*-pyrazol-3-yl)methylamine (39.83 mg), Pd₂(dba)₃ (32.79 mg), BrettPhos (38.42 mg) and potassium tert-butoxide (100.54 mg) were added to 1,4-dioxane (5 mL), and the microwave reaction was performed at 120°C for 3.5 hours. The system was filtrated with Celite, and the filtrate was concentrated, diluted with a small amount of methanol, and purified by preparative TLC (Eluent System A) to give Compound 17b (13 mg).

MS (ESI, m/z): 410.1[M+H]⁺.

### Step 2: Synthesis of N⁷-((1-methyl-1H-pyrazol-3-yl)methyl)-2-(1H-pyrazol-5-yl)thieno[3,2-b]pyridine-5,7-diamine (Compound 60)

Compound 17b (13 mg) was added to methanol (2 mL), and p-toluenesulfonic acid (10.93 mg) was added. The reaction proceeded at room temperature for 2 hours. The system was dried in a rotary dryer, separated and purified by Prep-HPLC (Elution Condition 2), and lyophilized to give Compound 60 (3 mg).

MS (ESI, m/z): 326.1 [M+H]⁺.

¹H NMR (DMSO-*d₆*, 400 MHz) δ 12.95 (s, 1H), 7.80 (s, 1H), 7.57 (d, *J=* 2.1 Hz, 1H), 7.27 (s, 1H), 6.70 (s, 1H), 6.62 (t, *J* = 2.0 Hz, 1H), 6.13 (d, *J* = 2.1 Hz, 1H), 5.68 (s, 1H), 5.49 (s, 2H), 4.30 (d, *J* = 5.9 Hz, 2H), 3.79 (s, 3H).

### Example 18:

(1-(5-amino-2-(1*H*-pyrazol-5-yl)thieno[3,2-b]pyridin-7-yl)amino)-2-methyl-2-propanol (Compound 28)

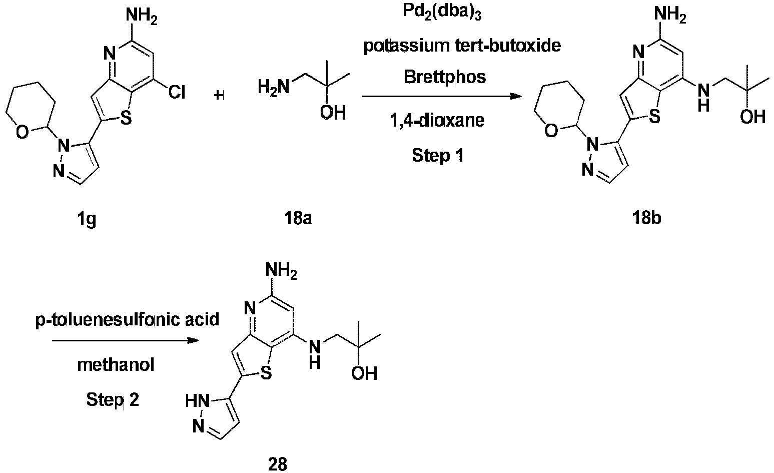

### Step 1: Synthesis of 1-((5-amino-2-(1-(tetrahydro-2H-pyran-2-yl)-1H-pyrazol-5-yl)thieno[3,2-b]pyridin-7-yl)amino)-2-methyl-2-propanol (Compound 18b)

Brettphos (8.00 mg), Pd₂(dba)₃ (6.83 mg) and potassium tert-butoxide (50.27 mg) were added to a solution of Compound 18a (16.82 mg) and Compound 1g (50 mg) in 1,4-dioxane (2 mL), and the reaction proceeded at an elevated temperature of 110°C for 5 hours. EA (10 mL) was added, and the mother liquor from filtration was concentrated, and was separated and purified by preparative TLC (Eluent System A) to give Compound 18b (15 mg).

MS (ESI, m/z): 387.50 [M+H]⁺.

### Step 2: Synthesis of (1-(5-amino-2-(1H-pyrazol-5-yl)thieno[3,2-b]pyridin-7-yl)amino)-2-methyl-2-propanol (Compound 28)

p-Toluenesulfonic acid (8.29 mg) was added to a solution of Compound 18b (15 mg) in methanol (4 mL) at room temperature, and the reaction proceeded at 25°C for 16 hours. Separation and purification by Prep-HPLC (Elution Condition 2) gave Compound 28 (3 mg).

MS (ESI, m/z): 303.38 [M+H]⁺.

¹H NMR (CD₃OD, 400 MHz) δ 7.71 (d, *J* = 1.8 Hz, 1H), 7.34 (s, 1H), 6.69 (d, *J* = 2.3 Hz, 1H), 5.89 (s, 1H), 4.66 (s, 2H), 3.28 (s, 2H), 1.30 (s, 6H).

### Example 19:

3-((5-amino-2-(1*H*-pyrazol-1-yl)thieno[3,2-b]pyridin-7-yl)amino)-1-propanol (Compound 32)

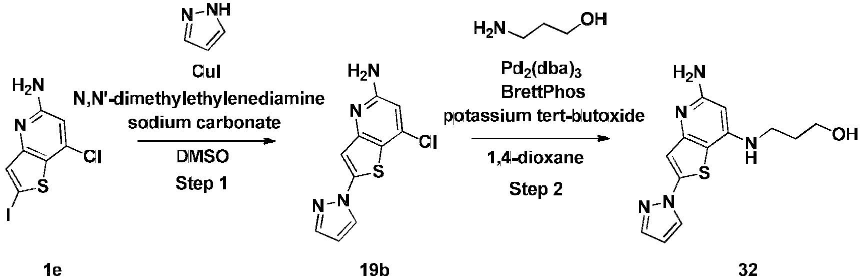

### Step 1: Synthesis of 7-chloro-2-(1H-pyrazol-1-yl)thieno[3,2-b]pyridine-5-amine (Compound 19b)

Compound 1e (150 mg), pyrazole (164.4 mg), sodium carbonate (234 mg) and cuprous iodide (184 mg) were added to a 25 mL three-necked flask, and nitrogen replacement was conducted. To the system was then sequentially added DMSO (9 mL) and *N*,*N*′-dimethylethylenediamine (128 mg), and nitrogen replacement was conducted. The reaction proceeded at an elevated temperature of 120°C for 4 hours. The system was filtered, washed with methanol, and the filtrate was concentrated and diluted with water. The solution was extracted with EA for three times, dried over anhydrous sodium sulfate, and purified by preparative TLC (Eluent System A) to give Compound 19b (49 mg).

MS (ESI, m/z): 251.0 [M+H]⁺.

### Step 2: Synthesis of 3-((5-amino-2-(1H-pyrazol-1-yl)thieno[3,2-b]pyridin-7-yl)amino)-1-propanol (Compound 32)

Compound 19b (28 mg), 3-amino-1-propanol (64.5 mg), Pd₂(dba)₃ (21 mg), BrettPhos (21 mg) and potassium tert-butoxide (58 mg) were added to 1,4-dioxane (2.5 mL), and the reaction proceeded at 120°C for 4 hours. The system was filtrated with Celite, washed with methanol, separated and purified by Prep-HPLC (Elution Condition 2), and lyophilized to give Compound 32 (1 mg).

MS (ESI, m/z): 290.1 [M+H]⁺.

¹H NMR (DMSO-*d₆*, 400 MHz) 8 8.55 (d, *J* = 2.4 Hz, 1H), 7.73 (d, *J* = 1.5 Hz, 1H), 7.25 (s, 1H), 6.58 (t, *J* = 2.4 Hz, 1H), 6.20 (t, *J* = 5.3 Hz, 1H), 5.65 (s, 1H), 5.52 (s, 2H), 4.55 (t, *J* = 5.2 Hz, 1H), 3.55-3.50 (m, 2H), 3.23-3.17 (m, 2H), 1.80-1.73 (m, 2H).

### Example 20:

2-(1*H*-pyrazol-5-yl)-*N*⁷-(tetrahydrofuran-3-yl)thieno[3,2-b]pyridine-5,7-diamine (Compound 61)

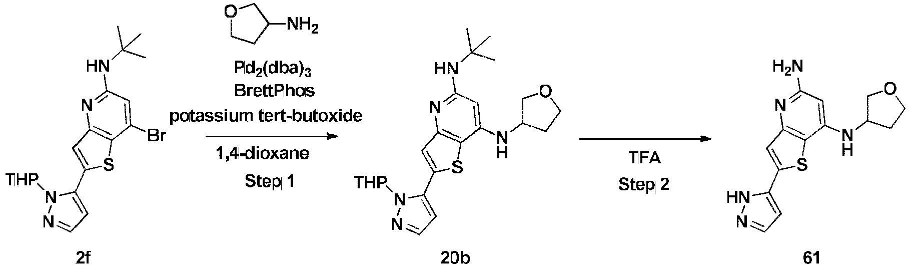

### Step 1: Synthesis of N⁵-(tert-butyl)-2-(1-(tetrahydro-2H-pyran-2-yl)-1H-pyrazol-5-yl)-N⁷-(tetrahydrofuran-3-yl)thieno[3,2-b)pyridine-5,7-diamine (Compound 20b)

Compound 2f (100 mg), tetrahydrofuran-3-amine (100 mg), Pd₂(dba)₃ (42 mg), BrettPhos (43 mg) and potassium tert-butoxide (129 mg) were added to 1,4-dioxane (2.5 mL), and the microwave reaction proceeded at 120°C for 5 hours. The system was filtrated with Celite, and the filtrate was concentrated, diluted with a small amount of methanol, and purified by preparative TLC (Eluent System A) to give Compound 20b (100 mg).

MS (ESI, m/z): 442.2 [M+H]⁺.

### Step 2: Synthesis of 2-(1H-pyrazol-5-yl)-N⁷-(tetrahydrofuran-3-yl)thieno[3,2-b]pyridine-5,7-diamine (Compound 61)

Compound 20b (100 mg) was added to TFA (3 mL), and the reaction proceeded at 70°C for 2 hours. The reaction liquid was concentrated under a reduced pressure, and methanol (5 ml) was added. The system was adjusted to pH = 8 with potassium carbonate and filtered under a reduced pressure. The filtrate was separated and purified by Prep-HPLC (Elution Condition 2), and lyophilized to give Compound 61 (28 mg).

MS (ESI, m/z): 302.1 [M+H]⁺.

¹H NMR (DMSO-*d₆*, 400 MHz) δ 12.96 (s, 1H), 7.80 (s, 1H), 7.28 (s, 1H), 6.71 (s, 1H), 6.26 (d, *J* = 5.2 Hz,1H), 5.65 (s, 1H), 5.52 (s, 2H), 4.10-4.04 (m, 1H), 3.95-3.90 (m, 1H), 3.90-3.83 (m, 1H), 3.77-3.71 (m, 1H), 3.69-3.66 (m, 1H), 2.26-2.17 (m, 1H), 2.04-1.92 (m, 1H).

### Example 21:

(1*S*,3*S*)-3-((5-atnino-2-(1*H*-pyrazol-5-yl)thieno[3,2-b]pyridin-7-yl)amino)cyclopentanol (Compound 26)

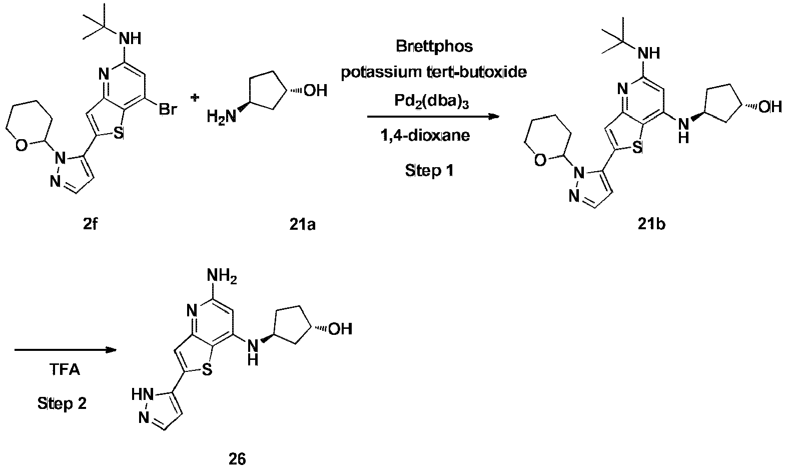

### Step 1: Synthesis of (1S,3S)-3-((5-(tert-butylamino)-2-(1-(tetrahydro-2H-pyran-2-yl)-1H-pyrazol-5-yl)thieno[3,2-b]pyridin-7-yl)amino)cyclopentanol (Compound 21b)

Pd₂(dba)₃ (8.20 mg), BrettPhos (9.61 mg) and potassium tert-butoxide (80.43 mg) were added to a solution of Compound 21a (36.25 mg) and Compound 2f (78.02 mg) in 1,4-dioxane (2 mL) at room temperature. The reaction proceeded at an elevated temperature of 110°C for 5 hours under N₂ protection. After dilution with EA, the system was filtered, and the filtrate was concentrated. Separation and purification by preparative TLC (Eluent System A) gave Compound 21b (20 mg).

MS (ESI, m/z): 456.2[M+H]⁺.

### Step 2: Synthesis of (1S,3S)-3-((5-amino-2-(1H-pyrazol-5-yl)thieno[3,2-b]pyridin-7-yl)amino)cyclopentanol (Compound 26)

Compound 21b (20 mg) was added to TFA (2 mL), and the reaction proceeded at an elevated temperature of 70°C for 2 hours. The reaction liquid was concentrated under a reduced pressure, and methanol (5 mL) and water (1 mL) were added to the residue. Sodium hydroxide was added to adjust to pH = 9. The reaction was stirred for 1 hour, concentrated, separated and purified by Prep-HPLC (Elution Condition 2) to give Compound 26 (4 mg).

MS (ESI, m/z): 316.2[M+H]⁺.

¹H NMR (DMSO-*d₆*, 400 MHz) δ 12.95 (s, 1H),7.80 (s, 1H), 7.26 (s, 1H), 6.70 (s, 1H), 5.99 (d, *J* = 6.0 Hz, 1H), 5.66 (s, 1H), 5.49 (s, 2H), 4.55 (d, *J* = 3.5 Hz, 1H), 4.24 (d, *J* = 3.1 Hz, 1H), 3.99 (dt, *J* = 13.9, 7.0 Hz, 1H), 2.20-2.11 (m, 1H), 1.98-1.87 (m, 2H), 1.80-1.72 (m, 1H), 1.57-1.47 (m, 2H).

### Example 22:

(*N*⁷-(2,6-dimethylmorpholinoethyl)-2-(1*H*-pyrazol-5-yl)thieno[3,2-b]pyridine-5,7-diamine (Compound 62)

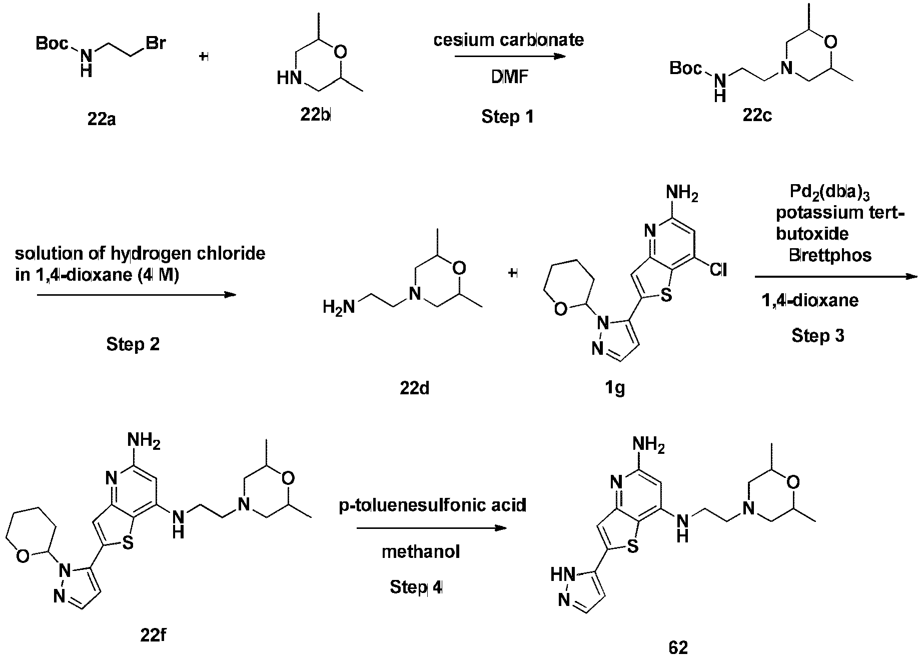

### Step 1: Synthesis of tert-butyl (2-(2,6-dimethylmorpholino)ethyl)carbamate (Compound 22c)

Cesium carbonate (5.64 g) was added to a solution of Compound 22b (1.0 g) and Compound 22a (2.33 g) in DMF (4 mL) at room temperature, and the reaction proceeded at 25°C for 16 hours. The reaction was quenched by adding water, and was extracted with EA. The EA layer was dried and concentrated to give Compound 22c (1.1 g).

MS (ESI, m/z): 259.3[M+H]⁺.

### Step 2: Synthesis of 2-(2,6-dimethylmorpholino)ethylamine (Compound 22d)

Compound 22c (1.1 g) was added to a solution of hydrogen chloride in 1,4-dioxane (4 M, 10 mL), and the reaction proceeded at room temperature for 5 hours. The reaction liquid was concentrated under a reduced pressure, and ethanol (5 mL) and water (1 mL) were added to the residue. Sodium hydroxide was added to adjust to pH = 8. The reaction liquid was concentrated, and DCM/MeOH (5 mL/1 mL) was added. The inorganic salts were removed by filtration. The mother liquor was concentrated to give Compound 22d (320 mg).

MS (ESI, m/z): 159.2[M+H]⁺.

### Step 3: Synthesis of N⁷-(2-(2,6-dimethylmorpholino)ethyl)-2-(1-(tetrahydro-2H-pyran-2-yl)-1H-pyrazol-5-yl)thieno[3,2-b]pyridine-5,7-diamine (Compound 22f)

Pd₂(dba)₃ (8.20 mg), BrettPhos (9.61 mg) and potassium tert-butoxide (60.32 mg) were added to a solution of

Compound 1g (60 mg) and Compound 22d (28.36 mg) in 1,4-dioxane (2 mL) at room temperature. The reaction proceeded at an elevated temperature of 110°C for 5 hours under N₂ protection. The reaction liquid was filtered, and the mother liquor was concentrated. Separation and purification by preparative TLC (Eluent System A) gave Compound 22f (15 mg).

MS (ESI, m/z): 475.2 [M+H]⁺.

### Step 4: Synthesis of N⁷-(2-(2,6-dimethylmorpholino)ethyl)-2-(1H-pyrazol-5-yl)thieno[3,2-b]pyridine-5,7-diamine (Compound 62)

p-Toluenesulfonic acid (6.89 mg) was added to a solution of Compound 22f (20 mg) in methanol (4 mL) at room temperature, and the reaction proceeded at 25°C for 16 hours. Separation and purification by Prep-HPLC (Elution Condition 2) gave Compound 62 (4 mg).

MS (ESI, m/z): 373.2.1 [M+H]⁺.

¹H NMR (DMSO-*d₆*, 400 MHz) δ 12.96 (s, 1H), 7.79 (s, 1H), 7.29 (s, 1H), 6.71 (s, 1H), 5.79 (s, 1H), 5.66 (s, 1H), 5.53 (s, 2H), 3.96-3.87 (m, 2H), 3.27-3.22 (m, 2H), 2.56 (dd, *J* = 12.4, 6.7 Hz, 2H), 2.47 (d, *J* = 2.7 Hz, 2H), 2.17 (dd, *J* = 10.9, 5.7 Hz, 2H), 1.15 (d, *J* = 6.4 Hz, 6H).

### Example 23:

*N*⁷-(1-methyl-1*H*-imidazol-4-yl)methyl-2-(1*H*-pyrazol-5-yl)thieno[3,2-b]pyridine-5,7-diamine (Compound 63)

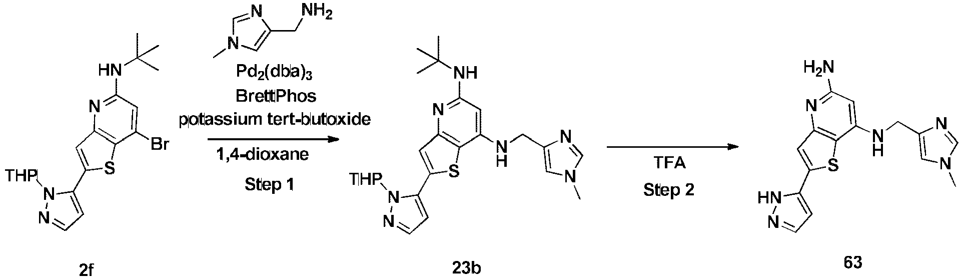

### Step 1: Synthesis of N⁵-tert-butyl-N⁷-((1-methyl-1H-imidazol-4-yl)methyl)-2-(1-(tetrahydro-2H-pyran-2-yl)-1H-pyrazol-5-yl)thieno[3,2-b]pyridine-5,7-diamine (Compound 23b)

Compound 2f (70 mg), (1-methyl-1*H*-imidazol-4-yl)methylamine (53.61 mg), Pd₂(dba)₃ (29.42 mg), BrettPhos (34.47 mg) and potassium tert-butoxide (90.21 mg) were added to 1,4-dioxane (3 mL), and the microwave reaction was performed at 120°C for 2.5 hours. The system was filtrated with Celite, and the filtrate was concentrated, then diluted with a small amount of methanol, and purified by preparative TLC (Eluent System A) to give Compound 23b (55 mg).

MS (ESI, m/z): 466.2[M+H]⁺.

### Step 2: Synthesis of N⁷-((1-methyl-1H-midazol-4-yl)methyl)-2-(1H-pyrazol-5-yl)thieno[3,2-b]pyridine-5,7-diamine (Compound 63)

Compound 23b (55 mg) was added to TFA (5 mL), and the reaction proceeded at 70°C for 2 hours. The system was dried in a rotary dryer to give a crude product, which was dissolved in 10 mL methanol again. The system was adjusted to pH 8-9 by the addition of a saturated Na₂CO₃ aqueous solution dropwise. Separation and purification by Prep-HPLC (Elution Condition 2) and lyophilization gave Compound 63 (22 mg).

MS (ESI, m/z): 326.1 [M+H]⁺.

¹H NMR (DMSO-*d₆*, 400 MHz) δ 12.96 (s, 1H), 7.78 (s, 1H), 7.50 (d, *J* = 0.9 Hz, 1H), 7.28 (s, 1H), 6.93 (s, 1H), 6.69 (d, *J=* 2.0 Hz, 1H), 6.50 (t, J= 5.7 Hz, 1H), 5.68 (s, 1H), 5.46 (s, 2H), 4.23 (d, *J* = 5.7 Hz, 2H), 3.59 (s, 3H).

### Example 24:

*N-*(2-(5-amino-2-(1*H*-pyrazol-5-yl)thieno[3,2-b]pyridin-7-yl)amino)ethyl)cyclopropyl carboxamide (Compound 64)

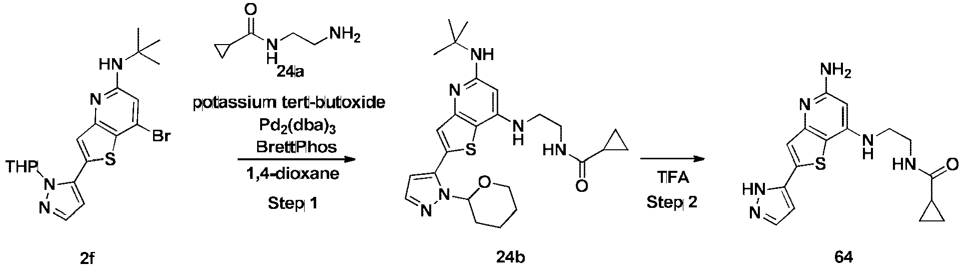

### Step 1: Synthesis of N-(2-((5-(tert-butylamino)-2-(1-(tetrahydro-2H-pyran-2-yl)-1H-pyrazol-5-yl)thieno[3,2-b]pyridin-7-yl)amino)ethyl)cyclopropyl formamide (Compound 24b)

Compound 2f (75 mg), Compound 24a (63.92 mg), potassium tert-butoxide (125.67 mg), Pd₂(dba)₃ (40.99 mg) and BrettPhos (48.03 mg) were added to 1,4-dioxane (2 mL), and the microwave reaction proceeded at 120°C for 2 hours under N₂ protection. The system was dried in a rotary dryer, and was purified by flash column chromatography (Eluent System A) to give Compound 24b (30 mg).

MS (ESI, m/z): 482.6 [M+H]⁺.

### Step 2: Synthesis of N-(2-(5-amino-2-(1H-pyrazol-5-yl)thieno[3,2-b]pyridin-7-yl)amino)ethyl)cyclopropyl carboxamide (Compound 64)

Compound 24b (30 mg) was added to TFA (2 mL), and the reaction proceeded at 75°C for 5 hours. The reaction liquid was concentrated under a reduced pressure, separated and purified by Prep-HPLC (Elution Condition 2), and lyophilized to give Compound 64 (4 mg).

MS (ESI, m/z): 342.4 [M+H]⁺.

¹H NMR (DMSO-*d₆*, 400 MHz) δ 12.95 (s, 1H), 8.28 (t, *J* = 5.5 Hz, 1H), 7.78 (s, 1H), 7.28 (s, 1H), 6.70 (d, *J* = 2.0 Hz, 1H), 6.15 (t, *J* = 5.1 Hz, 1H), 5.65 (s, 1H), 5.47 (s, 2H), 3.21 (dd, *J* = 11.7, 6.1 Hz, 4H), 1.60-1.49 (m, 1H), 0.75-0.70 (m, 2H), 0.69-0.62 (m, 2H).

### Example 25:

*N*-(2-((5-amino-2-(1*H*-pyrazol-5-yl)thieno[3,2-b]pyridin-7-yl)amino)ethyl)-*N*-methylpropionainide (Compound 88)

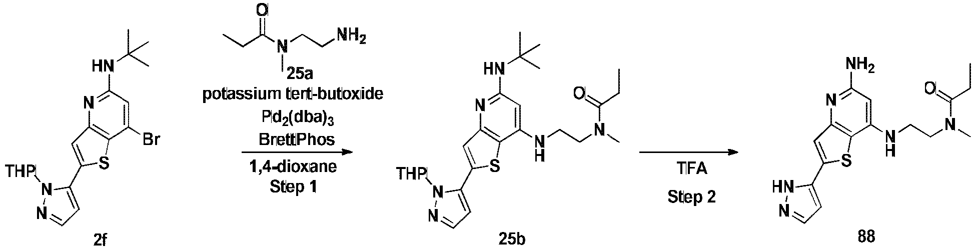

### Step 1: Synthesis of N-(2-((5-(tert-butylamino)-2-(1-(tetrahydro-2H-pyran-2-yl)-1H-pyrazol-5-yl)thieno[3,2-b]pyridin-7-yl)amino)ethyl)-N-methylpropionamide (Compound 25b)

Compound 25a (17.94 mg), Compound 2f (50 mg), Brettphos (24.66 mg), Pd₂(dba)₃ (21.03 mg) and potassium tert-butoxide (64.43 mg) were added to 1,4-dioxane (7 mL). The reaction proceeded at an elevated temperature of 110°C for 10 hours with stirring under N₂ protection. The system was dried in a rotary dryer, and was purified by flash column chromatography (Eluent System A) to give Compound 25b (50 mg).

MS (ESI, m/z): 485.3 [M+H]⁺.

### Step 2: Synthesis of N-(2-((5-amino-2-(1H-pyrazol-5-yl)thieno[3,2-b]pyridin-7-yl)amino)ethyl)-N-methylpropionamide (Compound 88)

Compound 25b (50 mg) was added to DCM (2 mL) and TFA (2 mL), and the reaction proceeded at 25°C for 10 hours with stirring. The reaction liquid was concentrated under a reduced pressure, separated and purified by Prep-HPLC (Elution Condition 2), and lyophilized to give Compound 88 (12 mg).

MS (ESI, m/z): 345.2 [M+H]⁺.

¹H NMR (DMSO-*d₆*, 400 MHz) δ 12.77 (s, 1H), 7.78-7.79 (m, 1H), 7.28-7.29 (m, 1H), 6.69-6.70 (m, 1H), 6.32 (t, *J* = 6.0 Hz, 0.4H), 6.17 (t, *J* = 5.2 Hz, 0.6H), 5.67-5.71 (m, 1H), 5.50-5.51 (m, 2H), 3.48-3.54 (m, 2H), 3.26-3.29 (m, 2H), 3.00 (s, 1.8 H), 2.87 (s, 1.2 H), 2.23-2.33 (m, 2H), 1.00 (t, *J* = 7.6 Hz, 1.8H), 0.91 (t, *J* = 7.6 Hz, 1.2H).

**Example** 26:

*N*⁷-(1-methylpiperidin-4-yl)-2-(1*H*-pyrazol-5-yl)thieno[3,2-b]pyridine-5,7-diamine (Compound 89)

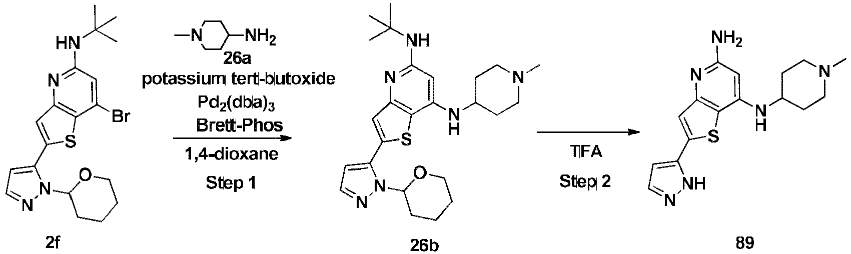

### Step 1: Synthesis of N⁵-(tert-butyl)-N⁷-(1-methylpiperidin-4-yl)-2-(1-(tetrahydro-2H-pyran-2-yl)-1H-pyrazol-5-yl)thieno[3,2-b]pyridine-5,7-diamine (Compound 26b)

Compound 2f (50 mg), Compound 26a (15.74 mg), Brettphos (64.43 mg), Pd₂(dba)₃ (21.02 mg) and Brett-Phos (24.62 mg) were added to 1,4-dioxane (2 mL). The reaction proceeded at an elevated temperature of 120°C for 2 hours under N₂ protection. The system was dried in a rotary dryer, and was purified by flash column chromatography (Eluent System A) to give Compound 26b (30 mg).

MS (ESI, m/z): 469.1 [M+H]⁺.

### Step 2: Synthesis of N⁷-(1-methylpiperidin-4-yl)-2-(1H-pyrazol-5-yl)thieno[3,2-b]pyridine-5,7-diamine (Compound 89)

Compound 26b (30 mg) was added to TFA (2 mL), and the reaction proceeded at an elevated temperature of 75°C for 2 hours with stirring. The reaction liquid was concentrated under a reduced pressure, separated and purified by Prep-HPLC (Elution Condition 3), and lyophilized to give Compound 89 (5 mg).

MS (ESI, m/z): 329.2 [M+H]⁺.

¹H NMR (DMSO-*d₆*, 400 MHz) δ 13.29 (s, 1H), 7.92-7.77 (m, 2H), 7.55-7.43 (m, 3H), 6.87 (s, 1H), 5.93 (s, 1H), 3.72-3.49 (m, 2H), 3.37-3.07 (m, 3H), 2.78 (s, 3H), 2.13-1.89 (m, 4H).

### Example 27:

3-((5-amino-2-(pyridin-4-yl)thieno[3,2-b]pyridin-7-yl)amino)-1-propanol (Compound 90)

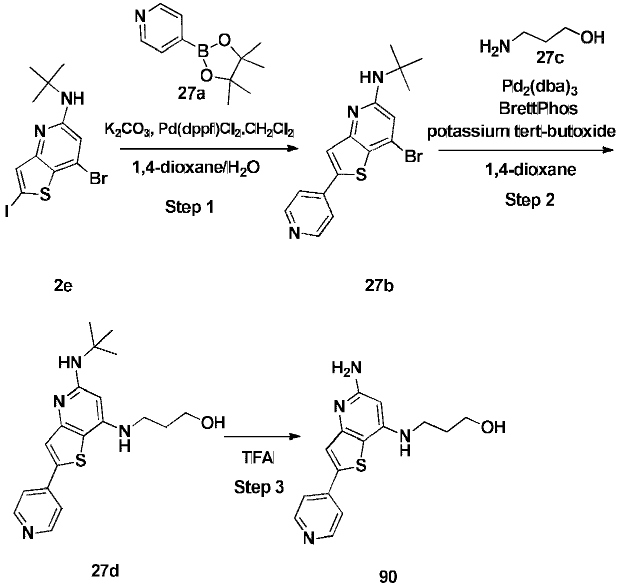

### Step 1: Synthesis of 7-bromo-N-(tert-butyl)-2-(pyridin-4-yl)thieno[3,2-b]pyridine-5-amine (Compound 27b)

Compound 2e (100 mg), Compound 27a (60 mg), Pd(dppf)Cl₂•CH₂Cl₂ (19.85 mg), and K₂CO₃ (67.14 mg) were added to 1,4-dioxane (5 mL) and water (0.5 mL). The reaction proceeded at an elevated temperature of 50°C for 4 hours under N₂ protection. The reaction liquid was filtered, and the filtrate was concentrated. Purification by preparative TLC (Eluent System A) gave Compound 27b (32 mg).

MS (ESI, m/z): 362.0 [M+H]⁺.

### Step 2: Synthesis of 3-((5-(tert-butylamino)-2-(pyridin-4-yl)thieno[3,2-b]pyridin-7-yl)amino)-1-propanol (Compound 27d)

Compound 27b (32 mg), Compound 27c (51 mg), Pd₂(dba)₃ (16.6 mg), BrettPhos (16.6 mg) and potassium tert-butoxide (45.6 mg) were added to 1,4-dioxane (3 mL). The reaction proceeded at an elevated temperature of 120°C for 4 hours under N₂ protection. The reaction liquid was filtered, and the filtrate was concentrated, and purified by preparative TLC (Eluent System A) to give Compound 27d (27 mg).

MS (ESI, m/z): 357.2 [M+H]⁺.

### Step 3: Synthesis of 3-((5-amino-2-(pyridin-4-yl)thieno[3,2-b]pyridin-7-yl)amino)-1-propanol (Compound 90)

Compound 27d (27 mg) was added to TFA (5 mL), and the reaction proceeded at an elevated temperature of 70°C for 2 hours. The reaction liquid was concentrated under a reduced pressure, separated and purified by Prep-HPLC (Elution Condition 2), and lyophilized to give Compound 90 (3 mg).

MS (ESI, m/z): 301.1 [M+H]⁺.

¹H NMR (DMSO-*d₆*, 400 MHz) δ 8.63 (d, *J* = 6.0 Hz, 2H), 7.74 (s, 1H), 7.71 (dd, *J* = 4.7, 1.4 Hz, 2H), 6.34 (t, *J* = 5.3 Hz, 1H), 5.72 (s, 1H), 5.65 (s, 2H), 4.60 (s, 1H), 3.54 (t, *J* = 6.0 Hz, 2H), 3.24 (dd, *J* = 12.6, 6.7 Hz, 2H), 1.84-1.74 (m, 2H).

### Example 28:

3-((5-amino-2-(6-methylpyridin-2-yl)thieno[3,2-b]pyridin-7-yl)amino)-1-propanol (Compound 91)

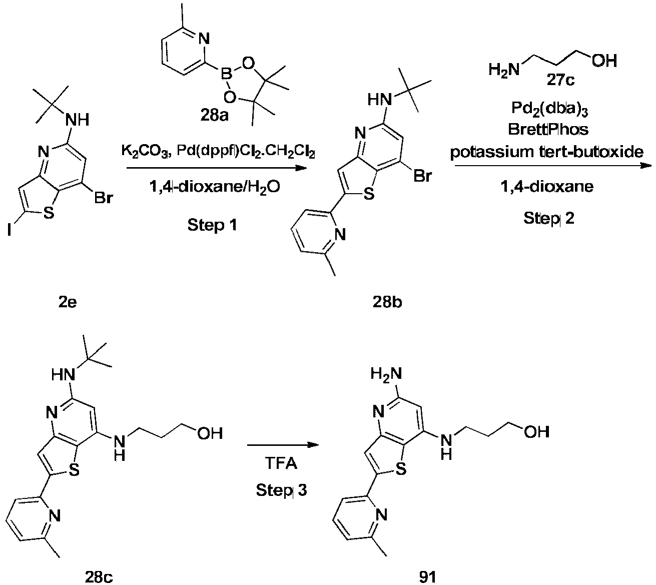

### Step 1: Synthesis of 7-bromo-N-(tert-butyl)-2-(6-methylpyridin-2-yl)thieno[3,2-b]pyridine-5-amine (Compound 28b)

Compound 2e (150 mg), Compound 28a (59.96 mg), potassium carbonate (151.28 mg) and Pd(dppf)Cl₂•CH₂Cl₂ (267.09 mg) were added to water (1 mL) and 1,4-dioxane (5 mL). The reaction proceeded at an elevated temperature of 80°C for 10 hours with stirring under N₂ protection. The reaction liquid was filtered, and the filtrate was concentrated. Purification by preparative TLC (Eluent System A) gave Compound 28b (25 mg).

MS (ESI, m/z): 378.0 [M+H]⁺.

### Step 2: Synthesis of 3-((5-(tert-butylamino)-2-(6-methylpyridin-2-yl)thieno[3,2-b]pyridin-7-yl)amino)-1-propanol (Compound 28c)

Compound 27c (23 mg), Compound 28b (50 mg), Brettphos (42.79 mg), Pd₂(dba)₃ (36.50 mg) and potassium tert-butoxide (82.63 mg) were added to 1,4-dioxane (7 mL). The reaction proceeded at an elevated temperature of 110°C for 10 hours with stirring under N₂ protection. The reaction liquid was filtered, and the filtrate was concentrated. Purification by preparative TLC (Eluent System A) gave Compound 28c (25 mg).

MS (ESI, m/z): 371.2[M+H]⁺.

### Step 3: Synthesis of 3-((5-amino-2-(6-methylpyridin-2-yl)thieno[3,2-b]pyridin-7-yl)amino)-1-propanol (Compound 91)

Compound 28c (25 mg) was added to DCM (2 mL) and TFA (3 mL), and the reaction proceeded at 25°C for 10 hours with stirring. The reaction liquid was filtered, and the filtrate was concentrated. Separation and purification by Prep-HPLC (Elution Condition 2) and lyophilization gave Compound 91 (4 mg).

MS (ESI, m/z): 315.1 [M+H]⁺.

¹H NMR (CD₃OD 400 MHz) δ 7.67-7.73 (m, 2H), 7.58 (s, 1H), 7.16-7.18 (m, 1H), 5.83 (s, 1H), 3.70-3.72 (m, 2H), 3.38-3.39 (m, 2H), 2.55 (s, 3H), 1.89-1.94 (m, 2H).

### Example 29:

3-((5-amino-2-phenylthieno[3,2-b]pyridin-7-yl)amino)-1-propanol (Compound 93)

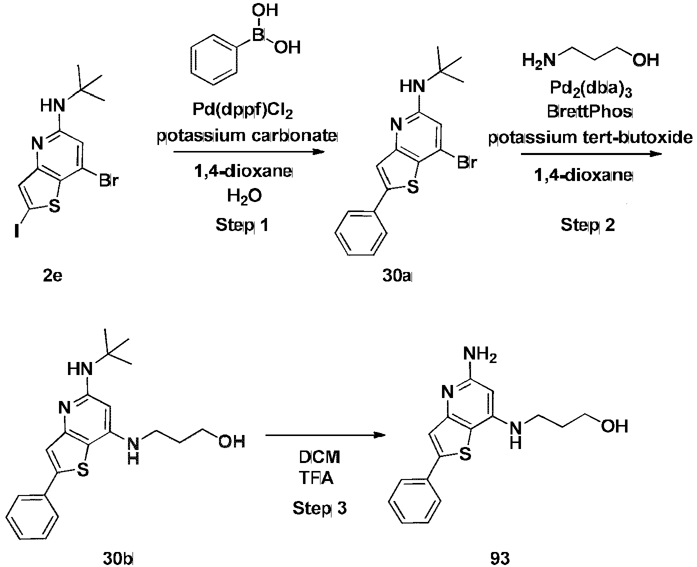

### Step 1: Synthesis of 7-bromo-N-(tert-butyl)-2-phenylthieno[3,2-b]pyridine-5-amine (Compound 30a)

Compound 2e (100 mg), phenylboronic acid (35.59 mg), potassium carbonate (67.14 mg), Pd(dppf)Cl₂•CH₂Cl₂ (19.85 mg) were added to a mixed system of 1,4-dioxane (5 mL) and water (0.5 mL). After nitrogen replacement, the reaction proceeded at an elevated temperature of 50°C for 4 hours. The reaction liquid was cooled to room temperature and filtered. The filtrate was concentrated under a reduced pressure and purified by TLC (Eluent System B) to give Compound 30a (50 mg).

MS (ESI, m/z): 361.2 [M+H]⁺.

### Step 2: Synthesis of 3-((5-(tert-butylamino)-2-phenylthieno[3,2-b]pyridin-7-yl)amino)-1-propanol (Compound 30b)

3-Amino-1-propanol (51.97 mg), Compound 30a (50 mg), Pd₂(dba)₃ (25.34 mg), BrettPhos (25.83 mg), potassium tert-butoxide (77.64 mg) were added to 1,4-dioxane (3 mL). After nitrogen replacement, the reaction proceeded at an elevated temperature of 110°C for 5 hours. The reaction liquid was cooled to room temperature and filtered. The solid was washed with 1,4-dioxane. The filtrate was concentrated under a reduced pressure and purified by TLC (Eluent System B) to give Compound 30b (30 mg).

MS (ESI, m/z): 356.2 [M+H]⁺.

### Step 3: Synthesis of 3-((5-amino-2-phenylthieno[3,2-b]pyridin-7-yl)amino)-1-propanol (Compound 93)

Compound 30b (35 mg) was added to a mixed system of DCM (3 mL) and TFA (5 mL), and the reaction proceeded at 25°C for 16 hours. The reaction liquid was concentrated to dryness under a reduced pressure, and methanol (5 ml) was added. The system was adjusted to pH = 8 with potassium carbonate, concentrated under a reduced pressure, and then separated and purified by Prep-HPLC (Elution Condition 4) and lyophilized to give Compound 93 (6 mg).

MS (ESI, m/z): 300.1 [M+H]⁺.

¹H NMR (DMSO-*d₆*, 400 MHz) δ 7.74-7.70 (m, 2H), 7.48-7.44 (m, 2H), 7.42 (s, 1H), 7.38-7.34 (m, 1H), 6.22 (t, *J* = 5.3 Hz, 1H), 5.66 (s, 1H), 5.55 (s, 2H), 4.58 (t, *J* = 4.9 Hz, 1H), 3.53 (dd, *J* = 11.1, 6.0 Hz, 2H), 3.22 (dd, *J* = 12.6, 6.8 Hz, 2H), 1.82 - 1.73 (m, 2H).

### Example 30:

(1S,2R)-2-((5-amino-2-(1H-pyrazol-5-yl)thieno[3,2-b]pyridin-7-yl)amino)cyclopentanol (Compound 96)

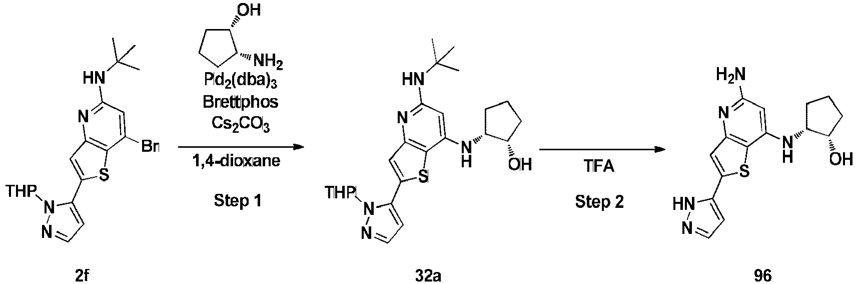

### Step 1: Synthesis of (1S,2R)-2-((5-(tert-butylamino)-2-(1-(tetrahydro-2H-pyran-2-yl)-1H-pyrazol-5-yl)thieno[3,2-b]pyridin-7-yl)amino)cyclopentanol (Compound 32a)

Compound 2f (100 mg), (1S,2R)-2-aminocyclopentanol (92.93 mg), Pd₂(dba)₃ (21.02 mg), BrettPhos (24.62 mg), Cs₂CO₃ (149.67 mg) were added to 1,4-dioxane (5 mL), and the microwave reaction proceeded at an elevated temperature of 120°C for 3 hours. The system was filtered. The filtrate was concentrated, diluted with a small amount of methanol, and purified by preparative TLC (Eluent System A) to give Compound 32a (45 mg).

MS (ESI, m/z): 456.3 [M+H]⁺.

### Step 2: Synthesis of (1S, 2R)-2-((5-amino-2-(1H-pyrazol-5-yl)thieno[3,2-b]pyridin-7-yl)amino)cyclopentanol (Compound 96)

Compound 32a (45 mg) was added to TFA (3 mL), and the reaction proceeded at an elevated temperature of 70°C for 2 hours. The system was concentrated, diluted with methanol, and adjusted to pH = 9 with a saturated sodium bicarbonate aqueous solution. The system was suction filtrated. The filtrate was separated and purified by Prep-HPLC (Elution Condition 4), and lyophilized to give Compound 96 (7 mg).

MS (ESI, m/z): 316.1 [M+H]⁺.

¹H NMR (DMSO-*d₆*, 400 MHz) δ 12.97 (s, 1H), 7.79 (s, 1H), 7.30 (s, 1H), 6.71 (d, *J* = 2.3 Hz, 1H), 5.72 (s, 1H), 5.51 (s, 2H), 5.28 (d, *J* = 6.8 Hz, 1H), 5.03 (s, 1H), 4.17 (s, 1H), 3.65 - 3.58 (m, 1H), 2.04 -1.99 (m, 1H), 1.87 - 1.71 (m, 2H), 1.67 - 1.50 (m, 3H).

**Example 31:**

*N*-(2-((5-amino-2-(1H-pyrazol-5-yl)thieno[3,2-b]pyridin-7-yl)amino)ethyl)-*N*-methylcyclopropionamide (Compound 75)

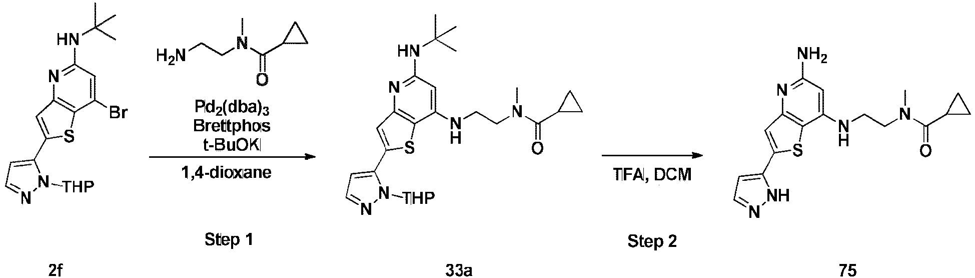

### Step 1: Synthesis of N-(2-((5-(tert-butylamino)-2-(1-(tetrahydro-2H-pyran-2-yl)-1H-pyrazol-5-yl)thieno[3,2-b]pyridin-7-yl)amino)ethyl)-N-methylcyclopropionamide (Compound 33a)

Compound 2f (80 mg), *N*-(2-aminoethyl)-*N*-methylcyclopropanamide (52.26 mg), Pd₂(dba)₃ (8.41 mg), BrettPhos (9.86 mg) and t-BuOK (82.47 mg) were added to 1,4-dioxane (2 mL), and the reaction proceeded at an elevated temperature of 100°C for 5 hours under N₂ protection. The system was filtered. The filtrate was concentrated, diluted with a small amount of DCM, and purified by preparative TLC (Eluent System B) to give Compound 33a (70 mg).

MS (ESI, m/z): 497.3 [M+H]⁺.

### Step 2: Synthesis of N-(2-((5-amino-2-(1H-pyrazol-5-yl)thieno[3,2-b]pyridin-7-yl)amino)ethyl)-N-methylpropionamide (Compound 75)

Compound 33a (30 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 4), and lyophilized to give Compound 75 (7 mg).

MS (ESI, m/z): 357.1 [M+H]⁺.

¹H NMR (CD₃OD, 400 MHz) δ 7.70 (s, 1H), 7.32 (s, 1H), 6.67 (s, 1H), 5.90 (s, 0.60H), 5.86 (s, 0.46H), 3.84 (t, *J=* 5.9 Hz, 0.85H), 3.65 (t, *J* = 6.5 Hz, 1.18H), 3.60 (t, *J* = 5.9 Hz, 0.82H), 3.47 (t, *J* = 6.5 Hz, 1.25H), 3.27 (s, 1.55H), 3.01 (s, 1.10H), 1.93 (ddd, *J* = 15.3, 8.0, 4.8 Hz, 0.80H), 1.81 (ddd, *J* = 12.4, 8.0, 4.5 Hz, 0.50H), 0.91 (dt, *J=* 5.6, 3.8 Hz, 1.12H), 0.85 (qd, *J* = 7.3, 4.4 Hz, 1.29H), 0.75 (dt, *J* = 7.9, 3.8 Hz, 0.91H), 0.56 (dt, *J* = 11.7, 3.7 Hz, 0.91H).

### Example 32:

*N*⁷-(2,2-difluoro-2-(pyridin-2-yl)ethyl)-2-(1H-pyrazol-5-yl)thieno[3,2-b]pyridine-5,7-diamine (Compound 97)

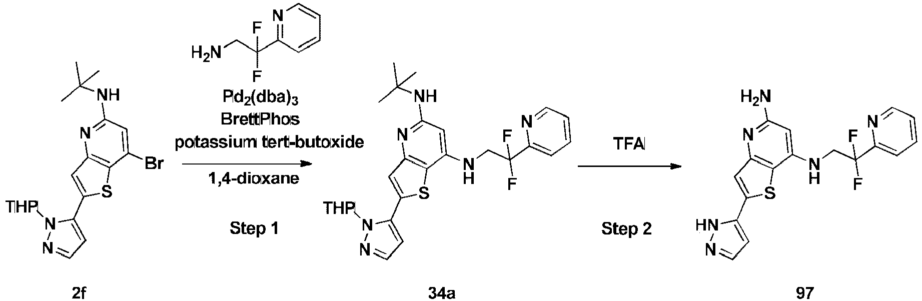

### Step 1: Synthesis of N⁵-(tert-butyl)-N⁷-(2,2-difluoro-2-(pyridin-2-yl)ethyl)-2-(1-(tetrahydro-2H-pyran-2-yl)-1H-pyrazol-5-yl)thieno[3,2-b]pyridine-5,7-diamine (Compound 34a)

Compound 2f (70 mg), 2,2-difluoro-2-(pyridin-2-yl)ethylamine (127 mg), Pd₂(dba)₃ (30.3 mg), BrettPhos (30.3 mg) and potassium tert-butoxide (83 mg) were added to 1,4-dioxane (3 mL). After nitrogen replacement, the reaction proceeded at an elevated temperature of 120°C for 4 hours. The system was filtered. The filtrate was concentrated, diluted with a small amount of methanol, and purified by preparative TLC (Eluent System A) to give Compound 34a (50 mg).

MS (ESI, m/z): 513.2 [M+H]⁺.

### Step 2: N⁷-(2,2-difluoro-2-(pyridin-2-yl)ethyl)-2-(1H-pyrazol-5-yl)thieno[3,2-b]pyridine-5,7-diamine (Compound 97)

Compound 34a (50 mg) was added to TFA (5 mL), and the reaction proceeded at an elevated temperature of 70°C for 2 hours. The system was concentrated to dryness, separated and purified by Prep-HPLC (Elution Condition 4), and lyophilized to give Compound 97 (15 mg).

MS (ESI, m/z): 373.1 [M+H]⁺.

¹H NMR (DMSO-*d₆*, 400 MHz) δ 12.98 (s, 1H), 8.75 (d, *J* = 4.3 Hz, 1H), 8.00 (td, *J* = 7.8, 1.7 Hz, 1H), 7.80 (s, 1H), 7.73 (d, *J* = 7.9 Hz, 1H), 7.59 (dd, *J* = 7.3, 5.0 Hz, 1H), 7.29 (s, 1H), 6.72 (s, 1H), 6.59 (s, 1H), 5.82 (s, 1H), 5.67 (s, 2H), 4.07 (td, *J* = 15.1, 6.4 Hz, 2H).

### Example 33:

*N*-(2-((5-amino-2-(1H-pyrazol-5-yl)thieno[3,2-b]pyridin-7-yl)amino)ethyl)-*N*-methylethanamide (Compound 73)

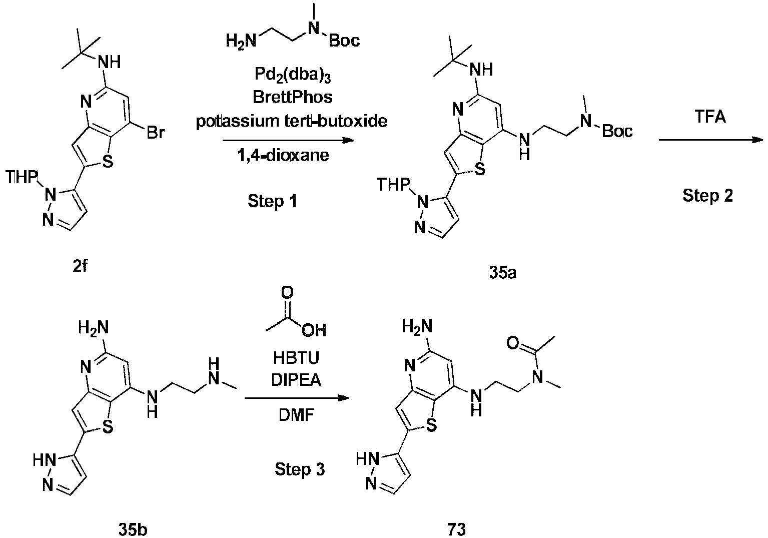

### Step 1: Synthesis of tert-butyl (2-((5-(tert-butylamino)-2-(1-(tetrahydro-2H-pyran-2-yl)-1H-pyrazol-5-yl)thieno[3,2-b]pyridin-7-yl)amino)ethyl)(methyl)carbamate (Compound 35a)

Compound 2f (70 mg), *N*-Boc-*N*-methylethylenediamine (140 mg), Pd₂(dba)₃ (30.3 mg), BrettPhos (30.3 mg) and potassium tert-butoxide (83 mg) were added to 1,4-dioxane (3 mL). After nitrogen replacement, the reaction proceeded at an elevated temperature of 120°C for 4 hours. The system was filtered. The filtrate was concentrated, diluted with a small amount of methanol, and purified by preparative TLC (Eluent System A) to give Compound 35a (52 mg).

MS (ESI, m/z): 529.2 [M+H]⁺.

### Step 2: Synthesis of N⁷-(2-(methylamino)ethyl)-2-(1H-pyrazol-5-yl)thieno[3,2-b]pyridine-5,7-diamine (Compound 35b)

Compound 35a (52 mg) was added to TFA (5 mL), and the reaction proceeded at an elevated temperature of 70°C for 4 hours. The system was concentrated to dryness to give Compound 35b (28.4 mg).

MS (ESI, m/z): 289.1 [M+H]⁺.

### Step 3: Synthesis of N-(2-((5-amino-2-(1H-pyrazol-5-yl)thieno[3,2-b]pyridin-7-yl)amino)ethyl)-N-methylethanamide (Compound 73)

Compound 35b (28.4 mg), acetic acid (7.1 mg) and HBTU (56 mg) were added to DMF (2 mL). DIPEA (64 mg) was then added to the system, and the reaction proceeded at room temperature for 4 hours. Separation and purification by Prep-HPLC (Elution Condition 2) and lyophilization gave Compound 73 (17 mg).

MS (ESI, m/z): 311.1 [M+H]⁺.

¹H NMR (DMSO-*d₆*, 400 MHz) δ 12.95 (s, 1H), 7.81 (s, 1H), 7.28 (s, 1H), 6.71 (s, 1H), 6.38 - 6.14 (m, 1H), 5.74 - 5.64 (m, 1H), 5.50 (s, 2H), 3.54 - 3.45 (m, 2H), 3.39 - 3.34 (m, 1H), 3.29 - 3.25 (m, 1H), 3.05 - 2.83 (m, 3H), 2.02 - 1.89 (m, 3H).

### Example 34:

1-(2-((5-amino-2-(1H-pyrazol-5-yl)thieno[3,2-b]pyridin-7-yl)amino)ethyl)-4-methylpiperidine-4-ol (Compound 98)

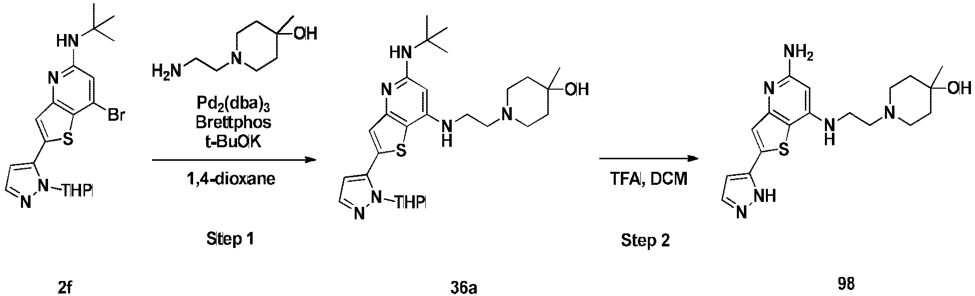

### Step 1: Synthesis of 1-(2-((5-(tert-butylamino)-2-(1-(tetrahydro-2H-pyran-2-yl)-1H-pyrazol-5-yl)thieno[3,2-b]pyridin-7-yl)amino)ethyl)-4-methylpiperidin-4-ol (Compound 36a)

Compound 2f (60 mg), 1-(2-aminoethyl)-4-methylpiperidin-4-ol (43.61 mg), Pd₂(dba)₃ (6.31 mg), BrettPhos (7.40 mg) and t-BuOK (61.85 mg) were added to 1,4-dioxane (2 mL), and the reaction proceeded at an elevated temperature of 110°C for 5 hours under N₂ protection. The system was filtered. The filtrate was concentrated, diluted with a small amount of DCM, and purified by preparative TLC (Eluent System B) to give Compound 36a (50 mg).

MS (ESI, m/z): 513.3 [M+H]⁺.

### Step 2: Synthesis of 1-(2-((5-amino-2-(1H-pyrazol-5-yl)thieno[3,2-b]pyridin-7-yl)amino)ethyl)-4-methylpiperidine-4-ol (Compound 98)

Compound 36a (30 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 6), and lyophilized to give Compound 98 (20 mg).

MS (ESI, m/z): 373.2 [M+H]⁺.

¹H NMR (DMSO-*d₆*, 400 MHz) δ 13.29 (s, 1H), 7.87 (d, *J* = 18.6 Hz, 2H), 7.45 (s, 3H), 6.86 (s, 1H), 5.91 (s, 1H), 4.72 (s, 1H), 3.63 (s, 2H), 3.11 (s, 2H), 1.81 - 1.57 (m, 4H), 1.21 (d, *J* = 18.9 Hz, 3H).

### Example 35:

1-(3-((5-amino-2-(1H-pyrazol-5-yl)thieno[3,2-b]pyridin-7-yl)amino)propyl)-4-methylpiperidine-4-ol (Compound 99)

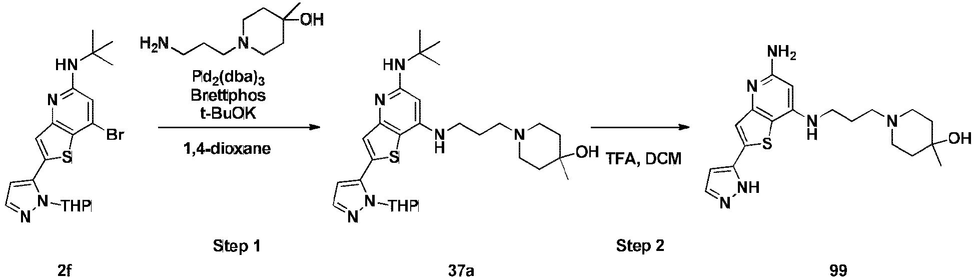

### Step 1: Synthesis of 1-(3-((5-(tert-butylamino)-2-(1-(tetrahydro-2H-pyran-2-yl)-1H-pyrazol-5-yl)thieno[3,2-b]pyridin-7-yl)amino)propyl)-4-methylpiperidin-4-ol (Compound 37a)

Compound 2f (60 mg), 1-(2-aminoethyl)-4-methylpiperidin-4-ol (47.48 mg), Pd₂(dba)₃ (6.31 mg), BrettPhos (7.40 mg) and t-BuOK (61.85 mg) were added to 1,4-dioxane (2 mL), and the reaction proceeded at an elevated temperature of 110°C for 5 hours under N₂ protection. The system was filtered. The filtrate was concentrated, diluted with a small amount of DCM, and purified by preparative TLC (Eluent System B) to give Compound 37a (60 mg).

MS (ESI, m/z): 527.3 [M+H]⁺.

### Step 2: Synthesis of 1-(3-((5-amino-2-(1H-pyrazol-5-yl)thieno[3,2-b]pyridin-7-yl)amino)propyl)-4-methylpiperidine -4-ol (Compound 99)

Compound 37a (50 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 6), and lyophilized to give Compound 99 (28 mg).

MS (ESI, m/z): 387.2 [M+H]⁺.

¹H NMR (400 MHz, DMSO) δ 7.78 (d, *J* = 1.8 Hz, 1H), 7.29 (s, 1H), 6.82 (s, 1H), 6.70 (d, *J* = 1.9 Hz, 1H), 5.91 (s, 2H), 5.63 (s, 1H), 3.20 (s, 2H), 2.52 (s, 2H), 2.48 - 2.44 (m, 2H), 2.33 (s, 2H), 1.84 - 1.74 (m, 2H), 1.63 (t, *J* = 10.0 Hz, 2H), 1.51 (d, *J* = 13.0 Hz, 2H), 1.17 (s, 3H).

**Example** 36:

(1s,3s)-3-((5-amino-2-(1H-pyrazol-5-yl)thieno[3,2-b]pyridin-7-yl)amino)-1-methylcyclobutanol (Compound 129)

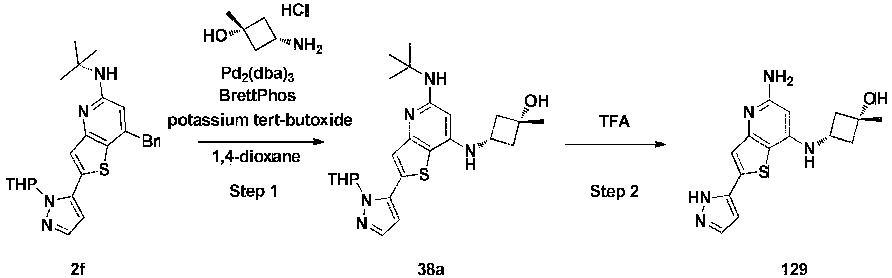

### Step 1: Synthesis of (1s,3s)-3-((5-(tert-butylamino)-2-(1-(tetrahydro-2H-pyran-2-yl)-1H-pyrazol-5-yl)thieno[3,2-b]pyridin-7-yl)amino)-1-methylcyclobutanol (Compound 38a)

Compound 2f (72 mg), 3-amino-1-methylcyclobutanol hydrochloride (100 mg), Pd₂(dba)₃ (31.2 mg), BrettPhos (31.2 mg) and potassium tert-butoxide (93 mg) were added to 1,4-dioxane (3 mL). After nitrogen replacement, the reaction proceeded at an elevated temperature of 120°C for 4 hours. The system was filtered. The filtrate was concentrated, diluted with a small amount of methanol, and purified by preparative TLC (Eluent System A) to give Compound 38a (9 mg).

MS (ESI, m/z): 456.3 [M+H]⁺.

### Step 2: Synthesis of (1s,3s)-3-((5-amino-2-(1H-pyrazol-5-yl)thieno[3,2-b]pyridin-7-yl)amino)-1-methylcyclobutanol (Compound 129)

Compound 38a (9 mg) was added to TFA (5 mL), and the reaction proceeded at an elevated temperature of 70°C for 2 hours. The system was concentrated, diluted with methanol, and adjusted to pH = 9 with a saturated potassium carbonate solution. The system was stirred at room temperature for 0.5 hours, separated and purified by Prep-HPLC (Elution Condition 4), and lyophilized to give Compound 129 (1.5 mg).

MS (ESI, m/z): 316.1 [M+H]⁺.

¹H NMR (DMSO-*d₆*, 400 MHz) δ 12.95 (s, 1H), 7.81 (s, 1H), 7.27 (s, 1H), 6.71 (s, 1H), 6.46 (s, 1H), 5.67 - 5.46 (m, 3H), 5.00 (s, 1H), 3.52 - 3.46 (m, 1H), 2.46 - 2.42 (m, 2H), 2.07 (t, *J* = 9.9 Hz, 2H), 1.29 (s, 3H).

### Example 37:

*N*⁷-(2-methoxyethyl)-2-(1H-pyrazol-5-yl)thieno[3,2-b]pyridine-5,7-diamine hydrochloride (Compound 100s)

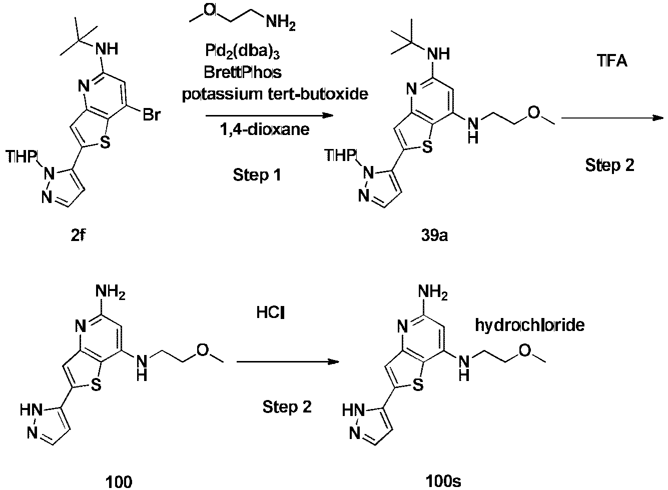

### Step 1: Synthesis of N⁵-(tert-butyl)-N⁷-(2-methoxyethyl)-2-(1-(tetrahydro-2H-pyran-2-yl)-1H-pyrazol-5-yl)thieno[3,2-b]pyridine-5,7-diamine (Compound 39a)

Compound 2f (400 mg), 2-methoxyethylamine (345 mg), Pd₂(dba)₃ (173 mg), BrettPhos (173 mg) and potassium tert-butoxide (474 mg) were added to 1,4-dioxane (18 mL). After nitrogen replacement, the reaction proceeded at an elevated temperature of 120°C for 4 hours. The system was filtered. The filtrate was concentrated, diluted with a small amount of methanol, and purified by TLC (Eluent System A) to give Compound 39a (400 mg).

MS (ESI, m/z): 430.2 [M+H]⁺.

### Step 2: Synthesis of N⁷-(2-methoxyethyl)-2-(1H-pyrazol-5-yl)thieno[3,2-b]pyridine-5,7-diamine hydrochloride (Compound 100s)

Compound 39a (370 mg) was added to TFA (10 mL), and the reaction proceeded at an elevated temperature of 70°C for 2 hours. The system was concentrated to dryness to give crude Compound 100. The system was separated and purified by Prep-HPLC (Elution Condition 4), concentrated to remove acetonitrile, and adjusted to pH = 1 by adding concentrated hydrochloric acid. The system was stirred at room temperature for 0.5 hours and lyophilized to give Compound 100s (135 mg).

MS (ESI, m/z): 290.1 [M+H]⁺.

¹H NMR (DMSO-*d₆*, 400 MHz) δ 12.97 (s, 1H), 7.99 (t, *J* = 5.6 Hz, 1H), 7.90 (d, *J=* 2.4 Hz, 1H), 7.48 (s, 1H), 7.25 (s, 2H), 6.87 (d, *J* = 2.4 Hz, 1H), 5.83 (d, *J=* 1.2 Hz, 1H), 3.57 (t, *J* = 5.6 Hz, 2H), 3.48 - 3.40 (m, 2H), 3.30 (s, 3H).

### Example 38:

(R)-*N*⁷-(2-methoxypropyl)-2-(1H-pyrazol-5-yl)thieno[3,2-b]pyridine-5,7-diamine hydrochloride (Compound 101s)

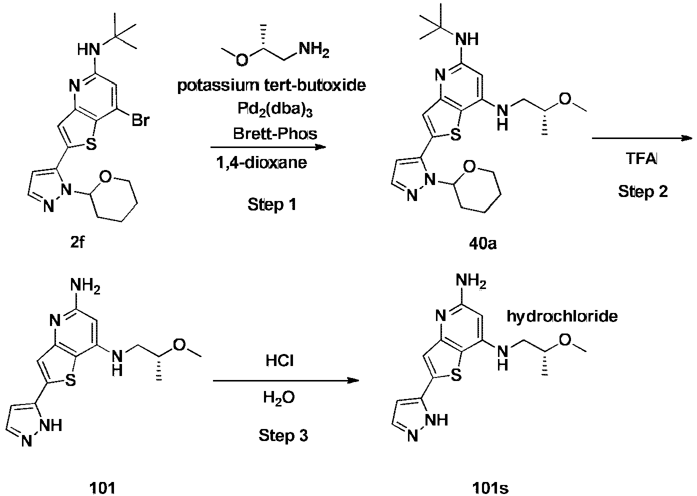

### Step 1: Synthesis of N⁵-(tert-butyl)-N⁷-((R)-2-methoxypropyl)-2-(1-(tetrahydro-2H-pyran-2-yl)-1H-pyrazol-5-yl)thieno[3,2-b]pyridine-5,7-diamine (Compound 40a)

Compound 2f (70 mg), (R)-2-methoxypropyl-1-amine hydrochloride (81.93 mg), potassium tert-butoxide (91.49 mg), Pd₂(dba)₃ (29.42 mg) and BrettPhos (34.47 mg) were added to 1,4-dioxane (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 40a (38 mg).

MS (ESI, m/z): 444.2 [M+H]⁺.

### Step 2: Synthesis of (R)-N⁷-(2-methoxypropyl)-2-(1H-pyrazol-5-yl)thieno[3,2-b]pyridine-5,7-diamine (Compound 101)

Compound 40a (38 mg) was added to TFA (2 mL), and the reaction proceeded at an elevated temperature of 60°C for 2 hours with stirring. The reaction solvent was removed by concentration under a reduced pressure to dryness. Separation and purification by Prep-HPLC (Elution Condition 4) and lyophilization gave Compound 101 (10 mg).

MS (ESI, m/z): 304.1 [M+H]⁺.

¹H NMR (DMSO-*d₆*, 400 MHz) δ 13.02 (s, 1H), 7.80 (s, 1H), 7.30 (s, 1H), 6.72 (s, 1H), 6.35 (s, 1H), 5.79 (s, 2H), 5.69 (s, 1H), 3.58 (dt, *J* = 18.0, 5.9 Hz, 3H), 3.30 (s, 3H), 3.24 (dd, *J* = 13.0, 6.7 Hz, 1H), 3.16 - 3.07 (m, 1H), 1.14 (d, *J* = 6.1 Hz, 3H).

### Step 3: Synthesis of (R)-N⁷-(2-methoxypropyl)-2-(1H-pyrazol-5-yl)thieno[3,2-b]pyridine-5,7-diamine hydrochloride (Compound 101s)

Compound 101 (30 mg) was added to water (5 mL), and concentrated hydrochloric acid was added dropwise until the system was clear. The system was stirred at 25°C for 15 minutes, and then lyophilized to give Compound 101s (25 mg).

MS (ESI, m/z): 304.1 [M+H]⁺.

¹H NMR (DMSO-*d₆*, 400 MHz) δ 13.17 (s, 1H), 8.01 (t, *J* = 5.6 Hz, 1H), 7.89 (d, *J* = 2.1 Hz, 1H), 7.50 (s, 1H), 7.30 (s, 2H), 6.87 (d, *J* = 2.2 Hz, 1H), 5.87 (s, 1H), 3.60 (dt, *J* = 12.3, 6.1 Hz, 1H), 3.36 - 3.31 (m, 1H), 3.30 (s, 3H), 3.27 - 3.20 (m, 1H), 1.15 (d, *J* = 6.1 Hz, 3H).

### Example 39:

*N*⁷-(cyclopropylmethyl)-2-(1H-pyrazol-5-yl)thieno[3,2-b]pyridine-5,7-diamine hydrochloride (Compound 102s)

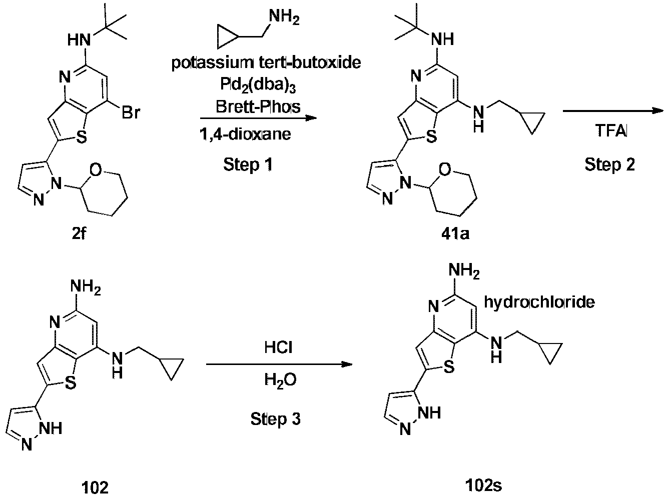

### Step 1: Synthesis of N⁵-(tert-butyl)-N⁷-(cyclopropylmethyl)-2-(1-(tetrahydro-2H-pyran-2-yl)-1H-pyrazol-5-yl)thieno[3,2-b]pyridine-5,7-diamine (Compound 41a)

Compound 2f (70 mg), cyclopropyl methylamine (57.99 mg), potassium tert-butoxide (54.90 mg), Pd₂(dba)₃ (29.42 mg) and BrettPhos (34.47 mg) were added to 1,4-dioxane (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 41a (45 mg).

MS (ESI, m/z): 426.2 [M+H]⁺.

### Step 2: Synthesis of N⁷-(cyclopropylmethyl)-2-(1H-pyrazol-5-yl)thieno[3,2-b]pyridine-5,7-diamine (Compound 102)

Compound 41a (45 mg) was added to TFA (2 mL), and the reaction proceeded at an elevated temperature of 60°C for 2 hours with stirring. The reaction solvent was removed by concentration under a reduced pressure to dryness. Separation and purification by Prep-HPLC (Elution Condition 4) and lyophilization gave Compound 102 (15 mg)

MS (ESI, m/z): 286.1 [M+H]⁺.

¹H NMR (DMSO-*d₆*, 400 MHz) δ 13.00 (s, 1H), 7.80 (s, 1H), 7.30 (s, 1H), 6.72 (s, 1H), 6.49 (t, *J* = 5.3 Hz, 1H), 5.76 (s, 2H), 5.71 (s, 1H), 3.05 (t*, J* = 6.1 Hz, 2H), 1.19 - 1.10 (m, 1H), 0.51 - 0.45 (m, 2H), 0.29 - 0.24 (m, 2H).

### Step 3: Synthesis of N⁷-(cyclopropylmethyl)-2-(1H-pyrazol-5-yl)thieno[3,2-b]pyridine-5,7-diamine hydrochloride (Compound 102s)

Compound 102 (30 mg) was added to water (5 mL), and concentrated hydrochloric acid was added dropwise until the system was clear. The system was stirred at 25°C for 15 minutes, and then lyophilized to give Compound 102s (23 mg).

MS (ESI, m/z): 286.1 [M+H]⁺.

¹H NMR (DMSO-*d₆*, 400 MHz) δ 13.14 (s, 1H), 8.12 (t, *J* = 5.3 Hz, 1H), 7.89 (d, *J* = 2.3 Hz, 1H), 7.50 (s, 1H), 7.28 (s, 2H), 6.87 (d, *J* = 2.3 Hz, 1H), 5.86 (s, 1H), 3.14 (t, *J=* 5.9 Hz, 2H), 1.20 - 1.08 (m, 1H), 0.56 - 0.50 (m, 2H), 0.33 - 0.29 (m, 2H).

### Example 40:

(R)-*N*⁷-(1-methylpiperidin-3-yl)-2-(1H-pyrazol-5-yl)thieno[3,2-b]pyridine-5,7-diamine (Compound 104)

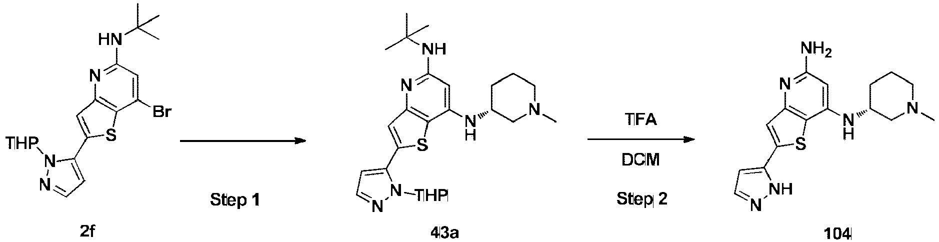

### Step 1: Synthesis of N⁵-(tert-butyl)-N⁷-((R)-1-methylpiperidin-3-yl)-2-(1-(tetrahydro-2H-pyran-2-yl)-1H-pyrazol-5-yl)thieno[3,2-b]pyridine-5,7-diamine (Compound 43a) 1-Methyl-(R)-3-aminopiperidine (137.39 mg), Compound 2f (102 mg), DavePhos (36.88 mg), Pd₂(dba)₃ (42.9 mg) and potassium tert-butoxide (131.44 mg) were added to 1,4-dioxane (8 mL). The reaction proceeded at an elevated temperature of 110°C for 10 hours with stirring under N₂ protection. The system was cooled and filtered. The filtrate was concentrated, and then purified and separated by TLC (Eluent System B) to give Compound 43a (101 mg).

MS (ESI, m/z): 469.1 [M+H]⁺.

### Step 2: Synthesis of (R)-N⁷-(1-methylpiperidin-3-yl)-2-(1H-pyrazol-5-yl)thieno[3,2-b]pyridine-5,7-diamine (Compound 104)

Compound 43a (227 mg) and TFA (1 mL) were added to methanol (5 mL), and stirred at 25°C for 3 hours. The reaction solvent was removed by concentration under a reduced pressure to dryness. Separation and purification by Prep-HPLC (Elution Condition 4) and lyophilization gave Compound 104 (181 mg).

MS (ESI, m/z): 346.2 [M+H]⁺.

¹H NMR (DMSO-*d₆*, 400 MHz) δ 13.28 (s, 2H), 9.92 (s, 1H), 7.91 (s, 1H), 7.86 (d, *J* = 7.0 Hz, 1H), 7.57 - 7.43 (m, 4H), 6.89 (s, 1H), 5.91 (s, 1H), 3.83 (s, 1H), 3.62 (d, *J* = 9.8 Hz, 1H), 2.91 (s, 2H), 2.87 (s, 3H), 2.16 - 2.06 (m, 1H), 1.99 (d, *J* = 13.4 Hz, 1H), 1.82 - 1.68 (m, 1H), 1.60 - 1.44 (m, 1H).

### Example 41:

(S)-2-(1H-pyrazol-5-yl)-*N⁷*-((tetrahydrofuran-2-yl)methyl)thieno[3,2-b]pyridine-5,7-diamine (Compound 105)

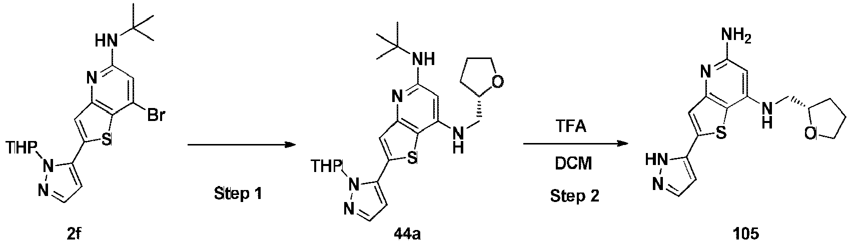

### Step 1: Synthesis of N⁵-(tert-butyl)-2-(1-(tetrahydro-2H-pyran-2-yl)-1H-pyrazol-5-yl)-N⁷-((S)-tetrahydrofuran-2-yl)methyl)thieno[3,2-b]pyridine-5,7-diamine (Compound 44a)

(S)-(Tetrahydrofuran-2-yl)formamide (93 mg), Compound 2f (200 mg), Brettphos (49.31 mg), Pd₂(dba)₃ (42.07 mg) and potassium tert-butoxide (154.64 mg) were added to 1,4-dioxane (8 mL), and the reaction proceeded at an elevated temperature of 110°C and stirred for 10 hours with stirring under N₂ protection. The system was cooled and filtered. The filtrate was concentrated, and then purified and separated by TLC (Eluent System B) to give Compound 44a (50 mg).

MS (ESI, m/z): 456.2[M+H]⁺.

### Step 2: Synthesis of (S)-2-(1H-pyrazol-5-yl)-N⁷-((tetrahydrofuran-2-yl)methyl)thieno[3,2-b]pyridine-5,7-diamine (Compound 105)

Compound 44a (50 mg) was added to DCM (2 mL) and TFA (2 mL), and stirred at 25°C for 10 hours. The mixture was concentrated under a reduced pressure to dryness, dissolved again with methanol, and adjusted to pH 8-9 with a saturated sodium bicarbonate aqueous solution. The system was filtrated. The filtrate was separated and purified by Prep-HPLC (Elution Condition 4), and lyophilized to give Compound 105 (20 mg).

MS (ESI, m/z): 316.1 [M+H]⁺.

¹HNMR (DMSO-*d₆*, 400 MHz) δ 12.96 (s, 1H), 7.80 (s, 1H), 7.28 (s, 1H), 6.71 (s, 1H), 6.19-6.20 (m, 1H), 5.70 (s, 1H), 5.56 (s, 2H), 4.06-4.10 (m, 1H), 3.70-3.71 (m, 1H), 3.61-3.62 (m, 1H), 3.15-3.17 (m, 2H), 1.85-1.86 (m, 1H), 1.82-1.83 (m, 2H), 1.61-1.68 (m, 1H).

### Example 42:

*N⁷*-(1-methyl-1H-pyrazol-4-yl)methyl-2-(1H-pyrazol-5-yl)thieno[3,2-b]pyridine-5,7-diamine (Compound 106)

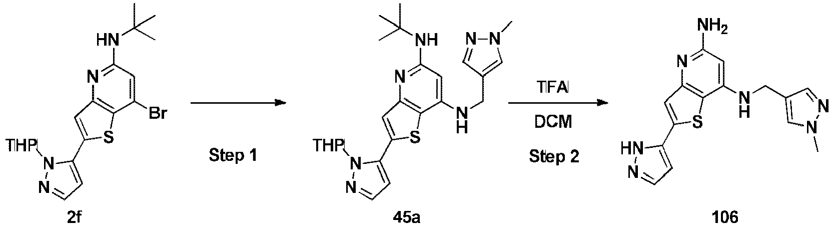

### Step 1: Synthesis of N⁵-(tert-butyl)-N⁷-((1-methyl-1H-pyrazol-4-yl)methyl)-2-(1-(tetrahydro-2H-pyran-2-yl)-1H-pyrazol-5-yl)thieno[3,2-b]pyridine-5,7-diamine (Compound 45a)

(1-Methyl-1H-pyrazol-4-yl)formamide (102 mg), Compound 2f (200 mg), Brettphos (49.31 mg), Pd₂(dba)₃ (42.07 mg) and potassium tert-butoxide (154.64 mg) were added to 1,4-dioxane (8 mL), and the reaction proceeded at an elevated temperature of 110°C for 10 hours with stirring under N₂ protection. The system was cooled and filtered. The filtrate was concentrated, purified and separated by TLC (Eluent System B) to give Compound 45a (40 mg).

MS (ESI, m/z): 466.2[M+H]⁺.

### Step 2: Synthesis of N⁷-((1-methyl-1H-pyrazol-4-yl)methyl)-2-(1H-pyrazol-5-yl)thieno[3,2-b]pyridine-5,7-diamine (Compound 106)

Compound 45a (40 mg, 85.8 µmol) was added to DCM (2 mL) and TFA (2 mL), and stirred at 25°C for 10 hours. The reaction solvent was removed by concentration under a reduced pressure to dryness. The residual was dissolved again with methanol, and was adjusted to pH 8-9 with a saturated sodium bicarbonate aqueous solution. The system was filtrated. The filtrate was separated and purified by Prep-HPLC (Elution Condition 4), and lyophilized to give

### Compound 106 (15 mg).

MS (ESI, m/z): 326.2 [M+H]⁺.

¹HNMR (DMSO-*d₆*, 400 MHz) δ 12.94 (s, 1H), 7.80 (s, 1H), 7.59 (s, 1H), 7.39 (s, 1H), 7.27 (s, 1H), 6.69(s, 1H), 6.53 (s, 1H), 5.68 (s, 1H), 5.47 (s, 2H), 4.20 (d, *J=* 6.0 Hz, 2H), 3.78 (s, 3H).

### Example 43:

(1R,3R)-3-((5-amino-2-(1H-pyrazol-5-yl)thieno[3,2-b]pyridin-7-yl)amino)cyclopentanol hydrochloride (Compound 30s)

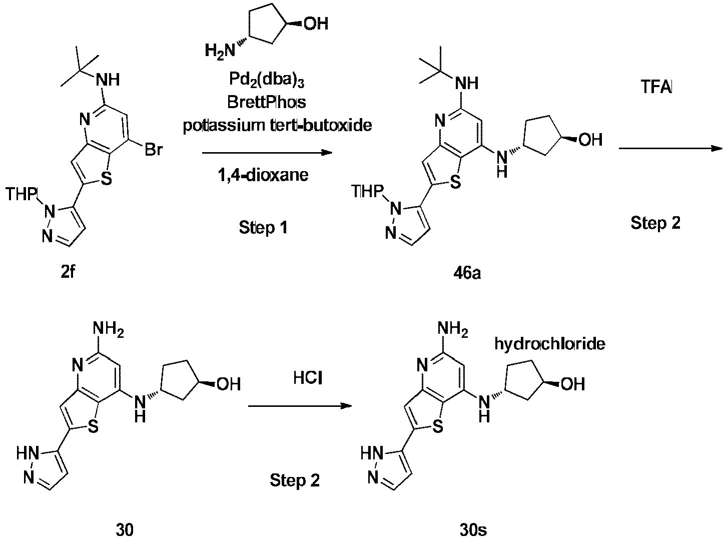

### Step 1: Synthesis of (1R,3R)-3-((5-(tert-butylamino)-2-(1-(tetrahydro-2H-pyran-2-yl)-1H-pyrazol-5-yl)thieno[3,2-b]pyridin-7-yl)amino)cyclopentanol (Compound 46a)

Compound 2f (300 mg), (1R,3R)-3-aminocyclopentanol (220 mg), Pd₂(dba)₃ (76 mg), BrettPhos (74 mg) and potassium tert-butoxide (232 mg) were added to 1,4-dioxane (15 mL), and the reaction. After nitrogen replacement, the reaction proceeded at an elevated temperature of 120°C for 4 hours. The system was filtered through Celite, and the filtrate was concentrated, diluted with a small amount of methanol, and purified by preparative TLC (Eluent System A) to give Compound 46a (52 mg).

MS (ESI, m/z): 456.2 [M+H]⁺.

### Step 2: Synthesis of (1R,3R)-3-((5-amino-2-(1H-pyrazol-5-yl)thieno[3,2-b]pyridin-7-yl)amino)cyclopentanol hydrochloride (Compound 30s)

Compound 46a (52 mg) was added to TFA (6 mL), and the reaction proceeded at an elevated temperature of 60°C for 2 hours. The system was concentrated, diluted with methanol, and adjusted to pH = 9 with a saturated potassium carbonate solution. The system was stirred at room temperature for 0.5 hours, separated and purified by Prep-HPLC (Elution Condition 4) to give an eluent containing Compound 30. The eluent containing Compound 30 was concentrated, and adjusted to pH = 1 by adding concentrated hydrochloric acid. The system was stirred at room temperature for 0.5 hours and lyophilized to give Compound 30s (17 mg).

MS (ESI, m/z): 316.1 [M+H]⁺.

¹H NMR (DMSO-*d₆*, 400 MHz) δ 13.16 (s, 1H), 7.90 (d, *J* = 2.0 Hz, 1H), 7.80 (d, *J* = 6.4 Hz, 1H), 7.50 (s, 1H), 7.32 (s, 2H), 6.87 (d, *J=* 2.0 Hz, 1H), 5.86 (s, 1H), 4.28 (s, 1H), 4.13 - 4.04 (m, 1H), 2.28 - 2.15 (m, 1H), 2.06 - 1.90 (m, 2H), 1.90 - 1.80 (m, 1H), 1.69 - 1.27 (m, 3H).

### Example 44:

3-((5-amino-2-(1-methyl-1H-pyrazol-4-yl)thieno [3,2-b]pyridin-7-yl)amino)-1 -propanol (Compound 109)

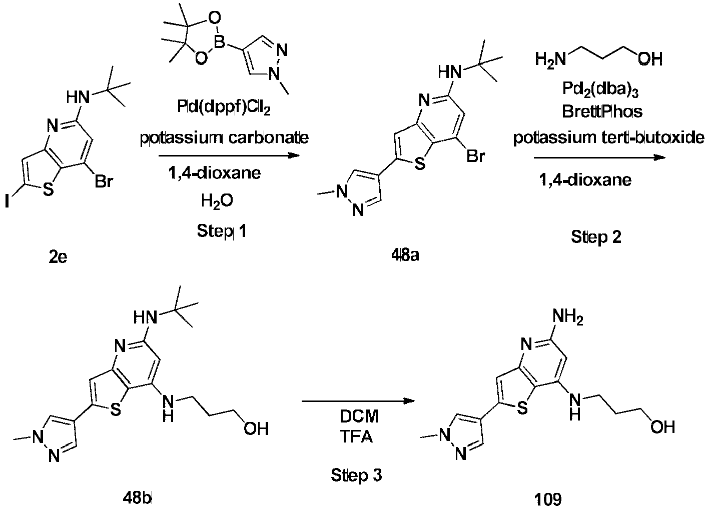

### Step 1: Synthesis of 7-bromo-N-(tert-butyl)-2-(1-methyl-1H-pyrazol-4-yl)thieno[3,2-b]pyridine-5-amine (Compound 48a)

Compound 2e (110 mg), 1-methyl-1H-pyrazol-4-boronic acid pinacol ester (66.81 mg), potassium carbonate (73.85 mg) and Pd(dppf)Cl₂•CH₂Cl₂ (21.83 mg) were added to a mixed system of 1,4-dioxane (5 mL) and water (0.5 mL). After nitrogen replacement, the reaction proceeded at an elevated temperature of 50°C for 4 hours. The reaction liquid was cooled to room temperature, filtered, and the filtrate was concentrated under a reduced pressure and purified by TLC (Eluent System B) to give Compound 48a (100 mg).

MS (ESI, m/z): 365 [M+H]⁺.

### Step 2: Synthesis of 3-((5-(tert-butylamino)-2-(1-methyl-1H-pyrazol-4-yl)thieno[3,2-b]pyridin-7-yl)amino)-1-propanol (Compound 48b)

3-Amino-1-propanol (102.81 mg), Compound 48a (100 mg), Pd₂(dba)3 (50.14 mg), BrettPhos (58.77 mg) and potassium tert-butoxide (92.15 mg) were added to 1,4-dioxane (3 mL). After nitrogen replacement, the reaction proceeded at an elevated temperature of 110°C for 5 hours. The reaction liquid was cooled to room temperature, filtered, and the filter cake was washed with dioxane. The filtrate was concentrated under a reduced pressure and purified by TLC (Eluent System A) to give Compound 48b (50 mg).

MS (ESI, m/z): 360.2 [M+H]⁺.

### Step 3: Synthesis of 3-((5-amino-2-(1-methyl-1H-pyrazol-4-yl)thieno[3,2-b]pyridin-7-yl)amino)-1-propanol (Compound 109)

Compound 48b (50 mg) was added to a mixed system of DCM (3 mL) and TFA (1 mL), and the reaction proceeded at 25°C for 16 hours. The system was concentrated to dryness under a reduced pressure, and methanol (5 mL) was added. The system was adjusted to pH = 8 with potassium carbonate. The solution was concentrated under a reduced pressure, separated and purified by Prep-HPLC (Elution Condition 4), and lyophilized to give Compound 109 (25 mg).

MS (ESI, m/z): 304.1 [M+H]⁺.

¹H NMR (DMSO-*d₆*, 400 MHz) δ 8.08 (s, 1H), 7.76 (s, 1H), 7.06 (s, 1H), 6.06 (t, *J=* 6.1 Hz, 1H), 5.61 (s, 1H), 5.46 (s, 2H), 4.6-4.56 (m, 1H), 3.87 (s, 3H), 3.54-3.50 (m, 2H), 3.20 (d, *J* = 5.8 Hz, 2H), 1.80 - 1.72 (m, 2H).

### Example 45:

*N*⁷-cyclopentyl-2-(1H-pyrazol-5-yl)thieno[3,2-b]pyridine-5,7-diamine hydrochloride (Compound 110s)

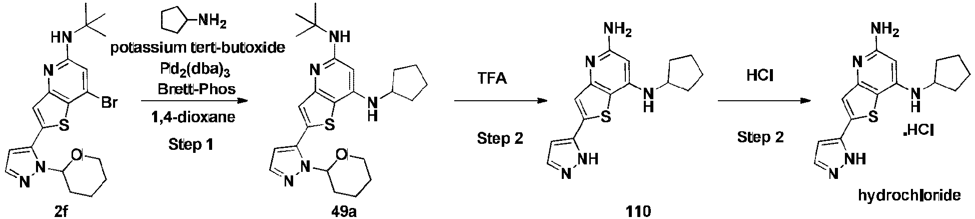

### Step 1: Synthesis of N⁵-(tert-butyl)-N⁷-cyclopentyl-2-(1-(tetrahydro-2H-pyran-2-yl)-1H-pyrazol-5-yl)thieno[3,2-b]pyridine-5,7-diamine (Compound 49a)

Compound 2f (70 mg), cyclopentylamine (16.43 mg), potassium tert-butoxide (90.21 mg), Pd₂(dba)₃ (29.42 mg) and BrettPhos (34.47 mg) were added to 1,4-dioxane (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 49a (40 mg).

MS (ESI, m/z): 440.1 [M+H]⁺.

### Step 2: Synthesis of N⁷-cyclopentyl-2-(1H-pyrazol-5-yl)thieno[3,2-b]pyridine-5,7-diamine hydrochloride (Compound 110s)

Compound 49a (40 mg) was added to TFA (2 mL), and the reaction proceeded at an elevated temperature of 70°C for 2 hours with stirring. The system was concentrated under a reduced pressure to dryness, separated and purified by Prep-HPLC (Elution Condition 3), and lyophilized to give Compound 110.

¹H NMR (DMSO-*d₆*, 400 MHz) δ 13.02 (s, 1H), 8.24 (s, 1H), 7.81 (d, *J=* 2.8 Hz, 1H), 7.29 (s, 1H), 6.72 (s, 1H), 6.34 (d, *J* = 7.2 Hz, 1H), 5.96 (s, 2H), 5.69 (s, 1H), 3.84-3.78 (m, 1H), 2.01-1.93 (m, 2H), 1.74-1.63 (m, 2H), 1.65-1.52 (m, 4H).

1 N hydrochloric acid was added to Compound 110. The system was lyophilized again to give Compound 110s (12 mg).

MS (ESI, m/z): 300.1 [M+H]⁺.

¹HNMR (DMSO-*d₆*, 400 MHz) δ13.14 (s, 1H), 7.88 (s, 1H), 7.81 (d, *J*= 2.8 Hz, 1H), 7.48 (s, 1H), 7.28 (s, 2H), 6.85 (s, 1H), 5.69 (s, 1H), 3.84-3.78 (m, 1H), 2.01-1.93 (m, 2H), 1.74-1.63 (m, 2H), 1.65-1.52 (m, 4H).

### Example 46:

*N*⁷-(2'-methoxy-2'-methylpropyl)-2-(1H-pyrazol-5-yl)thieno[3,2-b]pyridine-5,7-diamine (Compound 111)

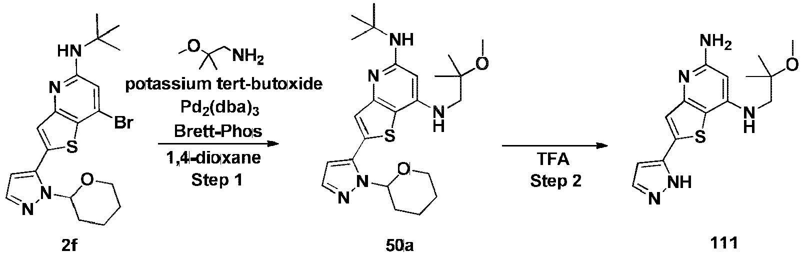

### Step 1: Synthesis of N⁵-(tert-butyl)-N⁷-(2-methoxy-2-methylpropyl)-2-(1-(tetrahydro-2H-pyran-2-yl)-1H-pyrazol-5-yl)thieno[3,2-b]pyridine-5,7-diamine (Compound 50a)

Compound 2f (70 mg), 2-methoxy-2-methylpropyl-1-amine (19.9 mg), potassium tert-butoxide (90.21 mg), Pd₂(dba)₃ (29.42 mg) and BrettPhos (34.47 mg) were added to 1,4-dioxane (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 50a (40 mg)

MS (ESI, m/z): 458.1 [M+H]⁺.

### Step 2: Synthesis of N⁷-(2-methoxy-2-methylpropyl)-2-(1H-pyrazol-5-yl)thieno[3,2-b]pyridine-5,7-diamine (Compound 111)

Compound 50a (40 mg, 0.09 mmol) was added to TFA (2 mL), and the reaction proceeded at an elevated temperature of 75°C for 2 hours with stirring. The system was concentrated under a reduced pressure to dryness, separated and purified by Prep-HPLC (Elution Condition 3), and lyophilized to give Compound 111 (5 mg).

MS (ESI, m/z): 318.1 [M+H]⁺.

¹H NMR (DMSO-*d₆*, 400 MHz) δ 13.05 (s, 1H), 8.31 (s, 1H), 7.81 (d, *J* = 2.4 Hz, 1H), 7.33 (s, 1H), 6.74 (d, *J* = 2.4 Hz, 1H), 6.04 (s, 2H), 5.94-5.91 (m, 1H), 5.81 (s, 1H), 3.20 (d, *J* = 2.0 Hz, 2H), 3.16 (s, 3H), 1.18 (s, 6H).

### Example 47:

*N*⁷-(oxetan-3-yl)-2-(1H-pyrazol-5-yl)thieno[3,2-b]pyridine-5,7-diamine (Compound 112)

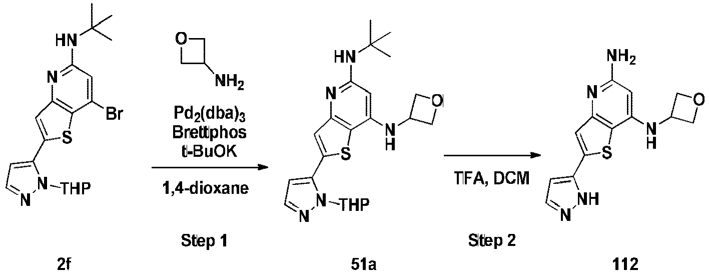

### Step 1: Synthesis of N⁵-(tert-butyl)-N⁷-(oxetan-3-yl)-2-(1-(tetrahydro-2H-pyran-2-yl)-1H-pyrazol-5-yl)thieno[3,2-b]pyridine-5,7-diamine (Compound 51a)

Compound 2f (60 mg), oxetan-3-amine (20.15 mg), Pd₂(dba)₃ (6.31 mg), BrettPhos (7.40 mg) and t-BuOK (61.85 mg) were added to 1,4-dioxane (2 mL), and the reaction proceeded at an elevated temperature of 110°C for 5 hours under N₂ protection. The system was filtered through Celite, and the filtrate was concentrated, diluted with a small amount of DCM, and purified by TLC (Eluent System B) to give Compound 51a (20 mg).

MS (ESI, m/z): 428.2 [M+H]⁺.

### Step 2: Synthesis of N⁷-(oxetan-3-yl)-2-(1H-pyrazol-5-yl)thieno[3,2-b]pyridine-5,7-diamine (Compound 112)

Compound 51a (20 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 4), and lyophilized to give Compound 112 (4 mg).

MS (ESI, m/z): 288.1 [M+H]⁺.

¹H NMR (CD₃OD, 400 MHz) δ 7.71 (d, *J* = 2.3 Hz, 1H), 7.32 (s, 1H), 6.70 (dd, *J* = 11.3, 2.4 Hz, 1H), 5.53 (s, 1H), 5.02 (t, *J=* 6.6 Hz, 2H), 4.79 (d, *J=* 6.0 Hz, 1H), 4.72 (t, *J=* 6.1 Hz, 2H).

### Example 48:

*N⁷*-(pent-3-yl)-2-(1H-pyrazol-5-yl)thieno[3,2-b]pyridine-5,7-diamine hydrochloride (Compound 113s)

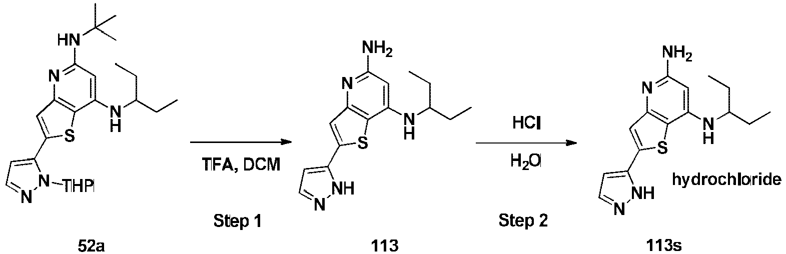

### Step 1: Synthesis of N⁷-(pent-3-yl)-2-(1H-pyrazol-5-yl)thieno[3,2-b]pyridine-5,7-diamine (Compound 113)

Compound 52a (50 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 3), and lyophilized to give Compound 113 (3 mg).

MS (ESI, m/z): 302.1 [M+H]⁺.

¹H NMR (CD₃OD, 400 MHz) δ 7.70 (d, *J* = 2.3 Hz, 1H), 7.30 (d, *J* = 8.4 Hz, 1H), 6.67 (d, *J=* 2.3 Hz, 1H), 5.81 (s, 1H), 3.49 - 3.39 (m, 1H), 1.74 - 1.54 (m, 4H), 0.98 (t, *J*= 7.4 Hz, 6H).

### Step 2: Synthesis of N⁷-(pent-3-yl)-2-(1H-pyrazol-5-yl)thieno[3,2-b]pyridine-5,7-diamine hydrochloride (Compound 113s)

Compound 113 (25 mg) was added to H₂O (2 mL), and concentrated hydrochloric acid was added until the system was clear. The reaction proceeded at 25°C for 0.5 hours. Lyophilization gave Compound 113s (27 mg).

MS (ESI, m/z): 302.1 [M+H]⁺.

¹H NMR (DMSO-*d₆*, 400 MHz) δ 13.26 (s, 1H), 12.80 (s, 1H), 7.90 (s, 1H), 7.57 (d, *J* = 8.0 Hz, 1H), 7.47 (s, 1H), 7.14 (s, 2H), 6.87 (s, 1H), 5.83 (s, 1H), 1.70 - 1.60 (m, 4H), 0.90 (t, *J* = 7.4 Hz, 6H).

### Example 49:

(1S,3R)-3-((5-amino-2-(1H-pyrazol-5-yl)thieno[3,2-b]pyridin-7-yl)amino)cyclopentanol (Compound 31)

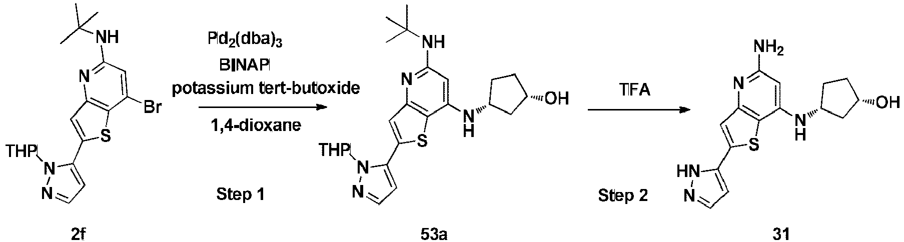

### Step 1: Synthesis of (1S,3R)-3-((5-(tert-butylamino)-2-(1-(tetrahydro-2H-pyran-2-yl)-1H-pyrazol-5-yl)thieno[3,2-b]pyridin-7-yl)amino)cyclopentanol (Compound 53a)

Compound 2f (75 mg), (1S,3R)-3-aminocyclopentanol (71 mg), Pd₂(dba)₃ (32 mg), BINAP (43 mg) and potassium tert-butoxide (96.5 mg) were added to 1,4-dioxane (2 mL). After nitrogen replacement, the reaction proceeded at an elevated temperature of 120°C for 3 hours. The system was filtered through Celite, and the filtrate was concentrated, diluted with a small amount of methanol, and purified by TLC (Eluent System A) to give Compound 53a (10 mg).

MS (ESI, m/z): 456.2 [M+H]⁺.

### Step 2: Synthesis of (1S,3R)-3-((5-amino-2-(1H-pyrazol-5-yl)thieno[3,2-b]pyridin-7-yl)amino)cyclopentanol (Compound 31)

Compound 53a (10 mg) was added to TFA (2 mL), and the reaction proceeded at 70°C for 2 hours. The system was concentrated, diluted with methanol, and was adjusted to pH = 8 with a saturated sodium carbonate solution. The system was stirred at room temperature for 0.5 hours, separated and purified by Prep-HPLC (Elution Condition 4), and lyophilized to give Compound 31 (1.44 mg).

MS (ESI, m/z): 316.1 [M+H]⁺.

¹H NMR (DMSO-*d₆*, 400 MHz) δ 12.95 (s, 1H), 7.80 (s, 1H), 7.27 (s, 1H), 6.70 (s, 1H), 5.92 (d, *J=* 6.8 Hz, 1H), 5.65 (s, 1H), 5.48 (s, 2H), 4.72 (d, *J=* 4.0 Hz, 1H), 4.24 - 4.08 (m, 1H), 3.89 - 3.72 (m, 1H), 2.26 - 2.15 (m, 1H), 2.02 - 1.92 (m, 1H), 1.82 - 1.70 (m, 2H), 1.69 - 1.54 (m, 2H).

### Example 50:

4-((5-amino-2-(1H-pyrazol-5-yl)thieno[3,2-b]pyridin-7-yl)amino)-1-cyclohexanol (Compound 114)

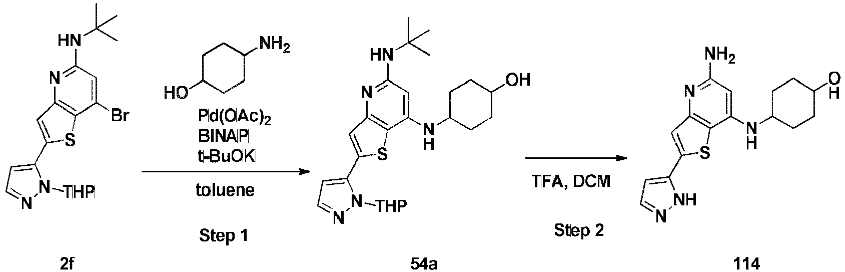

### Step 1: Synthesis of 4-((5-(tert-butylamino)-2-(1-(tetrahydro-2H-pyran-2-yl)-1H-pyrazol-5-yl)thieno[3,2-b]pyridin-7-yl)amino)-1-cyclohexanol (Compound 54a)

Compound 2f (60 mg), 4-aminocyclohexanol (31.74 mg), Pd(OAc)₂ (1.55 mg), BINAP (8.58 mg) and t-BuOK (46.39 mg) were added to toluene (2 mL), and the reaction proceeded at an elevated temperature of 120°C for 5 hours under N₂ protection. The system was filtered through Celite, and the filtrate was concentrated, diluted with a small amount of DCM, and purified by TLC (Eluent System B) to give Compound 54a (45 mg).

MS (ESI, m/z): 470.3 [M+H]⁺.

### Step 2: 4-((5-amino-2-(1H-pyrazol-5-yl)thieno[3,2-b]pyridin-7-yl)amino)-1-cyclohexanol (Compound 114)

Compound 54a (45 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 4), and lyophilized to give Compound 114 (15 mg).

MS (ESI, m/z): 330.2 [M+H]⁺.

¹H NMR (CD₃OD, 400 MHz) δ 7.70 (d, *J* = 2.2 Hz, 1H), 7.37 - 7.24 (m, 1H), 6.67 (t, *J* = 2.5 Hz, 1H), 5.84 (d, *J* = 10.0 Hz, 1H), 3.70 - 3.56 (m, 1H), 3.50 - 3.40 (m, 1H), 2.21 - 1.92 (m, 4H), 1.55 - 1.31 (m, 4H).

### Example 51:

3-((5-amino-2-(1H-pyrazol-5-yl)thieno[3,2-b]pyridin-7-yl)amino)-2,2-dimethyl-1-propanol hydrochloride (Compound 115s)

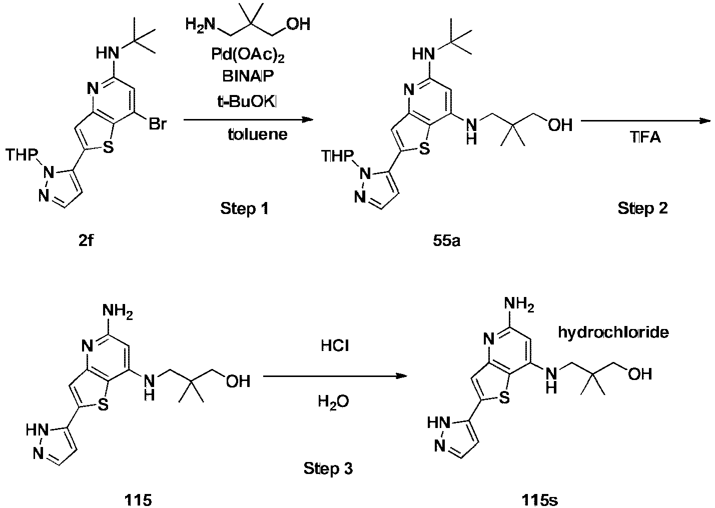

### Step 1: Synthesis of 3-((5-(tert-butylamino)-2-(1-(tetrahydro-2H-pyran-2-yl)-1H-pyrazol-5-yl)thieno[3,2-b]pyridin-7-yl)amino)-2,2-dimethyl-1-propanol (Compound 55a)

Compound 2f (70 mg), 3-amino-2,2-dimethyl-1-propanol (49.76 mg), Pd(OAc)₂ (3.61 mg), BINAP (20.02 mg) and t-BuOK (54.12 mg) were added to toluene (7 mL), and the reaction proceeded at an elevated temperature of 120°C for 5 hours under N₂ protection. The system was filtered. The filtrate was concentrated, diluted with a small amount of methanol, and purified by preparative TLC (Eluent System A) to give Compound 55a (34 mg).

MS (ESI, m/z): 458.2[M+H]⁺.

### Step 2: Synthesis of 3-((5-amino-2-(1H-pyrazol-5-yl)thieno[3,2-b]pyridin-7-yl)amino)-2,2-dimethyl-1-propanol (Compound 115)

Compound 55a (34 mg) was added to TFA (4 mL), and the reaction proceeded at 70°C for 2 hours. The system was concentrated, diluted with methanol, and adjusted to pH = 9 with a saturated sodium bicarbonate aqueous solution. The system was suction filtrated. The filtrate was separated and purified by Prep-HPLC (Elution Condition 4), and lyophilized to give Compound 115 (14 mg).

MS (ESI, m/z): 318.1 [M+H]⁺.

¹H NMR (DMSO-*d₆*, 400 MHz) δ 13.00 (s, 1H), 7.78 (s, 1H), 7.28 (s, 1H), 6.70 (s, 1H), 5.99 - 5.81 (m, 1H), 5.72 (s, 1H), 5.46 (s, 2H), 4.86 (s, 1H), 3.28 (s, 2H), 3.06 (d, *J=* 5.7 Hz, 2H), 0.90 (s, 6H).

### Step 3: Synthesis of 3-((5-amino-2-(1H-pyrazol-5-yl)thieno[3,2-b]pyridin-7-yl)amino)-2,2-dimethyl-1-propanol hydrochloride (Compound 115s)

Compound 115 (105 mg) was added to water (15 mL), and concentrated hydrochloric acid was added dropwise until the system was clear. The system was stirred at 25°C for 15 minutes, and then lyophilized to give Compound 115s (115 mg).

MS (ESI, m/z): 318.1 [M+H]⁺.

¹H NMR (DMSO-*d₆*, 400 MHz) δ 13.23 (s, 1H), 7.89 (d, *J* = 2.4 Hz, 1H), 7.77 (t, *J* = 6.1 Hz, 1H), 7.51 (s, 1H), 7.28 (s, 2H), 6.87 (d, *J* = 2.3 Hz, 1H), 5.97 (s, 1H), 4.80 (s, 1H), 3.24 (s, 2H), 3.18 (d, *J* = 6.1 Hz, 2H), 0.90 (s, 6H).

### Example 52:

*N⁷*-(2,2-difluoroethyl)-2-(1H-pyrazol-5-yl)thieno[3,2-b]pyridine-5,7-diamine hydrochloride (Compound 116s)

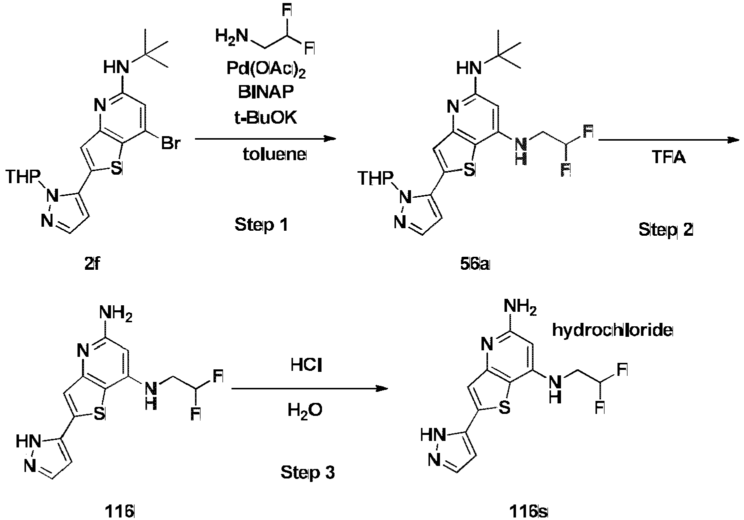

### Step 1: Synthesis of N⁵-(tert-butyl)-N⁷-(2,2-difluoroethyl)-2-(1-(tetrahydro-2H-pyran-2-yl)-1H-pyrazol-5-yl)thieno[3,2-b]pyridine-5,7-diamine (Compound 56a)

Compound 2f (70 mg), 2,2-difluoroethylamine (39.10 mg), Pd(OAc)₂ (3.61 mg), BINAP (20.02 mg) and t-BuOK (54.12 mg) were added to toluene (7 mL), and the reaction proceeded at an elevated temperature of 120°C for 5 hours under N₂ protection. The system was filtered through Celite, and the filtrate was concentrated, diluted with a small amount of methanol, and purified by TLC (Eluent System A) to give Compound 56a (40 mg).

MS (ESI, m/z): 436.3[M+H]⁺.

### Step 2: N⁷-(2,2-difluoroethyl)-2-(1H-pyrazol-5-yl)thieno[3,2-b]pyridine-5,7-diamine (Compound 116)

Compound 56a (40 mg) was added to TFA (4 mL), and the reaction proceeded at 70°C for 2 hours. The system was concentrated, diluted with methanol, and adjusted to pH = 9 with a saturated sodium bicarbonate aqueous solution. The system was suction filtrated. The filtrate was separated and purified by Prep-HPLC (Elution Condition 4), and lyophilized to give Compound 116 (20 mg).

MS (ESI, m/z): 296.1 [M+H]⁺.

¹H NMR (DMSO-*d₆*, 400 MHz) δ 12.99 (s, 1H), 7.79 (d, *J* = 2.3 Hz, 1H), 7.30 (s, 1H), 6.71 (d, *J* = 2.3 Hz, 1H), 6.49 (t, *J* = 6.2 Hz, 1H), 6.31 - 6.01 (m, 1H), 5.78 (s, 1H), 5.55 (s, 2H), 3.63 - 3.53 (m, 2H).

### Step 3: Synthesis of N⁷-(2,2-difluoroethyl)-2-(1H-pyrazol-5-yl)thieno[3,2-b]pyridine-5,7-diamine hydrochloride (Compound 116s)

Compound 116 (100 mg) was added to water (15 mL), and concentrated hydrochloric acid was added dropwise until the system was clear. The system was stirred at 25°C for 15 minutes, and then lyophilized to give Compound 116s (97 mg).

MS (ESI, m/z): 295.9 [M+H]⁺.

¹H NMR (DMSO-*d₆*, 400 MHz) δ 13.28 (s, 1H), 13.10 (s, 1H), 8.16 (t, *J* = 6.2 Hz, 1H), 7.91 (d, *J* = 2.4 Hz, 1H), 7.51 (s, 1H), 7.34 (s, 2H), 6.89 (d, *J=* 2.4 Hz, 1H), 6.27 (tt, *J* = 55.1, 3.6 Hz, 1H), 5.94 (s, 1H), 3.79 - 3.69 (m, 2H).

### Example 53:

*N*-(3-((5-amino-2-(1H-pyrazol-5-yl)thieno[3,2-b]pyridin-7-yl)amino)propyl)-*N-*methylcyclopropane formamide (Compound 76)

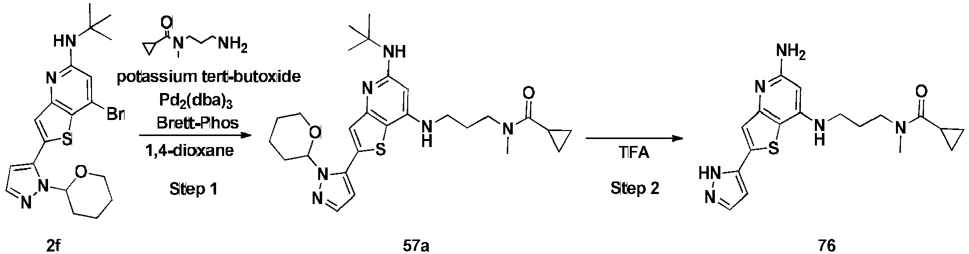

### Step 1: Synthesis of N-(3-((5-(tert-butylamino)-2-(1-(tetrahydro-2H-pyran-2-yl)-1H-pyrazol-5-yl)thieno[3,2-b)pyridin-7-yl)amino)propyl)-N-methylcyclopropane formamide (Compound 57a)

Compound 2f (70 mg), *N-*(3-aminopropyl)-*N-*methylcyclopropane formamide (29.64 mg), potassium tert-butoxide (90.21 mg), Pd₂(dba)₃ (29.42 mg) and BrettPhos (34.47 mg) were added to 1,4-dioxane (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 57a (40 mg)

MS (ESI, m/z): 511.2 [M+H]⁺.

### Step 2: Synthesis of N-(3-((5-amino-2-(1H-pyrazol-5-yl)thieno[3,2-b]pyridin-7-yl)amino)propyl)-N-methylcyclopropane formamide (Compound 76)

Compound 57a (40 mg, 0.08 mmol) was added to TFA (2 mL), and the reaction proceeded at an elevated temperature of 75°C for 2 hours with stirring. The reaction solvent was removed by concentration under a reduced pressure to dryness. Separation and purification by Prep-HPLC (Elution Condition 3) and lyophilization gave Compound 76 (7 mg).

MS (ESI, m/z): 371.2 [M+H]⁺.

¹H NMR (DMSO-*d₆*, 400 MHz) δ 13.00 (s, 1H), 7.80 (s, 1H), 7.29 (s, 1H), 6.71 (s, 1H), 6.41-6.30 (m, 1H), 5.75 (m, 2H), 5.65 (m, 1H), 3.61-3.57 (m, 1H), 3.41-3.38 (m, 2H), 3.21-3.08 (m, 4H), 1.98-1.87 (m, 2H), 1.82-1.75 (m, 1H), 0.74-0.57 (m, 4H).

### Example 54:

*N*⁷-(2-((2S,6R)-2,6-dimethylmorpholino)ethyl)-2-(1H-pyrazol-5-yl)thieno[3,2-b]pyridine-5,7-diamine (Compound 117)

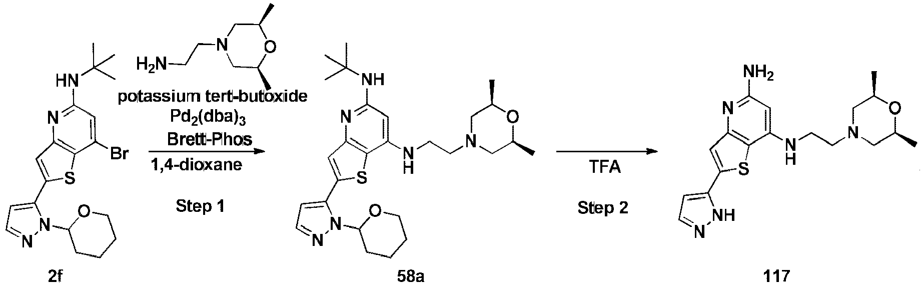

### Step 1: Synthesis of N⁵-(tert-butyl)-N⁷-(2-((2S,6R)-2,6-dimethylmorpholino)ethyl)-2-(1-(tetrahydro-2H-pyran-2-yl)-1H-pyrazol-5-yl)thieno[3,2-b]pyridine-5,7-diamine (Compound 58a)

Compound 2f (350 mg), 2-((2S,6R)-2,6-dimethylmorpholino)ethylamine (381.63 mg), potassium tert-butoxide (270.62 mg), Pd₂(dba)₃ (147.23 mg) and BrettPhos (172.60 mg) were added to 1,4-dioxane (20 mL), and the reaction proceeded at an elevated temperature of 120°C for 4 hours under N₂ protection. The reaction solvent was removed by drying in a rotary dryer, and the subsequent purification by flash column chromatography (Eluent System A) gave Compound 58a (330 mg).

MS (ESI, m/z): 513.3 [M+H]⁺.

### Step 2: Synthesis of N⁷-(2-((2S,6R)-2,6-dimethylmorpholino)ethyl)-2-(1H-pyrazol-5-yl)thieno[3,2-b]pyridine-5,7-diamine (Compound 117)

Compound 58a (350 mg) was added to TFA (8 mL), and the reaction proceeded at an elevated temperature of 60°C for 2 hours with stirring. The reaction solvent was removed by concentration under a reduced pressure to dryness. Separation and purification by Prep-HPLC (Elution Condition 4) and lyophilization gave Compound 117 (130mg).

MS (ESI, m/z): 373.2 [M+H]⁺.

¹H NMR (DMSO-*d₆*, 400 MHz) δ 13.00 (s, 1H), 7.78 (s, 1H), 7.29 (s, 1H), 6.70 (d, *J* = 2.1 Hz, 1H), 5.94 (t, *J=* 5.3 Hz, 1H), 5.66 (s, 1H), 5.52 (s, 2H), 3.58 (dqd, *J=* 12.4, 6.1, 1.7 Hz, 2H), 3.27 (dd, *J=* 12.8, 6.3 Hz, 2H), 2.80 (d, *J* = 10.2 Hz, 2H), 2.54 (t, *J=* 6.9 Hz, 2H), 1.70 (t, *J*= 10.7 Hz, 2H), 1.06 (d, *J*= 6.3 Hz, 6H).

### Example 55:

N-(3-((5-amino-2-(1H-pyrazol-5-yl)thieno[3,2-b]pyridin-7-yl)amino)propyl)-*N*-methylacetamide (Compound 74)

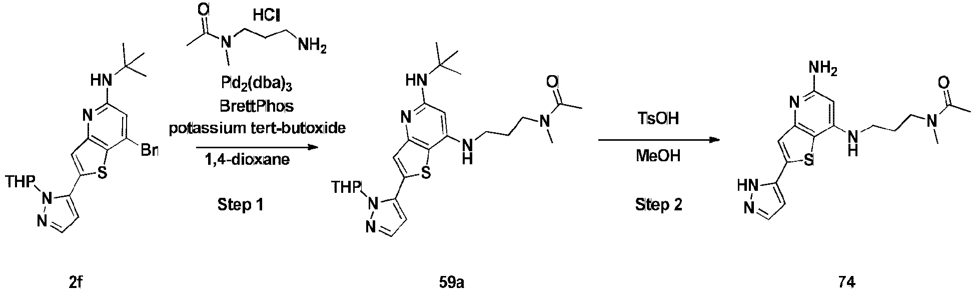

### Step 1: Synthesis of N-(3-((5-(tert-butylamino)-2-(1-(tetrahydro-2H-pyran-2-yl)-1H-pyrazol-5-yl)thieno[3,2-b]pyridin-7-yl)amino)propyl)-N-methylacetamide (Compound 59a)

Compound 2f (100 mg), *N*-(3-aminopropyl)-*N*-methyl-acetamide hydrochloride (153.11 mg), Pd₂(dba)₃ (42.07 mg), BrettPhos (49.24 mg) and t-BuOK (154.62 mg) were added to 1,4-dioxane (5 mL). After nitrogen replacement, the reaction proceeded at an elevated temperature of 120°C for 5 hours. The reaction liquid was cooled to room temperature and filtered. The filtrate was concentrated under a reduced pressure and purified by TLC (Eluent System B) to give Compound 59a (100 mg).

MS (ESI, m/z): 485.2 [M+H]⁺.

### Step 2: Synthesis of N-(3-((5-amino-2-(1H-pyrazol-5-yl)thieno[3,2-b]pyridin-7-yl)amino)propyl)-N-methylacetamide (Compound 74)

Compound 59a (120 mg) was added to a mixed system of DCM (5 mL) and TFA (5 mL), and the reaction proceeded at 25°C for 1 hour. The system was concentrated to dryness under a reduced pressure, and methanol (5 ml) was added. The system was adjusted to pH = 8 with potassium carbonate. Separation and purification by Prep-HPLC (Elution Condition 4) and lyophilization gave Compound 74 (22 mg).

MS (ESI, m/z): 345.1 [M+H]⁺.

¹H NMR (DMSO-*d₆*, 400 MHz) δ 12.98 (s, 1H), 7.79 (s, 1H), 7.28 (d, *J=* 1.3 Hz, 1H), 6.70 (d, *J=* 2.2 Hz, 1H), 6.20-6.10 (m, 1H), 5.64 (d, *J=* 4.4 Hz, 1H), 5.52-5.49 (m, 2H), 3.42 - 3.36 (m, 2H), 3.12 (td, *J=* 12.2, 6.6 Hz, 2H), 2.95 (s, 3H), 1.99 (s, 3H), 1.92 - 1.73 (m, 2H).

### Example 56:

1-((5-amino-2-(1H-pyrazol-5-yl)thieno[3,2-b]pyridin-7-yl)amino)methyl)cyclopropane formonitrile (Compound 119)

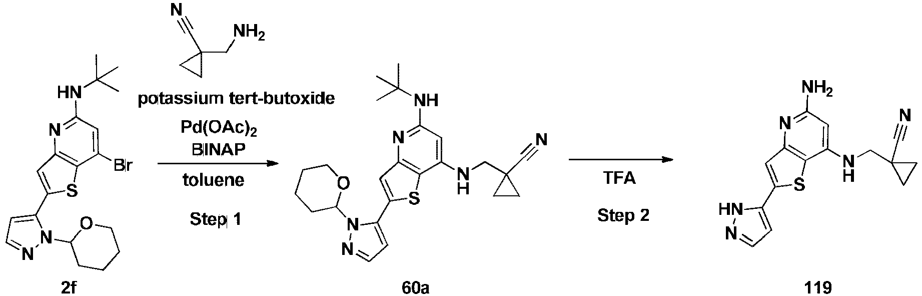

### Step 1: Synthesis of 1-((5-(tert-butylamino)-2-(1-(tetrahydro-2H-pyran-2-yl)-1H-pyrazol-5-yl)thieno[3,2-b]pyridin-7-yl)amino)methyl)cyclopropane formonitrile (Compound 60a)

Compound 2f (70 mg), 1-aminomethylcyclopropane formonitrile hydrochloride (25.08 mg), potassium tert-butoxide (54.12 mg), Pd(OAc)₂ (7.22 mg) and BINAP (40.04 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 60a (40 mg).

MS (ESI, m/z): 451.2 [M+H]⁺.

### Step 2: Synthesis of 1-((5-amino-2-(1H-pyrazol-5-yl)thieno[3,2-b]pyridin-7-yl)amino)methyl)cyclopropane formonitrile (Compound 119)

Compound 60a (40 mg) was added to TFA (2 mL), and the reaction proceeded at an elevated temperature of 75°C for 2 hours with stirring. The reaction solvent was removed by concentration under a reduced pressure to dryness. Separation and purification by Prep-HPLC (Elution Condition 4) and lyophilization gave Compound 119 (3 mg).

MS (ESI, m/z): 311.2 [M+H]⁺.

¹H NMR (DMSO-*d₆*, 400 MHz) δ 12.98 (s, 1H), 7.79 (s, 1H), 7.29 (s, 1H), 6.71 (d, *J* = 1.6 Hz, 1H), 6.53-6.50 (m, 1H), 5.76 (s, 1H), 5.54 (s, 2H), 3.40 (d, *J* = 6Hz, 2H), 1.25-1.22 (m, 2H), 1.11-1.08 (m, 2H).

### Example 57:

2-(1H-pyrazol-5-yl)-*N⁷*-((tetrahydro-2H-pyran-4-yl)methyl)thieno[3,2-b]pyridine-5,7-diamine hydrochloride (Compound 120s)

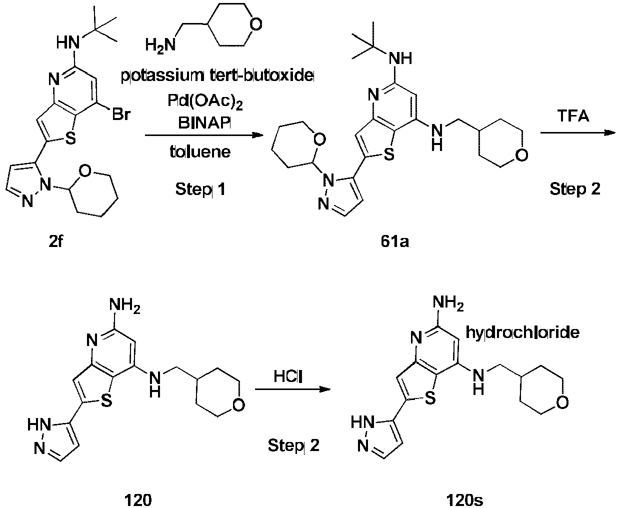

### Step 1: Synthesis of N⁵-(tert-butyl)-2-(1-(tetrahydro-2H-pyran-2-yl)-1H-pyrazol-5-yl)-N⁷-((tetrahydro-2H-pyran-4-yl)methyl)thieno[3,2-b]pyridine-5,7-diamine (Compound 61a)

Compound 2f (700 mg), 4-aminomethyltetrahydropyran (218.8 mg), potassium tert-butoxide (541.2 mg), Pd(OAc)₂ (72.2 mg) and BINAP (400.4 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 61a (300 mg).

MS (ESI, m/z): 470.1 [M+H]⁺.

### Step 2: 2-(1H-pyrazol-5-yl)-N⁷-((tetrahydro-2H-pyran-4-yl)methyl)thieno[3,2-b]pyridine-5,7-diamine hydrochloride (Compound 120s)

Compound 61a (300 mg, 0.6 mmol) was added to TFA (20 mL), and the reaction proceeded at an elevated temperature of 70°C for 2 hours with stirring. The reaction solvent was removed by concentration under a reduced pressure to dryness. Separation and purification by Prep-HPLC (Elution Condition 4) and lyophilization gave Compound 120.

¹H NMR (DMSO-*d₆*, 400 MHz) δ 12.94 (s, 1H), 7.80 (s, 1H), 7.26 (s, 1H), 6.70 (s, 1H), 6.25 (s, 1H), 5.65 (s, 1H), 5.46 (s, 2H), 3.88-3.84 (m, 2H), 3.31-3.24 (m, 2H), 3.05-3.02 (m, 2H), 1.94-1.88 (m, 1H), 1.68-1.64 (m, 2H), 1.27-1.16 (m, 2H).

Compound 120 was added to 1 N hydrochloric acid. The system was lyophilized again to give Compound 120s (55 mg).

MS (ESI, m/z): 330.1 [M+H]⁺.

¹H NMR (DMSO-*d₆*, 400 MHz) δ 13.42 (s, 1H), 8.09 (s, 1H), 7.88 (s, 1H), 7.52 (s, 1H), 6.88 (s, 1H), 5.92 (s, 1H), 3.88-3.84 (m, 2H), 3.31-3.24 (m, 2H), 3.17-3.14 (m, 2H), 1.98-1.92 (m, 1H), 1.68-1.64 (m, 2H), 1.30-1.20 (m, 2H).

### Example 58:

*N⁷*-((3-methyloxetan-3-yl)methyl)-2-(1H-pyrazol-5-yl)thieno[3,2-b]pyridine-5,7-diamine (Compound 121)

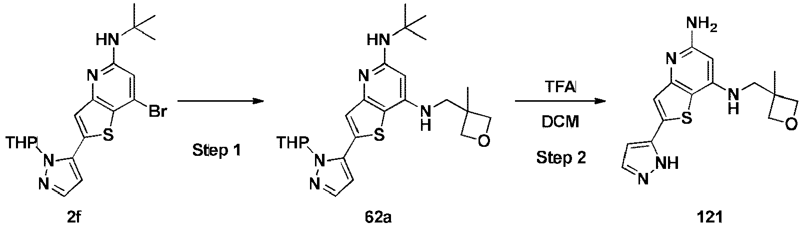

### Step 1: Synthesis of N⁵-(tert-butyl)-N⁷-((3-methyloxetan-3-yl)methyl)-2-(1-(tetrahydro-2H-pyran-2-yl)-1H-pyrazol-5-yl)thieno[3,2-b]pyridine-5,7-diamine (Compound 62a)

3-Methyl-3-aminomethyl-1-oxetane (93 mg), Compound 2f (200 mg), Brettphos (49.31 mg), Pd₂(dba)₃ (42.07 mg) and potassium tert-butoxide (154.64 mg) were added to 1,4-dioxane (8 mL). The reaction proceeded at an elevated temperature of 110°C for 10 hours with stirring under N₂ protection. The system was cooled and filtered. The filtrate was concentrated, purified and separated by TLC (Eluent System B) to give Compound 62a (40 mg).

MS (ESI, m/z): 456.2[M+H]⁺.

### Step 2: Synthesis of N⁷-((3-methyloxetan-3-yl)methyl)-2-(1H-pyrazol-5-yl)thieno[3,2-b]pyridine-5,7-diamine (Compound 121)

Compound 62a (40 mg) was added to DCM (2 mL) and TFA (2 mL), and stirred at 25 for 10 hours. The reaction solvent was removed by concentration under a reduced pressure to dryness, and the mixture was dissolved again with methanol. The system was adjusted to pH 8-9 with a saturated sodium bicarbonate aqueous solution. The system was filtrated. The filtrate was separated and purified by Prep-HPLC (Elution Condition 4), and lyophilized to give Compound 121 (5 mg).

MS (ESI, m/z): 316.1 [M+H]⁺.

¹HNMR (CD₃OD-*d4*, 400 MHz) δ 7.77 (d, *J* = 2.0 Hz, 1H), 7.43 (s, 1H), 6.76 (d, *J* = 2.4 Hz, 1H), 5.91 (s, 1H), 4.61 (d, *J=* 6.0 Hz, 2H), 4.43(d, *J=* 6.0 Hz, 2H), 3.59 (s, 2H),1.43 (s, 3H).

### Example 59:

2-((5-amino-2-(1H-pyrazol-5-yl)thieno[3,2-b]pyridin-7-yl)amino)methyl)-2-methylpropan-1,3-diol (Compound 122)

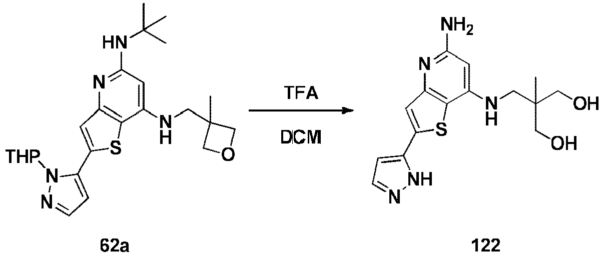

Compound 62a (40 mg) was added to DCM (2 mL) and TFA (2 mL), and stirred at 25 for 10 hours. The reaction solvent was removed by concentration under a reduced pressure to dryness. The residual was dissolved again with methanol, and was adjusted to pH 8-9 with a saturated sodium bicarbonate aqueous solution. The system was filtrated. The filtrate was separated and purified by Prep-HPLC (Elution Condition 4) to give Compound 122 (10 mg).

MS (ESI, m/z): 334.1 [M+H]⁺.

¹HNMR (DMSO-*d₆*, 400 MHz) δ 12.96 (s, 1H), 7.79 (s, 1H), 7.29 (s, 1H), 6.70 (s, 1H), 5.87-5.90 (m, 1H), 5.70 (s, 1H), 5.48 (s, 2H), 4.72-4.75 (m, 2H), 3.32-3.41 (m, 4H), 3.11-3.12 (m, 2H), 0.86 (s, 3H).

### Example 60:

3-((5-amino-2-(1H-pyrazol-5-yl)thieno[3,2-b]pyridin-7-yl)amino)-3 -phenyl-1-propanol (Compound 123)

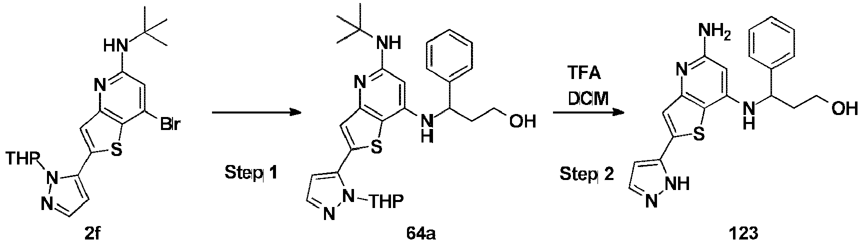

### Step 1: Synthesis of 3-((5-(tert-butylamino)-2-(1-(tetrahydro-2H-pyran-2-yl)-1H-pyrazol-5-yl)thieno[3,2-b]pyridin-7-yl)amino)-3-phenyl-1-propanol (Compound 64a)

3-Amino-3-phenyl-1-propanol (139 mg), Compound 2f (200 mg), Brettphos (49.31 mg), Pd₂(dba)₃ (42.07 mg) and potassium tert-butoxide (154.64 mg) were added to 1,4-dioxane (8 mL) The reaction proceeded at an elevated temperature of 110°C for 10 hours with stirring under N₂ protection. The system was cooled and filtered. The filtrate was concentrated, purified and separated by TLC (Eluent System B) to give Compound 64a (50 mg).

MS (ESI, m/z): 506.3[M+H]⁺.

### Step 2: Synthesis of 3-((5-amino-2-(1H-pyrazol-5-yl)thieno[3,2-b]pyridin-7-yl)amino)-3-phenyl-1-propanol (Compound 123)

Compound 64a (50 mg) was added to DCM (2 mL) and TFA (2 mL), and stirred at 25°C for 10 hours. The reaction solvent was removed by concentration under a reduced pressure to dryness. The residual was dissolved again with methanol, and was adjusted to pH 8-9 with a saturated sodium bicarbonate aqueous solution. The system was filtrated. The filtrate was separated and purified by Prep-HPLC (Elution Condition 4), to give Compound 123 (5 mg).

MS (ESI, m/z): 366.1 [M+H]⁺.

¹HNMR (DMSO-*d₆*, 400 MHz) δ 12.97 (s, 1H), 7.82 (s, 1H), 7.33-7.35 (m, 1H), 7.24-7.25 (m, 2H), 7.19-7.20 (m, 1H), 7.17-7.18 (m, 1H), 6.71 (s, 1H), 6.62-6.63 (m, 1H), 5.48 (s, 1H), 5.38-5.39 (m, 2H), 4.67-4.69 (m, 2H), 3.50-3.52 (m, 1H), 3.44-3.48 (m, 1H), 2.57-2.58 (m, 0.5H), 2.32-2.33 (m, 0.5H), 2.06-2.10 (m, 1H), 1.87-1.90 (m, 1H).

### Example 61:

*N*⁷-(2-((2R,6R)-2,6-dimethylmorpholino)ethyl)-2-(1H-pyrazol-5-yl)thieno[3,2-b]pyridine-5,7-diamine (Compound 124)

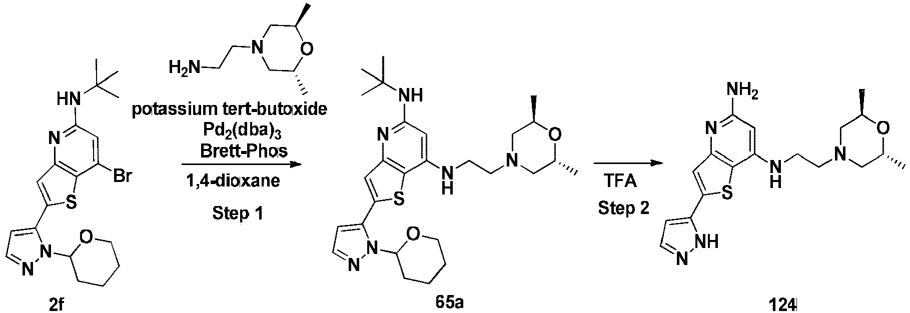

### Step 1: Synthesis of N⁵-(tert-butyl)-N⁷-(2-((2R,6R)-2,6-dimethylmorpholino)ethyl)-2-(1-(tetrahydro-2H-pyran-2-yl)-1H-pyrazol-5-yl)thieno[3,2-b]pyridine-5,7-diamine (Compound 65a)

Compound 2f (300 mg), 2-((2R,6R)-2,6-dimethylmorpholino)ethylamine (327.11 mg), potassium tert-butoxide (231.96 mg), Pd₂(dba)₃ (126.20 mg) and BrettPhos (147.94 mg) were added to 1,4-dioxane (15 mL), and the reaction proceeded at an elevated temperature of 120°C for 4 hours under N₂ protection. The reaction solvent was removed by drying in a rotary dryer, and the subsequent purification by flash column chromatography (Eluent System A) gave Compound 65a (235 mg).

MS (ESI, m/z): 513.3 [M+H]⁺.

### Step 2: Synthesis of N⁷-(2-((2R,6R)-2,6-dimethylmorpholino)ethyl)-2-(1H-pyrazol-5-yl)thieno[3,2-b]pyridine-5,7-diamine (Compound 124)

Compound 65a (235 mg) was added to TFA (8 mL), and the reaction proceeded at an elevated temperature of 60°C for 2 hours with stirring. The reaction solvent was removed by concentration under a reduced pressure to dryness. Separation and purification by Prep-HPLC (Elution Condition 4) and lyophilization gave Compound 124 (80mg).

MS (ESI, m/z): 373.2 [M+H]⁺.

¹H NMR (DMSO-*d₆*, 400 MHz) δ 13.00 (s, 1H), 7.79 (s, 1H), 7.30 (s, 1H), 6.71 (s, 1H), 5.80 (t, *J* = 4.9 Hz, 1H), 5.68 (s, 1H), 5.55 (s, 2H), 3.96 - 3.88 (m, 2H), 3.26 (dd, *J=* 12.1, 6.4 Hz, 2H), 2.60 - 2.51 (m, 2H), 2.50 - 2.43 (m, 2H), 2.16 (dd, *J* = 10.9, 5.7 Hz, 2H), 1.15 (d, *J* = 6.4 Hz, 6H).

### Example 62:

(S)-3-((5-amino-2-(1H-pyrazol-5-yl)thieno[3,2-b]pyridin-7-yl)amino)butanol hydrochloride (Compound 125s)

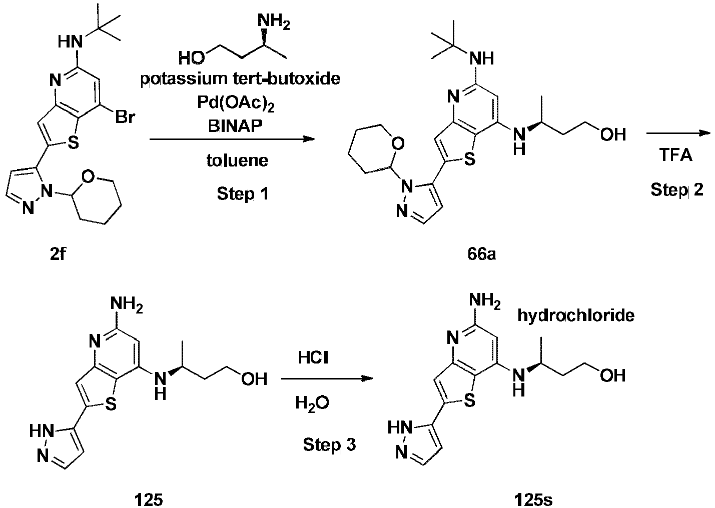

### Step 1: Synthesis of (3S)-3-((5-(tert-butylamino)-2-(1-(tetrahydro-2H-pyran-2-yl)-1H-pyrazol-5-yl)thieno(3,2-b]pyridin-7-yl)amino)butanol (Compound 66a)

Compound 2f (70 mg), (S)-3-aminobutanol (57.32 mg), potassium tert-butoxide (54.12 mg), Pd(OAc)₂ (7.22 mg) and BINAP (40.04 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 66a (30 mg).

MS (ESI, m/z): 444.2 [M+H]⁺.

### Step 2: Synthesis of (S)-3-((5-amino-2-(1H-pyrazol-5-yl)thieno[3,2-b]pyridin-7-yl)amino)butanol (Compound 125)

Compound 66a (30 mg) was added to TFA (2 mL), and the reaction proceeded at an elevated temperature of 60°C for 2 hours with stirring. The reaction solvent was removed by concentration under a reduced pressure to dryness. Separation and purification by Prep-HPLC (Elution Condition 4) and lyophilization gave Compound 125 (10 mg).

MS (ESI, m/z): 304.1 [M+H]⁺.

¹H NMR (DMSO-*d₆*, 400 MHz) δ 12.95 (s, 1H), 7.80 (s, 1H), 7.27 (s, 1H), 6.70 (s, 1H), 5.84 (d, *J=* 8.0 Hz, 1H), 5.67 (s, 1H), 5.45 (s, 2H), 4.52 (t, *J=* 4.8 Hz, 1H), 3.76 - 3.64 (m, 1H), 3.58 - 3.46 (m, 2H), 1.82 (td, *J=* 13.2, 6.1 Hz, 1H), 1.64 (td, *J* = 13.0, 6.3 Hz, 1H), 1.19 (d, *J* = 6.4 Hz, 3H).

### Step 3: Synthesis of (S)-3-((5-amino-2-(1H-pyrazol-5-yl)thieno[3,2-b]pyridin-7-yl)amino)butanol hydrochloride (Compound 125s)

Compound 125 (30 mg) was added to water (5 mL), and concentrated hydrochloric acid was added dropwise until the system was clear. The system was stirred at 25°C for 15 minutes, and then lyophilized to give Compound 125s (28 mg).

MS (ESI, m/z): 304.1 [M+H]⁺.

¹H NMR (DMSO-*d₆*, 400 MHz) δ 13.27 (s, 1H), 12.89 (s, 1H), 7.89 (d, *J* = 2.2 Hz, 1H), 7.64 (d, *J=* 7.9 Hz, 1H), 7.47 (s, 1H), 7.21 (s, 2H), 6.86 (d, *J=* 2.3 Hz, 1H), 5.83 (s, 1H), 3.86 - 3.76 (m, 1H), 3.51 (d, *J=* 6.0 Hz, 2H), 1.85 (td, *J* = 13.2, 5.7 Hz, 1H), 1.69 (td, *J* = 12.9, 6.4 Hz, 1H), 1.24 (d, *J* = 6.4 Hz, 3H).

### Example 63:

*N*⁷-(cyclobutylmethyl)-2-(1H-pyrazol-5-yl)thieno[3,2-b]pyridine-5,7-diamine (Compound 126)

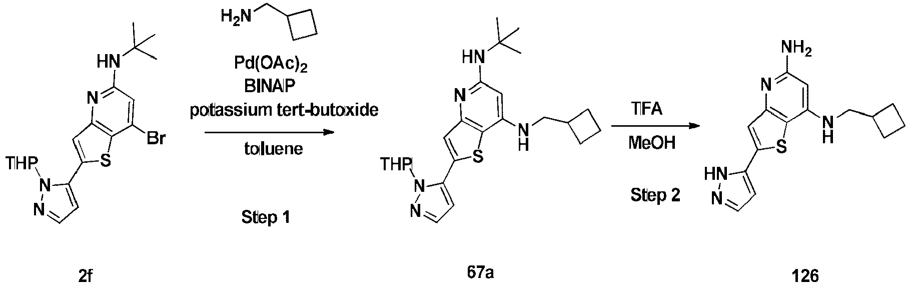

### Step 1: Synthesis of N⁵-(tert-butyl)-N⁷-(cyclobutylmethyl)-2-(1-(tetrahydro-2H-pyran-2-yl)-1H-pyrazol-5-yl)thieno[3,2-b)pyridine-5,7-diamine (Compound 67a)

Compound 2f (100 mg), cyclobutyl methylamine (78.23 mg), Pd(OAc)₂ (5.16 mg), BINAP (28.60 mg) and t-BuOK (77.31 mg) were added to toluene (10 mL). After nitrogen replacement, the reaction proceeded at an elevated temperature of 120°C for 5 hours under N₂ protection. The reaction liquid was cooled to room temperature and filtered. The filtrate was concentrated under a reduced pressure and purified by TLC (Eluent System B) to give Compound 67a (90 mg).

MS (ESI, m/z): 440.2 [M+H]⁺.

### Step 2: Synthesis of N⁷-(cyclobutylmethyl)-2-(1H-pyrazol-5-yl)thieno[3,2-b]pyridine-5,7-diamine (Compound 126)

Compound 67a (90 mg) was added to a mixed system of DCM (5 mL) and TFA (5 mL), and the reaction proceeded at 25°C for 1 hour. The reaction liquid was concentrated to dryness under a reduced pressure, and methanol (5 mL) was added. The system was adjusted to pH = 8 with potassium carbonate, separated and purified by Prep-HPLC (Elution Condition 4), and lyophilized to give Compound 126 (20 mg).

MS (ESI, m/z): 300.1 [M+H]⁺.

¹H NMR (DMSO-*d₆*, 400 MHz) δ 12.97 (s, 1H), 7.78 (s, 1H), 7.26 (s, 1H), 6.69 (d, *J* = 2.1 Hz, 1H), 6.16 (t, *J* = 5.5 Hz, 1H), 5.63 (s, 1H), 5.47 (s, 2H), 3.22 - 3.14 (m, 2H), 2.65 (dt, *J* = 14.9, 7.6 Hz, 1H), 2.09 - 2.00 (m, 2H), 1.91 - 1.82 (m, 2H), 1.78 - 1.67 (m, 2H).

### Example 64:

3-((5-amino-2-(1H-pyrazol-5-yl)thieno[3,2-b]pyridin-7-yl)(methyl)amino)-1-propanol (Compound 127)

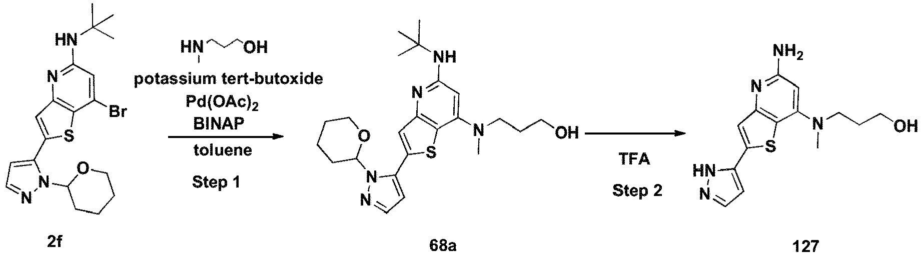

### Step 1: Synthesis of 3-((5-(tert-butylamino)-2-(1-(tetrahydro-2H-pyran-2-yl)-1H-pyrazol-5-yl)thieno[3,2-b]pyridin-7-yl)(methyl)amino)-1-propanol (Compound 68a)

Compound 2f (70 mg), 3-methylamino-1-propanol (16.91 mg), potassium tert-butoxide (54.12 mg), Pd(OAc)₂ (7.22 mg) and BINAP (40.04 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 68a (30 mg).

MS (ESI, m/z): 444.1 [M+H]⁺.

### Step 2: Synthesis of 3-((5-amino-2-(1H-pyrazol-5-yl)thieno[3,2-b]pyridin-7-yl)(methyl)amino)-1-propanol (Compound 127)

Compound 68a (30 mg) was added to TFA (2 mL), and the reaction proceeded at an elevated temperature of 75°C for 2 hours with stirring. The reaction solvent was removed by concentration under a reduced pressure to dryness. Separation and purification by Prep-HPLC (Elution Condition 3) and lyophilization gave Compound 127 (3 mg).

MS (ESI, m/z): 304.1 [M+H]⁺.

¹H NMR (DMSO-*d₆*, 400 MHz) δ 8.44 (s, 2H), 7.80 (s, 1H), 7.30 (s, 1H), 6.72 (s, 1H), 6.15 (d, *J* = 8.0 Hz, 1H), 5.98 (s, 2H), 5.71 (s, 1H), 3.76-3.70 (m, 1H), 3.58-3.48 (m, 2H), 1.87-1.80 (m, 1H), 1.69-1.63 (m, 1H), 1.21 (d, *J=* 6.3 Hz, 3H).

### Example 65:

*N*⁷-((1R,4R)-4-methylcyclohexyl)-2-(1H-pyrazol-5-yl)thieno[3,2-b]pyridine-5,7-diamine (Compound 128)

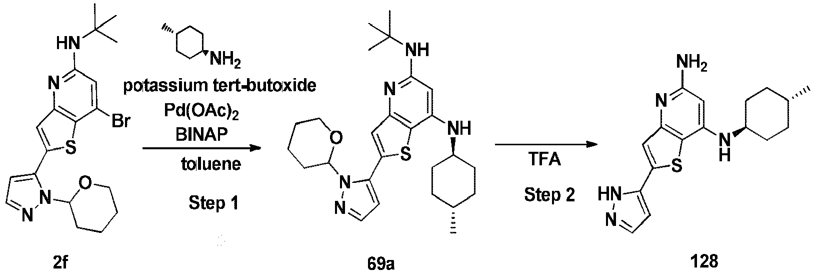

### Step 1: Synthesis of N⁵-(tert-butyl)-N⁷-((1R,4R)-4-methylcyclohexyl)-2-(1-(tetrahydro-2H-pyran-2-yl)-1H-pyrazol-5-yl)thieno[3,2-b]pyridine-5,7-diamine (Compound 69a)

Compound 2f (70 mg), trans 4-methylcyclohexylamine hydrochloride (28.43 mg), potassium tert-butoxide (54.12 mg), Pd(OAc)₂ (7.22 mg) and BINAP (40.04 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 69a (30 mg).

MS (ESI, m/z): 468.1 [M+H]⁺.

### Step 2: Synthesis of N⁷-((1R,4R)-4-methylcyclohexyl)-2-(1H-pyrazol-5-yl)thieno[3,2-b]pyridine-5,7-diamine (Compound 128)

Compound 69a (30 mg) was added to TFA (2 mL), and the reaction proceeded at an elevated temperature of 75°C for 2 hours with stirring. The reaction solvent was removed by concentration under a reduced pressure to dryness. Separation and purification by Prep-HPLC (Elution Condition 4) and lyophilization gave Compound 128 (4 mg).

MS (ESI, m/z): 328.1 [M+H]⁺.

¹H NMR (DMSO-*d₆*, 400 MHz) δ 13.02 (s, 1H), 7.82 (s, 1H), 7.30 (s, 1H), 6.73 (s, 1H), 6.26 (s, 1H), 5.84 (s, 2H), 5.71 (s, 1H), 1.98-1.94 (m, 2H), 1.76-1.71 (m, 2H), 1.41-1.31 (m, 3H), 1.09-0.98 (m, 2H), 0.91 (d, *J* = 6.4 Hz, 3H).

### Example 66:

(S)-2-((5-amino-2-(1H-pyrazol-5-yl)thieno[3,2-b]pyridin-7-yl)amino)propanol (Compound 108)

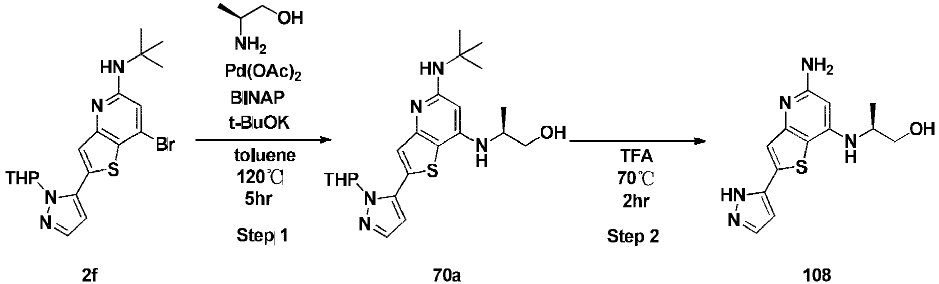

### Step 1: Synthesis of (2S)-2-((5-(tert-butylamino)-2-(1-(tetrahydro-2H-pyran-2-yl)-1H-pyrazol-5-yl)thieno[3,2-b]pyridin-7-yl)amino)propanol (Compound 70a)

Compound 2f (70 mg), (S)-2-amino-1-propanol (36.23 mg), Pd(OAc)₂ (3.61 mg), BINAP (20.02 mg) and t-BuOK (54.12 mg) were added to toluene (7 mL), and the reaction proceeded at an elevated temperature of 120°C for 5 hours under N₂ protection. The system was filtered. The filtrate was concentrated, diluted with a small amount of methanol, and purified by TLC (Eluent System A) to give Compound 70a (48 mg).

MS (ESI, m/z): 430.2[M+H]⁺.

### Step 2: Synthesis of (S)-2-((5-amino-2-(1H-pyrazol-5-yl)thieno[3,2-b]pyridin-7-yl)amino)propanol (Compound 108)

Compound 70a (48 mg) was added to TFA (6 mL), and the reaction proceeded at 70°C for 2 hours. The system was concentrated, diluted with methanol, and was adjusted to pH = 9 with a saturated sodium bicarbonate aqueous solution. The system was suction filtrated. The filtrate was separated and purified by Prep-HPLC (Elution Condition 4), and lyophilized to give Compound 108 (20 mg).

MS (ESI, m/z): 290.1 [M+H]⁺.

¹H NMR (400 MHz, DMSO-*d₆*) δ 12.98 (s, 1H), 7.78 (d, *J* = 2.3 Hz, 1H), 7.28 (s, 1H), 6.69 (d, *J* = 2.3 Hz, 1H), 5.69 (s, 1H), 5.65 (d, *J* = 7.6 Hz, 1H), 5.48 (s, 2H), 4.82 (s, 1H), 3.59 - 3.50 (m, 2H), 3.41-3.36 (m, 1H), 1.18 (d, *J* = 6.3 Hz, 3H).

### Example 67:

(R)-2-((5-amino-2-(1H-pyrazol-5-yl)thieno[3,2-b]pyridm-7-yl)amino)butanol (Compound 95)

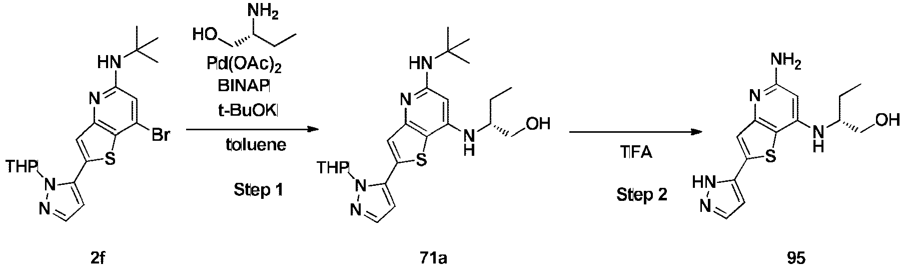

### Step 1: Synthesis of (2R)-2-((5-(tert-butylamino)-2-(1-(tetrahydro-2H-pyran-2-yl)-1H-pyrazol-5-yl)thieno(3,2-b]pyridin-7-yl)amino)butanol (Compound 71a)

Compound 2f (70 mg), (R)-2-amino-1-butanol (42.99 mg), Pd(OAc)₂ (3.61 mg), BINAP (20.02 mg) and t-BuOK (54.12 mg) were added to toluene (5 mL), and the reaction proceeded at an elevated temperature of 120°C for 5 hours under N₂ protection. The system was filtered. The filtrate was concentrated, diluted with a small amount of methanol, and purified by TLC (Eluent System A) to give Compound 71a (37 mg).

MS (ESI, m/z): 444.3[M+H]⁺.

### Step 2: Synthesis of (R)-2-((5-amino-2-(1H-pyrazol-5-yl)thieno[3,2-b]pyridin-7-yl)amino)butanol (Compound 95)

Compound 71a (37 mg) was added to TFA (4 mL), and the reaction proceeded at 70°C for 2 hours. The system was concentrated, diluted with methanol, and adjusted to pH = 9 with a saturated sodium bicarbonate aqueous solution. The system was suction filtrated. The filtrate was separated and purified by Prep-HPLC (Elution Condition 4), and lyophilized to give Compound 95 (12 mg).

MS (ESI, m/z): 304.1 [M+H]⁺.

¹H NMR (400 MHz, DMS0-d6) δ 12.98 (s, 1H), 7.78 (s, 1H), 7.27 (s, 1H), 6.69 (d, *J=* 2.3 Hz, 1H), 5.68 (s, 1H), 5.59 (d, *J*= 8.2 Hz, 1H), 5.45 (s, 2H), 4.75 (s, 1H), 3.58 - 3.49 (m, 1H), 3.47 - 3.36 (m, 2H), 1.75-1.64 (m, 1H), 1.58-1.47 (m, 1H), 0.92 (t, *J* = 7.4 Hz, 3H).

### Example 68:

1-(4-((5-amino-2-(1H-pyrazol-5-yl)thieno[3,2-b]pyridin-7-yl)amino)piperidin-1-yl)ethanone(Compound 92)

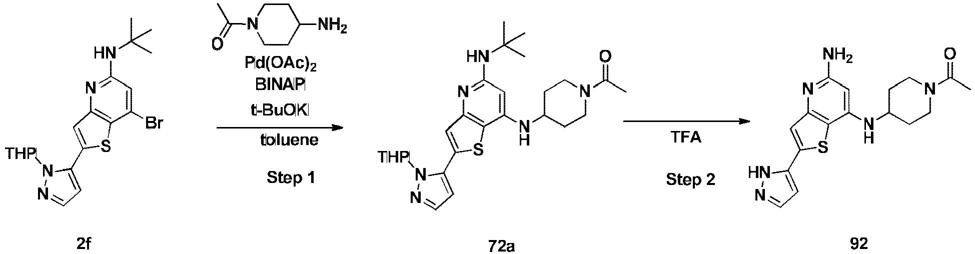

### Step 1: Synthesis of 1-(4-((5-(tert-butylamino)-2-(1-(tetrahydro-2H-pyran-2-yl)-1H-pyrazol-5-yl)thieno[3,2-b]pyridin-7-yl)amino)piperidin-1-yl)ethanone (Compound 72a)

Compound 2f (70 mg), 1-(4-aminopiperidin-1-yl)ethanone (68.59 mg), Pd(OAc)₂ (3.61 mg), BINAP (20.02 mg) and t-BuOK (54.12 mg) were added to toluene (5 mL), and the reaction proceeded at an elevated temperature of 120°C for 5 hours under N₂ protection. The system was filtered. The filtrate was concentrated, diluted with a small amount of methanol, and purified by TLC (Eluent System A) to give Compound 72a (65 mg).

MS (ESI, m/z):497.2[M+H]⁺.

### Step 2: Synthesis of 1-(4-((5-amino-2-(1H-pyrazol-5-yl)thieno[3,2-b]pyridin-7-yl)amino)piperidin-1-yl)ethanone (Compound 92)

Compound 72a (65 mg) was added to TFA (6 mL), and the reaction proceeded at 70°C for 2 hours. The system was concentrated, diluted with methanol, and adjusted to pH = 9 with a saturated sodium bicarbonate aqueous solution. The system was suction filtrated. The filtrate was separated and purified by Prep-HPLC (Elution Condition 4), and lyophilized to give Compound 92 (27 mg).

MS (ESI, m/z):357.2 [M+H].⁺

¹H NMR (400 MHz, DMSO-*d₆*) δ 12.95 (s, 1H), 7.78 (d, *J* = 2.3 Hz, 1H), 7.27 (s, 1H), 6.69 (d, *J* = 2.3 Hz, 1H), 5.95 (d, *J=* 7.8 Hz, 1H), 5.75 (s, 1H), 5.47 (s, 2H), 4.37 (d, *J=* 13.3 Hz, 1H), 3.87 (d, *J=* 14.1 Hz, 1H), 3.59 - 3.55 (m, 1H), 3.17 - 3.10 (m, 1H), 2.72-2.64 (m, 1H), 2.02 (s, 3H), 1.96 (t, *J* = 16.7 Hz, 2H), 1.53 - 1.44 (m, 1H), 1.40 - 1.33 (m, 1H).

### Example 69:

(S)-2-(1H-pyrazol-5-yl)-*N*⁷-(1-(3-(trifluoromethyl)phenyl)ethyl)thieno[3,2-b]pyridine-5,7-diamine (Compound 131)

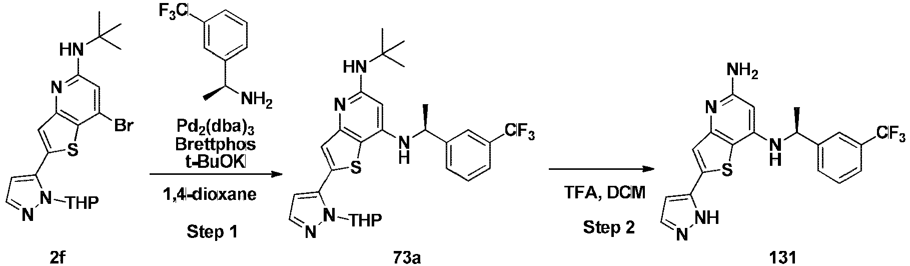

### Step 1: Synthesis of N⁵-(tert-butyl)-2-(1-(tetrahydro-2H-pyran-2-yl)-1H-pyrazol-5-yl)-N⁷-((S)-1-(3-(trifluoromethyl)phenyl)ethyl)thieno[3,2-b]pyridine-5,7-diamine (Compound 73a)

Compound 2f (88.99 mg), (S)-1-(3-(trifluoromethyl)phenyl)ethan-1-amine (77.34 mg), Pd₂(dba)₃ (5.88 mg), BrettPhos (10.97 mg) and t-BuOK (91.74 mg) were added to 1,4-dioxane (2 mL), and the reaction proceeded at an elevated temperature of 110°C for 5 hours under N₂ protection. The system was filtered. The filtrate was concentrated, diluted with a small amount of DCM, and purified by TLC (Eluent System B) to give Compound 73a (50 mg).

MS (ESI, m/z): 544.2 [M+H]⁺.

### Step 2: (S)-2-(1H-pyrazol-5-yl)-N⁷-(1-(3-(trifluoromethyl)phenyl)ethyl)thieno[3,2-b]pyridine-5,7-diamine (Compound 131)

Compound 73a (50 mg) was added to TFA (2 mL) and DCM (2 mL), and the reaction proceeded at 25°C for 12 hours. The system was concentrated and diluted with methanol, and adjusted to basic by adding 2 equivalents of NaOH. The system was suction filtrated. The filtrate was separated and purified by Prep-HPLC (Elution Condition 4), and lyophilized to give Compound 131 (9 mg).

MS (ESI, m/z): 404.1 [M+H]⁺.

¹H NMR (CD₃OD, 400 MHz) δ 7.77-7.62 (m, 3H), 7.52 (dd, *J* = 4.8, 1.8 Hz, 2H), 7.31 (s, 1H), 6.69 (d, *J* = 2.3 Hz, 1H), 5.54 (s, 1H), 1.63 (d, *J=* 6.9 Hz, 3H).

### Example 70:

(S)-2-((5-amino-2-(1H-pyrazol-5-yl)thieno[3,2-b]pyridin-7-yl)amino)-1-butanol (Compound 132)

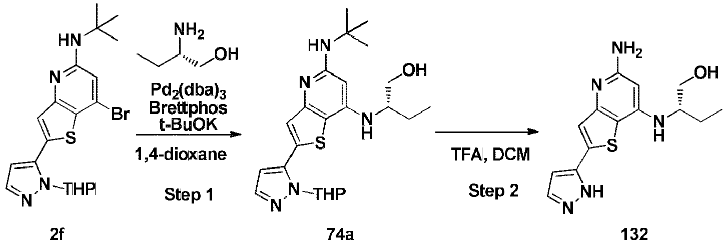

### Step 1: Synthesis of (2S)-2-((5-(tert-butylamino)-2-(1-(tetrahydro-2H-pyran-2-yl)-1H-pyrazol-5-yl)thieno(3,2-b]pyridin-7-yl)amino)-1-butanol (Compound 74a)

Compound 2f (80 mg), (2S)-2-amino-1-butanol (32.76 mg), Pd₂(dba)₃ (5.28 mg), BrettPhos (9.86 mg) and t-BuOK (82.47 mg) were added to 1,4-dioxane (2 mL), and the reaction proceeded at an elevated temperature of 110°C for 6 hours under N₂ protection. The system was filtered through Celite, and the filtrate was concentrated and then diluted with a small amount of DCM. Purification by TLC (Eluent System B) gave Compound 74a (30 mg).

MS (ESI, m/z): 444.2 [M+H]⁺.

### Step 2: Synthesis of (S)-2-((5-amino-2-(1H-pyrazol-5-yl)thieno[3,2-b]pyridin-7-yl)amino)-1-butanol (Compound 132)

Compound 74a (30 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 4), and lyophilized to give Compound 132 (2 mg).

MS (ESI, m/z): 304.2 [M+H]⁺.

¹H NMR (CD₃OD, 400 MHz) δ 7.70 (d, *J* = 2.3 Hz, 1H), 7.36 (d, *J* = 4.9 Hz, 1H), 6.69 (d, *J* = 2.4 Hz, 1H), 6.17 (s, 1H), 4.15 (dd, *J=* 9.4, 4.5 Hz, 1H), 4.01 (dd, *J=* 9.3, 6.9 Hz, 1H), 3.18 - 3.04 (m, 1H), 1.69 (qd, *J* = 13.5, 7.5 Hz, 1H), 1.54 (tt, *J* = 14.6, 7.4 Hz, 1H), 1.05 (t, *J* = 7.5 Hz, 3H).

### Example 71:

(R)-*N*⁷-(1-methoxyprop-2-yl)-2-(1H-pyrazol-5-yl)thieno[3,2-b]pyridine-5,7-diamine (Compound 133)

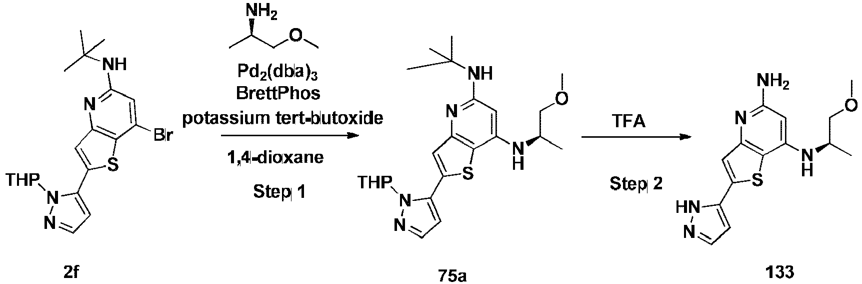

### Step 1: Synthesis of N⁵-(tert-butyl)-N⁷-((R)-1-methoxyprop-2-yl)-2-(1-(tetrahydro-2H-pyran-2-yl)-1H-pyrazol-5-yl)thieno[3,2-b]pyridine-5,7-diamine (Compound 75a)

Compound 2f (70 mg), (R)-1-methoxyprop-2-amine (71.7 mg), Pd₂(dba)₃ (30.3 mg), BrettPhos (30.3 mg) and potassium tert-butoxide (83 mg) were added to 1,4-dioxane (3 mL). After nitrogen replacement, the reaction proceeded at an elevated temperature of 120°C for 4 hours. The system was filtered. The filtrate was concentrated, then diluted with a small amount of methanol, and purified by TLC (Eluent System A) to give Compound 75a (25 mg).

MS (ESI, m/z): 444.2 [M+H]⁺.

### Step 2: Synthesis of (R)-N⁷-(1-methoxyprop-2-yl)-2-(1H-pyrazol-5-yl)thieno[3,2-b]pyridine-5,7-diamine (Compound 133)

Compound 75a (25 mg) was added to TFA (2 mL) and the reaction proceeded at 60°C for 1 hour. The system was concentrated, separated and purified by Prep-HPLC (Elution Condition 4), and lyophilized to give Compound 133 (10 mg).

MS (ESI, m/z): 304.1 [M+H]⁺.

¹H NMR (DMSO-*d₆*, 400 MHz) δ 13.25 (s, 1H), 12.89 (s, 1H), 7.91 (s, 1H), 7.64 (d, *J=* 8.0 Hz, 1H), 7.44 (s, 1H), 7.23 (s, 2H), 6.86 (s, 1H), 5.86 (s, 1H), 3.89 - 3.77 (m, 1H), 3.52 - 3.42 (m, 2H), 3.29 (s, 3H), 1.24 (s, 3H).

### Example 72:

*N*⁷-((3-methylpyridin-2-yl)methyl)-2-(1H-pyrazol-5-yl)thieno[3,2-b]pyridine-5,7-diamine (Compound 134)

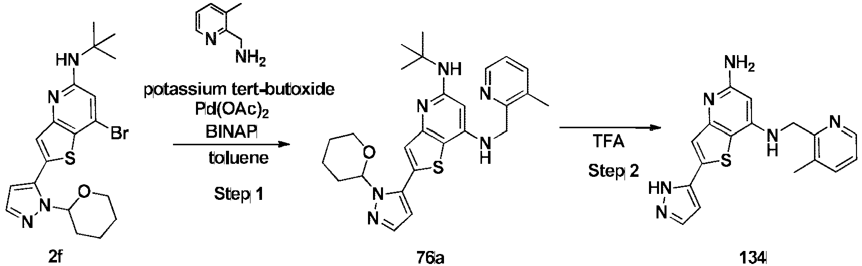

### Step 1: Synthesis of N⁵-(tert-butyl)-N⁷-((3-methylpyridin-2-yl)methyl)-2-(1-(tetrahydro-2H-pyran-2-yl)-1H-pyrazol-5-yl)thieno[3,2-b]pyridine-5,7-diamine (Compound 76a)

Compound 2f (70 mg), 2-aminomethyl-3-methylpyridine hydrochloride (30.14 mg), potassium tert-butoxide (54.12 mg), Pd(OAc)₂ (7.22 mg) and BINAP (40.04 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 76a (50 mg).

MS (ESI, m/z): 477.1 [M+H]⁺.

### Step 2: Synthesis of N⁷-((3-methylpyridin-2-yl)methyl)-2-(1H-pyrazol-5-yl)thieno[3,2-b]pyridine-5,7-diamine

### (Compound 134)

Compound 76a (30 mg) was added to TFA (2 mL), and the reaction proceeded at an elevated temperature of 75°C for 2 hours with stirring. The reaction solvent was removed by concentration under a reduced pressure to dryness. Separation and purification by Prep-HPLC (Elution Condition 4) and lyophilization gave Compound 134 (4 mg).

MS (ESI, m/z): 337.1 [M+H]⁺.

¹H NMR (DMSO-*d₆*, 400 MHz) δ 13.02 (s, 1H), 8.43 (s, 1H), 7.83 (s, 1H), 7.62 (s, 1H), 7.33-7.28 (m, 2H), 6.74 (s, 2H), 5.75 (m, 3H), 4.49 (s, 2H), 2.37 (s, 3H).

### Example 73:

1-(2-((5-amino-2-(1H-pyrazol-5-yl)thieno[3,2-b]pyridin-7-yl)amino)ethyl)pyrrolidin-2-one (Compound 135)

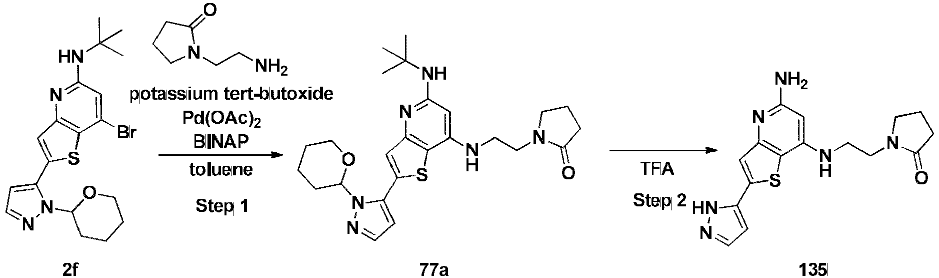

### Step 1: Synthesis of 1-(2-((5-(tert-butylamino)-2-(1-(tetrahydro-2H-pyran-2-yl)-1H-pyrazol-5-yl)thieno[3,2-b]pyridin-7-yl)amino)ethyl)pyrrolidin-2-one (Compound 77a)

Compound 2f (100 mg), 1-(2-aminoethyl)pyrrolidin-2-one (88.32 mg), potassium tert-butoxide (77.32 mg), Pd(OAc)₂ (10.31 mg) and BINAP (57.21 mg) were added to toluene (3 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 77a (50 mg).

MS (ESI, m/z): 483.2 [M+H]⁺.

### Step 2: Synthesis of 1-(2-((5-amino-2-(1H-pyrazol-5-yl)thieno[3,2-b]pyridin-7-yl)amino)ethyl)pymolidin-2-one (Compound 135)

Compound 77a (30 mg) was added to TFA (2 mL), and the reaction proceeded at an elevated temperature of 60°C for 2 hours with stirring. The reaction solvent was removed by concentration under a reduced pressure to dryness. Separation and purification by Prep-HPLC (Elution Condition 4) and lyophilization gave Compound 135 (12 mg).

MS (ESI, m/z): 343.1 [M+H]⁺.

¹H NMR (DMSO-*d₆*, 400 MHz) δ 12.75 (s, 1H), 7.79 (d, *J* = 2.2 Hz, 1H), 7.28 (s, 1H), 6.70 (d, *J* = 2.3 Hz, 1H), 6.21 (t, J= 5.5 Hz, 1H), 5.67 (s, 1H), 5.51 (s, 2H), 3.42 (d, *J=* 6.7 Hz, 4H), 3.32 - 3.27 (m, 2H)., 2.22 (t, *J=* 8.1 Hz, 2H), 1.96 - 1.87 (m, 2H).

### Example 74:

2-((5-amino-2-(1H-pyrazol-5-yl)thieno[3,2-b]pyridin-7-yl)amino)methyl)propane-1,3-diol (Compound136)

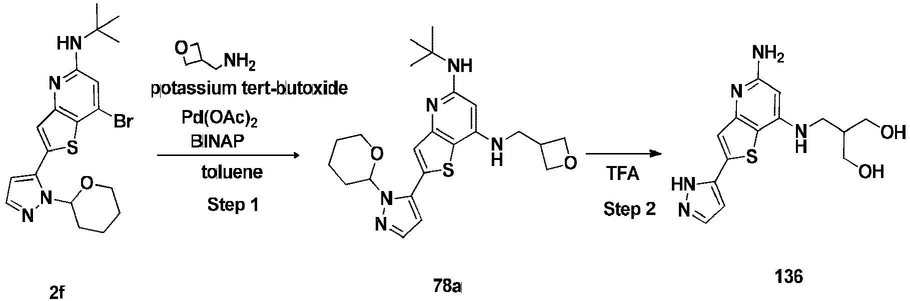

### Step 1: Synthesis of N⁵-(tert-butyl)-N⁷-(oxetan-3-ylmethyl)-2-(1-(tetrahydro-2H-pyran-2-yl)-1H-pyrazol-5-yl)thieno[3,2-b]pyridin-5,7-diamine (Compound 78a)

Compound 2f (100 mg), 3-aminomethyloxetane (24.01 mg), potassium tert-butoxide (77.32 mg), Pd(OAc)₂ (10.02 mg) and BINAP (57.21 mg) were added to toluene (3 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 78a (80 mg)

MS (ESI, m/z): 442.1 [M+H]⁺.

### Step 2: Synthesis of 2-((5-amino-2-(1H-pyrazol-5-yl)thieno[3,2-b]pyridin-7-yl)amino)methyl)propane-1,3-diol (Compound 136)

Compound 78a (30 mg) was added to TFA (2 mL), and the reaction proceeded at room temperature for 12 hours with stirring. The reaction solvent was removed by concentration under a reduced pressure to dryness. Separation and purification by Prep-HPLC (Elution Condition 4) and lyophilization gave Compound 136 (4 mg).

MS (ESI, m/z): 320.1 [M+H]⁺.

¹H NMR (DMS0-*d₆*, 400 MHz) δ 12.95 (s, 1H), 7.78 (s, 1H), 7.28 (s, 1H), 6.69 (s, 1H), 6.12-6.09 (m, 1H), 5.66 (s, 1H), 5.47 (s, 2H), 4.57 (s, 2H), 3.55-3.45 (m, 4H), 3.17-3.14 (m, 2H), 1.97-1.91 (m, 1H).

### Example 75:

2-((5-amino-2-(1H-pyrazol-5-yl)thieno[3,2-b]pyridin-7-yl)amino)-*N-*methylacetamide (Compound137)

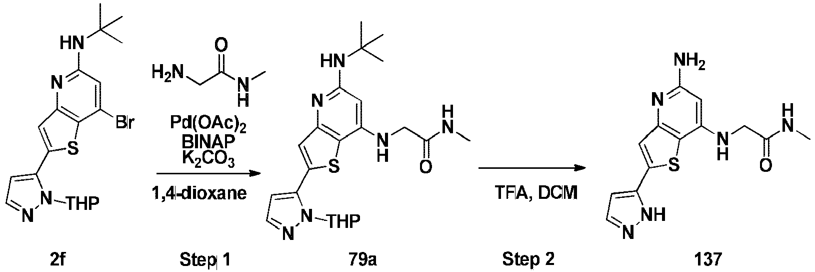

### Step 1: Synthesis of 2-((5-(tert-butylamino)-2-(1-(tetrahydro-2H-pyran-2-yl)-1H-pyrazol-5-yl)thieno[3,2-b]pyridin-7-yl)amino)-N-methylacetamide (Compound 79a)

Compound 2f (60 mg), 2-amino-*N*-methylacetamide (24.28 mg), Pd(OAc)₂ (1.55 mg), BINAP (8.58 mg) and K₂CO₃ (38.09 mg) were added to 1,4-dioxane (2 mL), and the reaction proceeded at an elevated temperature of 110°C for 6 hours under N₂ protection. The system was filtered. The filtrate was concentrated, diluted with a small amount of DCM, and purified by TLC (Eluent System B) to give Compound 79a (30 mg).

MS (ESI, m/z): 443.2 [M+H]⁺.

### Step 2: Synthesis of 2-((5-amino-2-(1H-pyrazol-5-yl)thieno[3,2-b]pyridin-7-yl)amino)-N-methylacetamide (Compound 137)

Compound 79a (30 mg) was added to TFA (2 mL) and DCM (2 mL), and the reaction proceeded at 25°C for 12 hours. The system was concentrated, diluted with methanol, and adjusted to basic by adding 2 equivalents of NaOH. The system was suction filtrated. The filtrate was separated and purified by Prep-HPLC (Elution Condition 4), and lyophilized to give Compound 137 (4 mg).

MS (ESI, m/z): 303.1 [M+H]⁺.

¹H NMR (CD₃OD, 400 MHz) δ 7.71 (d, *J=* 2.3 Hz, 1H), 7.33 (s, 1H), 6.68 (d, *J=* 2.3 Hz, 1H), 5.63 (s, 1H), 3.92 (s, 2H), 2.76 (s, 3H).

### Example 76:

2-((5-amino-2-(1H-pyrazol-5-yl)thieno[3,2-b]pyridin-7-yl)amino)-*N-*cyclopropylacetamide (Compound 138)

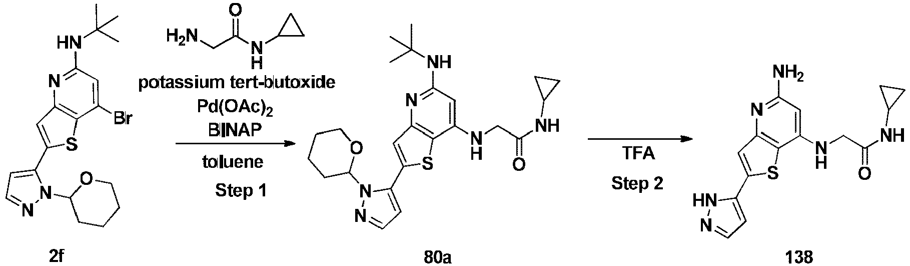

### Step 1: Synthesis of 2-((5-(tert-butylamino)-2-(1-(tetrahydro-2H-pyran-2-yl)-1H-pyrazol-5-yl)thieno[3,2-b]pyridin-7-yl)amino)-N-cyclopropylacetamide (Compound 80a)

Compound 2f (100 mg), 2-amino-*N-*cyclopropylacetamide (78.65 mg), potassium tert-butoxide (77.32 mg), Pd(OAc)₂ (10.31 mg) andBINAP (57.21 mg) were added to toluene (3 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 80a (70 mg).

MS (ESI, m/z): 469.3 [M+H]⁺.

### Step 2: Synthesis of 2-((5-amino-2-(1H-pyrazol-5-yl)thieno[3,2-b]pyridin-7-yl)amino)-N-cyclopropylacetamide (Compound 138)

Compound 80a (35 mg) was added to TFA (2 mL), and the reaction proceeded at an elevated temperature of 60°C for 2 hours with stirring. The reaction solvent was removed by concentration under a reduced pressure to dryness. Separation and purification by Prep-HPLC (Elution Condition 4) and lyophilization gave Compound 138 (12 mg).

MS (ESI, m/z): 329.1 [M+H]⁺.

¹H NMR (DMSO-*d₆*, 400 MHz) δ 12.94 (s, 1H), 8.03 (d*, J* = 4.1 Hz, 1H), 7.79 (d*, J* = 2.3 Hz, 1H), 7.30 (s, 1H), 6.71 (d, *J=* 2.3 Hz, 1H), 6.25 (t, *J=* 5.8 Hz, 1H), 5.56 (s, 2H), 5.45 (s, 1H), 3.71 (d, *J=* 5.8 Hz, 2H), 2.66 (tq, *J*= 7.8, 4.0 Hz, 1H), 0.62 (td, *J*= 7.0, 4.7 Hz, 2H), 0.48 - 0.42 (m, 2H).

### Example 77:

2-((5 -amino-2-(1H-pyrazol-5-yl)thieno[3,2-b]pyridin-7-yl)(methyl)amino)ethanol (Compound 139)

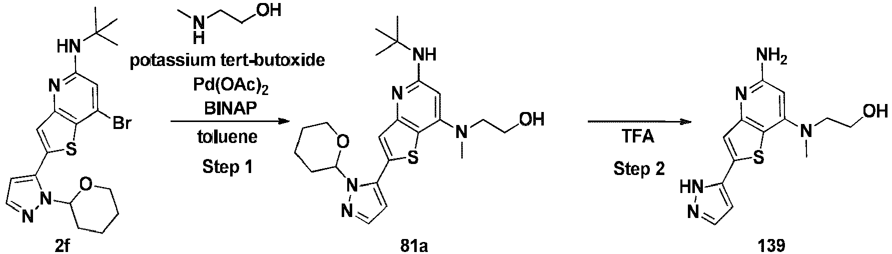

### Step 1: Synthesis of 2-((5-(tert-butylamino)-2-(1-(tetrahydro-2H-pyran-2-yl)-1H-pyrazol-5-yl)thieno[3,2-b]pyridin-7-yl)(methyl)amino)ethanol (Compound 81a)

Compound 2f (100 mg), *N*-methyl-2-hydroxyethylamine (24.36 mg), potassium tert-butoxide (77.32 mg), Pd(OAc)₂ (10.02 mg) and BINAP (57.21 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 81a (50 mg).

MS (ESI, m/z): 430.1 [M+H]⁺.

### Step 2: Synthesis of 2-((5-amino-2-(1H-pyrazol-5-yl)thieno[3,2-b]pyridin-7-yl)(methyl)amino)ethanol (Compound 139)

Compound 81a (30 mg, 0.06 mmol) was added to TFA (2 mL), and the reaction proceeded at an elevated temperature of 75°C for 2 hours with stirring. The reaction solvent was removed by concentration under a reduced pressure to dryness. Separation and purification by Prep-HPLC (Elution Condition 3) and lyophilization gave Compound 139 (3 mg).

MS (ESI, m/z): 290.1 [M+H]⁺.

¹H NMR (DMSO-*d₆*, 400 MHz) δ 13.28 (s, 1H), 13.04 (s, 1H), 7.91 (s, 1H), 7.46 (s, 1H), 7.26 (s, 2H), 6.87 (s, 1H), 5.84 (s, 1H), 5.03 (s, 1H), 3.78-3.71 (m, 4H), 3.32 (s, 3H).

### Example 78:

*N-*(2-((5-amino-2-(1H-pyrazol-5-yl)thieno[3,2-b]pyridin-7-yl)amino)ethyl)-*N-*ethyl-2-(m-tolyl)acetamide (Compound 140)

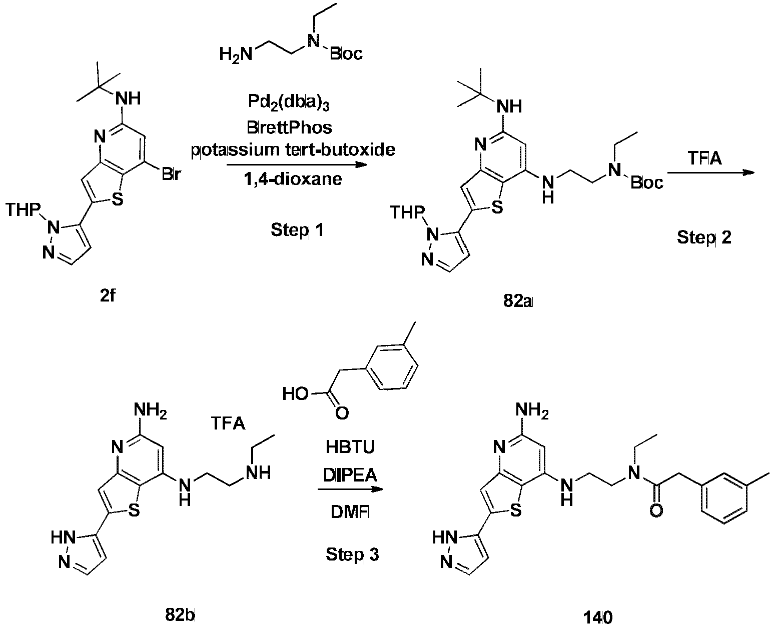

### Step 1: Synthesis of tert-butyl (2-((5-(tert-butylamino)-2-(1-(tetrahydro-2H-pyran-2-yl)-1H-pyrazol-5-yl)thieno[3,2-b]pyridin-7-yl)amino)ethyl)(ethyl)carbamate (Compound 82a)

Compound 2f (200 mg), tert-butyl (2-aminoethyl)(ethyl)carbamate (432.4 mg), Pd₂(dba)₃ (86.5 mg), BrettPhos (86.5 mg) and potassium tert-butoxide (237 mg) were added to 1,4-dioxane (10 mL). After nitrogen replacement, the reaction proceeded at an elevated temperature of 120°C for 4 hours. The system was filtered. The filtrate was concentrated, then diluted with a small amount of methanol, and purified by TLC (Eluent System A) to give Compound 82a (227 mg).

MS (ESI, m/z): 543.3 [M+H]⁺.

### Step 2: Synthesis of N⁷-(2-(ethylamino)ethyl)-2-(1H-pyrazol-5-yl)thieno[3,2-b]pyridine-5,7-diamine trifluoroacetate (Compound 82b)

Compound 82a (227 mg) was added to TFA (3 mL), and the reaction proceeded at 60°C for 2 hours. The system was concentrated to give Compound 82b (280 mg).

MS (ESI, m/z): 303.1 [M+H]⁺.

### Step 3: Synthesis of N-(2-((5-amino-2-(1H-pyrazol-5-yl)thieno[3,2-b]pyridin-7-yl)amino)ethyl)-N-ethyl-2-(m-tolyl)acetamide (Compound 140)

Compound 82b (70 mg), m-tolueneacetic acid (32 mg), HBTU (88.2 mg) and DIPEA (150 mg) were added to DMF (3 mL), and the mixture was reacted at 25°C for 1 hour. The reaction liquid was separated and purified by Prep-HPLC (Elution Condition 4), and lyophilized to give Compound 140 (7 mg).

MS (ESI, m/z): 435.2 [M+H]⁺.

¹H NMR (DMSO-*d₆*, 400 MHz) δ 12.97 (s, 1H), 7.80 (s, 1H), 7.29 (s, 1H), 7.18 - 6.84 (m, 4H), 6.71 (s, 1H), 6.47 - 6.21 (m, 1H), 5.80 - 5.51 (m, 3H), 3.68 - 3.46 (m, 8H), 2.20 (d, *J* = 5.6 Hz, 3H), 1.10 - 0.98 (m, 3H).

### Example 79: N⁷-isobutyl-2-(1H-pyrazol-5-yl)thieno[3,2-b]pyridin-5,7-diamine (Compound 141)

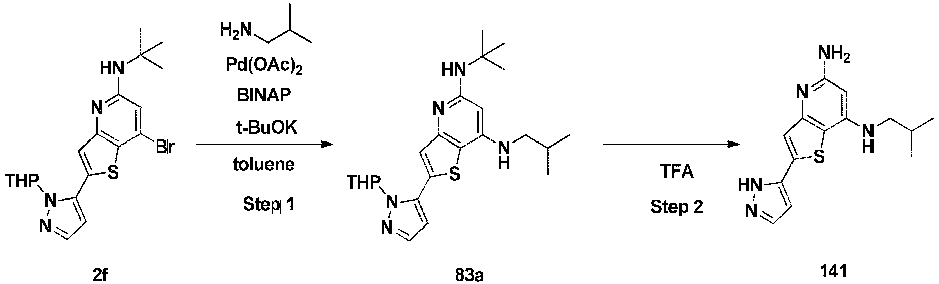

### Step 1: Synthesis of N⁵-(tert-butyl)-N⁷-isobutyl-2-(1-(tetrahydro-2H-pyran-2-yl)-1H-pyrazol-5-yl)thieno[3,2-b]pyridin-5,7-diamine (Compound 83a)

Compound 2f (80 mg), isobutyl amine (40.28 mg), Pd(OAc)₂ (4.12 mg), BINAP (22.88 mg) and t-BuOK (61.85 mg) were added to toluene (10 mL), and the reaction proceeded at an elevated temperature of 120°C for 5 hours under N₂ protection. The system was filtered. The filtrate was concentrated, then diluted with a small amount of methanol, and purified by TLC (Eluent System A) to give Compound 83a (50 mg).

MS (ESI, m/z): 428.1[M+H]⁺.

### Step 2: Synthesis of N⁷-isobutyl-2-(1H-pyrazol-5-yl)thieno[3,2-b]pyridin-5,7-diamine (Compound 141)

Compound 83a (40 mg) was added to TFA (3 mL), and the reaction proceeded at an elevated temperature of 70°C for 2 hours. The system was concentrated, diluted with methanol, and adjusted to pH = 9 with a saturated sodium bicarbonate aqueous solution. The system was suction filtrated. The filtrate was concentrated, then purified by Prep-HPLC (Elution Condition 4), and lyophilized to give Compound 141 (3 mg).

MS (ESI, m/z): 288.0[M+H]⁺.

¹H NMR (400 MHz, DMS0-*d₆*) δ 13.0 (s, 1H), 7.79 (d, *J=* 2.3 Hz, 1H), 7.28 (s, 1H), 6.70 (d, *J=* 2.3 Hz, 1H), 6.26 (t, *J* = 5.7 Hz, 1H), 5.65 (s, 1H), 5.46 (s, 2H), 2.96 (d, *J* =5.6 Hz, 2H), 2.02-1.92 (m, 1H), 0.94 (d, *J=* 6.6 Hz, 6H).

### Example 80:

*N*⁷-((1R,5S,6S)-3-azabicyclo[3.1.0]hex-6-yl)-2-(1H-pyrazol-5-yl)thieno[3,2-b]pyridin-5,7-diamine (Compound 142)

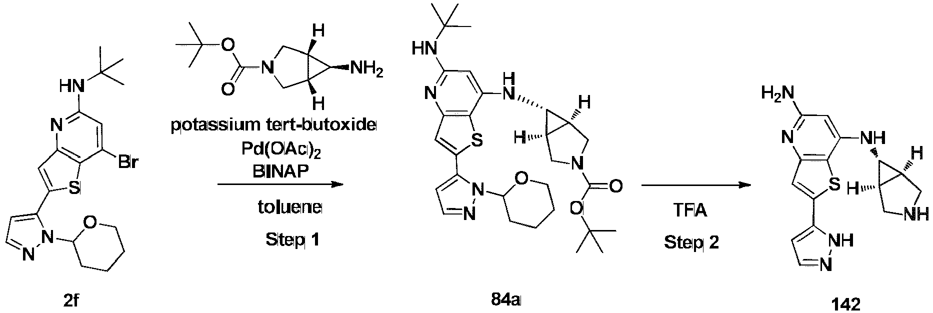

### Step 1: Synthesis of (1R,5S,6S)-tert-butyl 6-((5-(tert-butylamino)-2-(1-(tetrahydro-2H-pyran-2-yl)-1H-pyrazol-5-yl)thieno[3,2-b]pyridin-7-yl)amino)-3-azabicyclo[3.1.0]hexan-3-carboxylate (Compound 84a)

Compound 2f (120 mg), (1R,5S,6S)-tert-butyl 6-amino-3-azabicyclo[3.1.0]hexan-3-carboxylate (163.94 mg), potassium tert-butoxide (92.78 mg), Pd(OAc)₂ (12.38 mg) and BINAP (68.65 mg) were added to toluene (3 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 84a (110 mg).

MS (ESI, m/z): 553.3 [M+H]⁺.

### Step 2: N⁷-((1R,5S,6S)-3-azabicyclo[3.1.0]hex-6-yl)-2-(1H-pyrazol-5-yl)thieno[3,2-b]pyridin-5,7-diamine (Compound 142)

Compound 84a (50 mg) was added to TFA (3 mL), and the reaction proceeded at an elevated temperature of 60°C for 2 hours with stirring. The system was concentrated under a reduced pressure, separated and purified by Prep-HPLC (Elution Condition 4), and lyophilized to give Compound 142 (15 mg).

MS (ESI, m/z): 312.9 [M+H]⁺.

¹H NMR (DMS0-*d₆*, 400 MHz) δ 12.98 (s, 1H), 7.78 (s, 1H), 7.27 (d*, J* = 3.2 Hz, 1H), 6.69 (s, 1H), 6.54 (d*, J* = 68.0 Hz, 1H), 5.85 (d, *J* = 19.9 Hz, 1H), 5.61 (d, *J* = 17.9 Hz, 2H), 3.68 (d, *J* = 10.7 Hz, 1H), 3.47 (s, 2H), 3.08 (d, *J* = 11.0 Hz, 1H), 2.76 (d*, J* = 10.7 Hz, 1H), 2.23 (d*, J* = 63.0 Hz, 1H), 1.77 (s, 1H), 1.57 (s, 1H).

### Example 81:

*N*⁷-(4,4-difluorocyclohexyl)-2-(1H-pyrazol-5-yl)thieno[3,2-b]pyridin-5,7-diamine (Compound 143)

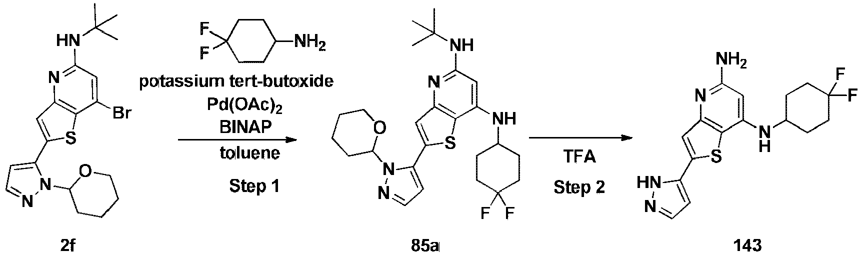

### Step 1: Synthesis of N⁵-(tert-butyl)-N⁷-(4,4-difluorocyclohexyl)-2-(1-(tetrahydro-2H-pyran-2-yl)-1H-pyrazol-5-yl)thieno[3,2-b]pyridin-5,7-diamine (Compound 85a)

Compound 2f (70 mg), 4,4-difluorocyclohexylamine (28.43 mg), potassium tert-butoxide (54.12 mg), Pd(OAc)₂ (7.22 mg) and BINAP (40.04 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 85a (30 mg).

MS (ESI, m/z): 490.1 [M+H]⁺.

### Step 2: Synthesis of N⁷-(4,4-difluorocyclohexyl)-2-(1H-pyrazol-5-yl)thieno[3,2-b]pyridin-5,7-diamine (Compound 143)

Compound 85a (30 mg) was added to TFA (2 mL), and the reaction proceeded at an elevated temperature of 75°C for 2 hours with stirring. The system was concentrated under a reduced pressure, separated and purified by Prep-HPLC (Elution Condition 4), and lyophilized to give Compound 143 (4 mg).

MS (ESI, m/z): 349.9 [M+H]⁺.

¹H NMR (DMS0-*d₆*, 400 MHz) δ 13.25 (s, 1H), 12.90 (s, 1H), 7.90 (s, 1H), 7.67 (d, *J* = 7.6 Hz, 1H), 7.44 (s, 1H), 7.26 (s, 2H), 6.87-6.86 (m, 1H), 5.88 (s, 1H), 3.69-3.66 (m, 1H), 2.15-1.91 (m, 6H), 175-1.66 (m, 2H).

### Example 82:

(R)-2-((5-amino-2-(1H-pyrazol-5-yl)thieno[3,2-b]pyridin-7-yl)amino)-4-methyl-1-pentanol (Compound 144)

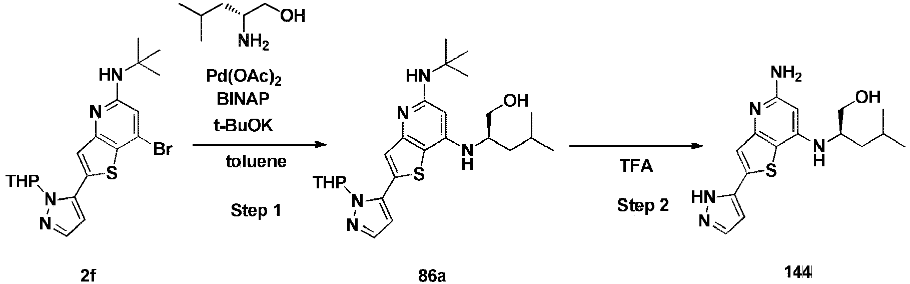

### Step 1: Synthesis of (2R)-2-((5-(tert-butylamino)-2-(1-(tetrahydro-2H-pyran-2-yl)-1H-pyrazol-5-yl)thieno[3,2-b]pyridin-7-yl)amino)-4-methyl-1-pentanol (Compound 86a)

Compound 2f (80 mg), (R)-2-amino-4-methyl-1-pentanol (56.52 mg), Pd(OAc)₂ (3.61mg), BINAP (20.02 mg) and t-BuOK (54.12 mg) were added to toluene (7 mL), and the reaction proceeded at an elevated temperature of 120°C for 5 hours under N₂ protection. The system was filtered. The filtrate was concentrated, then diluted with a small amount of methanol, and purified by TLC (Eluent System A) to give Compound 86a (40 mg).

MS (ESI, m/z): 472.3[M+H]⁺.

### Step 2: Synthesis of (R)-2-((5-amino-2-(1H-pyrazol-5-yl)thieno[3,2-b]pyridin-7-yl)amino)-4-methyl-1-pentanol (Compound 144)

Compound 86a (40 mg) was added to TFA (4 mL), and the reaction proceeded at an elevated temperature of 70°C for 2 hours. The system was concentrated, diluted with methanol, and adjusted to pH = 9 with a saturated sodium bicarbonate aqueous solution. The system was suction filtrated. The filtrate was concentrated, then separated and purified by Prep-HPLC (Elution Condition 4), and lyophilized to give Compound 144 (15 mg).

MS (ESI, m/z): 332.0[M+H]⁺.

¹H NMR (DMSO-*d₆*, 400 MHz) δ 13.01 (s, 1H), 7.79 (s, 1H), 7.29 (s, 1H), 6.70 (d, *J* = 2.3 Hz, 1H), 5.73 (s, 1H), 5.62 (d, *J* = 8.4 Hz, 1H), 5.46 (s, 2H), 4.79 (t, *J* = 5.3 Hz, 1H), 3.55-3.47 (m, 3H), 1.78-1.68 (m, 1H), 1.57-1.44 (m, 2H), 0.95 (d, *J* = 6.6 Hz, 3H), 0.87 (d, *J* = 6.6 Hz, 3H).

### Example 83:

*N-*(2-((5-amino-2-(1H-pyrazol-5-yl)thieno[3,2-b]pyridin-7-yl)amino)ethyl)-*N-*ethylpropionamide (Compound 145)

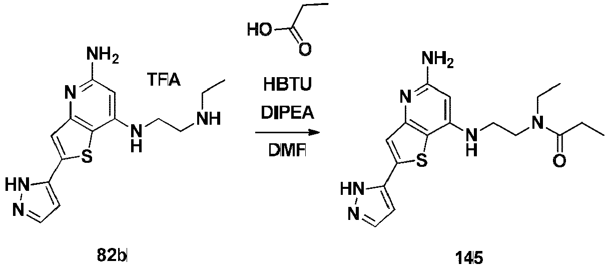

Compound 82b (70 mg), propionic acid (17.2 mg), HBTU (88.2 mg) and DIPEA (150 mg) were added to DMF (3 mL), and the reaction proceeded at 25°C for 1 hour. The reaction liquid was separated and purified by Prep-HPLC (Elution Condition 4), and lyophilized to give Compound 145 (0.85 mg).

MS (ESI, m/z): 359.2 [M+H]⁺.

¹H NMR (DMSO-*d₆*, 400 MHz) δ 12.95 (s, 1H), 7.81 (s, 1H), 7.32 - 7.24 (m, 1H), 6.72 (s, 1H), 6.32 - 6.16 (m, 1H), 5.73 - 5.65 (m, 1H), 5.58 - 5.42 (m, 2H), 3.53 - 3.44 (m, 2H), 3.39 - 3.35 (m, 2H), 3.31 - 3.25 (m, 2H), 2.37 - 2.26 (m, 2H), 1.17 - 0.85 (m, 6H).

### Example 84:

*N-*(2-((5-amino-2-(1H-pyrazol-5-yl)thieno[3,2-b]pyridin-7-yl)amino)ethyl)-*N-*ethylcyclopropyl formamide (Compound 146)

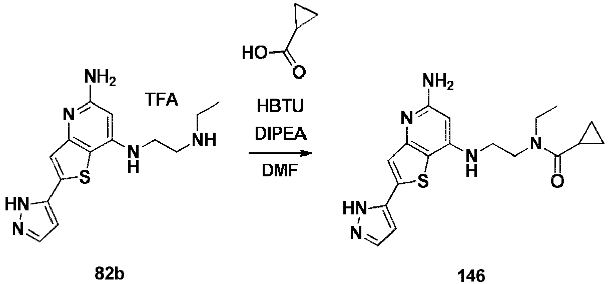

Compound 82b (70 mg), cyclopropanecarboxylic acid (20 mg), HBTU (88.2 mg) and DIPEA (150 mg) were added to DMF (3 mL), and the reaction proceeded at 25°C for 1 hour. The reaction liquid was separated and purified by Prep-HPLC (Elution Condition 4), and lyophilized to give Compound 146 (1 mg).

MS (ESI, m/z): 371.2 [M+H]⁺.

¹H NMR (DMSO-*d₆*, 400 MHz) δ 12.95 (s, 1H), 7.81 (s, 1H), 7.28 (s, 1H), 6.72 (s, 1H), 6.34 - 6.11 (m, 1H), 5.72 - 5.63 (m, 1H), 5.58 - 5.43 (m, 2H), 3.59 - 3.51 (m, 2H), 3.42 - 3.36 (m, 2H), 3.30 - 3.24 (m, 2H), 2.03 - 1.86 (m, 1H), 1.21 - 1.14 (m, 2H), 1.05 - 1.00 (m, 1H), 0.86 - 0.59 (m, 4H).

### Example 85:

(1R,4R)-4-((5-amino-2-(1H-pyrazol-5-yl)thieno[3,2-b]pyridin-7-yl)amino)cyclohexanol (Compound 147)

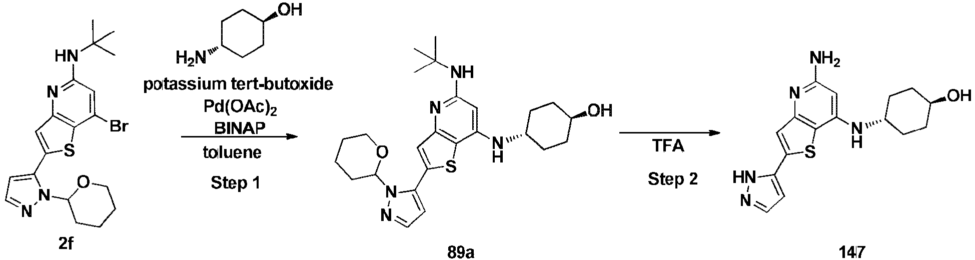

### Step 1: Synthesis of (1R,4R)-4-((5-(tert-butylamino)-2-(1-(tetrahydro-2H-pyran-2-yl)-1H-pyrazol-5-yl)thieno[3,2-b]pyridin-7-yl)amino)cyclohexanol (Compound 89a)

Compound 2f (100 mg), trans-4-aminocyclohexanol (79.36 mg), potassium tert-butoxide (77.32 mg), Pd(OAc)₂ (10.31 mg) and BINAP (57.21 mg) were added to toluene (3 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 89a (80 mg)

MS (ESI, m/z): 470.3 [M+H]⁺.

### Step 2: Synthesis of (1R,4R)-4-((5-amino-2-(1H-pyrazol-5-yl)thieno[3,2-b]pyridin-7-yl)amino)cyclohexanol (Compound 147)

Compound 89a (50 mg) was added to TFA (2 mL), and the reaction proceeded at an elevated temperature of 60°C for 2 hours with stirring. The system was concentrated under a reduced pressure, separated and purified by Prep-HPLC (Elution Condition 4), and lyophilized to give Compound 147 (17 mg).

MS (ESI, m/z): 329.9 [M+H]⁺.

¹H NMR (DMSO-*d₆*, 400 MHz) δ 13.02 (s, 1H), 7.78 (d, *J* = 2.2 Hz, 1H), 7.27 (s, 1H), 6.68 (d, *J* = 2.2 Hz, 1H),5.81 (d, *J=* 7.9 Hz, 1H), 5.69 (s, 1H), 5.46 (s, 2H), 4.62 (s, 1H), 3.49 - 3.42 (m, 1H), 3.30 - 3.23 (m, 1H), 1.99 - 1.84 (m, 4H), 1.40 - 1.22 (m, 4H).

### Example 86:

7-((2R,6R)-2,6-dimethylmorpholino)-2-(1H-pyrazol-5-yl)thieno[3,2-b]pyridine-5-amine (Compound 148)

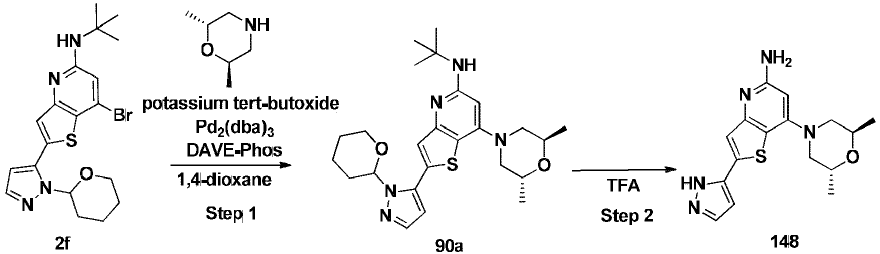

### Step 1: Synthesis of N-(tert-butyl)-7-((2R,6R)-2,6-dimethylmorpholino)-2-(1-(tetrahydro-2H-pyran-2-yl)-1H-pyrazol-5-yl)thieno[3,2-b]pyridine-5-amine (Compound 90a)

Compound 2f (100 mg), 2R,6R-dimethylmorpholine (32.20 mg), potassium tert-butoxide (77.32 mg), Pd₂(dba)₃ (18.30 mg) and DAVE-Phos (19.65 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 90a (60 mg).

MS (ESI, m/z): 469.9 [M+H]⁺.

### Step 2: Synthesis of 7-((2R,6R)-2,6-dimethylmorpholino)-2-(1H-pyrazol-5-yl)thieno[3,2-b]pyridine-5-amine (Compound 148)

Compound 90a (60 mg) was added to TFA (2 mL), and the reaction proceeded at an elevated temperature of 75°C for 2 hours with stirring. The system was concentrated under a reduced pressure, separated and purified by Prep-HPLC (Elution Condition 4), and lyophilized to give Compound 148 (20 mg).

MS (ESI, m/z): 329.9 [M+H]⁺.

¹H NMR (DMSO-*d₆*, 400 MHz) δ 13.03 (s, 1H), 7.83 (s, 1H), 7.37 (s, 1H), 6.78 (s, 1H), 5.98-5.96 (m, 3H), 4.13-4.10 (m, 2H), 3.24-3.20 (m, 2H), 3.12-3.08 (m, 2H), 1.28 (d, *J* = 6.4 Hz, 6H).

### Example 87

*N*⁷-((3,5-dimethylisoxazol-4-yl)methyl)-2-(1H-pyrazol-5-yl)thieno[3,2-b]pyridine-5,7-diamine (Compound 149)

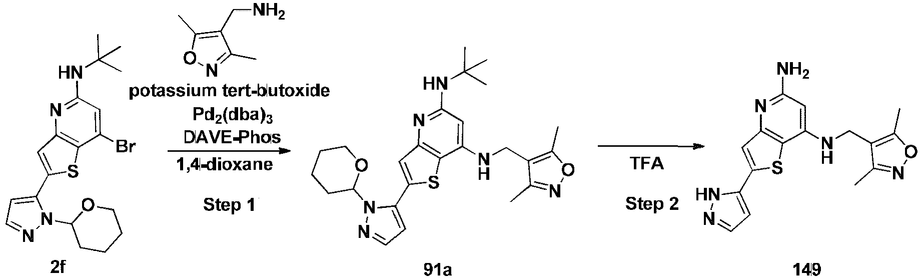

### Step 1: Synthesis of N⁵-(tert-butyl)-N⁷-((3,5-dimethylisoxazol-4-yl)methyl)-2-(1-(tetrahydro-2H-pyran-2-yl)-1H-pyrazol-5-yl)thieno[3,2-b]pyridin-5,7-diamine (Compound 91a)

Compound 2f (100 mg), 3,5-dimethyl-4-aminomethylisoxazole (35.28 mg), potassium tert-butoxide (77.32 mg), Pd₂(dba)₃ (18.30 mg) and DAVE-Phos (19.65 mg) were added to toluene (2 mL), and the reaction proceeded at an elevated temperature of 120°C for 2 hours under N₂ protection. The reaction solvent was removed by drying in a rotary dryer, and the subsequent purification by flash column chromatography (Eluent System A) gave Compound 91a (90 mg).

MS (ESI, m/z): 480.9 [M+H]⁺.

### Step 2: Synthesis of N⁷-((3,5-dimethylisoxazol-4-yl)methyl)-2-(1H-pyrazol-5-yl)thieno[3,2-b]pyridin-5,7-diamine (Compound 149)

Compound 91a (50 mg) was added to TFA (2 mL), and the reaction proceeded at an elevated temperature of 75°C for 2 hours with stirring. The reaction solvent was removed by concentration under a reduced pressure to dryness. Separation and purification by Prep-HPLC (Elution Condition 3) and lyophilization gave Compound 149 (8 mg).

MS (ESI, m/z): 340.9 [M+H]⁺.

¹H NMR (DMSO-*d₆*, 400 MHz) δ 13.05 (s, 1H), 8.17 (s, 1H), 7.82 (s, 1H), 7.33 (s, 1H), 7.01 (s, 1H), 6.75 (s, 1H), 6.09 (s, 2H), 5.59 (s, 1H), 4.18 (d, *J=* 4.8 Hz, 2H), 2.39 (s, 3H), 2.20 (s, 3H).

The compounds in the table below were synthesized using appropriate starting materials in accordance with methods similar to those described in the above examples.

| Compound structure | Compound No. | ¹H NMR (DMSO-*d₆*, 400 MHz) δ | MS (ESI, m/z) [M+H]⁺ |
|---|---|---|---|
| 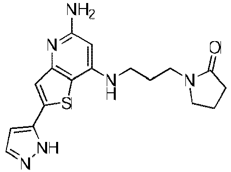 | 150 | 13.09 (s, 1H), 8.26 (s, 1H), 7.81 (d*, J* = 2.3 Hz, 1H), 7.31 (s, 1H), 6.74 (d*, J* = 2.3 Hz, 1H), 6.56 (t*, J* = 5.6 Hz, 1H), 6.18 (s, 2H), 5.65 (s, 1H), 3.36 (t, *J=* 7.0 Hz, 2H), 3.28 (t, *J* = 6.9 Hz, 2H), 3.15 (q, *J* = 6.6 Hz, 2H), 2.24 (t, *J* = 8.0 Hz, 2H), 1.97-1.89 (m, 2H), 1.83-1.76 (m, 2H). | 356.8 |
| 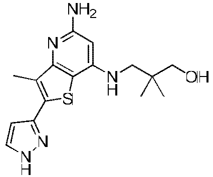 | 151 | δ 8.35 (s, 3H), 7.84 (s, 1H), 6.59 (d, *J* = 1.8 Hz, 1H), 5.76 (s, 1H), 3.27 (s, 3H), 3.07 (d, *J=* 5.7 Hz, 2H), 2.41 (s, 3H), 0.89 (s, 6H). | 331.4 |
| 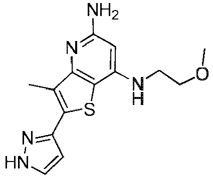 | 152 | δ 13.04 (s, 1H), 7.86 (s, 1H), 6.59 (s, 1H), 6.05 (s, 1H), 5.70 (s, 1H), 5.52 (s, 2H), 3.53 (t, *J=* 6.0 Hz, 2H), 3.33 - 3.26 (m, 5H), 2.41 (s, 3H). | 304.0 |
| 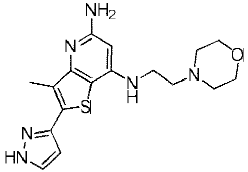 | 153 | δ 13.07 (s, 1H), 7.87 (s, 1H), 6.60 (s, 1H), 6.06 (s, 1H), 5.86 - 5.52 (m, 3H), 3.63 - 3.56 (m, 4H), 3.31 - 3.25 (m, 2H), 2.56 (t, *J* = 6.8 Hz, 2H), 2.48 - 2.35 (m, 7H). | 358.9 |
| 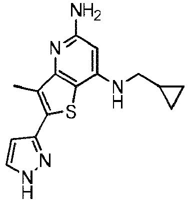 | 156 | δ 13.05 (s, 1H), 7.86 (s, 1H), 6.58 (s, 1H), 6.19 (s, 1H), 5.72 (s, 1H), 5.50 (s, 2H), 3.04 (t, J = 5.9 Hz, 2H), 2.41 (s, 3H), 1.19 - 1.09 (m, 1H), 0.47 (d, J = 7.2 Hz, 2H), 0.26 (d, J = 4.1 Hz, 2H). | 300.1 |
| 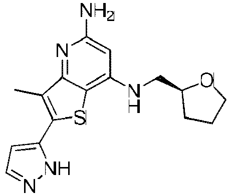 | 157 | δ 13.07 (s, 1H), 7.87 (s, 1H), 6.60 (s, 1H), 6.07 (t, *J* = 7.0 Hz, 1H), 5.75 (s, 1H), 5.52 (s, 2H), 4.12 - 4.06 (m, 1H), 3.87 - 3.76 (m, 1H), 3.68-3.63 (m, 1H), 3.31 - 3.17 (m, 2H), 2.42 (s, 3H), 2.00 - 1.91(m, 1H), 1.90 - 1.79 (m, 2H), 1.68-1.60 (m, 1H). | 330.0 |
| 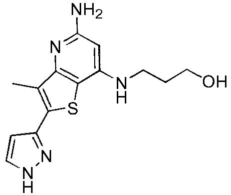 | 159 | δ 13.05 (s, 1H), 7.84 (s, 1H), 6.57 (d, *J=* 1.9 Hz, 1H), 6.06 (s, 1H), 5.67 (s, 1H), 5.50 (s, 2H), 4.56 (s, 1H), 3.60 - 3.46 (m, 2H), 3.21 (q, *J* = 6.7 Hz, 2H), 2.40 (s, 3H), 1.77 (p, *J* = 6.4 Hz, 2H). | 304 |
| 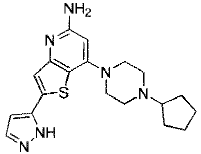 | 160 | 13.01 (s, 1H), 7.82 (s, 1H), 7.34 (s, 1H), 6.76 (s, 1H), 5.96 (s, 1H), 5.80 (s, 2H), 3.27 - 3.22 (m, 4H), 2.64 - 2.58 (m, 4H), 2.56 - 2.53 (m, 1H), 1.88 - 1.78 (m, 2H), 1.69 - 1.59 (m, 2H), 1.58 - 1.47 (m, 2H), 1.43 - 1.32 (m, 2H). | 369.2 |
| 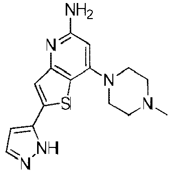 | 161 | 13.27 (s, 1H), 7.91 (s, 1H), 7.49 (s, 1H), 7.32 (s, 2H), 6.90 (s, 1H), 6.15 (s, 1H), 4.05 - 3.50 (m, 4H), 3.33 - 3.09 (m, 4H), 2.80 (s, 3H). | 315.1 |
| 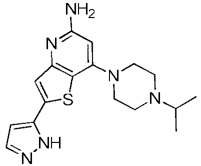 | 162 | 13.01 (s, 1H), 7.82 (s, 1H), 7.35 (s, 1H), 6.76 (s, 1H), 5.96 (s, 1H), 5.80 (s, 2H), 3.24 (s, 4H), 2.75 - 2.67 (m, 1H), 2.66 - 2.60 (m, 4H), 1.03 (d, *J=* 6.5 Hz, 6H). | 343.2 |
| 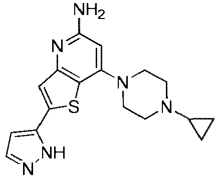 | 163 | 13.00 (s, 1H), 7.82 (s, 1H), 7.34 (s, 1H), 6.76 (t, *J=* 1.9 Hz, 1H), 5.96 (s, 1H), 5.80 (s, 2H), 3.24 - 3.17 (m, 4H), 2.77 - 2.70 (m, 4H), 1.76 - 1.68 (m, 1H), 0.51 - 0.43 (m, 2H), 0.40 - 0.33 (m, 2H). | 341.2 |
| 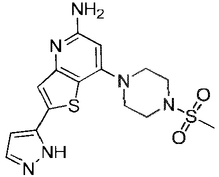 | 164 | 13.03 (s, 1H), 7.83 (s, 1H), 7.37 (s, 1H), 6.78 (t, *J=* 1.9 Hz, 1H), 6.02 (s, 1H), 5.91 (s, 2H), 3.37 - 3.35 (m, 4H), 3.34 - 3.32 (m, 4H), 2.97 (s, 3H). | 379.1 |
| 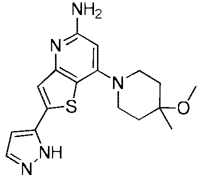 | 165 | 13.00 (s, 1H), 7.82 (s, 1H), 7.34 (s, 1H), 6.75 (s, 1H), 6.00 (s, 1H), 5.76 (s, 2H), 3.44 - 3.37 (m, 2H), 3.15 (s, 3H), 3.12 - 3.03 (m, 2H), 1.88 - 1.80 (m, 2H), 1.70 - 1.57 (m, 2H), 1.18 (s, 3H). | 344.2 |
| 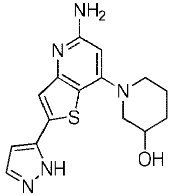 | 166 | 13.01 (s, 1H), 7.80 (d, *J* = 1.7 Hz, 1H), 7.34 (s, 1H), 6.74 (d, *J* = 2.2 Hz, 1H), 5.97 (s, 1H), 5.78 (s, 2H), 4.99 (s, 1H), 3.77 - 3.70 (m, 1H), 3.69 - 3.61 (m, 1H), 3.60 - 3.52 (m, 1H), 2.81 - 2.71 (m, 1H), 2.63 - 2.56 (m, 1H), 2.02 - 1.90 (m, 1H), 1.87 - 1.76 (m, 1H), 1.66 - 1.53 (m, 1H), 1.39 - 1.27 (m, 1H). | 316.1 |
| 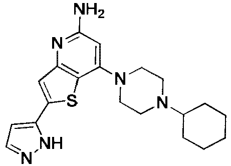 | 167 | 13.01 (s, 1H), 7.82 (s, 1H), 7.35 (s, 1H), 6.75 (s, 1H), 5.96 (s, 1H), 5.80 (s, 2H), 3.27 - 3.19 (m, 4H), 2.74 - 2.65 (m, 4H), 2.38 - 2.25 (m, 1H), 1.87 - 1.69 (m, 4H), 1.65 - 1.53 (m, 1H), 1.30 - 1.16 (m, 4H), 1.16 - 1.04 (m, 1H). | 383.2 |
| 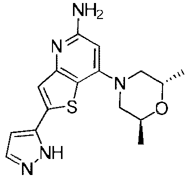 | 168 | 13.01 (s, 1H), 7.83 (s, 1H), 7.37 (s, 1H), 6.77 (d*, J* = 2.5 Hz, 1H), 5.98 (s, 1H), 5.89 (s, 2H), 4.13-4.10 (m, 2H), 3.22-3.19 (m, 2H), 3.10-3.06 (m, 2H), 1.28 (d*, J =* 6.4 Hz, 6H). | 330.1 |
| 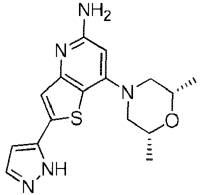 | 169 | 13.04 (s, 1H), 7.85 - 7.77 (m, 1H), 7.37 (s, 1H), 6.76 (d, *J=* 2.3 Hz, 1H), 6.00 (s, 1H), 5.83 (s, 2H), 3.80-3.76 (m, 2H), 3.65-3.60 (m, 2H), 2.48-2.42 (m, 2H), 1.17 (d, *J* = 6.2 Hz, 6H). | 330.1 |
| 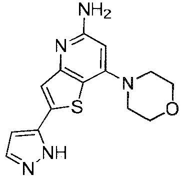 | 170 | 13.07 (s, 1H), 7.84 (s, 1H), 7.39 (s, 1H), 6.79 (s, 1H), 6.17 (s, 2H), 6.01 (s, 1H), 3.81-3.79 (m, 4H), 3.31-3.28 (m, 4H). | 301.3 |
| 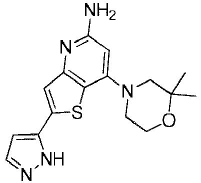 | 171 | 13.01 (s, 1H), 7.80 (d*, J* = 2.3 Hz, 1H), 7.37 (s, 1H), 6.75 (d, *J* = 2.3 Hz, 1H), 5.99 (s, 1H), 5.82 (s, 2H), 3.86 - 3.79 (m, 2H), 3.17 - 3.11 (m, 2H), 3.07 (s, 2H), 1.31 (s, 6H). | 330.1 |
| 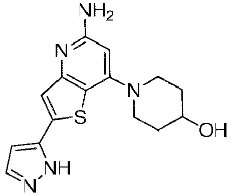 | 172 | 13.02 (s, 1H), 7.80 (s, 1H), 7.34 (s, 1H), 6.74 (d*, J* = 2.3 Hz, 1H), 5.99 (s, 1H), 5.76 (s, 2H), 4.78 (d, *J=* 4.3 Hz, 1H), 3.73-3.68 (m, 1H), 3.61-3.56 (m, 2H), 3.00-2.94 (m, 2H), 1.93-1.86 (m, 2H), 1.59-1.50 (m, 2H). | 316.1 |
| 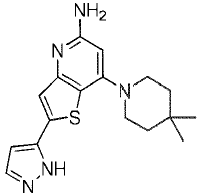 | 173 | 13.13 (s, 1H), 8.14 (s, 0H), 7.86 (s, 1H), 7.40 (s, 1H), 6.81 (s, 1H), 6.44 (s, 2H), 6.01 (s, 1H), 3.43 (d, *J=* 5.3 Hz, 4H), 1.57 - 1.45 (m, 4H), 1.01 (s, 6H). | 328.1 |
| 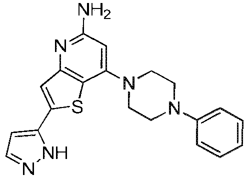 | 174 | 13.02 (s, 1H), 7.83 (s, 1H), 7.38 (s, 1H), 7.30 - 7.22 (m, 2H), 7.07 - 7.01 (m, 2H), 6.86 - 6.81 (m, 1H), 6.78 (s, 1H), 6.05 (s, 1H), 5.86 (s, 2H), 3.43 - 3.38 (m, 4H), 3.37 - 3.35 (m, 3H), 3.34 - 3.32 (m, 1H). | 377.2 |
| 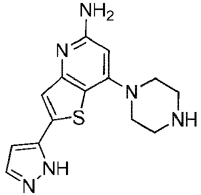 | 175 | 12.99 (s, 1H), 8.23 (s, 2H), 7.81 (d*, J* = 1.9 Hz, 1H), 7.37 (s, 1H), 6.76 (d, *J=* 2.2 Hz, 1H), 6.01 (s, 1H), 5.90 (s, 2H), 3.35 - 3.25 (m, 4H), 3.16 - 3.04 (m, 4H). | 301.1 |
| 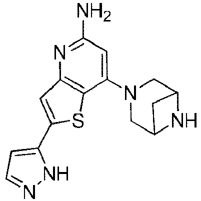 | 176 | 13.03 (s, 1H), 8.25 (s, 2H), 7.81 (d*, J* = 2.1 Hz, 1H), 7.34 (s, 1H), 6.75 (d, J*=* 2.3 Hz, 1H), 6.01 (s, 2H), 5.72 (s, 1H), 4.11 (d*, J* = 5.8 Hz, 2H), 4.04 - 3.95 (m, 4H), 2.75 - 2.64 (m, 1H), 1.73 (d*, J* = 9.3 Hz, 1H). | 313.1 |
| 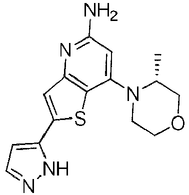 | 177 | 13.02 (s, 1H), 7.82 (s, 1H), 7.36 (s, 1H), 6.77 (s, 1H), 5.98 (s, 1H), 5.83 (s, 2H), 4.08-4.03 (m, 1H), 3.94-3.89 (m, 1H), 3.84-3.80 (m, 1H), 3.69-3.61 (m, 2H), 3.34-3.31 (m, 1H), 3.01-2.97 (m, 1H), 1.03 (d, *J* = 6.5 Hz, 3H). | 316.1 |
| 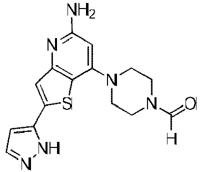 | 178 | 13.02 (s, 1H), 8.15 (s, 1H), 8.10 (s, 1H), 7.82 (s, 1H), 7.38 (s, 1H), 6.77 (s, 1H), 6.01 (s, 1H), 5.90 (s, 2H), 3.63 - 3.55 (m, 4H), 3.28 - 3.19 (m, 4H). | 329.1 |
| 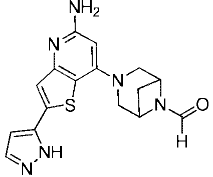 | 179 | 13.04 (s, 1H), 8.16 (s, 2H), 7.82 (s, 1H), 7.33 (s, 1H), 6.75 (s, 1H), 5.80 (s, 2H), 5.70 (s, 1H), 4.74 - 4.67 (m, 1H), 4.62 - 4.53 (m, 1H), 4.08 (t, *J=* 9.5 Hz, 2H), 4.01 - 3.96 (m, 1H), 3.84 (d*, J* = 10.2 Hz, 1H), 2.76 - 2.68 (m, 1H), 1.74 (d, *J* = 8.8 Hz, 1H). | 341.1 |
| 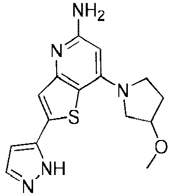 | 180 | 13.10 (s, 1H), 7.83 (s, 1H), 7.35 (s, 1H), 6.77 (s, 1H), 6.11 (s, 2H), 5.55 (s, 1H), 4.13 (s, 1H), 3.78 (dd, *J* = 10.5, 4.5 Hz, 1H), 3.69 (dd, *J* = 8.5, 2.9 Hz, 1H), 3.66 - 3.60 (m, 2H), 3.29 (s, 3H), 2.20 - 2.03 (m, 2H). | 316 |
| 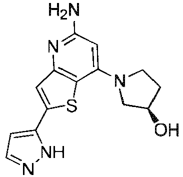 | 181 | 13.05 (s, 1H), 7.83 (s, 1H), 7.33 (s, 1H), 6.75 (s, 1H), 5.86 (s, 2H), 5.52 (s, 1H), 5.11 *(d, J* = 3.4 Hz, 1H), 4.43 (s, 1H), 3.77 (dd, *J* = 9.8, 4.4 Hz, 1H), 3.72 - 3.59 (m, 2H), 3.50 (d*, J* = 9.8 Hz, 1H), 2.11 - 1.89 (m, 2H). | 302 |
| 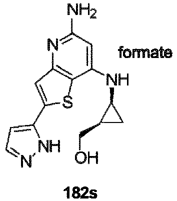 | 182s | 13.12 (s, 1H), 8.20 (s, 1H), 7.85 (s, 1H), 7.37 (s, 1H), 7.11 (s, 1H), 6.78 (s, 1H), 6.53 (s, 2H), 6.00 (s, 1H), 4.51 (s, 1H), 3.50-3.45 (m, 1H), 2.67-2.65 (m, 1H), 1.31 - 1.21 (m, 2H), 1.01 (dt, *J=* 8.6, 5.4 Hz, 1H), 0.58 (dd, *J* = 10.4, 5.3 Hz, 1H). | 302.2 |

### Activity Tests

### Experimental Example 1:|Agonistic effect of the compound of the invention on the expression of IL-1β in THP-1 cells after PMA-induced differentiation

In this test, a HTRF (homogeneous time-resolved fluorescence) detection method was used to test the effect of the compound of the invention on the level of downstream cytokine IL-1β of NLRP3 to evaluate the agonistic effect of the compound on hNLRP3 inflammasome or hNLRP3 inflammasome pathway at a cellular level.
Reagents: RPMI 1640 (Hyclone); heat-inactivated FBS (fetal bovine serum) (Gibco); PMA (phorbol myristyl acetate) (Beyotime)
Cell: THP-1 (Nanjing Cobioer)
Kit: IL-1β assay kit (CISBIO)

Experimental Procedures:
1) THP-1 cells in a logarithmic growth phase were seeded in a T75 culture flask at a density of 5×10⁵ cells/well, and cultured in a cell incubator at 37°C, 5% CO₂ for 24 hours, and then 1 µM PMA was used to induce THP-1 suspension cells to become adherent macrophages. The medium was RPMI 1640 containing 10% heat-inactivated FBS and 0.05 mM β-mercaptoethanol.
2) The cells were induced and cultured for 24 hours. Then the adherent cells were trypsinized and centrifuged at 1000 rpm for 5 min. The supernatant was removed, and the cells were resuspended to a density of 2×10⁶ cells/mL in RPMI 1640 medium containing 2% heat-inactivated FBS. 50 µL/well of cell resuspension was taken and spread on a 96-well plate such that the number of cells in each well was 1 × 10⁵.
3) An appropriate amount of 10 mM DMSO solution of the compound to be tested was taken and diluted to a concentration of 2× test concentration with RPMI 1640 medium containing 2% heat-inactivated FBS. 50 µL/well of the dilution was added to the cells in the 96-well plate, and mixed well. The plate was placed in a cell incubator at 37°C, 5% CO₂ for 6 hours. The supernatant was collected, and the level of IL-1β was measured in accordance with the instructions of the IL-1β detection kit.
4) EC₅₀ was fitted by the GraphPad software log(agonist) vs. response - Variable slope four-parameter method. The results are shown in Table 1.

### Experimental Example 2: Agonistic effect of the compound of the invention on the expression of IL-1β in NLRP3 deficient THP1 cells (THP1-_{def}NLRP3 cells) after PMA-induced differentiation

In this test, a HTRF detection method was used to test the effect of the compound of the invention on the level of IL-1β in THP1-_{def}NLRP3 cells to evaluate the specificity of the agonistic effect of the compound on hNLRP3 inflammasome or hNLRP3 inflammasome pathway.
Reagents: as described in Experimental Example 1
Cell: THP1-_{def}NLRP3 (InvivoGen)
Kit: IL-1β assay kit (CISBIO)

Experimental Procedures:
1) THP1-_{def}NLRP3 cells in a logarithmic growth phase were seeded in a T75 culture flask at a density of 5×10⁵ cells/well, and cultured in a cell incubator at 37°C, 5% CO₂ for 24 hours, and then 1 µM PMA was used to induce THP1-_{def}NLRP3 suspension cells to become adherent macrophages. The medium was RPMI 1640 containing 10% heat-inactivated FBS and 0.05 mM β-mercaptoethanol.
2) The cells were induced and cultured for 24 hours. Then the adherent cells were trypsinized and centrifuged at 1000 rpm for 5 min. The supernatant was removed, and the cells were resuspended to a density of 2×10⁶ cells/mL in RPMI 1640 medium containing 2% heat-inactivated FBS. 50 µL/well of the cell resuspension was taken and spread on a 96-well plate such that the number of cells in each well was 1×10⁵.
3) An appropriate amount of 10 mM DMSO solution of the compound to be tested was taken and diluted to a concentration of 2× test concentration with RPMI 1640 medium containing 2% heat-inactivated FBS. 50 µL/well of the dilution was added to the cells in the 96-well plate, and mixed well. The plate was incubated in a cell incubator at 37°C, 5% CO₂ for 6 hours. The supernatant was collected, and the level of IL-1β was measured in accordance with the instructions of the lL-1β detection kit.
4) EC₅₀ was fitted by the GraphPad software log(agonist) vs. response - Variable slope four-parameter method. The results are shown in Table 1.

### Experimental Example 3: Agonistic effect of the compound of the invention on hTLR7

In this test, the luciferase in HEK-hTLR7-NF-κB-reporter cells was detected to test the activating effect of the compound of the invention on TLR7 signaling pathway, so as to evaluate the specificity of the agonistic effect of the compound on NLRP3 pathway.
Reagents: DMEM (High glucose); FBS (fetal bovine serum) (Gibco); Bright-Glo^{™} Luciferase detection kit (Promega)
Cells: HEK-hTLR7-NF-κB-Luciferase gene cells (human TLR7 NF-κB-luciferase reporter gene cells) (Nanjing Cobioer)
   1) The HEK-hTLR7-NF-κB-Luciferase cells in a logarithmic growth phase were trypsinized and resuspended in the medium to a concentration of 2×10⁶ cells/mL. 50 µL/well of the cell resuspension was added to a 96-well plate such that the number of cells in each well was 1×10⁶. An appropriate amount of 10 mM DMSO solution of the compound to be tested was taken and diluted to a concentration of 2× test concentration with the medium. 50 µL/well was added to the cells in the 96-well plate, and the 96-well plate was incubated in an incubator at 37°C and 5% CO₂ for 16 hours. The medium was DMEM (High glucose) containing 10% FBS.
   2) After the cell incubation, 100 µL/well of Bright-Glo^{™} Luciferase detection reagent was added, and the mixture was incubated at room temperature for 5 minutes. Relative luciferase activity unit (RLU) was read using a microplate reader.
   3) The EC₅₀ of effect of the tested compound to stimulate hTLR7 was fitted by the GraphPad software log(agonist) vs. response - Variable slope four-parameter method. The results are shown in Table 1.

### Experimental Example 4: Agonistic effect of the compound of the invention on hTLR8

In this test, the secretion of alkaline phosphatase in HEK-Blue cells was measured to test the activating effect of the compound of the invention on TLR8 signaling pathway, so as to evaluate the specificity of the agonistic effect of the compound on NLRP3 pathway.
Reagents: DMEM (High glucose); FBS (fetal bovine serum) (Gibco); QUANTI-Blue/InvivoGen/rep-qb2
Cells: HEK-Blue^{™} hTLR8 cells (human TLR 8 cells) (InvivoGen)

Experimental Procedures:
1) The HEK-Blue^{™} hTLR8 cells in a logarithmic growth phase were trypsinized and resuspended in the medium to a concentration of 2×10⁶ cells/mL. 50 µL/well of the cell suspension was added to a 96-well plate. An appropriate amount of 10 mM DMSO solution of the compound to be tested was taken and diluted to a concentration of 2× test concentration with the medium. 50 µL/well was added to the cells in the 96-well plate. The plate was incubated in an incubator at 37°C and 5% CO₂ for 16 hours. The medium was DMEM (High glucose) containing 10% FBS.
2) After the cell incubation, 10 µL of cell culture supernatant was transferred to a 96-well plate. 90 µL/well of QUANTI-Blue detection solution was added, and the mixture was incubated at 37°C for 3 hours. OD₆₂₀ was read using a microplate reader.
3) The EC₅₀ of the effect of the tested compound to stimulate hTLR8 was fitted by the GraphPad software log(agonist) vs. response - Variable slope four-parameter method. The results are shown in Table 1.

**Table 1**

| Compound No. | hNLRP3 EC₅₀ (µM) (THP-1 ) | hNLRP3 EC₅₀ (µM) (THP-1_{def}NLRP3) | hTLR7 EC₅₀ (µM) | hTLR8 EC₅₀ (µM) |
|---|---|---|---|---|
| 26 | 0.10 | >30 | >100 | >100 |
| 28 | 0.54 | >30 | >100 | >100 |
| 29 | 0.16 | >30 | >100 | >100 |
| 30 | 0.03 | >30 | >100 | >100 |
| 30s | 0.02 | >30 | >100 | >100 |
| 31 | 0.11 | >30 | >100 | >100 |
| 33 | 0.26 | >30 | >100 | >100 |
| 40 | 0.39 | >30 | >100 | >100 |
| 51 | 0.33 | >30 | >100 | >100 |
| 52 | 0.20 | >30 | >100 | >100 |
| 53 | 0.18 | >30 | >100 | >100 |
| 54 | 0.92 | >30 | >100 | >100 |
| 55 | 0.44 | >30 | >100 | >100 |
| 57 | 0.57 | >30 | >100 | >100 |
| 58 | 0.26 | >30 | >100 | >100 |
| 59 | 0.1 | >30 | >100 | >100 |
| 60 | 0.1 | >30 | >100 | >100 |
| 61 | 0.10 | >30 | >100 | >100 |
| 62 | 0.06 | >30 | >100 | >100 |
| 63 | 0.07 | >30 | >100 | >100 |
| 64 | 0.05 | >30 | >100 | >100 |
| 73 | 0.18 | >30 | >100 | >100 |
| 74 | 0.26 | >30 | >100 | >100 |
| 75 | 0.11 | >30 | >100 | >100 |
| 76 | 0.27 | >30 | >100 | >100 |
| 88 | 0.04 | >30 | >100 | >100 |
| 89 | 0.16 | >30 | >100 | >100 |
| 90 | 6.03 | >30 | >100 | >100 |
| 91 | 0.11 | >30 | >100 | >100 |
| 92 | 1.12 | >30 | >100 | >100 |
| 93 | 0.18 | >30 | >100 | >100 |
| 95 | 0.09 | >30 | >100 | >100 |
| 96 | 0.07 | >30 | >100 | >100 |
| 97 | 0.03 | >30 | >100 | >100 |
| 98 | 0.35 | >30 | >100 | >100 |
| 99 | 0.54 | >30 | >100 | >100 |
| 100 | 0.03 | >30 | >100 | >100 |
| 100s | 0.01 | >30 | >100 | >100 |
| 101 | 0.03 | >30 | >100 | >100 |
| 101s | 0.03 | >30 | >100 | >100 |
| 102 | 0.01 | >30 | >100 | >100 |
| 102s | 0.01 | >30 | >100 | >100 |
| 104 | 0.17 | >30 | >100 | >100 |
| 105 | 0.03 | >30 | >100 | >100 |
| 105s | 0.01 | >30 | >100 | >100 |
| 106 | 0.04 | >30 | >100 | >100 |
| 108 | 0.34 | >30 | >100 | >100 |
| 109 | 4.48 | >30 | >100 | >100 |
| 110 | 0.02 | >30 | >100 | >100 |
| 110s | 0.03 | >30 | >100 | >100 |
| 111 | 0.23 | >30 | >100 | >100 |
| 112 | 0.28 | >30 | >100 | >100 |
| 113 | 0.03 | >30 | >100 | >100 |
| 113s | 0.01 | >30 | >100 | >100 |
| 114 | 0.06 | >30 | >100 | >100 |
| 115 | 0.04 | >30 | >100 | >100 |
| 115s | 0.04 | >30 | >100 | >100 |
| 116 | 0.03 | >30 | >100 | >100 |
| 116s | 0.05 | >30 | >100 | >100 |
| 117 | 0.27 | >30 | >100 | >100 |
| 119 | 0.01 | >30 | >100 | >100 |
| 120 | 0.05 | >30 | >100 | >100 |
| 120s | 0.03 | >30 | >100 | >100 |
| 121 | 0.03 | >30 | >100 | >100 |
| 122 | 0.6 | >30 | >100 | >100 |
| 123 | 0.1 | >30 | >100 | >100 |
| 124 | 0.05 | >30 | >100 | >100 |
| 125 | 0.03 | >30 | >100 | >100 |
| 126 | 0.35 | >30 | >100 | >100 |
| 127 | 0.11 | >30 | >100 | >100 |
| 128 | 0.11 | >30 | >100 | >100 |
| 129 | 0.03 | >30 | >100 | >100 |
| 131 | 0.06 | >30 | >100 | >100 |
| 132 | 3.48 | >30 | >100 | >100 |
| 133 | 0.58 | >30 | >100 | >100 |
| 134 | 0.03 | >30 | >100 | >100 |
| 135 | 0.32 | >30 | >100 | >100 |
| 136 | 0.05 | >30 | >100 | >100 |
| 137 | 0.95 | >30 | >100 | >100 |
| 138 | 0.2 | >30 | >100 | >100 |
| 139 | 0.07 | >30 | >100 | >100 |
| 140 | 0.5 | >30 | >100 | >100 |
| 141 | 0.03 | >30 | >100 | >100 |
| 142 | 0.14 | >30 | >100 | >100 |
| 143 | 0.08 | >30 | >100 | >100 |
| 144 | 0.38 | >30 | >100 | >100 |
| 145 | 0.31 | >30 | >100 | >100 |
| 146 | 0.29 | >30 | >100 | >100 |
| 147 | 0.03 | >30 | >100 | >100 |
| 148 | 0.008 | >30 | >100 | >100 |
| 149 | 0.04 | >30 | >100 | >100 |
| 150 | 0.16 | >30 | >100 | >100 |
| 151 | 3.66 | >30 | >100 | >100 |
| 152 | 2.75 | >30 | >100 | >100 |
| 153 | 4.29 | >30 | >100 | >100 |
| 156 | 5.01 | >30 | >100 | >100 |
| 157 | 3.47 | >30 | >100 | >100 |
| 159 | 1.29 | >30 | >100 | >100 |
| 160 | 0.11 | >30 | >100 | >100 |
| 161 | 0.18 | >30 | >100 | >100 |
| 162 | 0.01 | >30 | >100 | >100 |
| 163 | 0.27 | >30 | >100 | >100 |
| 164 | 0.006 | >30 | >100 | >100 |
| 165 | 0.03 | >30 | >100 | >100 |
| 166 | 0.34 | >30 | >100 | >100 |
| 167 | 0.01 | >30 | >100 | >100 |
| 168 | 0.01 | >30 | >100 | >100 |
| 169 | 0.01 | >30 | >100 | >100 |
| 170 | 0.02 | >30 | >100 | >100 |
| 171 | 0.03 | >30 | >100 | >100 |
| 172 | 0.03 | >30 | >100 | >100 |
| 173 | 0.10 | >30 | >100 | >100 |
| 174 | 0.34 | >30 | >100 | >100 |
| 175 | 0.34 | >30 | >100 | >100 |
| 176 | 0.23 | >30 | >100 | >100 |
| 177 | 0.03 | >30 | >100 | >100 |
| 178 | 0.17 | >30 | >100 | >100 |
| 179 | 0.05 | >30 | >100 | >100 |
| 180 | 0.01 | >30 | >100 | >100 |
| 181 | 0.009 | >30 | >100 | >100 |
| 182s | 0.11 | >30 | >100 | >100 |

The results show that the compounds of the invention represented by the compounds listed in Table 1 have a significant agonistic effect on the expression of IL-1β in THP-1 cells after PMA-induced differentiation, but have no agonistic effect on the expression of IL-1β in THP-1_{def}NLRP3 cells even at the highest test concentration (30 µM) of the compounds. All the tested compounds have no activating effect on hTLR7 and hTLR8 at 100 µM. In summary, the compounds of the invention represented by the compounds listed in Table 1 have significant specific agonistic activity on hNLRP3 and/or its signaling pathway.

### Experimental Example 5: hERG Test

1. Predictor^{™} hERG Fluorescence Polarization Assay Kit (ThermoFisher) was used to test the inhibitory effect of the compounds on hERG potassium channel according to the instructions of the kit. The test concentrations of the compounds were 1 µM and 10 µM, and the results are shown in Table 2.

**Table 2**

| Compound No. | IC₅₀ (µM) |
|---|---|
| 111 | >10 |
| 112 | >10 |

2. The inhibitory effect of the compounds on hERG was tested by manual patch clamp method.
Cells: HEK-293 overexpressing hERG potassium channel (human embryonic kidney cells)
Negative control: extracellular fluid containing 0.1% DMSO
Positive control: Quinidine
Test concentration of the compounds: 10 µM
Reaction temperature: 22 to 24°C
Data collection: PATCHMASTER V2X60.

Experimental Procedures:
1) The compound was dissolved in DMSO to prepare a 10 mM solution. 30 µL of the solution was taken and added to 30 mL of extracellular fluid for electrophysiological testing.
2) The cells for the test were transferred to a cell bath embedded in an inverted microscope platform, and the extracellular fluid was perfused. After stabilizing for 5 minutes, the membrane voltage was clamped to -80 mV. The cells were stimulated with +20 mV voltage for 2 s to activate the hERG potassium channel, and then repolarized to -50 mV for 5 s to generate outward tail current. The stimulation frequency was 15 seconds/time.
3) Extracellular fluid (2 ml/min) was perfused, and recording went on. After the current was stable, the extracellular fluid containing the compound to be tested was perfused and recording went on until the inhibitory effect of the compound on the hERG current reached a steady state.

In this test, the positive control quinidine inhibited the hERG potassium channel at a rate of 90.7% at 30 µM, indicating that the test system was normal. The results are shown in Table 3.

**Table 3**

| Compound No. | 10 µM average inhibition (%) |
|---|---|
| 30 | 1.91 ± 1.40 |
| 100 | 5.42 ± 2.20 |
| 115 | 8.26 ± 3.66 |
| 116 | 18.54 ± 4.98 |
| 120 | 11.65 ± 0.70 |

The results show that the IC₅₀ values of Compounds 30, 100, 115, 116 and 120 are greater than 10 µM. The results show that the compounds of the invention represented by Compounds 30, 100, 115, 116 and 120 have no apparent inhibitory effect on hERG, and in turn low possibility of prolonging the cardiac QT interval.

### Experimental Example 6: CYP Enzyme Inhibition Test

CYP450 is the most important enzyme system in drug metabolism. The enzymes involved in metabolism interact with drugs, and the most important ones are CYP1A2, CYP2D6 and CYP3A4. P450-Glo^{™} CYP1A2 Screening System, Vivid^{®} CYP2D6 Cyan Screening Kit and Vivid^{®} CYP3A4 Red Screening Kit were used in this test, and the inhibitory effects of the compounds on CYP1A2, CYP2D6 and CYP3A4 were determined according to the instructions of the kit. The results are shown in Table 4.

**Table 4**

| Compound No. | IC₅₀ (µM) | | |
|---|---|---|---|
| | CYP1A2 | CYP2D6 | CYP3A4 |
| 31 | >10 | >10 | >10 |
| 111 | >10 | >10 | >10 |
| 112 | >10 | >10 | >10 |
| 114 | >10 | >10 | >10 |
| 115 | >10 | >10 | >10 |
| 124 | >10 | >10 | >10 |
| 125 | >10 | >10 | >10 |

The results show that Compounds 31, 111, 112, 114, 115, 124 and 125 of the invention have no apparent inhibitory effects on CYP1A2, CYP2D6 and CYP3A4 enzymes.

### Experimental Example 7: In vivo efficacy of the compounds of the invention on bilaterally inoculated CT26 mouse colon cancer subcutaneously transplanted tumor

Test method:
1) CT26 mouse colon cancer cells were cultured in a monolayer in vitro. When the cells were in a logarithmic growth phase, the cells were collected by centrifugation and counted.
2) 0.1 ml of PBS resuspension containing 3×10⁵ CT26 cells was inoculated subcutaneously in the bilateral shoulder blades of mice respectively. When the bilateral scapular tumors of the mice grew to a volume of about 100 mm³, the mice were randomly divided into 4 groups according to the bilateral tumor volumes, 8 mice in each group.
3) Compound 115s was injected into the tumor at the right scapular tumor of mice at 10 µg/time, 50 µg/time, and 250 µg/time, respectively, twice a week for two consecutive weeks. The bilateral tumor volumes and the body weight of the animals were measured.
4) Tumor inhibition rate, TGI (%), was calculated based on the tumor volumes to evaluate the inhibitory effect of Compound 115s on the growth of transplanted tumors in the mice. The calculation formula was: TGI (%) = 100% - (T_{Vt} - T_{V0}) / (C_{Vt} - C_{V0}) × 100%. When the tumor was regressing, the calculation formula was: TGI (%) = 100% - (T_{Vt} - T_{V0}) / T_{V0} × 100%, wherein T_{V0} was the average tumor volume of the tested compound group at the time of grouping; T_{Vt} was the average tumor volume of the tested compound group at the time of measurement on the t^{th} day; Cvo was the average tumor volume of the vehicle group at the time of grouping; and Cvt was the average tumor volume of the vehicle group at the time of measurement on the t^{th} day. For statistical analysis of differences between the two groups, student's t-test was used to calculate the P value.

The results are shown in Tables 5A and 5B.

**Table 5A. Inhibitory effect of Compound 115s on subcutaneous colon tumor in CT26 mice at the administration side**

| Group | tumor volume (mm³) | | TGI (%) | P value *(vs.* vehicle group) |
|---|---|---|---|---|
| | day 0 | day 14 | | |
| vehicle | 76.35±5.11 | 1959.38±142.67 | / | / |
| 10 (µg/time | 74.63±6.26 | 499.05±211.28 | 77.46 | <0.0001 |
| 50 µg/time | 74.03±4.69 | 61.66±41.15 | 144.99 | <0.0001 |
| 250 µg/time | 74.00±4.41 | 0.00±0.00 | 200.00 | <0.0001 |

| | | | | |
|---|---|---|---|---|
| "/" means not present. | | | | |

**Table 5B. Inhibitory effect of Compound 115s on subcutaneous colon tumor in CT26 mice at the non-administration side**

| Group | tumor volume (mm³) | | TGI (%) | P value (vs. vehicle group) |
|---|---|---|---|---|
| | day 0 | day 14 | | |
| vehicle | 86.98±5.69 | 2027.44±112.81 | / | / |
| 10 µg/time | 85.21±7.40 | 1269.99±140.11 | 38.94 | <0.0001 |
| 50 µg/time | 85.31±6.01 | 869.87±99.95 | 59.57 | <0.0001 |
| 250 µg/time | 85.17±6.28 | 462.85±52.91 | 80.54 | <0.0001 |

| | | | | |
|---|---|---|---|---|
| "/" means not present. | | | | |

The results in Tables 5A and 5B show that in the bilateral inoculated CT26 subcutaneously transplanted tumor model, intratumoral injection of Compound 115s of the invention, compared with the vehicle group, has a significant dose-dependent anti-tumor effect on the subcutaneous tumor at the administration side, wherein for the 10 µg/time dose group, the administration side showed complete regression of the subcutaneous tumors in 2 among 8 mice, for the 50 µg/time dose group, the administration side showed complete regression of the subcutaneous tumors in 6 among 8 mice, and for the 250 µg/time dose group, the administration side showed complete regression of the subcutaneous tumors in 8 among 8 mice. Moreover, Compound 115s of the invention also has a significant dose-dependent tumor inhibitory effect on the non-administration side tumors.

In summary, intratumoral injection of Compound 115s of the invention has a significant dose-dependent tumor inhibitory effect on bilateral CT26 subcutaneous tumors.

Various modifications of the invention in addition to those described herein above will become apparent to those skilled in the art from the foregoing description. Such modifications are intended to be covered within the scope of the appended claims. Each of the references, including all patents, patent applications, journal articles, books and any other disclosure, referred to herein is hereby incorporated by reference in its entirety.

## Claims

1. A compound having the structure of Formula I, a stereoisomer, tautomer or mixture thereof, a pharmaceutically acceptable salt, co-crystal, polymorph or solvate thereof, or a stable isotope derivative, metabolite or prodrug thereof: 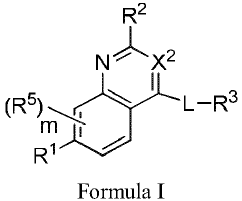 wherein:
X² is selected from the group consisting of C-L-R³ and N;
R¹ is selected from the group consisting of C₁₋₈ alkyl, C₃₋₈ cycloalkyl, 4- to 10-membered heterocyclyl, C₆₋₁₂ aryl, 5-to 10-membered heteroaryl and 9- to 12-membered aryl fused heterocyclyl, wherein the C₁₋₈ alkyl, C₃₋₈ cycloalkyl, 4- to 10-membered heterocyclyl, C₆₋₁₂ aryl, 5- to 10-membered heteroaryl and 9- to 12-membered aryl fused heterocyclyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², OC(O)R³⁰, OC(O)NR³¹R³², NR³³C(O)NR³¹R³², NR³³C(O)OR³⁰, S(O)NR³¹R³², S(O)₂NR³¹R³², S(O)R³⁵, S(O)₂R³⁵, OR³⁷ and SR³⁷;
R² is selected from the group consisting of H, NR^{41a}R^{41b}, C₁₋₈ alkyl, C₂₋₈ alkenyl, C₂₋₈ alkynyl, C₃₋₈ cycloalkyl and 4-to 10-membered heterocyclyl, wherein the C₁₋₈ alkyl, C₂₋₈ alkenyl, C₂₋₈ alkynyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, CN, NO₂, OR³⁷, SR³⁷, C(O)R³⁰, C(O)NR³¹R³², NR³³C(O)R³⁴, C(O)OR³⁰, OC(O)R³⁰, OC(O)NR³¹R³², NR³³C(O)NR³¹R³² and NR³¹R³²; preferably, R² is C₁₋₆ alkyl, preferably C₁₋₄ alkyl, and more preferably methyl;
R³ at each occurrence is independently selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, C(=NR³⁸)NR³¹R³², NR³³C(=NR³⁸)NR³¹R³², P(R³⁹)₂, P(OR³⁹)₂, P(O)R³⁹R⁴⁰, P(O)OR³⁹OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂,| C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³²_{,} NR³³C(O)NR³¹R³², NR³³C(O)OR³⁰, C(=NR³⁸)NR³¹R³², NR³³C(=NR³⁸)NR³¹R³², =NNR³¹R³², P(R³⁹)₂, P(OR³⁹)₂, P(O)R³⁹R⁴⁰ and P(O)OR³⁹OR³⁰;
R⁵ is null or selected from the group consisting of halogen, C₁₋₆ alkyl, C₁₋₄ alkoxy, C₃₋₈ cycloalkyl and 4- to 10-membered heterocyclyl, wherein the C₁₋₆ alkyl, C₁₋₄ alkoxy, C₃₋₈ cycloalkyl and 4- to 10-membered heterocyclyl are each optionally substituted by one or more of the following groups: halogen, OH, CN, C₁₋₄ alkoxy, C₁₋₄ hydroxyalkyl and NR³¹R³²; preferably, R⁵ is null or selected from the group consisting of halogen, C₁₋₄ alkyl and C₃₋₆ cycloalkyl, wherein the C₁₋₄ alkyl and C₃₋₆ cycloalkyl are each optionally substituted by one or more of the following groups: halogen, OH, CN, C₁₋₄ alkoxy, C₁₋₄ hydroxyalkyl and NR³¹R³²; and more preferably, R⁵ is null or F;
m is 0, 1 or 2, preferably 0 or 1;
L is -(L¹)ₙ-(L²)ₚ-(L³)_{q}-, wherein L¹, L² and L³ are the same or different and at each occurrence are each independently selected from the group consisting of C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl, C₁₋₄ alkoxy and NR³¹R³²;
n, p and q are each independently 0, 1 or 2 at each occurrence;
R³⁰, R³⁷, R³⁹ and R⁴⁰ are each independently selected from the group consisting of H, C₁₋₈ alkyl (e.g., C₁₋₆ alkyl or C₁₋₄ alkyl), C₁₋₈ alkoxy (e.g., C₁₋₆ alkoxy or C₁₋₄ alkoxy), C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl) are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)R³⁵, S(O)₂R³⁵, S(O)NR³¹R³² and S(O)₂NR³¹R³²;
R³¹, R³², R³³ and R³⁴ are each independently selected from the group consisting of H, C₁₋₈ alkyl, C₁₋₈ alkoxy, C₃₋₈ cycloalkyl, 4- to 10-membered heterocyclyl, C₆₋₁₂ aryl and 5- to 10-membered heteroaryl, or R³¹ and R³² together with the N atom to which they are attached form a 4- to 8-membered heterocyclyl, or R³³ and R³⁴ together with the C and N atoms to which they are attached form a 4- to 8-membered heterocyclyl, wherein the C₁₋₈ alkyl, C₁₋₈ alkoxy, C₃₋₈ cycloalkyl, 4- to 8-membered heterocyclyl, 4- to 10-membered heterocyclyl, C₆₋₁₂ aryl and 5- to 10-membered heteroaryl are each optionally substituted by one or more of the following substituents: OH, CN, halogen, NO₂, C₁₋₄ alkyl, C₁₋₄ alkoxy, C₁₋₄ hydroxyalkyl, C₁₋₄ haloalkyl, C₁₋₄ haloalkoxy, 4- to 10-membered heterocyclyl, C₆₋₁₂ aryl and 5- to 10-membered heteroaryl;
R³⁵ is selected from the group consisting of C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl and 5- to 10-membered heteroaryl are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)Me, S(O)₂Me, S(O)NR³¹R³² and S(O)₂NR³¹R³², wherein R³¹, R³², R³³ and R³⁴ are as defined above;
R^{36a} and R^{36b} are the same or different and are each independently selected from the group consisting of H, C₁₋₈ alkyl and C₁₋₈ alkoxy, wherein the C₁₋₈ alkyl and C₁₋₈ alkoxy are each optionally substituted by one or more of the following groups: OH, CN, halogen, NH₂, NHCH₃ and N(CH₃)₂, or R^{36a} and R^{36b} together with the C atom to which they are attached form 3- to 7-membered cycloalkyl or heterocyclyl;
R³⁸ is selected from the group consisting of H, OH, CN, NO₂, S(O)R³⁵ and S(O)₂R³⁵;
R^{41a} and R^{41b} are each independently selected from the group consisting of H, C₁₋₆ alkyl, C₁₋₆ alkoxy and C₃₋₈ cycloalkyl, or R^{41a} and R^{41b} together with the N atom to which they are attached form a 4- to 7-membered heterocyclyl, wherein the C₁₋₆ alkyl, C₁₋₆ alkoxy, C₃₋₈ cycloalkyl and 4- to 7-membered heterocyclyl are each optionally substituted by one or more of the following groups: OH, CN and NR³¹R³², and R^{41a} and R^{41b} are not simultaneously H; and
when multiple R³⁰ are simultaneously present, each R³⁰ may be the same or different;
when multiple R³¹ are simultaneously present, each R³¹ may be the same or different;
when multiple R³² are simultaneously present, each R³² may be the same or different;
when multiple R³³ are simultaneously present, each R³³ may be the same or different;
when multiple R³⁴ are simultaneously present, each R³⁴ may be the same or different;
when multiple R³⁵ are simultaneously present, each R³⁵ may be the same or different;
when multiple R³⁷ are simultaneously present, each R³⁷ may be the same or different;
when multiple R³⁸ are simultaneously present, each R³⁸ may be the same or different;
when multiple R³⁹ are simultaneously present, each R³⁹ may be the same or different;
when multiple R⁴⁰ are simultaneously present, each R⁴⁰ may be the same or different;
preferably, the compound has the structure of Formula III:

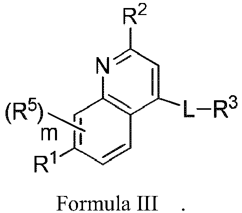

2. The compound according to claim 1, a stereoisomer, tautomer or mixture thereof, a pharmaceutically acceptable salt, co-crystal, polymorph or solvate thereof, or a stable isotope derivative, metabolite or prodrug thereof, wherein the compound has the structure of Formula III-A or Formula III-B: 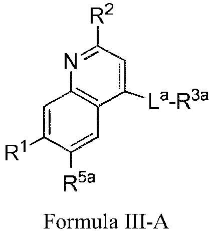 wherein:
R^{5a} is H, C₁₋₃ alkyl, F or Cl;
L^{a} is -L^{1a}-(L²)ₚ-(L³)_{q}-, wherein L^{1a} is O, S or NR³³;
R^{3a} is selected from the group consisting of C₁₋₈ alkyl, C₃₋₈ cycloalkyl, 4- to 10-membered heterocyclyl, C₆₋₁₂ aryl, 5-to 10-membered heteroaryl, 9- to 12-membered aryl fused heterocyclyl, 9- to 12-membered aryl fused heteroaryl, 9-to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³² NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9-to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³² NR³³C(O)NR³¹R³² and NR³³C(O)OR³⁰; and
the remaining groups are as defined in claim 1; 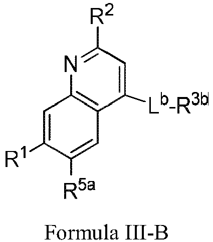 wherein:
R^{5a} is H, C₁₋₃ alkyl, F or Cl;
L^{b} is -(L^{1b|})ₙ-(L^{2b})ₚ-(L^{3b})_{q}-, wherein L^{1b}, L^{2b} and L^{3b} are the same or different and are each independently selected from the group consisting of C₁₋₈ alkylene, C₂₋₈ alkenylene, C₂₋₈ alkynylene, C₁₋₈ alkyleneoxy, C₃₋₈ cycloalkylene, 4-to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl and C₁₋₄ alkoxy;
n, p and q are each independently 0, 1 or 2;
R^{3b} is selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³¹, OC(O)NR³¹R³² NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³² NR³³C(O)NR³¹R³² and NR³³C(O)OR³⁰;
the remaining groups are as defined in claim 1; and
for Formula III-B, provided that:
when n+p+q≥1, L^{1b} or L^{2b} or L^{3b} of -(L^{1b})ₙ-(L^{2b})ₚ-(L^{3b})_{q}- attached to the C atom of the pyridine ring in Formula III-B is not O, S, NR³³, S(O), S(O)₂ or C(O);
when n+p+q=0, R^{3b} is not H, halogen, CN, NO2, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³², NR³³C(O)NR³¹R³², NR³³C(O)OR³⁰, S(O)R³⁵, S(O)NR³¹R³² or S(O)₂NR³¹R³².

3. A compound having the structure of Formula II, a stereoisomer, tautomer or mixture thereof, a pharmaceutically acceptable salt, co-crystal, polymorph or solvate thereof, or a stable isotope derivative, metabolite or prodrug thereof: 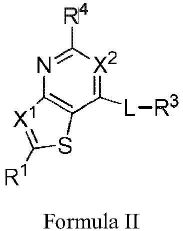 wherein:
X¹ is selected from the group consisting of CR⁶ and N,
X² is selected from the group consisting of C-L-R³ and N,
R¹ is selected from the group consisting of C₁₋₈ alkyl, C₃₋₈ cycloalkyl, 4- to 10-membered heterocyclyl, C₆₋₁₂ aryl, 5-to 10-membered heteroaryl and 9- to 12-membered aryl fused heterocyclyl, wherein the C₁₋₈ alkyl, C₃₋₈ cycloalkyl, 4- to 10-membered heterocyclyl, C₆₋₁₂ aryl, 5- to 10-membered heteroaryl and 9- to 12-membered aryl fused heterocyclyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², OC(O)R³⁰, OC(O)NR³¹R^{3a}, NR³³C(O)NR³¹R³², NR³³C(O)OR³⁰, S(O)NR³¹R³², S(O)₂NR³¹R³², S(O)R³⁵, S(O)₂R³⁵, OR³⁷ and SR³⁷;
R³ at each occurrence is independently selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³² NR³³C(O)NR³¹R³², NR³³C(O)OR³⁰, C(=NR³⁸)NR³¹R³², NR^{33C}(=NR³⁸)NR³¹R³², P(R³⁹)₂, P(OR³⁹)₂, P(O)R³⁹R⁴⁰ P(O)OR³⁹OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³², NR³³C(O)NR³¹R³², NR³³C(O)OR³⁰, C(=NR³⁸)NR³¹R³², NR³³C(=NR³⁸)NR³¹R³², =NNR³¹R³², P(R³⁹)₂, P(OR³⁹)₂, P(O)R³⁹R⁴⁰ and P(O)OR³⁹OR³⁰;
R⁴ is selected from the group consisting of H, NR^{41a}R^{41b}, C₁₋₁₅ alkyl, C₁₋₈ alkoxy, C₂₋₈ alkenyl, C₂₋₈ alkynyl, C₃₋₈ cycloalkyl and 4- to 10-membered heterocyclyl, wherein the C₁₋₁₅ alkyl, C₁₋₈ alkoxy, C₂₋₈ alkenyl, C₂₋₈ alkynyl, C₃₋₈ cycloalkyl and 4- to 10-membered heterocyclyl are each optionally substituted by one or more of the following substituents: halogen, C₁₋₄ haloalkyl, C₁₋₄ hydroxyalkyl, 4- to 7-membered heterocyclyl, CN, NO₂, OR³⁷, SR³⁷, C(O)R³⁰, C(O)NR³¹R³², NR³³C(O)R³⁴, C(O)OR³⁰, OC(O)R³⁰, OC(O)NR³¹R³², NR³³C(O)NR³¹R³² and NR³¹R³²; preferably, R⁴ is C₁₋₆ alkyl, preferably C₁₋₄ alkyl, more preferably methyl; or R⁴ is NR^{41a}R^{41b}, wherein R^{41a} and R^{41b} are each independently selected from the group consisting of H, C₁₋₄ alkyl and C₃₋₆ cycloalkyl, more preferably R⁴ is NH₂;
R⁶ is selected from the group consisting of H, halogen, C₁₋₆ alkyl, C₁₋₄ alkoxy, C₃₋₈ cycloalkyl and 4- to 10-membered heterocyclyl, wherein the C₁₋₆ alkyl, C₁₋₄ alkoxy, C₃₋₈ cycloalkyl and 4- to 10-membered heterocyclyl are each optionally substituted by one or more of the following groups: halogen, OH, CN, C₁₋₄ alkoxy, C₁₋₄ hydroxyalkyl and NR³¹R³²; preferably, R⁶ is H, halogen, C₁₋₄ alkyl or C₃₋₆ cycloalkyl, and more preferably, R⁶ is H, Cl or methyl;
L is -(L¹)ₙ-(L²)ₚ-(L³)_{q}-, wherein L¹, L² and L³ are the same or different and at each occurrence are each independently selected from the group consisting of C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl, C₁₋₄ alkoxy and NR³¹R³²;
n, p and q are each independently 0, 1 or 2 at each occurrence;
R³⁰, R³⁷, R³⁹ and R⁴⁰ are each independently selected from the group consisting of H, C₁₋₈ alkyl (e.g., C₁₋₆ alkyl or C₁₋₄ alkyl), C₁₋₈ alkoxy (e.g., C₁₋₆ alkoxy or C₁₋₄ alkoxy), C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl) are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁-₆ alkyl), CONR³¹R³², NR³¹R³², NR³³C(O)R³⁴, S(O)R³⁵, S(O)₂R³⁵, S(O)NR³¹R³² and S(O)₂NR³¹R³²;
R³¹, R³², R³³ and R³⁴ are each independently selected from the group consisting of H, C₁₋₈ alkyl, C₁₋₈ alkoxy, C₃₋₈ cycloalkyl, 4- to 10-membered heterocyclyl, C₆₋₁₂ aryl and 5- 10-membered heteroaryl, or R³¹ and R³² together with the N atom to which they are attached form a 4- to 8-membered heterocyclyl, or R³³ and R³⁴ together with the C and N atoms to which they are attached form a 4- to 8-membered heterocyclyl, wherein the C₁₋₈ alkyl, C₁₋₈ alkoxy, C₃₋₈ cycloalkyl, 4- to 8-membered heterocyclyl, 4- to 10-membered heterocyclyl, C₆₋₁₂ aryl and 5- to 10-membered heteroaryl are each optionally substituted by one or more of the following substituents: OH, CN, halogen, NO₂, C₁₋₄ alkyl, C₁₋₄ alkoxy, C₁₋₄ hydroxyalkyl, C₁₋₄ haloalkyl, C₁₋₄ haloalkoxy, 4- to 10-membered heterocyclyl, C₆₋₁₂ aryl and 5- to 10-membered heteroaryl;
R³⁵ is selected from the group consisting of C₁₋₈ alkyl, C₁₋₈ alkoxy, C₃₋₈ cycloalkyl, 4- to 10-membered heterocyclyl, C₆₋₁₂ aryl, 5- to 10-membered heteroaryl, -C₁₋₈ alkyl-C₆₋₁₂ aryl and -C₁₋₈ alkyl-(5- to 10-membered heteroaryl), wherein the C₁₋₈ alkyl, C₁₋₈ alkoxy, C₃₋₈ cycloalkyl, 4- to 10-membered heterocyclyl, C₆₋₁₂ aryl and 5- to 10-membered heteroaryl are each optionally substituted by one or more of the following substituents: OH, CN, NO₂, C₁₋₄ alkyl, C₁₋₄ alkoxy, C₁₋₄ haloalkyl, halogen, C₁₋₄ haloalkoxy, CO₂(C₁₋₆ alkyl), CONR³¹R³², NR³¹R³², NR³³C(O)R³⁴, S(O)Me, S(O)₂Me, S(O)NR³¹R³² and S(O)₂NR³¹R³², wherein R³¹, R³², R³³ and R³⁴ are as defined above;
R^{36a} and R^{36b} are the same or different and are each independently selected from the group consisting of H, C₁₋₈ alkyl and C₁₋₈ alkoxy, wherein the C₁₋₈ alkyl and C₁₋₈ alkoxy are each optionally substituted by one or more of the following groups: OH, CN, halogen, NH₂, NHCH₃ and N(CH₃)₂, or R^{36a} and R^{36b} together with the C atom to which they are attached form 3- to 7-membered cycloalkyl or heterocyclyl;
R³⁸ is selected from the group consisting of H, OH, CN, NO₂, S(O)R³⁵ and S(O)₂R³⁵;
R^{41a} and R^{41b} are each independently selected from the group consisting of H, C₁₋₆ alkyl, C₁₋₆ alkoxy and C₃₋₈ cycloalkyl, or R^{41a} and R^{41b} together with the N atom to which they are attached form a 4- to 7-membered heterocyclyl, wherein the C₁₋₆ alkyl, C₁₋₆ alkoxy, C₃₋₈ cycloalkyl and 4- to 7-membered heterocyclyl may optionally be substituted by one or more of the following groups: OH, CN and NR³¹R³²; and
when multiple R³⁰ are simultaneously present, each R³⁰ may be the same or different;
when multiple R³¹ are simultaneously present, each R³¹ may be the same or different;
when multiple R³² are simultaneously present, each R³² may be the same or different;
when multiple R³³ are simultaneously present, each R³³ may be the same or different;
when multiple R³⁴ are simultaneously present, each R³⁴ may be the same or different;
when multiple R³⁵ are simultaneously present, each R³⁵ may be the same or different;
when multiple R³⁷ are simultaneously present, each R³⁷ may be the same or different;
when multiple R³⁸ are simultaneously present, each R³⁸ may be the same or different;
when multiple R³⁹ are simultaneously present, each R³⁹ may be the same or different;
when multiple R⁴⁰ are simultaneously present, each R⁴⁰ may be the same or different;
preferably, the compound has the structure of Formula IV:

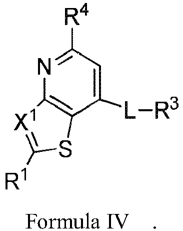

4. The compound according to claim 3, a stereoisomer, tautomer or mixture thereof, a pharmaceutically acceptable salt, co-crystal, polymorph or solvate thereof, or a stable isotope derivative, metabolite or prodrug thereof, wherein the compound has the structure of Formula IV-A or Formula IV-B: 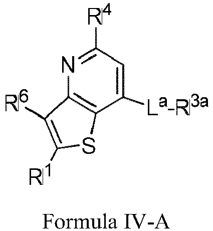 wherein:
L^{a} is -L^{1a}-(L²)ₚ-(L³)_{q}-, wherein L^{1a} is O, S or NR³³;
R^{3a} is selected from the group consisting of C₁₋₈ alkyl, C₃₋₈ cycloalkyl, 4- to 10-membered heterocyclyl, C₆₋₁₂ aryl, 5-to 10-membered heteroaryl, 9- to 12-membered aryl fused heterocyclyl, 9- to 12-membered aryl fused heteroaryl, 9-to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³² NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9-to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³² NR³³C(O)NR³¹R³² and NR³³C(O)OR³⁰; and
the remaining groups are as defined in claim 3; 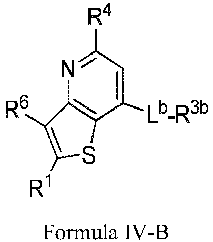 wherein:
L^{b} is -(L^{1b|})ₙ-(L^{2b})ₚ-(L^{3|b})_{q}-, wherein L^{1b}, L^{2b} and L^{3b} are the same or different and are each independently selected from the group consisting of C₁₋₈ alkylene, C₂₋₈ alkenylene, C₂₋₈ alkynylene, C₁₋₈ alkyleneoxy, C₃₋₈ cycloalkylene, 4-to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₁₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl and C₁₋₄ alkoxy;
n, p and q are each independently 0, 1 or 2;
R^{3b} is selected from the group consisting of H, halogen, CN, NO₂, C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl, 9- to 12-membered aryl fused cycloalkyl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³² NR³³C(O)NR³¹R³², NR³³C(O)OR³⁰, S(O)R³⁵, S(O)NR³¹R³² and S(O)₂NR³¹R³², wherein the C₁₋₈ alkyl, C₃₋₈ cycloalkyl, 4- to 10-membered heterocyclyl, C₆₋₁₂ aryl, 5- to 10-membered heteroaryl, 9- to 12-membered aryl fused heterocyclyl, 9- to 12-membered aryl fused heteroaryl and 9- to 12-membered aryl fused cycloalkyl are each optionally substituted by one or more of the following substituents: halogen, CN, NO₂, C₁₋₄ alkyl, C₃₋₈ cycloalkyl, C₁₋₄ haloalkyl, C₁₋₄ hydroxyalkyl, 4- to 10-membered heterocyclyl, C₆₋₁₂ aryl, 5- to 10-membered heteroaryl, 9- to 12-membered aryl fused heterocyclyl, CO₂R³⁰, C(O)R³⁰, C(O)NR³¹R³², NR³³C(O)R³⁴, NR³¹R³², S(O)R³⁵, S(O)₂R³⁵, S(O)NR³¹R³², S(O)₂NR³¹R³², OR³⁷, SR³⁷, OC(O)R³⁰, OC(O)NR³¹R³² NR³³C(O)NR³¹R³² and NR³³C(O)OR³⁰;
the remaining groups are as defined in claim 3; and
for Formula IV-B, provided that:
when n+p+q≥1, L^{1b} or L^{2b} or L^{3b} of -(L^{1b})ₙ-(L^{2b})ₚ-(L^{3b})_{q}- attached to the C atom of the pyridine ring in Formula IV-B is not O, S, NR³³, S(O), S(O)₂ or C(O);
when n+p+q=0, R^{3b} is not H, halogen, CN, NO₂, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, OC(O)NR³¹R³² NR³³C(O)NR³¹R³², NR³³C(O)OR³⁰, S(O)R³⁵, S(O)NR³¹R³² or S(O)₂NR³¹R³².

5. The compound according to claim 1 or 3, a stereoisomer, tautomer or mixture thereof, a pharmaceutically acceptable salt, co-crystal, polymorph or solvate thereof, or a stable isotope derivative, metabolite or prodrug thereof, wherein
R³ at each occurrence is independently selected from the group consisting of H, halogen, CN, C₁₋₆ alkyl, C₃₋₆ cycloalkyl, 4- to 7-membered heterocyclyl, C₆₋₁₀ aryl, 5- to 6-membered heteroaryl, CO₂R³⁰, C(O)NR³¹R³², NR³³C(O)R³⁴, NR³¹R³², S(O)₂R³⁵, OR³⁷, SR³⁷, C(O)R³⁰, OC(O)R³⁰, NR³³C(O)NR³¹R³² and S(O)₂NR³¹R³², wherein the C₁₋₆ alkyl, C₃₋₆ cycloalkyl, 4- to 7-membered heterocyclyl, C₆₋₁₀ aryl and the 5- to 6-membered heteroaryl are each optionally substituted by one or more of the following substituents: halogen, CN, C₁₋₄ alkyl, C₃₋₆ cycloalkyl, C₁₋₄ haloalkyl, C₁₋₄ hydroxyalkyl, 4- to 7-membered heterocyclyl, C₆₋₁₀ aryl, 5- to 6-membered heteroaryl, CO₂R³⁰, C(O)R³⁰, C(O)NR³¹R³², NR³³C(O)R³⁴, NR³¹R³², S(O)₂R³⁵, S(O)₂NR³¹R³², OR³⁷, SR³⁷ and NR³³C(O)NR³¹R³²;
preferably, R³ at each occurrence is independently selected from the group consisting of H, halogen, CN, C₁₋₆ alkyl, C₃₋₆ cycloalkyl, 4- to 7-membered heterocyclyl, C₆₋₁₀ aryl, 5- to 6-membered heteroaryl, C(O)NR³¹R³², NR³³C(O)R³⁴, S(O)₂R³⁵, OR³⁷ and C(O)R³⁰, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₆₋₁₀ aryl, C(O)R³⁰, S(O)₂R³⁵ and OR³⁷;
preferably, R³ at each occurrence is independently selected from the group consisting of methyl, difluoromethyl, trifluoromethyl, ethyl, propyl, methoxy, cyclopropyl, cyclobutyl, cyclopentyl, piperazinyl, morpholinyl, piperidinyl, 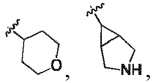 tetrahydrofuranyl, tetrahydropyrrolyl, 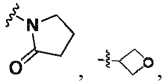 pyrazolyl, pyridyl, pyridazinyl, phenyl, 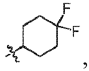 CN, OH, C(O)R³⁰, S(O)₂CH₃ and 3,6-diazabicyclo[3.1.1]hept-3-yl, each of which is optionally substituted by one or more of the following substituents: C₁₋₄ alkyl, C₃₋₁₆ cycloalkyl, S(O)₂R³⁵, OR³⁷, C(O)R³⁰ and phenyl; preferably, R³ at each occurrence is independently selected from the group consisting of methyl, difluoromethyl, trifluoromethyl, ethyl, propyl, methoxy, S(O)₂R³⁵, cyclopropyl, cyclobutyl, cyclopentyl, 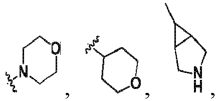 tetrahydrofuranyl, tetrahydropyrrolyl, 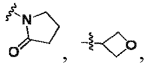 pyrazolyl, pyridyl, pyridazinyl, phenyl, 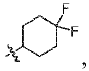 CN, OH and C(O)R³⁰; or R³ at each occurrence is independently selected from the group consisting of piperazinyl, morpholinyl, piperidinyl, tetrahydropyrrolyl and 3,6-diazabicyclo[3.1.1]hept-3-yl, each of which is optionally substituted by one or more of the following substituents: C₁₋₄ alkyl, C₃₋₆ cycloalkyl, S(O)₂R³⁵, OR³⁷, C(O)R³⁰ and phenyl, and which are preferably selected from the group consisting of methyl, propyl, cyclopropyl, cyclopentyl, cyclohexyl, S(O)₂CH₃, methoxy, -OH, -C(O)H and phenyl; and more preferably, R³ at each occurrence is independently selected from the group consisting of methyl, difluoromethyl, trifluoromethyl, ethyl, methoxy, cyclopropyl, cyclobutyl, cyclopentyl, 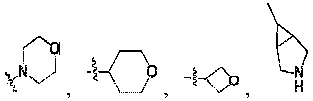 (e.g., 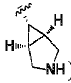 ), tetrahydrofuranyl, pyrazolyl, pyridyl, phenyl, CN and OH.

6. The compound according to claim 1, 3 or 5, a stereoisomer, tautomer or mixture thereof, a pharmaceutically acceptable salt, co-crystal, polymorph or solvate thereof, or a stable isotope derivative, metabolite or prodrug thereof, wherein
L is -(L¹)ₙ-(L²)ₚ-(L³)_{q}-, wherein L¹, L² and L³ are the same or different and at each occurrence are each independently selected from the group consisting of C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene, 5- to 10-membered heteroarylene, O, S, NR³³, S(O), S(O)₂, C(O) and C(R^{36a}R^{36b}), wherein the C₁₋₈ alkylene, C₂₋₈ alkenylene, C₂₋₈ alkynylene, C₁₋₈ alkyleneoxy, C₃₋₈ cycloalkylene, 4- to 10-membered heterocyclylene, C₆₋₁₂ arylene and 5- to 10-membered heteroarylene are each optionally substituted by one or more of the following substituents: halogen, OH, CN, NO₂, C₁₋₆ alkyl, C₁₋₄ haloalkyl, C₁₋₄ hydroxyalkyl, C₁₋₄ alkoxy and NR³¹R³²;
preferably, L is -(L¹)ₙ-(L²)ₚ-(L³)_{q}-, wherein L¹, L² and L³ are the same or different and at each occurrence are each independently selected from the group consisting of C₁₋₄ alkylene (e.g., methylene, ethylene and n-propylene), C₃₋₆ cycloalkylene (e.g., cyclopropylene, cyclobutylene, cyclopentylene, and cyclohexylene), 4- to 6-membered heterocyclylene (e.g., 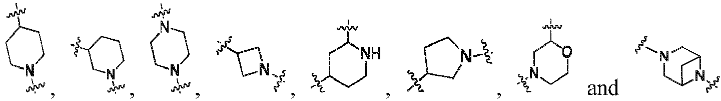 ), 5- to 6-membered heteroarylene (e.g., imidazolylene, pyrazolylene, isoxazolylene and pyridylene), phenylene, O, NH, N(CH₂CH₃), N(CH₃), C(O) and C(R^{36a}R^{36b}), wherein the C₁₋₄ alkylene, C₃₋₆ cycloalkylene, 4- to 6-membered heterocyclylene and 5- to 6-membered heteroarylene are each optionally substituted by one or more of the following substituents: halogen (e.g., F), OH, C₁₋₃ hydroxyalkyl (e.g., hydroxymethyl and hydroxyethyl) and C₁₋₃ alkyl (e.g., methyl and ethyl);
preferably, L is -(L¹)ₙ-(L²)ₚ-(L³)_{q}-, wherein L¹, L² and L³ are the same or different and at each occurrence are each independently selected from the group consisting of methylene, ethylene, n-propylene, cyclopropylene, cyclobutylene, cyclopentylene, cyclohexylene, 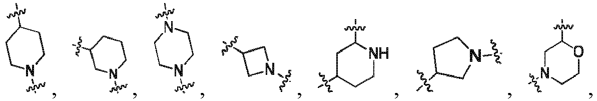 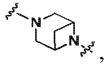 imidazolylene, pyrazolylene, isoxazolylene, pyridylene, phenylene, O, NH, N(CH₂CH₃), N(CH₃) and C(O), wherein the methylene, ethylene, n-propylene, cyclopropylene, cyclobutylene, cyclopentylene, cyclohexylene, 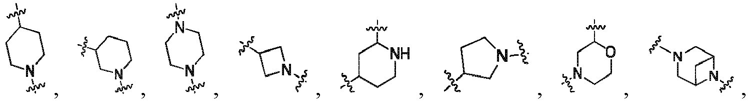 imidazolylene, pyrazolylene, isoxazolylene, pyridylene and phenylene are each optionally substituted by one or more of the following substituents: F, OH, hydroxymethyl, hydroxyethyl, methyl and ethyl; and n, p and q are each independently 1 or 2 at each occurrence; and
more preferably, L is -(L¹)ₙ-(L²)ₚ-(L³)_{q}-, wherein L¹, L² and L³ are the same or different and are each independently selected from the group consisting of C₁₋₆ alkylene (e.g., methylene, ethylene and n-propylene) and C₃₋₆ cycloalkylene (e.g., cyclopropylene, cyclobutylene, cyclopentylene and cyclohexylene).

7. The compound according to any one of claims 1 to 6, a stereoisomer, tautomer or mixture thereof, a pharmaceutically acceptable salt, co-crystal, polymorph or solvate thereof, or a stable isotope derivative, metabolite or prodrug thereof, wherein -L-R³, -L^{a}-R^{3a} or -L^{b}-R^{3b} is selected from the group consisting of:

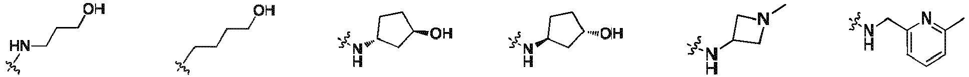

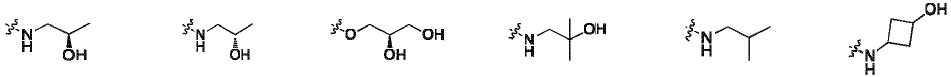

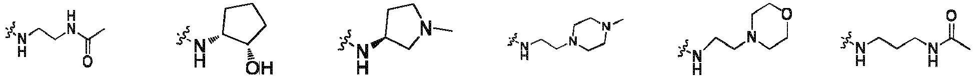

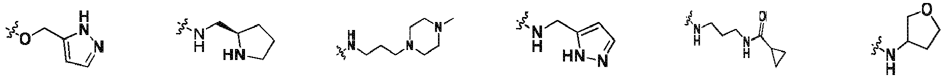

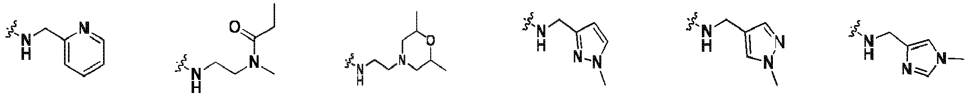

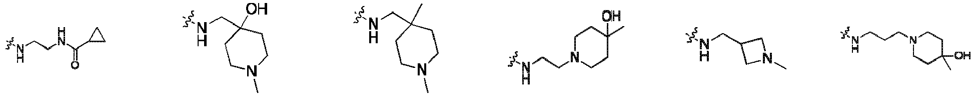

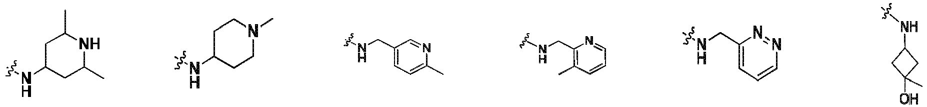

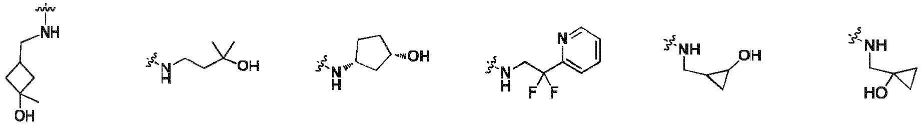

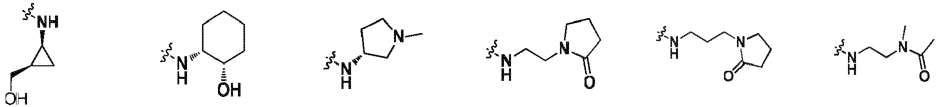

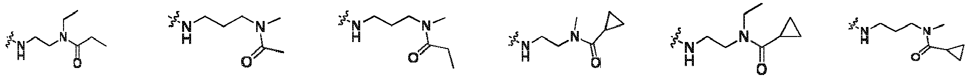

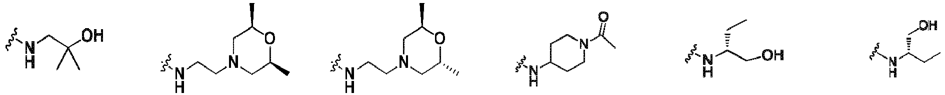

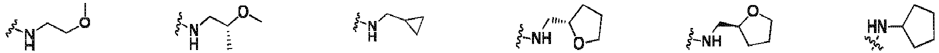

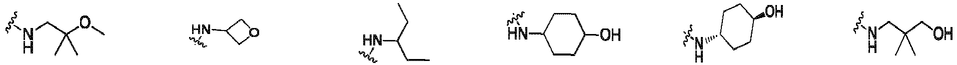

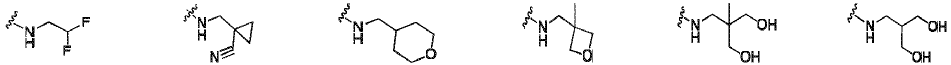

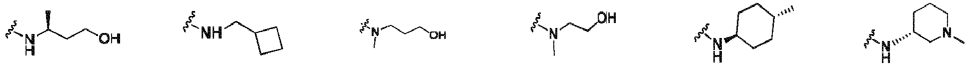

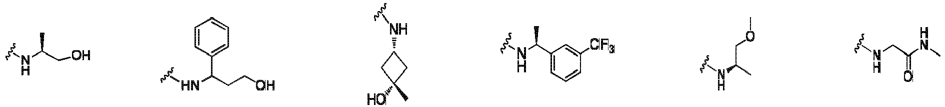

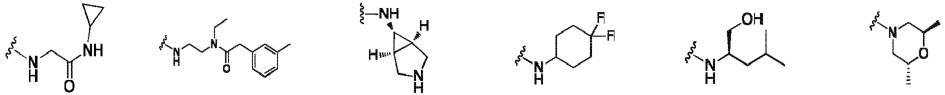

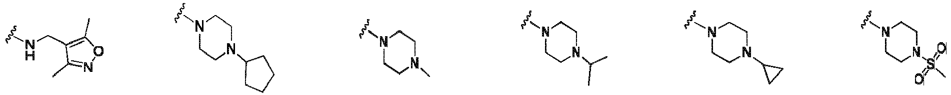

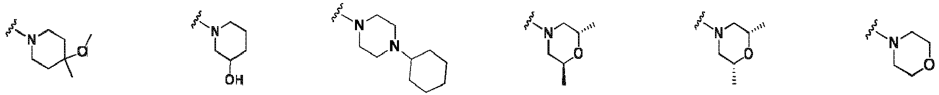

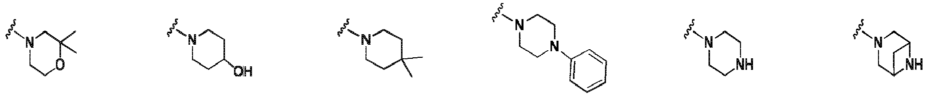

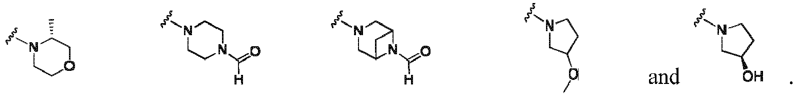

8. The compound according to any one of claims 1 to 7, a stereoisomer, tautomer or mixture thereof, a pharmaceutically acceptable salt, co-crystal, polymorph or solvate thereof, or a stable isotope derivative, metabolite or prodrug thereof, wherein
R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl; and preferably, R¹ is 5- to 6-membered heteroaryl, especially 5- to 6-membered nitrogen-containing heteroaryl such as pyrazolyl or pyridyl, which is optionally substituted by one or more of the following substituents: halogen (preferably F) and C₁₋₃ alkyl (preferably methyl); or R¹ is phenyl.

9. The compound according to claim 4, a stereoisomer, tautomer or mixture thereof, a pharmaceutically acceptable salt, co-crystal, polymorph or solvate thereof, or a stable isotope derivative, metabolite or prodrug thereof, wherein the compound is a compound of Formula IV-A, and wherein
R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl;
R⁴ is NR^{41a}R^{41b}, wherein R^{41a} and R^{41b} are each independently selected from the group consisting of H, C₁₋₄ alkyl and C₃₋₆ cycloalkyl;
R⁶ is selected from the group consisting of H, halogen, C₁₋₆ alkyl and C₃₋₆ cycloalkyl; -L^{a}-R^{3a} is selected from the group consisting of:

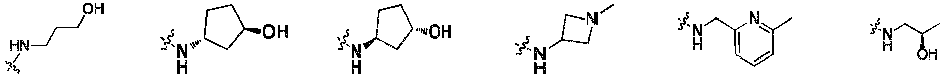

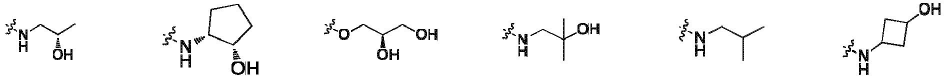

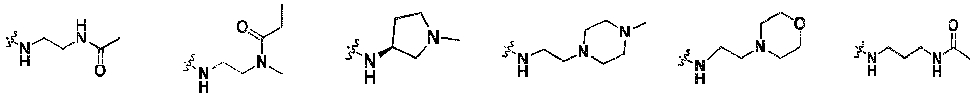

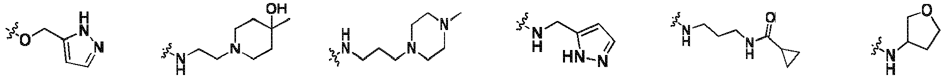

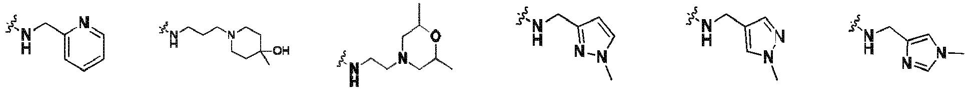

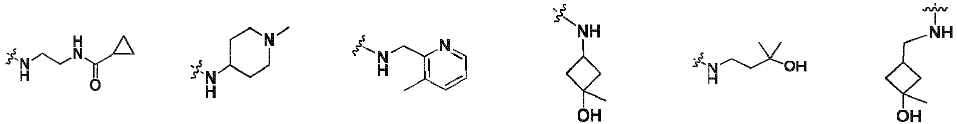

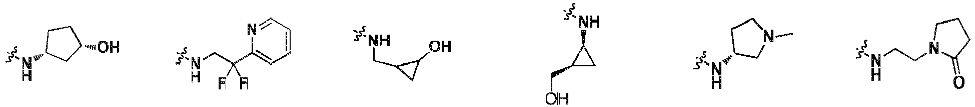

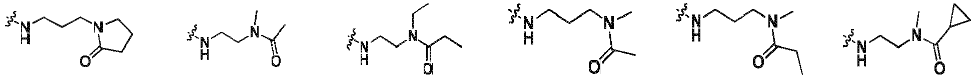

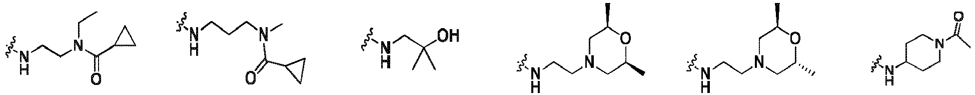

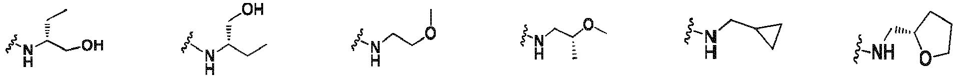

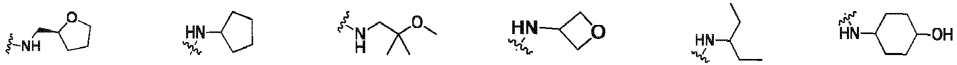

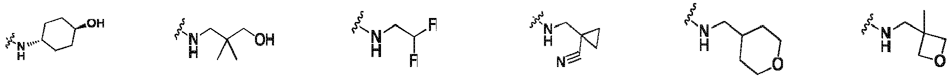

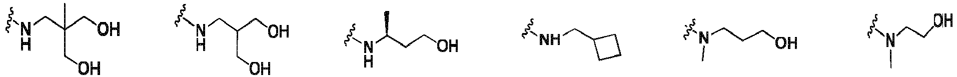

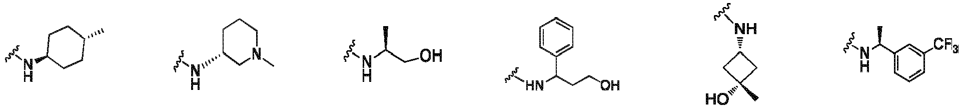

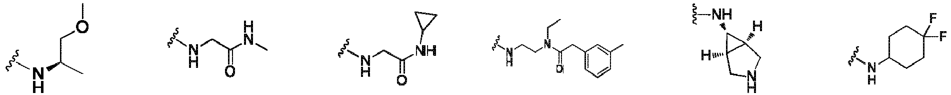

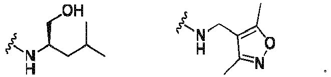

10. The compound according to claim 4, a stereoisomer, tautomer or mixture thereof, a pharmaceutically acceptable salt, co-crystal, polymorph or solvate thereof, or a stable isotope derivative, metabolite or prodrug thereof, wherein the compound is a compound of Formula IV-A, and wherein:
R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl; preferably, R¹ is 5- to 6-membered heteroaryl (especially 5- to 6-membered nitrogen-containing heteroaryl such as pyrazolyl or pyridyl), and the 5- to 6-membered heteroaryl is optionally substituted by one or more of the following substituents: halogen (e.g., F), 4- to 10-membered heterocyclyl (e.g., 2-tetrahydropyranyl) and C₁₋₃ alkyl (e.g., methyl); or R¹ is phenyl;
R⁴ is NR^{41a}R^{41b}, Wherein R^{41a} and R^{41b} are each independently selected from the group consisting of H, C₁₋₄ alkyl and C₃₋₆ cycloalkyl; preferably, R⁴ is NH₂;
R⁶ is selected from the group consisting of H, halogen, C₁₋₄ alkyl and C₃₋₆ cycloalkyl; preferably, R⁶ is H, Cl or methyl;
L^{a} is -NH-(L²)ₚ-(L³)_{q}-, wherein L² and L³ are the same or different and at each occurrence are independently selected from the group consisting of methylene, ethylene, n-propylene, cyclopropylene, cyclobutylene, cyclopentylene, cyclohexylene, 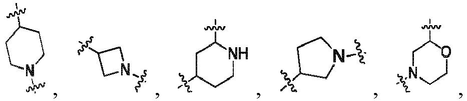 imidazolylene, pyrazolylene, isoxazolylene, pyridylene, phenylene, O, NH, N(CH₂CH₃), N(CH₃) and C(O), wherein the methylene, ethylene, n-propylene, cyclopropylene, cyclobutylene, cyclopentylene, cyclohexylene, 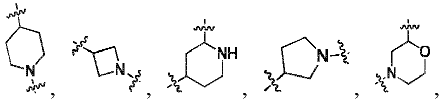 imidazolylene, pyrazolylene, isoxazolylene, pyridylene and phenylene are each optionally substituted by one or more of the following substituents: F, OH, hydroxymethyl, hydroxyethyl, methyl and ethyl; and p and q are each independently 1 or 2 at each occurrence; and
R^{3a} is selected from the group consisting of methyl, difluoromethyl, trifluoromethyl, ethyl, methoxy, cyclopropyl, cyclobutyl, cyclopentyl, 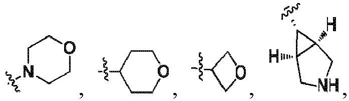 tetrahydrofuranyl, pyrazolyl, pyridyl, phenyl, CN and OH.

11. The compound according to claim 4, a stereoisomer, tautomer or mixture thereof, a pharmaceutically acceptable salt, co-crystal, polymorph or solvate thereof, or a stable isotope derivative, metabolite or prodrug thereof, wherein the compound is a compound of Formula IV-B, and wherein:
R¹ is selected from the group consisting of C₁₋₆ alkyl, C₃₋₆ cycloalkyl, 4- to 7-membered heterocyclyl, C₆₋₁₀ aryl and 5- to 6-membered heteroaryl, wherein the C₁₋₆ alkyl, C₃₋₆ cycloalkyl, 4- to 7-membered heterocyclyl, C₆₋₁₀ aryl and 5- to 6-membered heteroaryl are each optionally substituted by one or more of the following substituents: halogen, CN, C₁₋₄ alkyl, C₃₋₆ cycloalkyl, C₁₋₄ haloalkyl and C₁₋₄ hydroxyalkyl;
R⁴ is NR^{41a}R^{41b}, Wherein R^{41a} and R^{41b} are each independently selected from the group consisting of H, C₁₋₄ alkyl and C₃₋₆ cycloalkyl;
R⁶ is selected from the group consisting of H, halogen, C₁₋₄ alkyl and C₃₋₆ cycloalkyl;
when n+p+q=0, R^{3b} is selected from the group consisting of piperazinyl, morpholinyl, piperidinyl, tetrahydropyrrolyl and 3,6-diazabicyclo[3.1.1]hept-3-yl, each of which is optionally substituted by one or more of the following substituents: C₁₋₄ alkyl, C₃₋₆ cycloalkyl, S(O)₂R³⁵, OR³⁷, C(O)R³⁰ and phenyl; preferably, the substituents are selected from the group consisting of methyl, propyl, cyclopropyl, cyclopentyl, cyclohexyl, S(O)₂CH₃, methoxy, -OH, -C(O)H and phenyl; and
when n+p+q≥1, -L^{b}- is selected from the group consisting of methylene, ethylene, butylene, and R^{3b} is OH.

12. The compound according to claim 11, a stereoisomer, tautomer or mixture thereof, a pharmaceutically acceptable salt, co-crystal, polymorph or solvate thereof, or a stable isotope derivative, metabolite or prodrug thereof, wherein:
R¹ is 5- to 6-membered heteroaryl (especially 5- to 6-membered nitrogen-containing heteroaryl such as pyrazolyl or pyridyl), and the 5- to 6-membered heteroaryl is optionally substituted by one or more of the following substituents: halogen (e.g., F), 4- to 10-membered heterocyclyl (e.g., 2-tetrahydropyranyl) and C₁₋₃ alkyl (e.g., methyl).

13. The compound according to any one of claims 1 to 12, a stereoisomer, tautomer or mixture thereof, a pharmaceutically acceptable salt, co-crystal, polymorph or solvate thereof, or a stable isotope derivative, metabolite or prodrug thereof, wherein the compound is selected from the group consisting of:

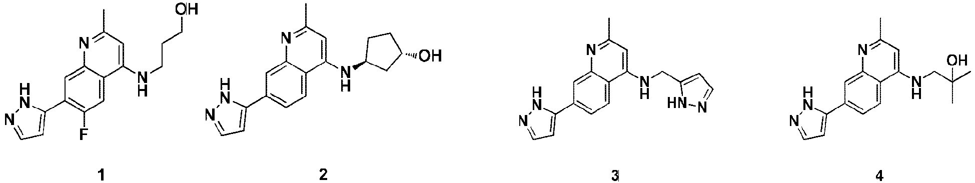

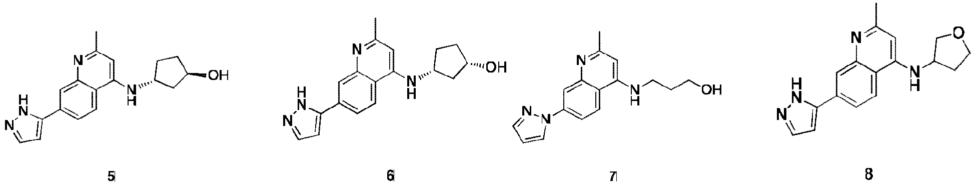

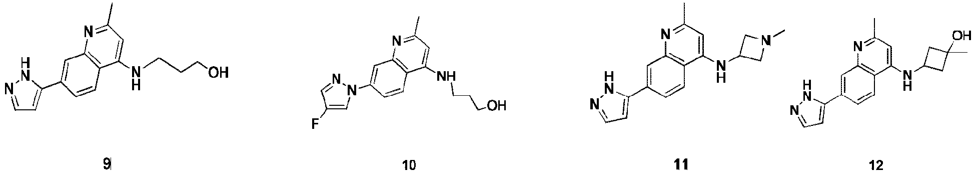

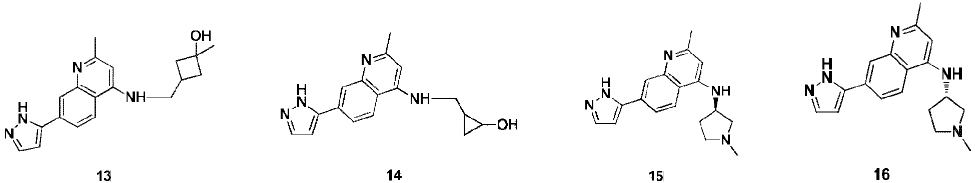

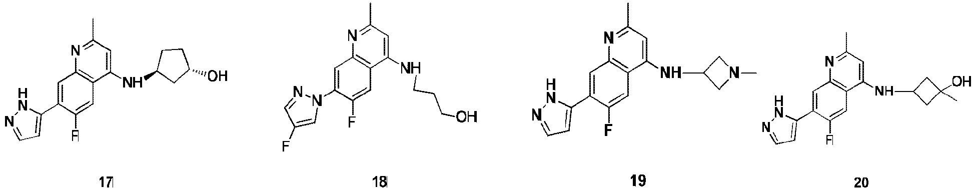

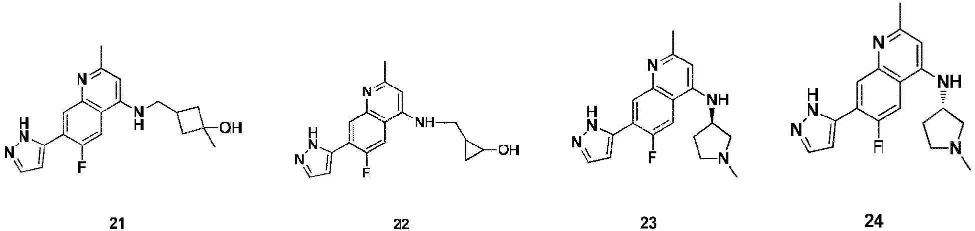

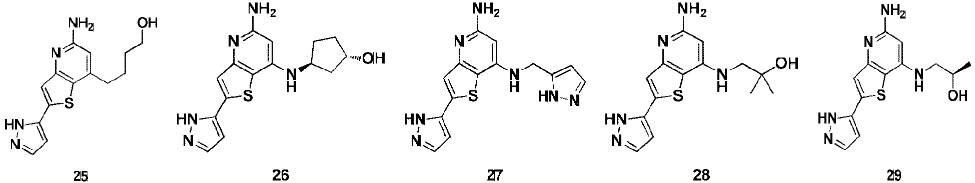

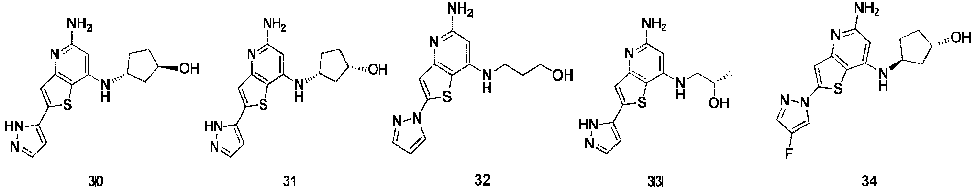

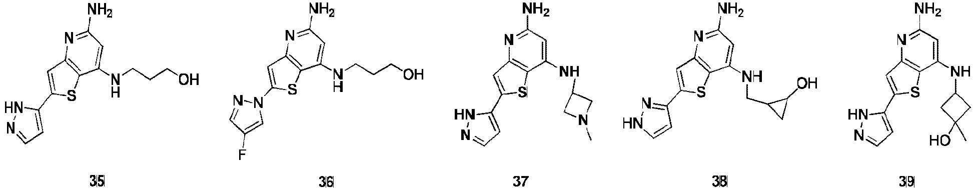

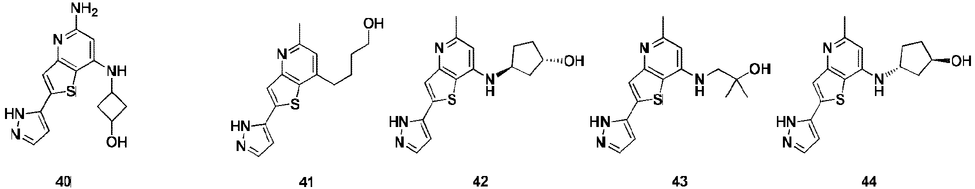

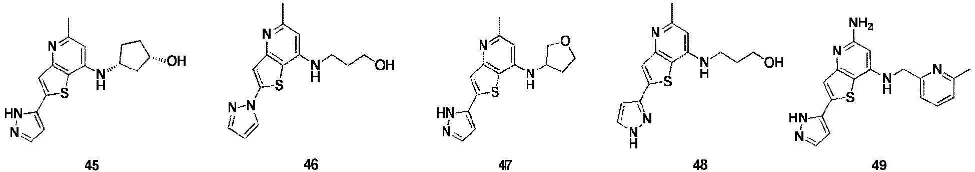

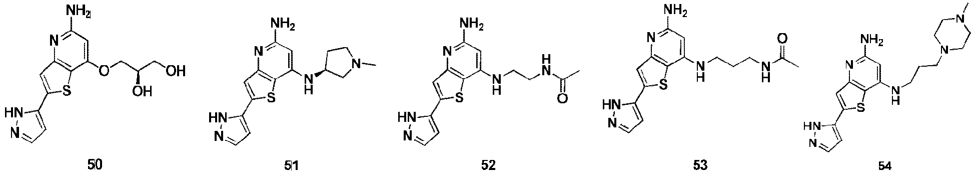

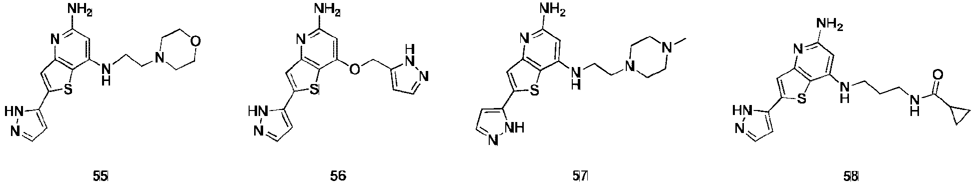

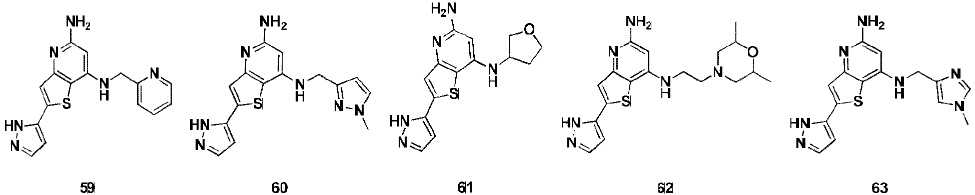

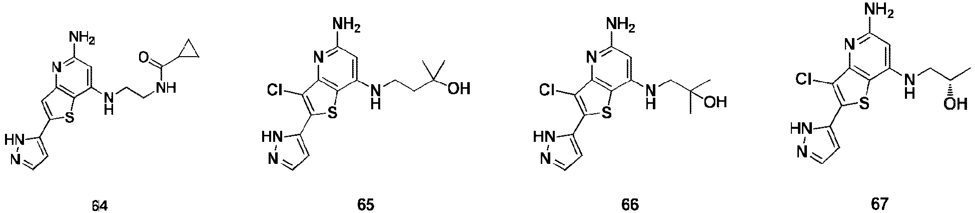

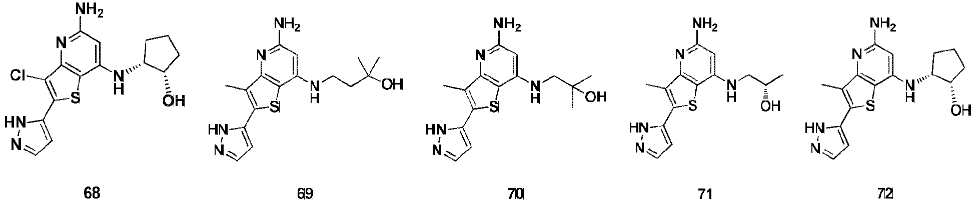

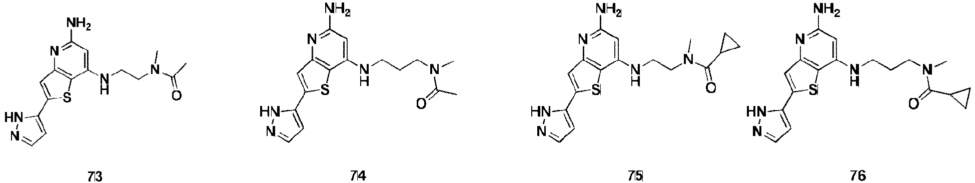

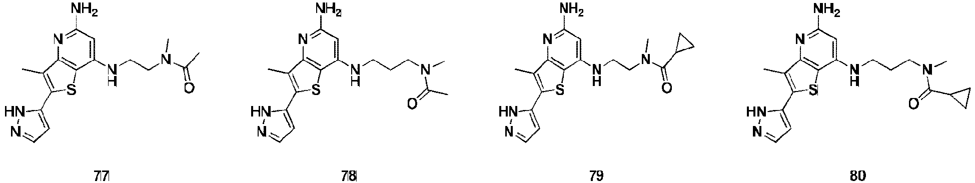

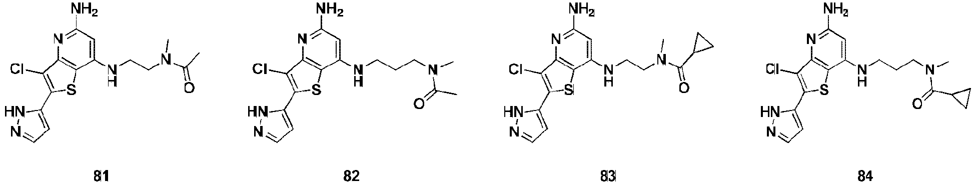

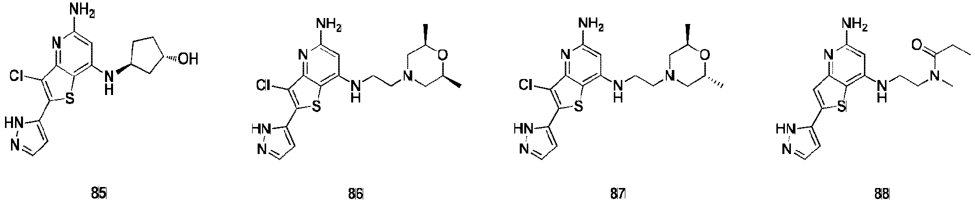

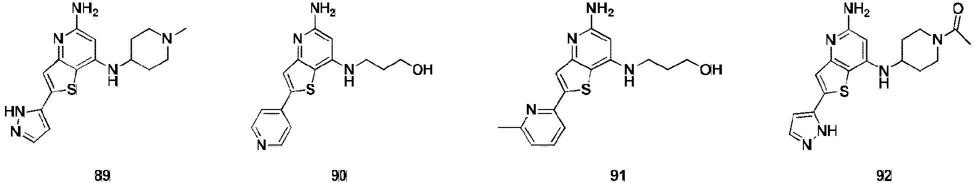

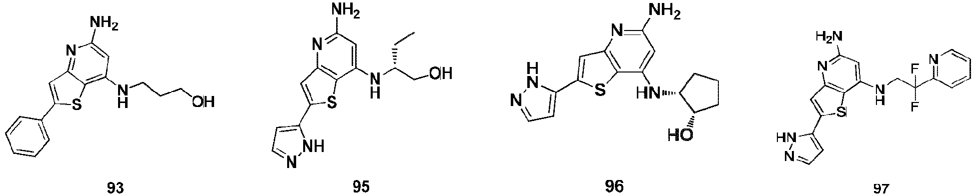

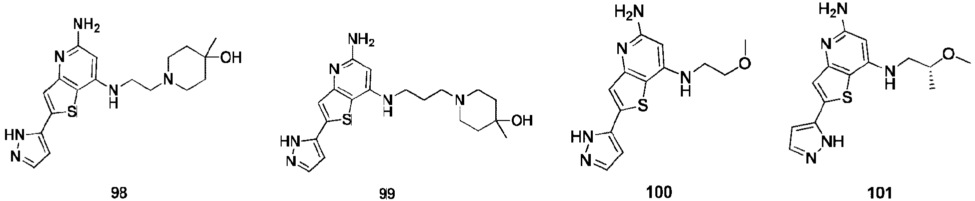

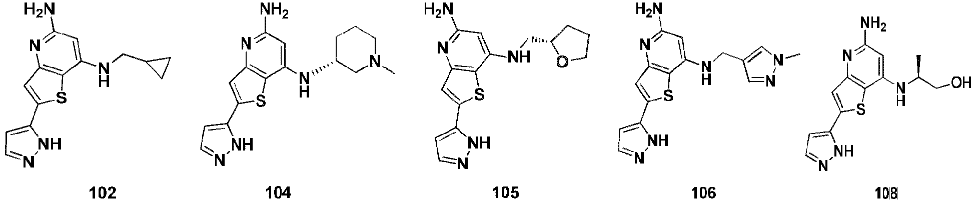

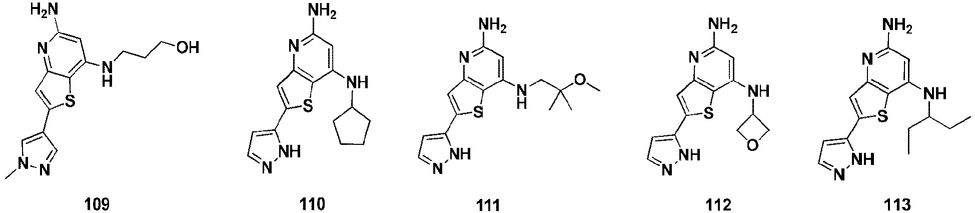

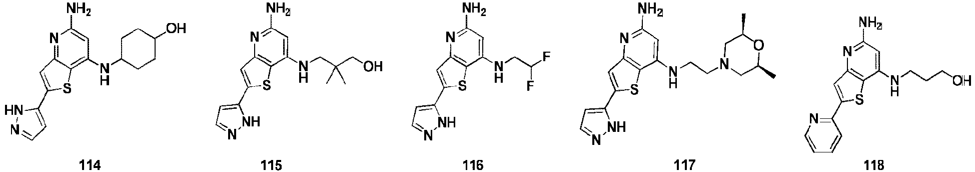

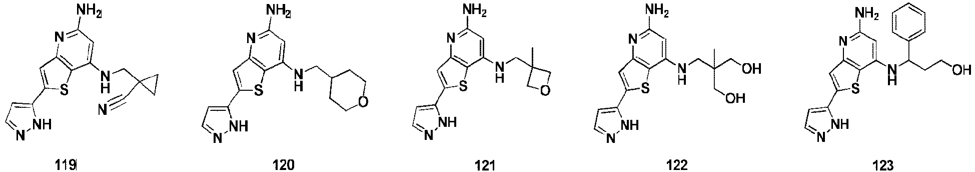

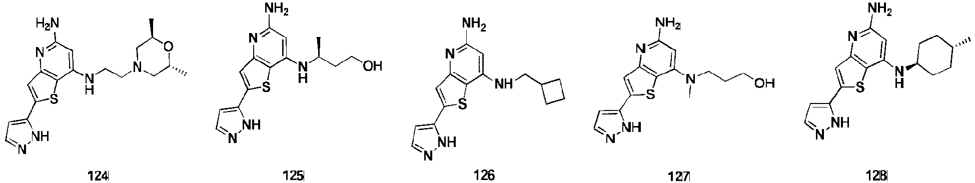

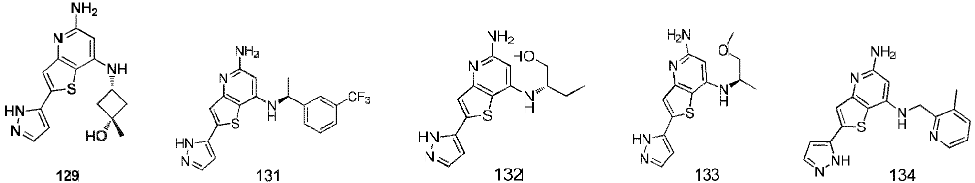

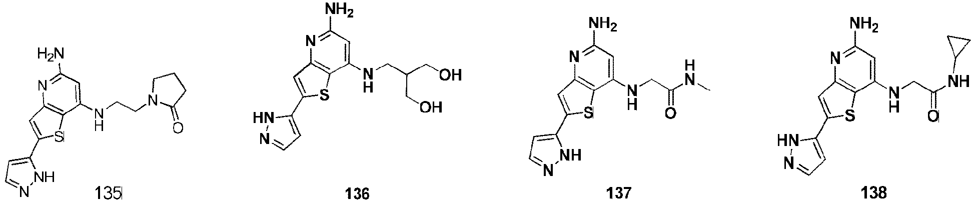

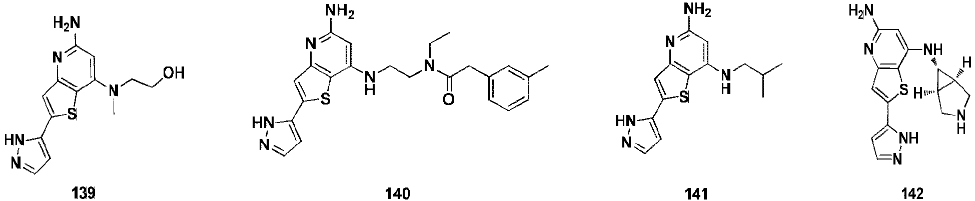

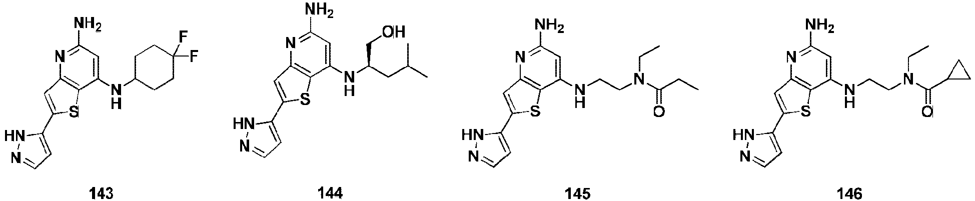

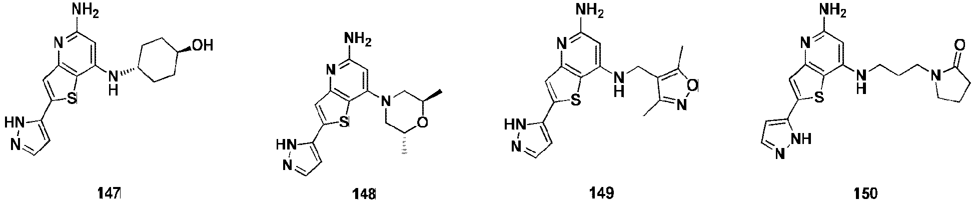

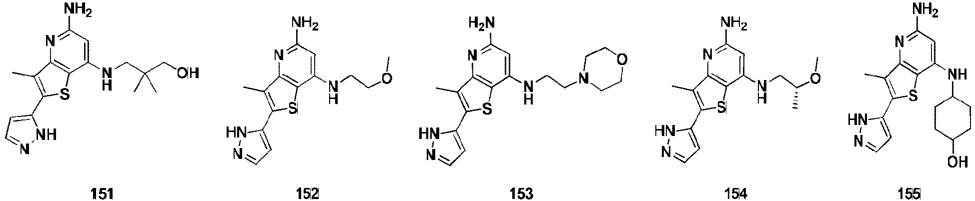

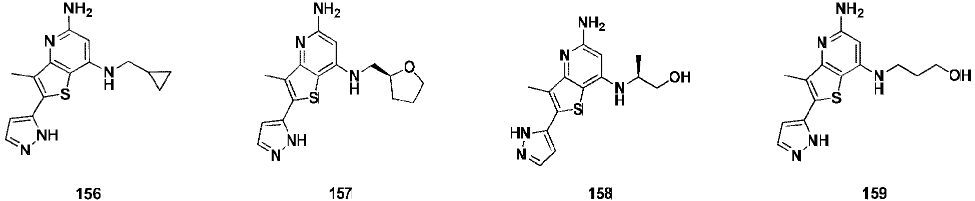

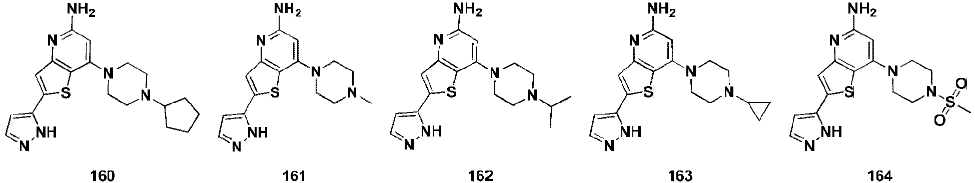

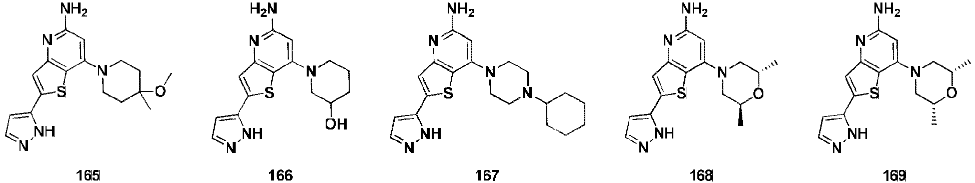

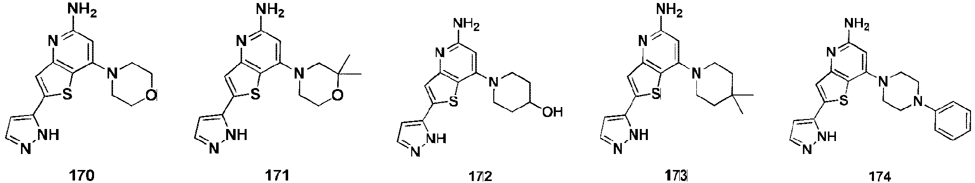

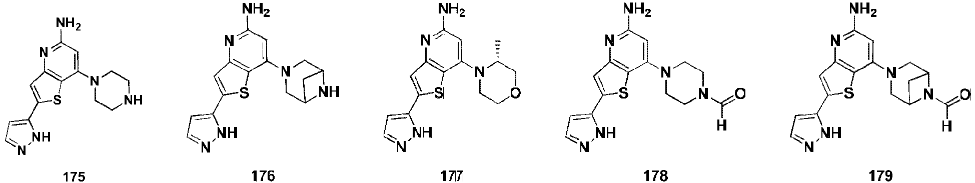

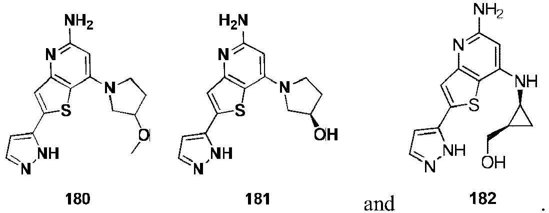

14. A pharmaceutical composition comprising the compound according to any one of claims 1 to 13, a stereoisomer, tautomer or mixture thereof, a pharmaceutically acceptable salt, co-crystal, polymorph or solvate thereof, or a stable isotope derivative, metabolite or prodrug thereof, and optionally one or more pharmaceutically acceptable carriers.

15. A pharmaceutical preparation comprising the compound according to any one of claims 1 to 13, a stereoisomer, tautomer or mixture thereof, a pharmaceutically acceptable salt, co-crystal, polymorph or solvate thereof, or a stable isotope derivative, metabolite or prodrug thereof, or comprising the pharmaceutical composition according to claim 14.

16. A use of the compound according to any one of claims 1 to 13, a stereoisomer, tautomer or mixture thereof, a pharmaceutically acceptable salt, co-crystal, polymorph or solvate thereof, or a stable isotope derivative, metabolite or prodrug thereof, or of the pharmaceutical composition according to claim 14 in the manufacture of a medicament for the prophylaxis and/or treatment of a disease related to the activity of NLRP3 inflammasomes, which is preferably a neoplastic disease, and preferably, the neoplastic disease is selected from the group consisting of brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, esophageal squamous cell carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma and sarcoma.

17. The compound according to claim any one of claims 1 to 13, a stereoisomer, tautomer or mixture thereof, a pharmaceutically acceptable salt, co-crystal, polymorph or solvate thereof, or a stable isotope derivative, metabolite or prodrug thereof, or the pharmaceutical composition according to claim 14 for use in the prophylaxis and/or treatment of a disease related to the activity of NLRP3 inflammasomes, which is preferably a neoplastic disease, and preferably, the neoplastic disease is selected from the group consisting of brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, esophageal squamous cell carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma and sarcoma.

18. A method for the treatment and/or prophylaxis of a disease related to the activity of NLRP3 inflammasomes, comprising administering to a subject in need thereof a therapeutically and/or prophylactically effective amount of the compound according to any one of claims 1 to 13, a stereoisomer, tautomer or mixture thereof, a pharmaceutically acceptable salt, co-crystal, polymorph or solvate thereof, a stable isotope derivative, metabolite or prodrug thereof, the pharmaceutical composition according to claim 14, or the pharmaceutical preparation according to claim 15.

19. The method according to claim 18, wherein the disease related to the activity of NLRP3 inflammasomes is a neoplastic disease, which is preferably selected from the group consisting of brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, esophageal squamous cell carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma and sarcoma.
